# EUROPEAN PATENT APPLICATION

(11) **EP 4 549 442 A1**
(43) Date of publication of application: **07.05.2025**
(21) Application number: 23830538.7
(22) Date of filing: 03.07.2023
(51) Int. Cl.: C07F 5/02, C07D 487/14, C07D 491/14, C07D 491/04, A61K 31/4353, A61P 35/00

(54) **PRMT5 INHIBITORS AND USE THEREOF**

(30) Priority: 01.07.2022 CN 202210775564; 23.09.2022 CN 202211168268
(71) Applicant: Cytosinlab Therapeutics Co., Ltd., Shanghai 201203 (CN)
(72) Inventor: WANG, Long, Shanghai 201203 (CN); WU, Haiping, Shanghai 201203 (CN); MI, Yuan, Shanghai 201203 (CN); LIU, Yilin, Shanghai 201203 (CN); FU, Xingnian, Shanghai 201203 (CN); WANG, Meng, Shanghai 201203 (CN); SHI, Hui, Shanghai 201203 (CN); GUO, Jiannan, Shanghai 201203 (CN)
(74) Representative: Lavoix
(86) International application number: PCT/CN2023/105593
(87) International publication number: WO 2024/002376

(57) **Abstract**

The present invention provides a class of compounds with inhibitory activity against methyltransferases. Particularly, the present invention provides a class of compounds with inhibitory activity against PRMT5. The compounds can be used for preparing pharmaceutical compositions for treating PRMT5 activity-related diseases. Formula (I) and formula (II).

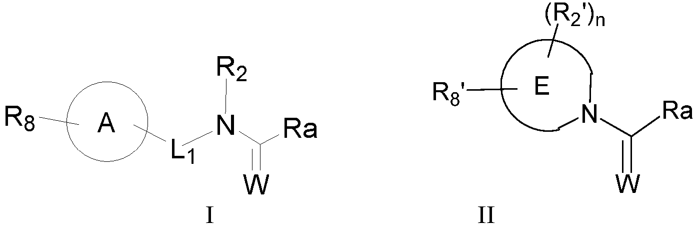

## Description

### Technical field

The present invention relates to the field of pharmaceutical compounds. Specifically, the present invention provides a class of compounds for inhibiting PRMT5 and its use in pharmaceutical compositions.

### Background

The epigenetic regulation of gene expression is an important biological determinant of protein production and cell differentiation, and plays a crucial pathological role in many human diseases.

The epigenetic regulation involves heritable modifications of genetic material without altering its nucleotide sequence. Typically, the epigenetic regulation is mediated by the selective and reversible modifications (such as methylation) of DNA and proteins (such as histones), which control conformational transitions between transcriptionally active and inactive states of chromatin. These covalent modifications can be controlled by enzymes such as methyltransferases (such as PRMT5), many of which are associated with specific genetic changes that may lead to human diseases. PRMT5 plays a role in diseases such as proliferative disorders, metabolic disorders, and hematological disorders.

PRMT5 is a known essential gene for cells. Conditional knockout of *PRMT5* and siRNA knockdown studies have shown that depletion of PRMT5 in normal tissues is associated with a range of diseases, such as pancytopenia, infertility, skeletal muscle loss, and cardiac hypertrophy. Therefore, new strategies are needed to exploit the metabolic vulnerability and priority targeting PRMT5 in MTAP-null tumors, while retaining PRMT5 activity in normal (*MTAP*^{WT}) tissues. Targeting PRMT5 with MTA in conjunction with a small molecule inhibitor can preferentially target the MTA-bound state of PRMT5, which is enriched in MTAP-null tumor cells, and provide a therapeutic index superior to that of normal cells with intact MTAP and low MTA levels.

Therefore, there is a need in the field to provide novel small molecule compounds targeting PRMT5 in MTAP-null tumors.

### SUMMARY OF THE INVENTION

The purpose of the present invention is to provide a novel class of small molecule compounds targeting PRMT5 in MTAP-null tumors.

In a first aspect of the present invention, provided is a compound of formula I shown below, or a pharmaceutically acceptable stereoisomer, a salt or a deuterated product thereof: 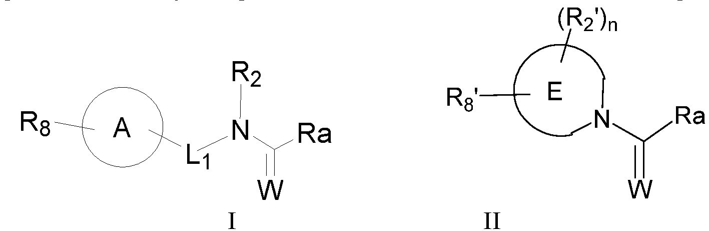 wherein,
Ra is 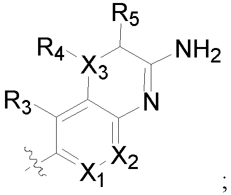
W is O or S;
X₁, and X₂ are each independently selected from the group consisting of: CR and N; X₃ is N;
L₁ is selected from the group consisting of: chemical bond, -CHR-, and -C(R)R-;
ring A is selected from the group consisting of: substituted or unsubstituted 7-12 membered bridged ring (including carbocycle or heterocycle), substituted or unsubstituted 7-12 membered spirocyclic ring (including carbocycle or heterocycle), substituted or unsubstituted 8-12 membered fused bicyclic heterocyclyl (including carbocycle or heterocycle, preferably five-membered fused six-membered ring), substituted or unsubstituted 7-10 membered fused bicyclic heteroaryl (preferably five-membered fused six-membered ring), or ring A is substituted or unsubstituted 3-7 membered carbocycle or heterocycle, and substituted or unsubstituted 5-6 membered aromatic ring or heteroaromatic ring;
ring E is selected from the group consisting of: substituted or unsubstituted 3-7 membered monocyclic heterocycle, substituted or unsubstituted 7-12 membered bridged heterocycle, substituted or unsubstituted 7-12 membered spirocyclic heterocycle, substituted or unsubstituted 8-12 membered fused polycyclic heterocycle (such as fused bicyclic ring);
R₈ is selected from the group consisting of: H, deuterium, halogen, cyano, alkynyl, SF₅, amino, nitro, hydroxyl, thiol, aldehyde group, carboxyl, substituted or unsubstituted or halogenated C₁-C₆ alkyl, and substituted or unsubstituted or halogenated C₁-C₆ alkoxyl, or
R₈ is 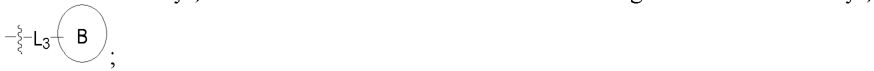
R₈' is selected from the group consisting of: H, deuterium, halogen, cyano, amino, nitro, hydroxyl, thiol, aldehyde group, carboxyl, unsubstituted or halogenated C₁-C₆ alkyl, substituted or unsubstituted benzene ring, substituted or unsubstituted 5-12 membered heteroaromatic ring, substituted or unsubstituted C₃-C₁₀ carbocycle (including saturated or partially unsaturated situations), and substituted or unsubstituted 3-12 membered heterocycle (including saturated or partially unsaturated situations), or R₈' is 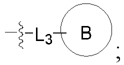
L₃ is selected from the group consisting of: chemical bonds, -O-, -CHR-, -C(R)R-, carbonyl, S and -NH-;
ring B is selected from the group consisting of: substituted or unsubstituted benzene ring, substituted orunsubstituted 5-6-membered heteroaromatic ring, substituted or unsubstituted C₃-C₆ carbocycle (including saturated and partially unsaturated situations), substituted or unsubstituted 3-7-membered heterocycle (including saturated and partially unsaturated situations);
R₂ and R₂' are independently selected from the group consisting of: R₇, and -L₂R₇; wherein, L₂ is selected from the group consisting of: -O-, -CHR-, and -C(R)R-; wherein, R₇ is selected from the group consisting of: hydrogen, none, substituted or unsubstituted C₁-C₆ alkyl, substituted or unsubstituted C₆₋₁₀ aromatic ring, substituted or unsubstituted 5-12 membered heteroaromatic ring, substituted or unsubstituted C₃-C₁₀ carbocycle (including saturated and partially unsaturated situations, including single ring, fused ring, spirocyclic ring and bridged ring), and substituted or unsubstituted 3-10 membered heterocycle (including saturated and partially unsaturated situations, including single ring, fused ring, spirocyclic ring and bridged ring); n is 0, 1, 2 or 3;
R₃ is selected from the group consisting of H, deuterium, halogen, cyano, and substituted or unsubstituted C₁-C₆ alkyl;
R₄ and R₅ together with the attached ring atoms form a 5-12 membered saturated or unsaturated ring, and the ring can be substituted or unsubstituted;
R is H, deuterium, halogen, substituted or unsubstituted C₁-C₄ alkyl, substituted or unsubstituted C₁-C₄ alkoxyl, and substituted or unsubstituted C₃-C₆ cycloalkyl;
unless otherwise specified, in each of the above formulas, the "substituted" refers to that hydrogen atoms on the corresponding group is substituted by one or more substituents selected from the group consisting of: deuterium, tritium, halogen, hydroxyl, carboxyl, thiol, benzyl, C₁-C₁₂ alkoxycarbonyl, C₁-C₆ aldehyde group, amino, C₁-C₆ amide group, nitro, cyano, unsubstituted or halogenated C₁-C₆ alkyl, unsubstituted or halogenated C₁-C₆ alkyl-O-C₁-C₆ alkyl-, unsubstituted or halogenated C₁-C₆ alkyl-O-C₁-C₆ alkyl-O-, unsubstituted or halogenated C₁-C₆ alkylene-OH, unsubstituted or halogenated C₃-C₈ cycloalkyl, C₂-C₁₀ alkenyl, unsubstituted or halogenated C₁-C₆ alkoxyl, C₁-C₆ alkyl-amino, C₆-C₁₀ aryl, five-membered or six-membered heteroaryl, five-membered or six-membered non-aromatic heterocyclyl, -O-(C₆-C₁₀ aryl), -O-(five-membered or six-membered heteroaryl), C₁-C₁₂ alkylamino carbonyl, unsubstituted or halogenated C₂-C₁₀ acyl, sulfonyl (-SO₂-OH), phosphoryl-(-PO₃-OH), unsubstituted or halogenated C₁-C₄ alkyl-S(O)₂-, unsubstituted or halogenated C₁-C₄ alkyl-SO-, and -SF₆.

In another preferred embodiment, Ra is selected from the group consisting of: 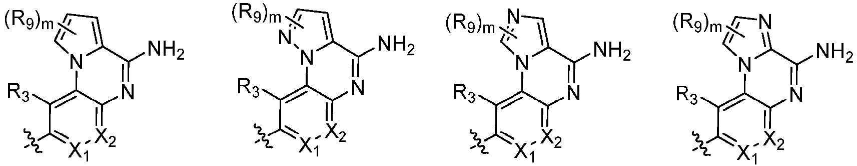 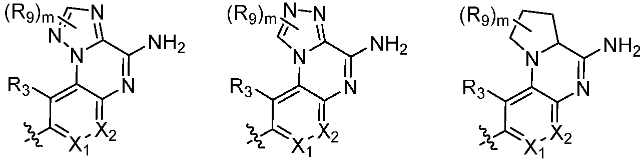 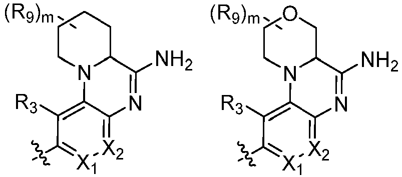 wherein, R₉ is selected from the group consisting of: deuterium, tritium, halogen, hydroxyl, carboxyl, unsubstituted or halogenated C₁-C₆ alkyl, unsubstituted or halogenated C₁-C₆ alkoxyl, unsubstituted or substituted C₁-C₆ alkyl-OH, - NH (unsubstituted or halogenated C₁-C₆ alkyl), - N(unsubstituted or halogenated C₁-C₆ alkyl)₂; m is selected from 0, 1, 2, and 3; preferably, R₉ is selected from the group consisting of: deuterium, tritium, halogen, and unsubstituted or halogenated C₁-C₆ alkyl.

In another preferred embodiment, L₁ is -CHR- or -C(R)R-; ring A is selected from the group consisting of: substituted or unsubstituted 8-12 membered fused bicyclic heterocyclyl, and substituted or unsubstituted 7-10 membered fused bicyclic heteroaryl; R₈ is selected from the croup consisting of: H, halogen cyano, amino, alkynyl, SF₅, hydroxyl, thiol aldehyde group, carboxyl, unsubstituted or halogenated C₁-C₆ alkyl, and 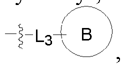 and ring B is selected from the group consisting of: substituted or unsubstituted benzene ring, substituted or unsubstituted 5-6 membered heteroaromatic ring, substituted or unsubstituted C₃-C₆ carbocycle, and substituted or unsubstituted 3-6 membered heterocycle; L₃ is selected from the group consisting of: chemical bond, -O-, -CHR-, carbonyl, S, and -NH-.

In another preferred embodiment, R₂ is selected from the group consisting of: R₇, and -L₂R₇; wherein, L₂ is selected from the group consisting of: -O-, -CHR-, carbonyl, S, and - NH-; wherein, R₇ is selected from the group consisting of: substituted or unsubstituted C₁-C₆ alkyl, substituted or unsubstituted C₆₋₁₀ aromatic ring, and substituted or unsubstituted 5-12 membered heteroaromatic ring.

In another preferred embodiment, R₂ is ortho-substituted 5 or 6-membered heteroaromatic ring, as shown below: 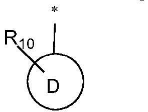
wherein, ortho-substituent R₁₀ is selected from the group consisting of: hydrogen, deuterium, halogen, halogenated or unhalogenated C₁-C₃ alkyl, and halogenated or unhalogenated C₁-C₃ alkoxyl;
ring D is selected from the group consisting of: substituted or unsubstituted benzene ring, substituted or unsubstituted 5-6 membered heteroaromatic ring, preferably, ring D is selected from the group consisting of:

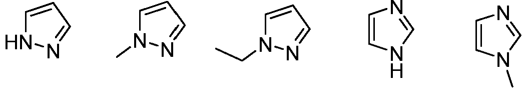

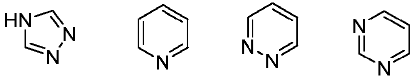

In another preferred embodiment, L₁ is -CH₂- or CH(CH₃)-; ring A is selected from the group consisting of: 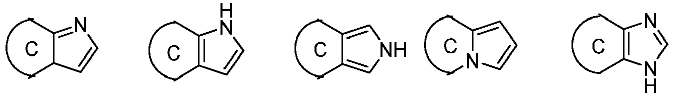 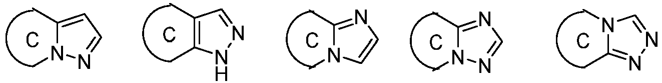 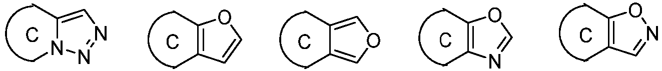 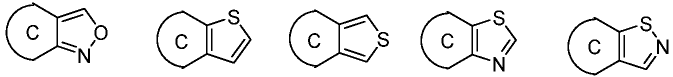 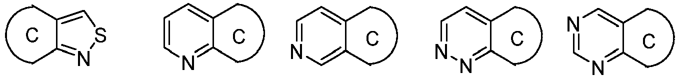 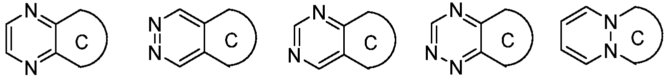
wherein, ring C is selected from the group consisting of: substituted or unsubstituted benzene ring, substituted or unsubstituted 5-6-membered heteroaromatic ring, substituted or unsubstituted C₃-C₆ carbocycle (including saturated and partially unsaturated situations), and substituted or unsubstituted 3-6-membered heterocycle (including saturated and partially unsaturated situations);
or, ring A is selected from the group consisting of: 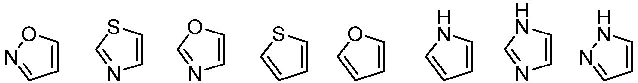 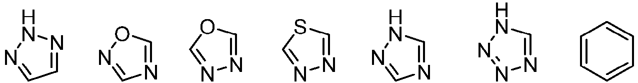 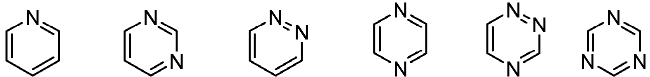
R₈ is 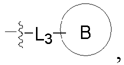 and ring B is selected form the group consisting of: substituted or unsubstituted benzene ring, substituted or unsubstituted 5-6 membered heteroaromatic ring, substituted or unsubstituted C₃-C₆ carbocycle, and substituted or unsubstituted 3-6 membered heterocycle; L₃ is selected from the group consisting of: chemical bond, -O-, - CHR-, carbonyl, S, and -NH-.

In another preferred embodiment, L₃ is a chemical bond.

In a preferred embodiment, ring B is substituted or unsubstituted benzene ring, or substituted or unsubstituted 5-6-membered heteroaromatic ring.

In a preferred embodiment, R₂ is selected from the group consisting of: R₇, and - (CHR)R₇; wherein, R₇ is selected from the group consisting of: hydrogen and none, substituted or unsubstituted C₁-C₆ alkyl, substituted or unsubstituted C₆₋₁₀ aromatic ring, substituted or unsubstituted 5-12 membered heteroaromatic ring, substituted or unsubstituted C₃-C₈ carbocycle (including saturated or partially unsaturated situations, including monocyclic, fused, spirocyclic or bridged rings), and substituted or unsubstituted 3-8-membered heterocycle (including saturated or partially unsaturated situations, including monocyclic, fused, spiral or bridged rings).

In another preferred embodiment, the compound has a structure shown in the following formula: 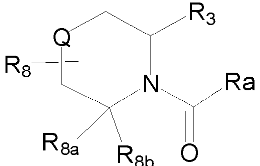
wherein, Q is O, NH, CH₂, or a chemical bond (i.e., 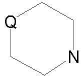 is a five membered ring);
R₈ is as described above;
R₈ₐ and R_{8b} are independently selected from H; or R₈ₐ and R_{8b} together with the attached carbon atom form a 4-7 membered carbocycle or heterocycle;
and when R₈ₐ and R_{8b} are independently H; R₈ₐ or R_{8b} may optionally substituted by R₈; when R₈ₐ and R_{8b} together with the attached carbon atom form a 4-7 membered carbocycle or heterocycle, R₈ may located on the carbocycle or heterocycle.

In another preferred embodiment, R₃ is selected from the group consisting of H, deuterium, halogen, cyano, and substituted or unsubstituted C₁-C₆ alkyl.

In another preferred embodiment, R₂ is substituted or unsubstituted 5-7 membered heteroaromatic ring; and ring A is selected from the group consisting of: substituted or unsubstituted 5-6-membered aromatic ring or heteroaromatic ring, and substituted or unsubstituted 7-10-membered fused bicyclic heteroaromatic group; R₈ is CF₃.

In a second aspect of the present invention, provided is a pharmaceutical composition comprising a therapeutically effective amount of one or more of the compound according to the first aspect of the present invention, or a pharmaceutically acceptable salt, a racemate, an optical isomer, a stereoisomer, or a tautomer thereof, and one or more pharmaceutically acceptable carriers, excipients, adjuvants, accessories, and/or diluents.

In a third aspect of the present invention, provided is a use of the compound according to the first aspect of the present invention, or a racemate, an optical isomer, or a pharmaceutically acceptable salt thereof in the preparation of drugs for treatment or prevention of diseases associated with abnormal gene levels or abnormal expression of PRMT5(such as corresponding nucleic acid mutations, deletions, or the methyltransferase is ectopic or fused or overexpressed).

In another preferred embodiment, the disease is selected from the group consisting of: the disease or disorder ovarian cancer, lung cancer, lymphatic cancer, glioblastoma, colon cancer, melanoma, gastric cancer, pancreatic cancer or bladder cancer.

It should be understood that, within the scope of the present invention, each of the above technical features of the present invention and each of the technical features specifically described in the following (such as the embodiments) can be combined with each other to constitute a new or preferred technical solution. Due to space limitations, It will not be repeated herein.

### EMBODIMENTS FOR CARRYING OUT THE INVENTION

After long and intensive research, the inventors unexpectedly discovered a class of compound with PRMT5 regulatory effects for the first time. The present invention is completed on this basis.

### TERMS

As used herein, halogen refers to F, Cl, Br or I.

As used herein, unless otherwise specified, the terms used have a general meaning known to those skilled in the art. As used herein, unless otherwise specified, all chemical formulas are intended to encompass any possible optical or geometric isomers (such as R-type, S-type or racemate, or cis-trans isomers of olefins, etc.).

As used herein, the term "C1-C6 alkyl" refers to a linear or branched alkyl having 1 to 6 carbon atoms, but not limited to methyl, ethyl, propyl, isopropyl, butyl, isobutyl , sec-butyl, tert-butyl, pentyl and hexyl and the like; preferably ethyl, propyl, isopropyl, butyl, isobutyl, sec-butyl and tert-butyl.

As used herein, the term "C1-C6 alkoxyl" refers to a linear or branched alkoxy having 1 to 6 carbon atoms, including but not limited to methoxy, ethoxy, propoxy, isopropoxy and butoxy and the like.

As used herein, the term "C2-C6 alkenyl" refers to a linear or branched alkenyl having 2 to 6 carbon atoms and containing a double bond, including but not limited to vinyl, propenyl, butenyl, isobutenyl, pentenyl, hexenyl, and the like.

As used herein, the term "C2-C6 alkynyl" refers to a linear or branched alkynyl group having 2 to 6 carbon atoms and containing a triple bond, including but not limited to ethynyl, propynyl, butynyl, isobutynyl, pentynyl, hexynyl, and the like.

As used herein, the term "C3-C10 cycloalkyl" refers to a cyclic alkyl having 3 to 10 carbon atoms on the ring, including but not limited to cyclopropyl, cyclobutyl, cyclopentyl, cyclohexyl, cycloheptyl, cyclooctyl, cyclodecyl and the like. The terms "C3-C8 cycloalkyl", "C3-C7 cycloalkyl", and "C3-C6 cycloalkyl" have similar meanings.

As used herein, the term "C3-C10 cycloalkenyl" refers to a cyclic alkenyl having 3 to 10 carbon atoms on the ring, including but not limited to cyclopropenyl, cyclobutenyl, cyclopentenyl, cyclohexenyl, cycloheptenyl, cyclooctenyl, cyclodecenyl and the like. The term "C3-C7 cycloalkenyl" has a similar meaning.

As used herein, the term "C1-C12 alkoxycarbonyl" refers to an alkoxycarbonyl having 1 to 12 carbon atoms on the alkyl chain, including but not limited to methoxycarbonyl, ethoxycarbonyl, propoxycarbonyl, isopropoxycarbonyl, tert butoxycarbonyl, benzyloxycarbonyl, and the like.

As used herein, the term "C1-C12 alkylaminocarbonyl" refers to an alkylaminocarbonyl having 1 to 12 carbon atoms on the alkyl chain, including but not limited to methylamino carbonyl, ethylamino carbonyl, propylamino carbonyl, isopropylamino carbonyl, tert butylamino carbonyl, benzylamino carbonyl, dimethylamino carbonyl and the like.

As used herein, the terms "aromatic ring" or "aryl" have the same meaning, preferably "aryl" is "C6-C12 aryl" or "C6-C10 aryl". The term "C6-C12 aryl" refers to an aromatic ring group having 6 to 12 carbon atoms without any heteroatoms on the ring, such as phenyl, naphthyl and the like. The term "C6-C10 aryl" has a similar meaning.

As used herein, the terms "heteroaromatic ring" or "heteroaryl" have the same meaning, referring to heteroaromatic groups containing one to more heteroatoms. The heteroatoms referred to herein include oxygen, sulfur, and nitrogen. For example, furyl, thienyl, pyridinyl, pyrazolyl, pyrrolyl, N-alkyl pyrrolyl, pyrimidinyl, pyrazinyl, imidazolyl, tetrazolyl, and the like. Heteroaryl may be fused onto an aromatic, heterocyclic, or cycloalkyl ring, wherein the ring connected to the parent structure is a heteroaryl ring. Heteroaryl may be optionally substituted or unsubstituted.

As used herein, the term "3-12 membered heterocyclyl" refers to a saturated or unsaturated 3-12 membered cyclic group containing 1-3 heteroatoms selected from oxygen, sulfur, and nitrogen on the ring, such as dioxocyclopentyl and the like. The term "3-7-membered heterocyclyl" has a similar definition.

As used herein, the term "substituted" indicates that one or more hydrogen atoms on a specific group are substituted by specific substituents. The specific substituents are the substituents described in the previous text, or the substituents appeared in each example. Unless otherwise specified, a substituted group may have a substituent selected from a specific group at any substitutable site of that group, and the substituents may be the same or different in each position. A cyclic substituent, such as a heterocyclicalky, can be linked to another ring, such as a cycloalkyl, thereby forming a spiro-dicyclic ring system, where the two rings share a common carbon atom. It should be understood by those skilled in the art that the combinations of substituents contemplated by the present invention are those that are stable or chemically achievable. The substituents, such as (but not limited to): C1-8 alkyl, C2-8 alkenyl, C2-8 alkynyl, C3-8 cycloalkyl, 3- to 12-membered heterocyclyl, aryl, heteroaryl, halogen, hydroxyl, carboxyl (-COOH), C1-8 aldehyde group, C2-10 acyl, C2-10 ester, C1-C12 alkoxycarbonyl, amino, alkoxyl, C1-10 sulfonyl, etc.

When using expressions such as "C1-8" and the like, it means that the functional group can have 1, 2, 3, 4, 5, 6, 7, or 8 carbon atoms.

When using expressions such as "3-12 membered" and the like, it refers to that the group has 3, 4, 5, 6, 7, 8, 9, 10, 11, or 12 carbon atoms or heteroatoms as ring atoms.

### PRMT5 regulatory compound

The present invention provides a class of compounds with PRMT5 regulatory activity: 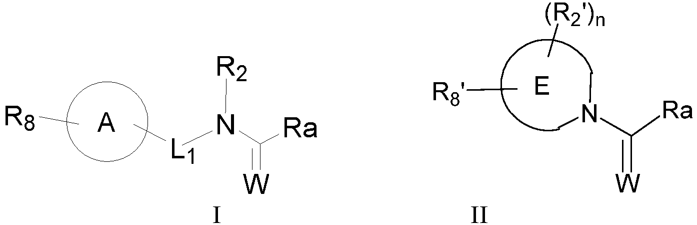 wherein,
Ra is 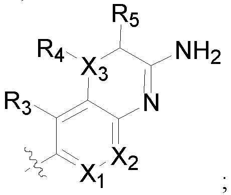
W is O or S;
X₁, and X₂ are each independently selected from the group consisting of: CR and N; X₃ is N;
L₁ is selected from the group consisting of: chemical bond, -CHR-, and -C(R)R-;
ring A is selected from the group consisting of: substituted or unsubstituted 7-12 membered bridged ring (including carbocycle or heterocycle), substituted or unsubstituted 7-12 membered spirocyclic ring (including carbocycle or heterocycle), substituted or unsubstituted 8-12 membered fused bicyclic heterocyclyl (including carbocycle or heterocycle, preferably five-membered fused six-membered ring), substituted or unsubstituted 7-10 membered fused bicyclic heteroaryl (preferably five-membered fused six-membered ring), or ring A is substituted or unsubstituted 3-7 membered carbocycle or heterocycle, and substituted or unsubstituted 5-6 membered aromatic ring or heteroaromatic ring;
ring E is selected from the group consisting of: substituted or unsubstituted 3-7 membered monocyclic heterocycle, substituted or unsubstituted 7-12 membered bridged heterocycle, substituted or unsubstituted 7-12 membered spirocyclic heterocycle, substituted or unsubstituted 8-12 membered fused polycyclic heterocycle (such as fused bicyclic ring);
R₈ is selected from the group consisting of: H, deuterium, halogen, cyano, alkynyl, SF₅, amino nitro, hydroxyl, thiol, aldehyde group, carboxyl, halogenated or unhalogenated C₁-C₆ alkyl, and halogenated or unhalogenated C₁-C₆ alkoxyl, or R₈ is 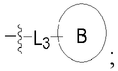 and when ring A is a substituted or unsubstituted 3-7 membered carbocycle or heterocycle, or 5-6-membered aromatic ring or heteroaromatic ring, R₈ is 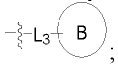
R₈' is selected from the group consisting of: H, deuterium, halogen, cyano, amino, nitro, hydroxyl, thiol, aldehyde group, carboxyl, halogenated or unhalogenated C₁-C₆ alkyl, substituted or unsubstituted benzene ring, substituted or unsubstituted 5-12 membered heteroaromatic ring, substituted or unsubstituted C₃-C₁₀ carbocycle (including saturated or partially unsaturated situations), and substituted or unsubstituted 3-12 membered heterocycle (including saturated or partially unsaturated situations), or R₈' is 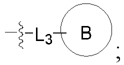
L₃ is selected from the group consisting of: chemical bonds, -O-, -CHR-, -C(R)R-, carbonyl, Sand -NH-;
ring B is selected from the group consisting of: substituted or unsubstituted benzene ring, substituted or unsubstituted 5-6-membered heteroaromatic ring, substituted or unsubstituted C₃-C₆ carbocycle (including saturated and partially unsaturated situations), substituted or unsubstituted 3-7-membered heterocycle (including saturated and partially unsaturated situations);
R₂ and R₂' are independently selected from the group consisting of: R₇, and -L₂R₇; wherein, L₂ is selected from the group consisting of: -O-, -CHR-, and -C(R)R-; wherein, R₇ is selected from the group consisting of: hydrogen, none, substituted or unsubstituted C₁-C₆ alkyl, substituted or unsubstituted C₆₋₁₀ aromatic ring, substituted or unsubstituted 5-12 membered heteroaromatic ring, substituted or unsubstituted C₃-C₁₀ carbocycle (including saturated and partially unsaturated situations, including single ring, fused ring, spirocyclic ring and bridged ring), and substituted or unsubstituted 3-10 membered heterocycle (including saturated and partially unsaturated situations, including single ring, fused ring, spirocyclic ring and bridged ring); n is 0, 1, 2 or 3;
R₃ is selected from the group consisting of H, deuterium, halogen, cyano, substituted or unsubstituted C₁-C₆ alkyl;
R₄ and R₅ together with the attached ring atoms form a 5-12 membered saturated or unsaturated ring, and the ring can be substituted or unsubstituted;
R is H, deuterium, halogen, substituted or unsubstituted C₁-C₄ alkyl, substituted or unsubstituted C₁-C₄ alkoxyl, substituted or unsubstituted C₃-C₆ cycloalkyl;
unless otherwise specified, in each of the above formulas, the "substituted" refers to that hydrogen atoms on the corresponding group is substituted by one or more substituents selected from the group consisting of: deuterium, tritium, halogen, hydroxyl, carboxyl, thiol, benzyl, C₁-C₁₂ alkoxycarbonyl, C₁-C₆ aldehyde group, amino, C₁-C₆ amide group, nitro, cyano, unsubstituted or halogenated C₁-C₆ alkyl, unsubstituted or halogenated C₁-C₆ alkyl-O-C₁-C₆ alkyl -, unsubstituted or halogenated C₁-C₆ alkyl-O-C₁-C₆ alkyl-O-, unsubstituted or halogenated C₁-C₆ alkylene-OH, unsubstituted or halogenated C₃-C₈ cycloalkyl, C₂-C₁₀ alkenyl, unsubstituted or halogenated C₁-C₆ alkoxyl, C₁-C₆ alkyl-amino, C₆-C₁₀ aryl, five-membered or six-membered heteroaryl, five-membered or six-membered non-aromatic heterocyclyl, -O-(C₆-C₁₀ aryl), -O- (five-membered or six-membered heteroaryl), C₁-C₁₂ alkylamino carbonyl, unsubstituted or halogenated C₂-C₁₀ acyl, sulfonyl (-SO₂-OH), phosphoryl-(-PO₃-OH), unsubstituted or halogenated C₁-C₄ alkyl-S(O)₂-, unsubstituted or halogenated C₁-C₄ alkyl-SO-, and -SF₆.

### Pharmaceutical composition and mode of administration

Due to the excellent methyltransferase inhibitory activity of the compound of the present invention, the compound of the invention and various crystal forms, pharmaceutically acceptable inorganic or organic salts, hydrates or solvates thereof, and pharmaceutical compositions containing the compound of the present invention as main active ingredients can be used in the treatment, prevention and alleviation of the related diseases induced by the abnormal expression or activity of methyltransferase (such as PRMT5).

The pharmaceutical composition of the present invention comprises a safe and effective amount of the compound of the present invention,or a pharmaceutically acceptable salt thereof and pharmaceutically acceptable excipients or carriers. Wherein "safe and effective amount" refers to the amount of compound which is sufficient to significantly improve the condition, and not to generate severe side effects. Generally, the pharmaceutical composition contains 1-2000 mg polymorphs of the invention per dose, preferably, 5-200mg polymorphs of the invention per dose. Preferably, the "one dose" is one capsule or one pill.

"Pharmaceutically acceptable carrier" means one or more compatible solid or liquid fillers, or gelatinous materials which are suitable for human use and should be of sufficient purity and sufficiently low toxicity. "Compatible" herein refers to the ability of each component of a composition can be mixed with the compound of the present invention and can be mixed with each other without appreciably reducing the efficacy of the compound. Examples of pharmaceutically acceptable carrier include cellulose and derivatives thereof (such as sodium carboxymethylcellulose, sodium ethylcellulose, cellulose acetate, etc.), gelatin, talc, solid lubricant (such as stearic acid, magnesium stearate), calcium sulfate, vegetable oil (such as soybean oil, sesame oil, peanut oil, olive oil, etc.), polyol (such as propylene glycol, glycerol, mannitol, sorbitol, etc.), emulsifier (such as Tween^{®}), wetting agent (such as lauryl sodium sulfate), colorant, flavoring, stabilizer, antioxidant, preservative, pyrogen-free water, etc.

There is no special limitation of administration mode for the compound or pharmaceutical compositions of the present invention, and the representative administration mode includes (but is not limited to): oral, intratumoral, rectal, parenteral (intravenous, intramuscular or subcutaneous), and topical administration.

The solid dosage forms used for oral administration include capsules, tablets, pills, powders, and granules. In these solid dosage forms, the active compounds are mixed with at least one conventional inert excipient (or carrier), such as sodium citrate or dicalcium phosphate, or mixed with any of the following components: (a) fillers or compatibilizer, for example, starch, lactose, sucrose, glucose, mannitol and silicic acid; (b) binders, for example, hydroxymethyl cellulose, alginates, gelatin, polyvinylpyrrolidone, sucrose and arabic gum; (c) humectant, such as, glycerol; (d) disintegrating agents such as agar, calcium carbonate, potato starch or tapioca starch, alginic acid, certain composite silicates, and sodium carbonate; (e) dissolution-retarding agents, such as paraffin; (f) absorption accelerators, for example, quaternary ammonium compounds; (g) wetting agents, such as cetyl alcohol and glyceryl monostearate; (h) adsorbents, for example, kaolin; and (i) lubricants such as talc, stearin calcium, magnesium stearate, solid polyethylene glycol, sodium lauryl sulfate, or the mixtures thereof. In capsules, tablets, and pills, the dosage form may also include buffers.

Solid dosage forms such as tablets, sugar pills, capsules, pills, and granules can be prepared using coating and shell materials, such as casings and other materials well-known in the art. They can contain opacifiers, and the release of active compounds or compounds in the composition can be delayed in a certain part of the digestive tract. Examples of embedding components that may be employed are polymeric substances and waxes. If necessary, the active compound may also be formed into a microcapsules with one or more of the above excipients.

Liquid dosage forms for oral administration include pharmaceutically acceptable lotion, solutions, suspensions, syrups or tinctures. In addition to the active compounds, the liquid dosage forms may contain any conventional inert diluents known in the art such as water or other solvents, solubilizers and emulsifiers, for example, ethanol, isopropanol, ethyl carbonate, ethyl acetate, propylene glycol, 1,3-butanediol, dimethyl carboxamide, as well as oil, in particular, cottonseed oil, peanut oil, corn germ oil, olive oil, castor oil and sesame oil, or the combination thereof.

In addition to these inert diluents, the composition can also include additives such as wetting agents, emulsifiers and suspensions, sweeteners, correctors, and spices.

In addition to active compounds, suspensions can include suspending agents such as ethoxylated isooctadecanol, polyoxyethylene sorbitol and dehydrated sorbitol esters, microcrystalline cellulose, methanol aluminum and agar, or mixtures of these substances.

Compositions for parenteral injection may include physiologically acceptable sterile aqueous or anhydrous solutions, dispersion liquid, suspensions or lotions, and sterile powders for re dissolution into sterile injectable solutions or dispersions. Suitable aqueous and non-aqueous carriers, diluents, solvents or excipients include water, ethanol, polyols, and their suitable mixtures.

The dosage forms of the compounds of the present invention used for local administration include ointments, powders, patches, sprays, and inhalants. The active ingredients are mixed under sterile conditions with physiologically acceptable carriers and any preservatives, buffers, and propellants if necessary.

The compounds of the present invention may be administered alone or in combination with other pharmaceutically acceptable compound. In some preferred embodiments, the compounds of the present invention can form PROTAC with other small molecule compounds, or jointly form ADC with other large molecule compounds such as monoclonal antibodies for application.

When the pharmaceutical compositions are used, a safe and effective amount of compound of the present invention is applied to a mammal (such as human) in need of, wherein the dose of administration is a pharmaceutically effective dose. For a person weighed 60 kg, the daily dose is usually 1- 2000 mg, preferably 5-500 mg. Of course, the particular dose should also depend on various factors, such as the route of administration, patient healthy status, which are well within the skills of an experienced physician.

The present invention was further described hereafter in combination with specific embodiments. It should be understood that these examples are only used to illustrate the and not to limit the scope of the invention. The experimental methods without specific conditions in the following examples generally follow the conventional conditions or the conditions suggested by the manufacturer. Unless otherwise stated, percentages and parts are caculated by weight.

The definitions of each abbreviation are as follows:

| | |
|---|---|
| Xantphos | 4,5-Bis(diphenylphosphino)-9,9-dimethylxanthene |
| NBS | N-Bromosuccinimide |
| Pd(dppf)Cl2 | 1,1'-Bis(diphenylphosphino)ferrocene]dichloropalladium(II) |
| Pd2(dba)3 | Tris(dibenzylideneacetone)dipalladium |
| Pd(dba)2 | Bis(dibenzylideneacetone)palladium |
| BINAP | 1.1'-Binaphthyl-2,2'-diphemyl phosphine |
| X-Phos | 2-dicyclohexylphosphino-2',4',6'-triiso-propyl-1,1'-biphenyl |
| tBuXPhos | 2-Di-tert-butylphosphino-2',4',6'-triiso-propyl-1,1'-biphenyl |
| tBuXPhos Pd G3 | Methanesulfonate (2-di-tert-butylphospho-2', 4', 6'- triisopropyl-1,1' - biphenyl) (2'- amino-1,1' - biphenyl-2-yl) palladium (II) |
| EDCI | n-(3-dimethylaminopropyl)-n'-ethylcarbodiimide hydrochloride |
| HOBT | 1-Hydroxybenzotriazole |
| LDA | Lithium diisopropylamide |
| HATU | 2- (7-Azobenzotriazole) - N, N, N', N' - Tetramethylurea hexafluorophosphate |
| | Supercritical fluid chromatography |

The raw materials can be obtained commercially or prepared by methods already known or disclosed in the art.

Purification of intermediates and compounds were carried out by normal phase or reverse chromatography or conventional chemical laboratory operations such as recrystallisation. Normal phase chromatography was either preloaded silica gel columns or preparative thin layer chromatography. Silica gel columns are mainly glass columns or fast preparative chromatographs. The mobile phase for normal phase chromatography was selected and proportioned for elution from petroleum ether/ethyl acetate, dichloromethane/methanol or other suitable solvents. Reverse phase preparative liquid chromatography was carried out on a C18 column using a preparative liquid chromatograph or rapid preparative chromatograph. Detection was performed using 214nM and 254nM wavelength or preparative liquid chromatography-mass spectrometry instrument, using water/acetonitrile containing 0.1% hydrochloric acid, water/acetonitrile, water/acetonitrile containing 0.1% ammonium bicarbonate, water/acetonitrile containing 0.1% formic acid, 0.1% ammonia/acetonitrile, water/acetonitrile containing 0.1% trifluoroacetic acid or other suitable solvent system as the mobile phase for gradient elution.

Structural characterisation of intermediates and compounds were carried out by nuclear magnetic resonance (NMR) and mass spectrometry (LCMS). The nuclear magnetic resonance spectrometers used for nuclear magnetic resonance are Bruker Ascend 400 or Varian 400, or ZKNJ BIXI-1 300MHz, Bruker Avance III 400 MHz, or Bruker AVANCE Neo 400 MHz. The solvents used are deuterated dimethyl sulfoxide, deuterated chloroform, deuterated methanol, or other labeled deuterated solvents. Spectral data reported in patterns: chemical shifts δ (Peak splitting number, coupling constant J (Hz), number of hydrogen). Tetramethylsilane was used as an internal standard for chemical shifts and the chemical shifts of which was set to zero (δ, 0 ppm). The meanings of some of the abbreviations are: s (single peak), d (double peak), t (triple peak), q (quadruple peak), m (multiple peaks), br (broad peak).

Representative methods for liquid chromatography-mass spectrometry (LCMS) in the structural characterization of intermediates and compounds are listed below:
Method 1: Conducted on an Agilent LC1260 system coupled with a single quadrupole mass spectrometer.
column: Waters CORTECS C-18, 2.7 µm, 4.6*30 mm. Solvent A: 0.05% formic acid aqueous solution, solvent 20 B: a solution of 0.05% formic acid in acetonitrile, lasting for one minute from 5% acetonitrile to 95% acetonitrile, holding for one minute, for a total of 2.5 minutes; Flow rate: 1.8 mL/min; column temperature 40°C.
column: XSelect CSH C18, 3.5 µm, 4.6*50 mm. Solvent A: 0.05% ammonia aqueous solution, solvent B: a solution of 0.05% ammonia in acetonitrile, lasting for one minute from 5% acetonitrile to 95% acetonitrile, holding for one minute, for a total of 2.5 minutes; Flow rate: 1.8 mL/min; column temperature 40°C.
Method 2: Conducted on an Agilent LC/MSD 1200 system coupled with a quadrupole mass spectrometer.
column: ODS2000 (50 x 4.6 mm, 5 µ m) (ES (+) or (-) ionization mode), temperature 30 °C; Flow rate: 1.5 mL/min.

### General method for Example synthesis:

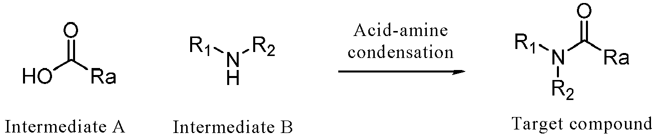

### General method: Synthesis of intermediate A2

### Synthetic route:

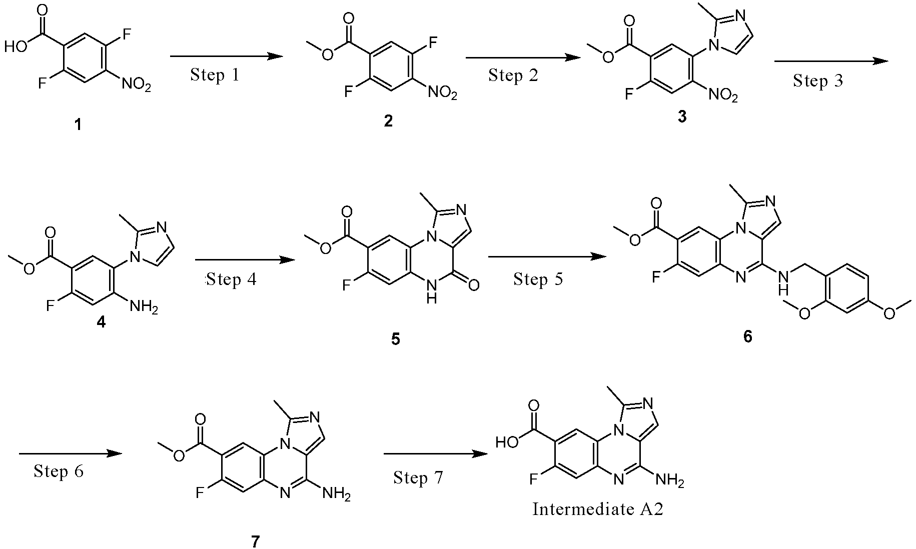

### Step 1: methyl 2,5-difluoro-4-nitrobenzoate (2)

To a solution of 2,5-difluoro-4-nitrobenzoic acid (1) (50g, 246.18 mmol, 1 equiv) in methanol (500 mL) was added thionyl chloride (43.93 g, 369.28 mmol, 26.79 mL, 1.5 equiv) at 0 °C. The reaction solution was reacted for 16 hours at 40°C. LCMS showed the reaction was completed. The reaction solution was concentrated under reduced pressure until dry, then diluted with 300 mL of water and extracted with 1 L of ethyl acetate three times. The organic phase was washed with saturated salt water (400ml), dried with anhydrous magnesium sulfate, and filtered. The filtrate was concentrated under reduced pressure until dry. The crude product was pulped with petroleum ether at 25 °C for 60 minutes to obtain white solid methyl 2,5-difluoro-4-nitrobenzoate (2) (103 g, 474.38 mmol, 96.35% yield). ¹H NMR (400 MHz, CDCl3) δ ppm 7.89 (td, J=9.51, 5.63 Hz, 2 H) 4.00 (s, 3 H). ¹⁹F NMR (376 MHz, CDCl3) δ ppm -110.27 (s, 1 F) -121.56 (m, 1 F)

### Step 2: methyl 2-fluoro-5- (2-methyl-1H-imidazol-1-yl) -4-nitrobenzoate (3)

Methyl 2,5-difluoro-4-nitrobenzoate (2) (80 g, 368.45 mmol, 1 equiv) and 2-methyl-1H-imidazole (36.30 g, 442.14 mmol, 1.2 equiv) were dissolved in dimethyl sulfoxide (1.2 L). The reaction solution was reacted at 50°C for 16 hours. LCMS showed the reaction was completed. The reaction solution was diluted with 4 L of water and extracted with 4.5 L of ethyl acetate. The organic phase was washed with saturated salt water (3 L), dried with anhydrous magnesium sulfate, and filtered. The filtrate was concentrated under reduced pressure to dryness. The crude product was pulped with methyl tert butyl ether at 25°C for 60 minutes (25.5 g). (2) The mother liquor was purified by column chromatography (silica, 50% tetrahydrofuran in petroleum ether) to obtain a yellow liquid, which was then pulped in MTBE at 25 °C for 60 minutes to obtain a yellow solid (8.23 g). Yellow solid methyl 2-fluoro-5- (2-methyl-1H-imidazol-1-yl) -4-nitrobenzoate (3) (25.5 g, 91.32 mmol, 24.79% yield), methyl 2,5-difluoro-4-nitrobenzoate (2) (raw material recovery) (20.34 g, 93.68 mmol, 25.43% yield). yellow solid methyl 2-fluoro-5- (2-methyl-1H-imidazol-1-yl) -4-nitrobenzoate (3) (8.2 g, 29.07 mmol, 7.89% yield, 99% purity) HNMR: ES23714-67-P1A1, 1H NMR (400 MHz, DMSO-d6) δ ppm 8.39 (d, J=9.90 Hz, 1 H) 8.08 - 8.20 (m, 1 H) 7.23 (d, J=1.32 Hz, 1 H) 6.92 (s, 1 H) 3.91 (s, 3 H) 2.12 (s, 3 H). 19F NMR (376 MHz, DMSO-d6) δ ppm -105.45 (br s, 1 F)

### Step 3: methyl 4-amino-2-fluoro-5- (2-methyl-1H-imidazol-1-yl)benzoate (4)

Methyl 2-fluoro-5- (2-methyl-1H-imidazol-1-yl) -4-nitrobenzoate (3) (13.9 g, 49.78 mmol, 1 equiv) was dissolved in tetrahydrofuran (300 mL), and added with palladium carbon hydroxide (2.8 g, 49.78 mmol, 20% purity, 1 equiv) under hydrogen atmosphere. The reaction system was replaced three times with hydrogen gas. The reaction solution was heated to 50°Cunder hydrogen atmosphere(1 equiv) (50 psi)and reacted for 32 hours . LCMS showed the reaction was completed. The reaction solution was filtered through diatomite, and the filter cake was washed four times with 300 mL of ethyl acetate. The filtrate was concentrated to dry under reduced pressure to obtain a gray solid. The crude product was directly used in the next step. Methyl 4-amino-2-fluoro-5- (2-methyl-1H-imidazol-1-yl)benzoate (4) (12 g, 48.15 mmol, 96.72% yield) ¹H NMR (400 MHz, DMSO-*d*₆) : 7.46 (d, J=7.63 Hz, 1H), 7.07 (d, J=1.38 Hz, 1H), 6.93 (d, J=1.25 Hz, 1H), 6.59 (d, J=13.51 Hz, 1H), 6.15 (br s, 2H), 3.68-3.77 (m, 3H), 2.01-2.12 (m, 3H).¹⁹F NMR (376 MHz, DMSO-*d*₆) : -109.31--108.47 (m, 1F).

### Step 4: methyl 7-fluoro-1-methyl-4-oxo-4,5-dihydroimidazolo [1,5-a] quinoxalin-8-carboxylate (5)

Methyl 4-amino-2-fluoro-5- (2-methyl-1H-imidazol-1-yl)benzoate (4) (12 g, 48.15 mmol, 1 equiv) was added to 1-methyl-2-pyrrolidone at 25°C, and then 1,1-carbonyldiimidazole (19.52 g, 120.37 mmol, 2.5 equiv) was added. The reaction solution was heated to 115°C and reacted for 16 hours. LCMS showed the reaction was completed. The reaction solutions of the two batches were combined for processing. The reaction solution was added with 600 mL of ethyl acetate and 600 mL of water, and pulped for 16 hours at 25°C. The slurry was filtered under reduced pressure, and the filter cake was washed with 100 mL of ethyl acetate. The solid was concentrated under reduced pressure to obtain gray solid methyl 7-fluoro-1-methyl-4-oxo-4,5-dihydroimidazolo [1,5-a] quinoxalin-8-carboxylate (5)(24.1 g, 87.56 mmol, 86.60% yield). The crude product was directly used in the next step reaction. ¹H NMR (400 MHz, DMSO-*d*₆) : 11.71 (br s, 1H), 8.40 (d, J=6.38 Hz, 1H), 7.76 (s, 1H), 7.08 (d, J=11.38 Hz, 1H), 3.88 (s, 3H), 2.89 (s, 3H). ¹⁹F NMR (376 MHz, DMSO-*d*₆) : -111.43--110.58 (m, 1F)

### Step 5: methyl 4- ((2,4-dimethoxybenzyl) amino) -7-fluoro-1-methylimidazolo [1,5-a] quinoxalin-8-carboxylate (6)

Methyl 7-fluoro-1-methyl-4-oxo-4,5-dihydroimidazolo [1,5-a] quinoxalin-8-carboxylate (5) (12.0 g, 43.60 mmol, 1.0 equiv), 2,4-dimethoxybenzylamine (10.9 g, 65.19 mmol, 9.82 mL, 1.50 equiv), and 1.8-diazabicyclo [5.4.0]undec-7-ene (19.92 g, 130.80 mmol, 19.72 mL, 3.0 equiv) were added to acetonitrile (240 mL), and benzotriazol-1-oxo-tris (dimethylaminophosphate) hexafluorophosphate salt(25.07 g, 56.68 mmol, 1.3 equiv) was added in batches at 15-20 °C. The reaction solution was slightly exothermic, and became homogeneous and solids precipitated. The reaction solution was reacted for 16 hours at 15-20°C under nitrogen protection. LCMS showed the starting material was completely consumed and the target compound was detected. The reaction suspension was filtered under reduced pressure and the filter cake was washed with 100 mL of acetonitrile. The solid was collected and drained to dryness under reduced pressure to obtain offwhite solid methyl 4- ((2,4-dimethoxybenzyl) amino) -7-fluoro-1-methylimidazolo [1,5-a] quinoxalin-8-carboxylate (6) (15.3 g, 36.05 mmol, 82.68% yield). LCMS: (ESI) m/z= 425.3[M+1]⁺; RT= 1.721 min. ¹H NMR (400 MHz, DMSO-*d*₆) Shift: 8.49 (d, J=7.00 Hz, 1H), 8.45 (t, J=5.57 Hz, 1H), 7.95 (s, 1H), 7.23 (d,J=12.51 Hz, 1H), 7.18 (d, J=8.38 Hz, 1H), 6.58 (d, J=2.38 Hz, 1H), 6.47 (dd, J=2.38, 8.38 Hz, 1H), 4.66 (d,J=5.25 Hz, 2H), 3.88 (s, 3H), 3.82 (s, 3H), 3.73 (s, 3H), 2.93 (s, 3H). ¹⁹F NMR (376.5 MHz, DMSO-*d*₆) Shift: - 113.02.

### Step 6: methyl 4-amino-7-fluoro-1-methylimidazolo [1,5-a] quinoxalin-8-carboxylate (7)

Methyl 4- ((2,4-dimethoxybenzyl) amino) -7-fluoro-1-methylimidazolo [1,5-a] quinoxalin-8-carboxylate (6) (16.3 g, 38.40 mmol, 1.0 equiv) was added to dichloromethane(50 mL) and trifluoroacetic acid (250 mL) was added. The reaction solution was heated to 50°C and reacted for 16 hours. LCMS showed the starting material was completely consumed and the target compound was detected. The reaction solution was concentrated to dryness under reduced pressure to obtain purple solid methyl 4-amino-7-fluoro-1-methylimidazolo [1,5-a] quinoxalin-8-carboxylate (7) (28.3 g, crude product). The crude product was directly used in the next step reaction. LCMS ES15882-1150-P1A: (ESI) m/z=275.3 [M+1]⁺; RT= 0.607 min

### Step 7: 4-amino-7-fluoro-1-methylimidazolo [1,5-a] quinoxalin-8-carboxylic acid (intermediate A2)

Methyl 4-amino-7-fluoro-1-methylimidazolo [1,5-a] quinoxalin-8-carboxylate (7)(crude product from the previous step) (28.3 g, 38.70 mmol, 1 equiv) was added to tetrahydrofuran (80 mL) and methanol (80 mL). Sodium hydroxide (7.74 g, 193.48 mmol, 5 equiv) was dissolved in water (80 mL) and added to the reaction solution. The reaction solution was heated to 50°C and reacted for 4 hours. LCMS detected the desired compound. After cooling to 20°C, the reaction solution was concentrated under reduced pressure to remove the organic solvent. The residue was diluted with water/methanol in a ratio of 10 to 1 (300 mL), and filtered through diatomite. The filter cake was washed three times with water/methanol in a ratio of 10 to 1(300 mL). All filtrates were combined and concentrated under reduced pressure to remove methanol. The pH of the residue was adjusted to 5~6 using acetic acid. The resulting slurry was stirred at 15-20°C for 12 hours and filtered under reduced pressure to obtain a solid, which was washed with water. The solid was collected and lyophilized to obtain white solid 4-amino-7-fluoro-1-methylimidazolo [1,5-a] quinoxalin-8-carboxylic acid (intermediate A2) (9.9 g, 37.55 mmol, 97.05% yield, 98.71% purity). LCMS ES15882-1154-P1C: (ESI) m/z= 261.1 [M+1]⁺; RT= 0.422 min. ¹H NMR (400 MHz, DMSO-*d*₆): 13.15 (br s, 1H), 8.53 (d, J=7.04 Hz, 1H), 7.85 (s, 1H), 7.68 (s, 2H), 7.16 (d, J=12.10 Hz, 1H), 2.94 (s, 3H)

### General method: Synthesis of intermediate A3

### Synthetic route:

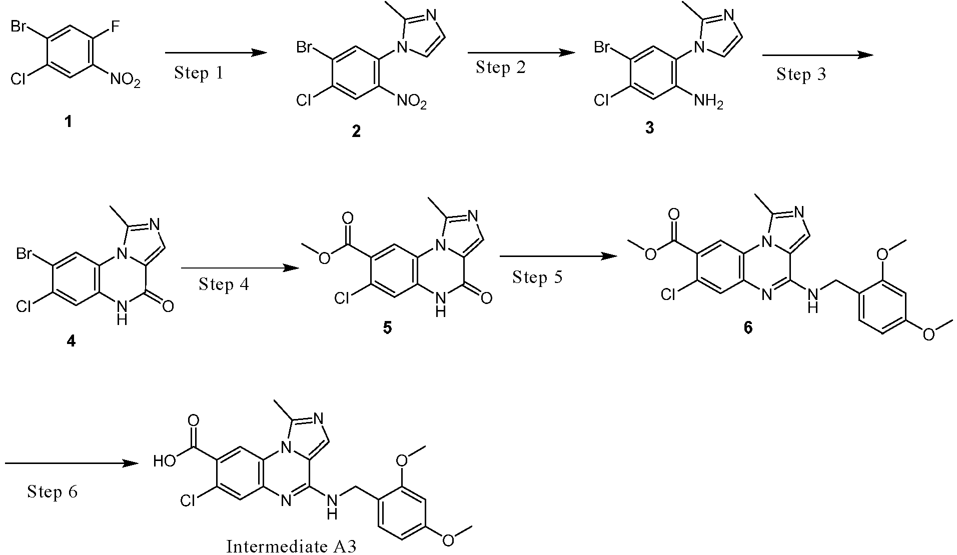

### Step 1:1- (5-bromo-4-chloro-2-nitro-phenyl) -2-methyl-imidazole (2)

1-bromo-2-chloro-5-fluoro-4-nitrobenzene (1) (3 g, 11 mmol, 1 equivalent) was addded to a solution of 2-methyl-1H-imidazole (1.2 g, 14 mmol, 1.2 equivalent) in acetonitrile (50 mL), and then the reaction solution was added with potassium carbonate (4 g, 29 mmol, 2.5 equivalent). The reaction solution was heated to 80°C and stirred for 16 hours. The starting material was detected to be completely consumed and the target compound was generated. The reaction solution was concentrated under reduced pressure to remove acetonitrile, added with water(40 mL) and extracted with ethyl acetate(3 x 50 mL). The organic phase was dried over magnesium sulfate anhydrous and filtered. The filtrate was concentrated to dryness under reduced pressure and purified by column chromatography(silica, 35% tetrahydrofuran in petroleum ether) to obtain white solid 1- (5-bromo-4-chloro-2-nitro-phenyl) -2-methyl-imidazole (2) (4 g). H NMR: 1H NMR (400 MHz, DMSO-d6) δ 8.55 (s, 1H), 8.32 (s, 1H), 7.22 (d, J=1.32 Hz, 1H), 6.91 (d, J=1.32 Hz, 1H), 2.08-2.23 (m, 3H).

### Step 2: 4-bromo-5-chloro-2- (2-methylimidazol-1-yl) aniline (3)

1- (5-bromo-4-chloro-2-nitro-phenyl) -2-methyl-imidazole (2) (3.5 g, 11 mmol, 1 equivalent) was dissolved in a mixed solution of water (8 mL), ethanol (16 mL), and tetrahydrofuran (16 mL). The reaction solution was added with ammonium chloride (8.9 g, 166 mmol, 15 equivalents) and iron powder (2.5 g, 44 mmol, 4 equivalent) was added to the reaction solution after raising to 70°C. The reaction was stirred at 90°C for 2 hours. LC-MS detected that the raw material was completely consumed and the target product was generated. The reaction solution was filtered through diatomite, washed with ethyl acetate (40mL x 3), and concentrated under reduced pressure to obtain black solid 4-bromo-5-chloro-2- (2-methylimidazol-1-yl) aniline (3) (3.1 g, 10.8 mmol, 98% yield). H NMR: 1H NMR (400 MHz, DMSO-d6) δ 7.41 (s, 1H), 7.12 (s, 1H), 7.07 (s, 1H), 6.99 (s, 1H), 5.45 (s, 2H), 2.13 (s, 3H).

### Step 3: 8-bromo-7-chloro-1-methyl-5-hydro-imidazol [1,5-a] quinoxalin-4-one (4)

4-bromo-5-chloro-2- (2-methylimidazol-1-yl) aniline (3) (3 g, 10.5 mmol, 1 equivalent) and 1,1-carbonyl diimidazole (2.6 g, 15.7 mmol, 1.5 equivalent) were dissolved successively in 1,2-dichlorobenzene solution(30 mL). The reaction solution was stirred at 130°C for 16 hours. LC-MS detected that the raw material was completely consumed and the target product was generated. The reaction solution was stirred in a solution of ethyl acetate and water (2/1 15 mL) for 30 minutes and filtered to obtain a filter cake. The filter cake was concentrated under vacuum to obtain black solid 8-bromo-7-chloro-1-methyl-5-hydro-imidazol [1,5-a] quinoxalin-4-one (4) (2.2 g, 7 mmol, 67% yield). H NMR: 1H NMR (400 MHz, DMSO-d6) δ 8.04 (s, 1H), 7.33 (d, J=3.30 Hz, 2H), 2.83 (s, 3H)

### Step 4: methyl 7- chloro-1-methyl-4-oxo-5H-imidazol [1,5-a] quinoxalin-8-carboxylate (5)

8-bromo-7-chloro-1-methyl-5-hydro-imidazol [1,5-a] quinoxalin-4-one (4) (500 mg, 1.6 mmol, 1 equivalent) was dissolved in ethanol (5 mL) solution and 1.8-diazadicyclo [5.4.0] undec-7-ene (365 mg, 2.4 mmol, 362 µL, 1.5 equivalent) was added. The reaction was replaced with nitrogen gas for three times. Tributylphosphorus tetrafluoroborate (46 mg, 160 µmol, 0.1 equivalent), molybdenum hexacarbonyl (232 mg, 880 µmol, 118 µL, 0.55 equivalent) and palladium acetate (36 mg, 160 µmol, 0.1 equivalent). The reaction was stirred at 90°C for two hours. LC-MS detected that the raw material was completely consumed and the target product was generated. The reaction solution was concentrated under reduced pressure to remove ethanol, added with water (10 mL) and extracted with 30 mL of ethyl acetate (10 mL*3). The organic phase was dried with anhydrous magnesium sulfate, and filtered. The filtrate was concentrated under reduced pressure to dryness, and purified by column chromatography (silica, 30% tetrahydrofuran in petroleum ether) to obtain yellow solid methyl 7- chloro-1-methyl-4-oxo-5H-imidazol [1,5-a] quinoxalin-8-carboxylate (5) (500 mg).

### Step 5: methyl 7-chloro-4- ((2,4-dimethoxybenzyl) amino) -1-methylimidazolo [1,5-a] quinoxalin-8-carboxylate (6)

Methyl 7- chloro-1-methyl-4-oxo-5H-imidazol [1,5-a] quinoxalin-8-carboxylate (5) (480 mg, 1.6 mmol, 1 equivalent) was dissolved in acetonitrile(5 mL), and 2,4-dimethoxybenzylamine (341 mg, 2 mmol, 306 µL, 1.3 equivalent), benzotriazol-1-oxo-tri (dimethylaminophosphate) hexafluorophosphate salt (1 g, 2.4 mmol, 1.2 mL, 1.5 equivalents) and 1.8-diazabicyclic [5.4.0] undec-7-ene (1.2 g, 7.9 mmol, 1.2 mL, 5 equivalents) were added. The reaction was stirred at room temperature for 16 hours. LC-MS detected that the raw material was completely consumed and the target product was generated. The reaction solution was concentrated under reduced pressure to remove acetonitrile, and added with water (8 mL) and extracted with 30 mL of ethyl acetate (10 mL *3). The organic phase was dried with anhydrous magnesium sulfate, and filtered. The filtrate was concentrated under reduced pressure to dryness, and purified by column chromatography (silica, 30% tetrahydrofuran in petroleum ether) to obtain yellow solid methyl 7-chloro-4- ((2,4-dimethoxybenzyl) amino) -1-methylimidazolo [1,5-a] quinoxalin-8-carboxylate (6) (500 mg, 1.1 mmol, 70% yield).

### Step 6: 7-chloro-4- ((2,4-dimethoxybenzyl)amino) -1-methylimidazolo [1,5-a] quinoxalin-8-carboxylic acid (Intermediate A3)

Methyl 7-chloro-4- ((2,4-dimethoxybenzyl) amino) -1-methylimidazolo [1,5-a] quinoxalin-8-carboxylate (6) (500 mg, 1.1 mmol, 1 equivalent) was dissolved in water(5 mL) and ethanol(5 mL) and sodium hydroxide(132 mg, 3.3 mmol, 3 equivalents) was added. The reaction solution was stirred at 50°C for 5 hours. LC-MS detected that the raw material was completely consumed and the target product was generated. The reaction solution was added with 6 mol of HCl(0.5 mL) and concentrated to dryness under reduced pressure. The crude product was directly used in the next step to obtain white solid 7-chloro-4- ((2,4-dimethoxybenzyl)amino) -1-methylimidazolo [1,5-a] quinoxalin-8-carboxylic acid (Intermediate A3) (350 mg, 820 µmol, 74.6 % yield).

### General method: Synthesis of intermediate A4

### Synthetic route:

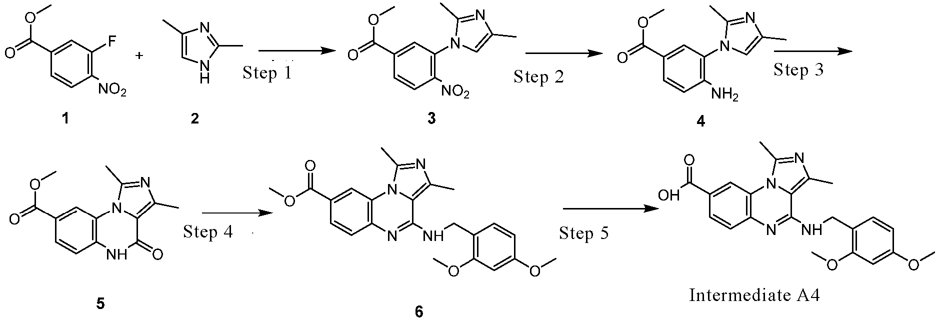

### Step 1: methyl 3- (2,4-dimethylimidazol-1-yl) -4-nitrobenzoate

Methyl 3-fluoro-4-nitrobenzoate (1) (2.00 g, 10.04 mmol, 1 equivalent) was added to acetonitrile (40 equivalent), and potassium carbonate (4.16 g, 30.13 mmol, 3 equivalents) and 2,4-dimethyl-1H-imidazole (2) (965 mg, 10.04 mmol, 1 equivalent) were addd. The reaction solution was reacted at 85 °C for 16 hours. LC-MS showed that the raw material was completely consumed and the target product was generated. The reaction solution was concentrated under reduced pressure to dryness, and the residue was diluted with dichloromethane (80 equivalents), and filtered. The filtrate was dried with magnesium sulfate, filtered and concentrated under reduced pressure to dryness to obtain yellow solid methyl 3- (2,4-dimethylimidazol-1-yl) -4-nitrobenzoate (3) (2.6 g, 9.45 mmol, 94.05% yield). The crude product was directly used in the next step without further purification.

### Step 2: methyl 4-amino-3- (2,4-dimethylimidazol-1-yl)benzoate

Methyl 3- (2,4-dimethylimidazol-1-yl) -4-nitrobenzoate (3) (2.5 g, 9.08 mmol, 1 equivalent) was dissolved in ethanol (20 equivalents), tetrahydrofuran (20 equivalents) and water(10 equivalents), and iron powder(5.07 g, 90.82 mmol, 10 equivalents) and ammonium chloride(2.43 g, 45.41 mmol, 5 equivalents) were added at room temperature. The reaction solution was reacted for 16 hours at 90°C. LC-MS showed that the raw material was completely consumed and the target product was generated. The reaction solution wad filtered and concentrated under reduced pressure to dryness. The residue was diluted with water (50 equivalents), and extracted with ethyl acetate (40 equivalents * 3). The organic phases were combined and dried with magnesium sulfate, filtered, and concentrated under reduced pressure to dryness to obtain yellow solid methyl 4-amino-3- (2,4-dimethylimidazol-1-yl) benzoate (4) (1.9 g, 6.13 mmol, 67.49% yield, 79.13% purity). The crude product was directly used in the next step without further purification. LCMS: ES19974-375-P1C2, (ESI) m/z=246.1 [M+1] +; RT=0.61 min, purity: 79.13%

### Step 3: methyl 1,3- dimethyl-4-oxo-4,5-dihydroimidazolo [1,5-a] quinoxalin-8-carboxylate

Methyl 4-amino-3- (2,4-dimethylimidazol-1-yl) benzoate (4) (500 mg, 2.04 mmol, 1 equivalent) and 1,1-carbonyl diimidazole (495 mg, 3.06 mmol, 1.5 equivalents) were dissolved in 1,2-dichlorobenzene (10 equivalents). The reaction solution was reacted at 120°C for 16 hours under N₂ protection. LC-MS showed that the raw material was completely consumed and the target product was generated. The reaction solution was suction filtrated and the filter cake was pulped with water and then dried under reduced pressure to obtain brown solid methyl 1,3- dimethyl-4-oxo-4,5-dihydroimidazolo [1,5-a] quinoxalin-8-carboxylate (5) (460 mg, 1.62 mmol, 79.48% yield, 95.55% purity). The crude product was directly used in the next step without further purification.
LCMS: ES19974-392-P1B1, (ESI) m/z= 272.0 [M+1] +; RT=0.61 min, purity: 95.55%

### Step 4: methyl 4- ((2,4-dimethoxybenzyl) amino) -1,3- dimethylimidazolo [1,5-a] quinoxalin-8-carboxylate

Methyl dimethyl-4-oxo-4,5-dihydroimidazolo [1,5-a] quinoxalin-8-carboxylate (5) (700 mg, 2.58 mmol, 1 equivalent) was dissolved in acetonitrile (25 equivalents), and benzotriazole-1-oxo-tris (dimethylaminophosphate) hexafluorophosphate salt (1.83 g, 4.13 mmol, 1.6 equivalents) and 1.8 diazabicyclo [5.4.0] undec-7-ene (1.96 g, 12.90 mmol, 1.94 equivalents, 5 equivalents) were added. The reaction solution was reacted at room temperature for 0.5 hours and then (2,4-dimethoxyphenyl) methylamine (647.2 mg, 3.87 mmol, 583.1 µL, 1.5 equivalents) was added. The reaction solution was reacted at 50°C for 15.5 hours. LC-MS showed that the raw material was completely consumed and the target product was generated. The reaction solution was filtered and the filter cake was dried under reduced pressure to obtain brown solid methyl 4- ((2,4-dimethoxybenzyl) amino) -1,3-dimethylimidazolo [1,5-a] quinoxalin-8-carboxylate (6) (900 mg, 2.08 mmol, 80.57% yield, 97.13% purity). LCMS: ES19974-397-P1B1, (ESI) m/z= 421.1 [M+1] +; RT=0.78 min, purity: 97.13%

### Step 5: 4- ((2,4-dimethoxybenzyl) amino) -1,3- dimethylimidazolo [1,5-a] quinoxalin-8-carboxylic acid

Methyl 4- ((2,4-dimethoxybenzyl) amino) -1,3- dimethylimidazolo [1,5-a] quinoxalin-8-carboxylate (6) (850 mg, 2.02 mmol, 1 equivalent) was dissolved in methanol(10 equivalents), tetrahydrofuran(10 equivalents) and water (5 mL), and Lithium hydroxide(424.2 mg, 10.11 mmol, 5 equivalents) was added. The reaction solution was reacted for 16 hours at 50°C. LC-MS showed that the raw material was completely consumed and the target product was generated. The reaction solution was concentrated under reduced pressure to dryness, and the residue was adjusted to pH=6-7 with acetic acid. After filtration, the filter cake was dried under reduced pressure to obtain brown solid 4-((2,4-dimethoxybenzyl) amino) -1,3- dimethylimidazolo [1,5-a] quinoxalin-8-carboxylic acid (Intermediate A4) (800 mg, 1.97 mmol, 97.37% yield). The crude product was directly used in the next step without further purification.

### General method: Synthesis of intermediate A6

### Synthetic route:

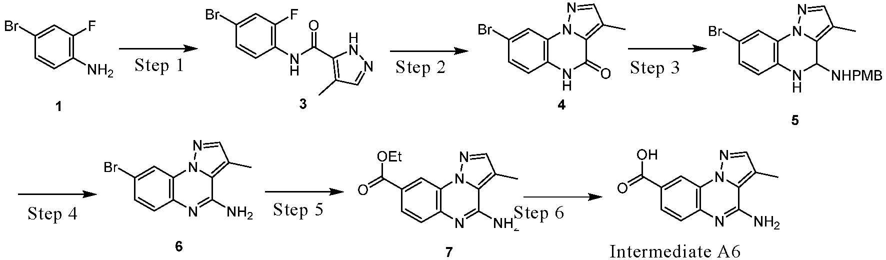

### Step 1: N-(4-bromo-2-fluorophenyl)-4-methyl-1H-pyrazol-5-formamide(3)

To a solution of 4-bromo-2-fluoroaniline (5.78g, 30.4mmol, 1 eq) and 4-methyl-1H-pyrazol-5-carboxylic acid (4.60g, 36.5mmol, 1.2 eq) in Py (120mL) was added POCl₃ (4.66g, 30.4mmol, 2.82mL, 1 eq) and stirred at 0 °C for 1 hour. LC-MS (ET63399-4-R1A1) showed that the starting material was completely consumed and a main peak with the desired m/z was detected. The reaction mixture was quenched with iced water(200mL) and extracted with ethyl acetate (60 mL×6). The combined organic layer was washed with brine(30.0mL) and dried with Na₂SO₄, filtered and concentrated under reduced pressure to obtain a residue. The crude product was ground with ethyl acetate (50.0mL) to obtain a white solid N - (4-bromo-2-fluorophenyl) -4-methyl-1H-pyrazol-5-formamide (3) (7.09g, 23.8mmol, 78.2% yield). ¹H NMR (400 MHz, DMSO-*d*₆) δ 13.26 (br s, 1H) 9.52 (s, 1H) 7.92 (t, *J* = 8.52 Hz, 1H) 7.70 (s, 1H) 7.62 (dd, *J* = 10.31, 1.85 Hz, 1H) 7.41 (br d, *J* = 8.70 Hz, 1H) 2.25 (s, 3H). LC-MS; [MH]⁺ 298.0

### Step 2: 8-bromo-3-methylpyrazolo [1,5-a] quinoxalin-4(5H)-one (4)

To a solution of N - (4-bromo-2-fluorophenyl) -4-methyl-1H-pyrazol-5-formamide (3) (7.09g, 23.8mmol, 1 equivalent) in DMA (70mL) was add NaH (1.43g, 35.7mmol, 60% purity, 1.5 equivalents). The mixture was stirred at 120°C for 16 hours. LC-MS showed that the starting material was consumed completely and a main peak had the desired m/z. The reaction mixture was quenched with saturated ammonium chloride (300mL), and the precipitate was collected and washed with water (50mL), then concentrated under reduced pressure to obtain a white solid of 8-bromo-3-methylpyrazolo [1,5-a] quinoxalin-4(5H)-one (4) (6.50g, crude product). ¹H NMR (400 MHz, DMSO-*d*₆) δ 8.11 (d, *J =* 2.00 Hz, 1H) 7.91 (s, 1H) 7.52 (dd, *J* = 8.63, 2.00 Hz, 1H) 7.28 (d, *J* = 8.63 Hz, 1H) 2.42 (s, 3H). LC-MS; [MH]⁺ 278.0

### Step 3: 8-bromo-N - (4-methoxybenzyl) -3-methyl-4,5-dihydropyrazolo [1,5-a] quinoxalin-4-amine (5)

To a solution of 8-bromo-3-methylpyrazolo [1,5-a] quinoxalin-4(5H)-one (4) (1.00g, 3.60mmol, 1 equivalent) in MeCN (10.0mL) was added PMBNH 2 (1.23g, 8.99mmol, 1.16mL, 2.5 equivalents), BOP (3.18g, 7.19mmol, 2 equivalents), and DBU (2.74g, 18.0mmol, 2.71ml, 5 equivalents). The mixture was stirred at 50°C for 16 hours. LC-MS showed that the starting material was consumed completely and a main peak had the desired m/z. The reaction mixture was diluted with saturated ammonium chloride (10.0mL) and ethanol (5.00mL). The precipitate was collected and washed with water (10.0mL), then concentrated under reduced pressure to obtain a yellow solid of 8-bromo-N - (4-methoxybenzyl) -3-methyl-4,5-dihydropyrazolo [1,5-a] quinoxalin-4-amine (5) (1.09g, crude prroduct). ¹H NMR (400 MHz, DMSO-*d*₆) δ 8.20 (d, *J =* 1.88 Hz, 1H) 7.95 (s, 1H), 7.44-7.48 (m, 2H) 7.38 (d, *J=* 8.63 Hz, 2H) 7.28 (br s, 1H) 6.87 (d, *J=* 8.75 Hz, 2H) 4.70 (d, *J* = 5.88 Hz, 2H) 3.71 (s, 3H) 2.53 (s, 3H). LC-MS; [MH]⁺ 399.0

### Step 4: 8-bromo-3-methylpyrazolo [1,5-a] quinoxalin-4-amine (6)

A solution of 8-bromo-N-(4-methoxybenzyl)-3-methyl-4,5-dihydropyrazolo [1,5-a] quinoxalin-4-amine (5) (500mg, 1.26mmol, 1 equivalent) in TFA (5mL) was stirred at 60 °C for 12 hours. LC-MS showed that the starting material was consumed completely and a main peak had the desired m/z. Te reaction mixture was concentrated under reduced pressure to obtain compound 8-bromo-3-methylpyrazolo [1,5-a] quinoxalin-4-amine (6) (400mg, crude product), as a yellow solid. LC-MS: [MH]⁺ 277.0

### Step 5: ethyl 4-amino-3-methylpyrazolo [1,5-a] quinoxalin-8-carboxylate (7)

To an a solution of 8-bromo-3-methylpyrazolo [1,5-a] quinoxalin-4-amine (6) (400mg, 1.08mmol, 1 equivalent) in EtOH (4mL) was added Mo(CO)₆ (143mg, 541umol, 72.9uL, 0.5 equivalents), DBU (659mg, 4.33 mmol, 652 uL, 4 eq), (t-Bu)3PBF₄ (94.2 mg, 325 umol, 0.3 eq) and Pd(OAc)₂ (36.5 mg, 162 umol, 0.15 eq). The mixture was stirred at 90°C for 12 hours. LC-MS(ET63399-28-R1A1) showed that the starting material was consumed completely and a desired mass was detected. The reaction mixture was diluted with saturated ammonium chloride (10mL) and ethanol (10mL). The precipitate was collected and washed with water (10.0mL), then concentrated under reduced pressure to obtain a yellow solid of ethyl 4-amino-3-methylpyrazolo [1,5-a] quinoxalin-8-carboxylate (7) (250mg, 925umol, 85.4% yield). LC-MS; [MH]⁺ 271.1

### Step 6: 4-amino-3-methylpyrazolo [1,5-a] quinoxalin-8-carboxylic acid (Intermediate A6)

To a solution of ethyl 4-amino-3-methylpyrazolo [1,5-a] quinoxalin-8-carboxylate (7) (250mg, 925umol, 1 equivalent) in EtOH (3mL) and H₂O (1mL) was added LiOH·H₂O (116mg, 2.77mmol, 3 equivalents). The mixture was stirred at 25°C for 12 hours. LC-MS(ET63399-32-R1A1) showed that the starting material was consumed completely and a desired mass was detected. The reaction mixture was concentrated, diluted with water (10mL), and washed with DCM (10.0mL × 3) to remove impurities. The aqueous phase was adjusted to pH=5 with 1M HCl, and the precipitate was collected and purified by preparative HPLC (column: Phenomenex Luna C18 75*30mm*3um; mobile phase: [water (FA)-ACN]; B%: 1%-35%, 8 min, UV 220 nm and 254 nm) to afford a yellow solid of 4-amino-3-methylpyrazolo [1,5-a] quinoxalin-8-carboxylic acid (Intermediate A6) (60.0 mg, 248 umol, 26.8% yield). ¹H NMR (400 MHz, DMSO-*d*₆) δ 12.94 (br s, 1H) 8.67 (d, *J =* 1.96 Hz, 1H) 7.96 (s, 1H) 7.89 (dd, *J* = 8.44, 1.96 Hz, 1H) 7.52 (d, *J* = 8.44 Hz, 1H) 7.19 (br s, 2H) 2.49 (br s, 3H). LC-MS, [MH]⁺ 243.1

### General method: Synthesis of intermediate A7

### Synthetic route:

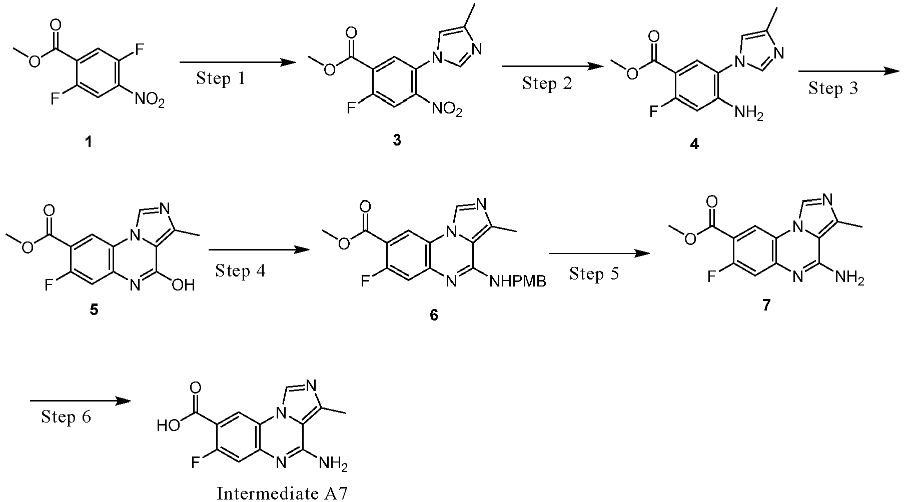

### Step 1: Methyl 2-fluoro-5-(4-methyl-1H-imidazol-1-yl)-4-nitrobenzoate (3)

A mixture of methyl 2,5-difluoro-4-nitrobenzoate (20.0 g, 92.1 mmol, 1 eq.) and 5-methyl-1H-imidazole (7.56 g, 92.1 mmol, 1 eq.) in DMSO (200mL) was stirred at 50°C for 12 hours. LC-MS showed the remaining starting material and detected the desired mass. The residue was diluted with H₂O(400mL) and extracted with EtOAc (200mL×3). The combined organic layer was washed with brine(200mL) and dried with Na₂SO₄, filtered and concentrated under reduced pressure to obtain a residue. The residue was purified by column chromatography (SiO₂, petroleum ether/ethyl acetate=20/1 to 0/1) to obtain a yellow solid compound methyl 2-fluoro-5-(4-methyl-1H-imidazol-1-yl)-4-nitrobenzoate (9.30g, 33.3mmol, 34.2% yield). LC-MS (ESI) m/z = 280.1 [M+H]⁺

### Step 2: methyl 4-amino-2-fluoro-5- (4-methyl-1H-imidazol-1-yl)benzoate (4)

To a solution of methyl 2-fluoro-5-(4-methyl-1H-imidazol-1-yl)-4-nitrobenzoate (9.30g, 33.3mmol, 1 equivalent) and NH₄Cl (26.7g, 499mmol, 15 equivalents) in EtOH (90.0mL)/THF (90.0mL)/H₂O (45.0mL) was added Fe powder (7.44 g, 133 mmol, 4 equivalents). The mixture was stirred at 80°C for 2 hours. LC-MS showed that the starting material was consumed and the desired mass was detected. The reaction mixture was filtered and the filtrate was diluted with water (300 mL) and extracted with EtOAc(200mL×3). The combined organic layer was washed with salt water (100mL), dried with Na₂SO₄, and cncentrated under reduced pressure to obtain compound methyl 4-amino-2-fluoro-5- (4-methyl-1H-imidazol-1-yl)benzoate (8.00g, 32.1mmol, 95.2% yield), as a yellow solid. LC-MS (ESI) m/z = 250.0 [M+H]⁺

### Step 3: Methyl 7-fluoro-4-hydroxy-3-methylimidazolo [1,5-a]quinoxalin-8-carboxylate (5)

Methyl 4-amino-2-fluoro-5- (4-methyl-1H-imidazol-1-yl) benzoate (500 mg, 2.01 mmol, 1 eq) and CDI (487 mg, 2.41 mmol, 1.2 eq) was added to 1,2-dichlorobenzene (20.0 mL) in a microwave tube. The sealed tube was heated under microwave at 150 °C for 3 hours. 11 parallel reactions were performed. LCMS showed that the starting material has been consumed and the desired mass has been detected. The reaction mixture was filtered, and the filter cake was dried under reduced pressure to obtain the residue. The crude product was ground with MTBE(20 mL) at 25°C for 30min to obtain a yellow solid of methyl 7-fluoro-4-hydroxy-3-methylimidazolo [1,5-a]quinoxalin-8-carboxylate (6.00 g, 21.80 mmol, 85.0% yield). LC-MS (ESI) m/z = 276.0 [M+H]⁺

### Step 4: methyl 7-fluoro-4-((4-methoxybenzyl)amino)-3-methylimidazo[1,5-a]quinoxalin-8-carboxylate (6)

To a solution of methyl 7-fluoro-4-hydroxy-3-methylimidazolo [1,5-a]quinoxalin-8-carboxylate 5 (6.00g, 21.8mmol, 1 equivalent) in MeCN (120mL) was added BOP (19.2g, 43.6mmol, 2 equivalents), DBU (16.5g, 109mmol, 16.4mL, 5 equivalents), and PMBNH₂ (7.48g, 54.5mmol, 7.05ml, 2.5 equivalents). The mixture was stirred at 70°C for 16h. LC-MS showed that the starting material was consumed completely and the desired mass was detected. The reaction mixture was diluted with saturated ammonium chloride (20.0mL). The filter cake was washed with water(10.0mL) and concentrated under reduced pressure to obtain yellow solid methyl 7-fluoro-4-((4-methoxybenzyl)amino)-3-methylimidazo[1,5-a]quinoxalin-8-carboxylate 6 (5.00 g, 12.6 mmol, 50.0% yield). LC-MS (ESI) m/z = 395.1 [M+H]⁺

### Step 5: methyl 4-amino-7-fluoro-3-methylimidazolo [1,5-a] quinoxalin-8-carboxylate (6)

A solution of methyl 7-fluoro-4-((4-methoxybenzyl)amino)-3-methylimidazo[1,5-a]quinoxalin-8-carboxylate 6 in TFA(20mL) was stirred at 75°C for 16 hours. LC-MS showed that the starting material was consumed and the desired mass was detected. The reaction mixture was concentrated under reduced pressure to obtain a yellow solid of methyl 4-amino-7-fluoro-3-methylimidazolo [1,5-a] quinoxalin-8-carboxylate 7 (1.39 g, crude product). LC-MS (ESI) m/z = 275.0 [M+H]⁺

### Step 6: 4-amino-7-fluoro-3-methylimidazolo [1,5-a] quinoxalin-8-carboxylic acid (Intermediate A7)

To a solution of methyl 4-amino-7-fluoro-3-methylimidazolo [1,5-a] quinoxalin-8-carboxylate 7 (1.39g, 5.07mmol, 1 equivalent) in EtOH (15mL)/H₂O (5.00mL) was added LiOH (638mg, 15.2mmol, 3 equivalents). The mixture was stirred at 25°C for 12 hours. LC-MS showed that the starting material was consumed and a main peak with the desired mass was detected. The reaction mixture was diluted with H₂O (20.0mL) and extracted with (DCM 10.0mL × 2) to remove impurities. The aqueous layer was adjusted to pH=6 with 2M HCl and the precipitate was collected and dried under reduced pressure to afford 4-amino-7-fluoro-3-methylimidazolo [1,5-a] quinoxalin-8-carboxylic acid (Intermediate A7) (1.30 g, 5.00 mmol, 98.5% yield) as a white solid. ¹H NMR (400MHz, DMSO-d6) *δ* 13.15 (br s, 1H), 9.12 (s, 1H), 8.56 (br d, *J =* 6.4 Hz, 1H), 7.35 (br s, 2H), 7.13 (br d, *J =* 12.1 Hz, 1H), 2.62 (s, 3H). LC-MS (ESI) m/z = 261.0 [M+H]⁺

### General method: Synthesis of intermediate B1

### Synthetic route:

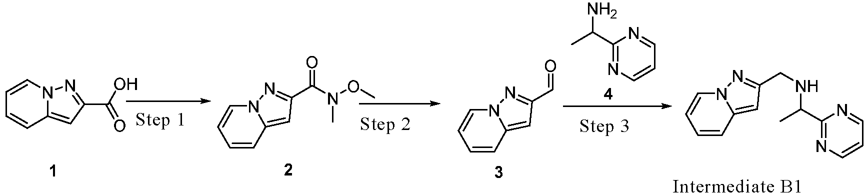

### Step 1: N-methoxy-N-methylpyrazolo[1,5-a]pyridine-2-carboxamide (1)

To a solution of pyrazolo [1,5-a] pyridin-2-carboxylic acid (2.0 g, 12.3 mmol) and HATU (7.0 g, 18.5 mmol) in DMF (100 mL) was added Et3N (6.2 g, 61.7 mmol) and methoxy (methyl) amine (1.9 g, 30.83 mmol) at 25 °C. The mixture was then stirred at 25°C for 12 hours. The reaction was quenched with water(100 mL) and extracted with EA(100mL×3). The organic solution was washed with brine(100 mL). The organic phase was dried over Na₂SO₄ and then filtered. The filtrate was concentrated under reduced pressure. The residue was purified by silica gel chromatography and eluted with a solution of MeOH in DCM from 0% to 10% within 20 minutes to obtain N-methoxy-N-methylpyrazolo[1,5-a]pyridin-2-carboxamide (2.01 g, 79% yield) as a yellow solid. LC-MS: Rt = 1.049 min, (ESI) m/z. [M+H]⁺ 206.1; C10H11N3O2.

### Step 2: Pyrazolo[1,5-a]pyridin-2-carbaldehyde (3)

To a solution of N-methoxy-N-methylpyrazolo[1,5-a]pyridin-2-carboxamide (1.5 g, 7.31 mmol) in THF (20 mL) was added LiAlH₄ (439 mg, 10.96 mmol)at -60 °C. The mixture was then stirred at -60°C for 2 hours. The reaction was quenched with water(40mL) and then filtered. The filtrate was concentrated under reduced pressure. The residue was purified by silica gel chromatography and eluted with a solution of MeOH in DCM from 0% to 10% within 20 minutes to obtain yellow oily pyrazolo[1,5-a]pyridin-2-carbaldehyde (450 mg, 42% yield). LC-MS: Rt = 1.071 min, (ESI) m/z. [M+H]⁺ 147.1; C8H6N2O

### Step 3: N-(pyrazolo[1,5-a]pyridin-2-ylmethyl) -1- (pyrimidin-2-yl) ethan-1-amine(Intermediate B1)

To a solution of pyrazolo [1,5-a] pyridin-2-carbaldehyde (100mg, 0.68mmol) in MeOH (5mL) was added 1-(pyrimidin-2-yl) ethylamine (126mg, 1.03mmol) and NaBH3CN (86mg, 1.37mmol) and reacted at 25 °C. The mixture was then stirred at 25°C for 2 hours. The reaction was quenched with water(1mL) and concentrated under reduced pressure. The residue was purified by silica gel chromatography and eluted with a solution of MeOH in DCM from 0% to 5% within 20 minutes to obtain N-(pyrazolo[1,5-a]pyridin-2-ylmethyl) - 1- (pyrimidin-2-yl) ethylamine (Intermediate B1) (40 mg, 23% yield) as yellow oily substance. LC-MS: Rt = 0.521 min, (ESI) m/z. [M+H]⁺ 254.2; C14H15N5

### General method: Synthesis of intermediate B2

### Synthetic route:

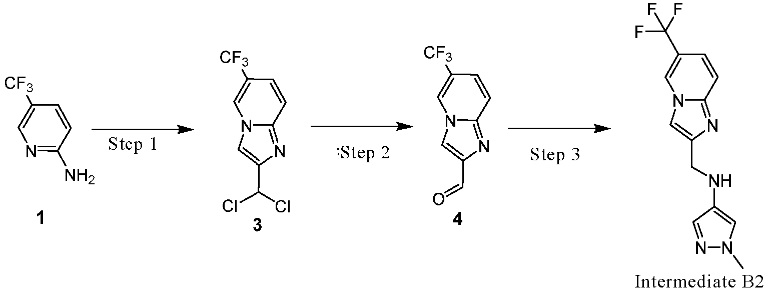

### Step 1: 2-Dichloromethyl-6-trifluoromethylimidazolo[1,2-a]pyridine(3)

A mixture of 5- (trifluoromethyl) pyridin-2-amine (1) (30 g, 185 mmol, 1 equivalent), chlorobenzene (450 mL), and 1,1,3-trichloro-2-acetone (45 g, 277 mmol, 1.5 equivalent) was reacted at 135 °C for 4 hours. LC-MS detected that the target product was generated. The reaction solution was adjusted to pH around 8 with sodium carbonate, and extracted with ethyl acetate (500 mL * 3). The combined organic phase was dried with magnesium sulfate and filtered. The filtrate was concentrated under reduced pressure to dryness, and purified by column chromatography (silica, 15% ethyl acetate in petroleum ether) to obtain a yellow solid of 2-dichloromethyl-6-trifluoromethylimidazolo [1,2-a] pyridine (3) (30 g, 111 mmol, 60% yield). HNMR: ES19506-784-P1A, 1H NMR (400 MHz, DMSO-d6) δ 9.24 (s, 1H), 8.27 (s, 1H), 7.79 (d, J=9.68 Hz, 1H), 7.65 (s, 1H), 7.56 (dd, J=1.65, 9.57 Hz, 1H).

### Step 2: 6-(trifluoromethyl)imidazolo[1,2-a]pyridin-2-carbaldehyde (4)

2-dichloromethyl-6-trifluoromethylimidazolo [1,2-a] pyridine (3) (30 g, 111 mmol, 1 equivalent), water (600 mL), and calcium carbonate (33 g, 334 mmol, 3 equivalent) was heated to 100 °C to react for 2 hours. LC-MS detected that the target product was generated. The reaction solution was added with diatomite and ethyl acetate (600ml) and stirred at room temperature for 30 minutes, filtered, and extracted with ethyl acetate (600ml * 2). The combined organic phase was dried with magnesium sulfate, and filtered. The filtrate was concentrated under reduced pressure to dryness to obtain a brown solid of 6-(trifluoromethyl)imidazolo[1,2-a]pyridin-2-carbaldehyde (4) (35 g). The crude product was directly used in the next step without further purification. H NMR: ES19506-789-P1A1, 1H NMR (400 MHz, CHLOROFORM-d) δ 10.09-10.29 (m, 1H), 8.59 (s, 1H), 8.27 (s, 1H), 7.82 (br d, J=9.46 Hz, 1H), 7.44 (br d, J=9.02 Hz, 1H).

### Step 3: 1-methyl-N-((6-(trifluoromethyl)imidazolo[1,2-a]pyridin-2-yl)methyl)-1H-pyrazol-4-amine (Intermediate B2)

To a solution of 6- (trifluoromethyl) imidazolo [1,2-a] pyridin-2-carbaldehyde (4) (100mg, 467umol, 1 equivalent) in DCM (2.00mL) was added KOAc (91.7mg, 934umol, 2 equivalents) and 1-methyl-1H-pyrazol-4-amine (45.4mg, 467umol, 1 equivalent) at -5 °C, and the reaction mixture was stirred at -5 °C for 1 hour, then added with NaBH(OAc)₃ (198mg, 934umol, 2 equivalents) and stirred at -5 °C for another 3 hours. LCMS (ET63219-45-P1A1) showed that Cpd.4 was consumed completely and several new peaks were displayed. The reaction mixture was diluted with saturated aqueous Na₂CO₃ solution (3.00 mL) and extracted with dichloromethane (2.00mL×4). The combined organic layer was dried over Na₂SO₄ and concentrated under reduced pressure to obtain a residue. The residue was purified by preparative TLC (ethyl acetate/methanol=8/1) to obtain a yellow solid of 1-methyl-N-((6-(trifluoromethyl)imidazolo[1,2-a]pyridin-2-yl)methyl)-1H-pyrazol-4-amine (Intermediate B2) (80.0mg, 271umol, 58.0% yield). ¹H NMR (400 MHz, CDCl3) δ 8.47 (s, 1H), 7.69 (d, *J =* 9.5 Hz, 1H), 7.64 (s, 1H), 7.35 (d, *J =* 1.5, 9.5 Hz, 1H), 7.30 (s, 2H), 6.97 (s, 1H), 4.39 (s, 2H), 3.81 (s, 3H); LC-MS, [MH]⁺ 217.0.

### General method: Synthesis of intermediate B3

### Synthetic route:

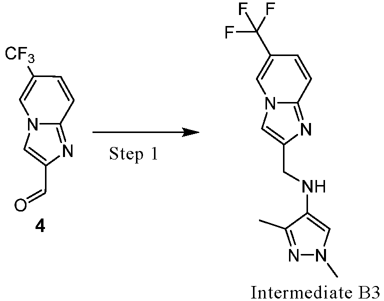

### Step 1: 1,3-Dimethyl-N-((6-(trifluoromethyl)imidazolo[1,2-a]pyridin-2-yl)methyl)-1H-pyrazol-4-amine (Intermediate B3)

6- (trifluoromethyl) imidazolo [1,2-a] pyridin-2-formaldehyde (4) (10 g, 46 mmol, 1 equivalent) and 1,3-dimethylpyrazol-4-amine (6 g, 56 mmol, 1.2 equivalent) were dissolved in dichloromethane (150 mL), and then added with acetic acid (3 g, 56 mmol, 3 mL, 1.2 equivalents). The reaction solution was reacted at to 50°C and reacted for 1 hour, then added with sodium triacetoxyborohydride (25 g, 117 mmol, 2.5 equivalents), and reacted at 25 °C for 3 hours. LCMS detection showed that the target product was generated. The reaction solution was quenched with 200 mL of sodium bicarbonate and extracted with ethyl acetate (150 mL * 2). The combined organic phase was washed with saturated salt water (400ml), dried with anhydrous magnesium sulfate, and filtered. The filtrate was concentrated under reduced pressure to dryness, and purified by column chromatography(silica, 35% ethyl acetate: ethanol (3:1) in petroleum ether) to obtain a brown solid of 1,3-Dimethyl-N-((6-(trifluoromethyl)imidazolo[1,2-a]pyridin-2-yl)methyl)-1H-pyrazol-4-amine (Intermediate B3) (14 g, 45 mmol, 97% yield). HNMR: ES19506-795-P1A, 1H NMR (400 MHz, DMSO-d6) δ 9.19 (s, 1H), 7.92 (s, 1H), 7.67 (d, J=9.46 Hz, 1H), 7.41 (dd, J=1.76, 9.46 Hz, 1H), 6.93 (s, 1H), 4.56 (br s, 1H), 4.17 (br d, J=3.96 Hz, 2H), 3.52-3.61 (m, 3H), 2.04 (s, 3H).LCMS: ES19506-795-P1B, (ESI) m/z=310.3 (M+1) +, RT= 0.64 min.

### General method: Synthesis of intermediate B5

### Synthetic route:

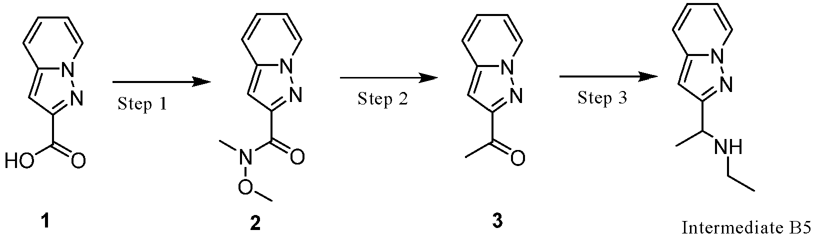

### Step 1: Pyrazolo [1,5-a] pyridin-2-carboxylic acid (2)

To a solution of pyrazolo [1,5-a] pyridin-2-carboxylic acid (10.0g, 61.67 mmol, 1 equivalent), HATU (28.14 g, 74.01 mmol, 1.2 equivalent), and DIEA (31.88 g, 246.69 mmol, 4 equivalents) in DCM (500mL) was added N, O-dimethylhydroxylamine hydrochloride (12.03 g, 123.35 mmol, 2 equivalents). The mixture was stirred at 25°C for 16 hours. LC-MS (ET63565-18-P1A) showed that the starting material was consumed completely and the product was detected. The reaction mixture was concentrated under reduced pressure to obtain the residue. The residue was purified by column chromatography (petroleum ether/ethyl acetate=1/1 to 0/1) to obtain a white solid compound pyrazolo [1,5-a] pyridin-2-carboxylic acid (2) (12.1g, 58.9mmol, yield 95.6%). ¹H NMR (400 MHz, CHLOROFORM-d) δ 8.49 (d, *J* = 7.1 Hz, 1H), 7.57 (d, *J* = 8.9 Hz, 1H), 7.15 (dd, *J* = 7.3, 8.4 Hz, 1H), 7.00 (s, 1H), 6.85 (t, *J* = 6.9 Hz, 1H), 3.79 (s, 3H), 3.49 (s, 3H); LC-MS, [MH]⁺ 206.22.

### Step 2: 1- (pyrazolo [1,5-a] pyridin-2-yl) ethane-1-one (3)

To a solution of pyrazolo [1,5-a] pyridin-2-carboxylic acid (2) (2g, 9.75mmol, 1 equivalent) in THF (20.0mL) was added MeLi (11.70mL, 1.2 equivalents) at -60 °C under N₂ atmosphere, and then stirred at 25 °C for 16 hours. LC-MS (ET63565-13-P1A1) showed that the starting material was consumed completely and the product was detected. The residue was purified by column chromatography (petroleum ether/ethyl acetate=50/1 to 3/1) to obtain a white solid of 1- (pyrazolo [1,5-a] pyridin-2-yl) ethane-1-one (3) (277mg, 1.73mmol, 17.7% yield). LC-MS, [MH]⁺ 161.1.

### Step 3: N-ethyl-1- (pyrazolo [1,5-a] pyridin-2-yl) ethyl-1-amine (Intermediate B5)

A mixture of 1- (pyrazolo [1,5-a] pyridin-2-yl) ethane-1-one (3) (0.1 g, 624.33 umol, 1 eq) and ethylamine (112.58 mg, 2.50 mmol, 163.39 uL, 4 eq) in DCM (2 mL) was stirred at -60 °C for 0.5 hours, then added with NaBH₄ (264.64 mg, 1.25 mmol, 2 equivalents) at 0 °C and stirred at 25 °C for 16 hours under N₂ atmosphere. LC-MS (ET63565-9-P1A2) showed that the starting material was consumed completely and the product was detected. The reaction mixture was quenched with H₂O (5mL) and diluted with DCM (5mL × 3). The combined organic layer was concentrated under reduced pressure to obtain the residue. The residue was purified by preparative TLC (DCM: MeOH=10:1) to obtain a white solid of N-ethyl-1- (pyrazolo [1,5-a] pyridin-2-yl) ethyl-1-amine (Intermediate B5) (50mg, 264umol, 42.3% yield). ¹H NMR (400 MHz, CHLOROFORM-d) δ 8.39 (br d, *J* = 6.9 Hz, 1H), 7.46 (br d, *J* = 8.5 Hz, 1H), 7.12-7.03 (m, 1H), 6.70 (br t, *J* = 6.8 Hz, 1H), 6.44 (s, 1H), 4.13 (br d, *J =* 6.8 Hz, 1H), 3.32-3.21 (m, 1H), 2.77-2.56 (m, 2H), 1.53 (d, *J =* 6.6 Hz, 4H), 1.17-1.14 (m, 3H): LC-MS, [MH]⁺ 190.1.

### General method: Synthesis of intermediate B6

### Synthetic route:

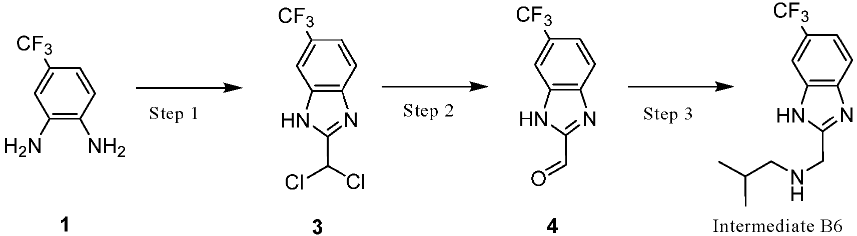

### Step 1: 2-(Dichloromethyl)-6-(trifluoromethyl)-1H-benzo[d]imidazole(3)

A mixture of 4- (trifluoromethyl) benzene-1,2-diamine (5 g, 28.3 mmol, 1 eq) and 2,2-dichloroacetic acid (7.32 g, 56.7 mmol, 4.66 mL, 2 eq) in HCl (125 mL) (4 M) was stirred at 100 °C for 10 minutes. 16 hours. LC-MS (ET60224-68-P1A) showed that Cpd.1was consumed and the desired mass was detected. The reaction mixture was filtered and the filter cake was washed with water. The combined filtrate was extracted with DCM (20ml * 3). The combined organic layer was washed with brine(100mL) and dried with MgSO₄, filtered and concentrated under reduced pressure to obtain the residue. The residue was purified by column chromatography (petroleum ether/ethyl acetate=20/1 to 5/1) to obtain a yellow oily compound 2- (dichloromethyl) -6- (trifluoromethyl) -1H-benzo [d] imidazole (3) (4.4g, 16.3mmol, yield 57.6%). ¹H NMR (400 MHz, CHLOROFORM-d) δ 8.01 (s, 1H), 7.79 (d, *J =* 8.6 Hz, 1H), 7.66 (d, *J =* 8.2 Hz, 1H), 7.26 (s, 1H); LCMS: [M+H]⁺ 268.9

### Step 2: 6- (trifluoromethyl) -1H-benzo [d] imidazol-2-carbaldehyde (4)

To a suspension of 2- (dichloromethyl) -6- (trifluoromethyl) -1H-benzo [d] imidazole (3) (1g, 3.72mmol, 1 equivalent) in H₂O (20mL) was added CaCO₃ (1.12g, 11.1mmol, 3 equivalents). The mixture was stirred at 100°C for 8 hours. LC-MS (ET60224-74-P1B) showed that Cpd.3 was consumed and the desired mass was detected. The reaction mixture was diluted with water(50 mL) and extracted with ethyl acetate(30mL×3). The combined organic layer was concentrated under reduced pressure to obtain a white solid of 6-(trifluoromethyl) -1H-benzo [d] imidazol-2-carbaldehyde (4) (310mg, 1.45mmol, 38.9% yield). (¹H NMR (400 MHz, DMSO-d6) δ 14.28-13.59 (m, 1H), 10.02 (s, 1H), 8.40-8.30 (m, 2H), 8.23-8.05 (m, 3H), 8.00 (dd, *J =* 5.4, 8.5 Hz, 1H), 7.89 (br s, 1H), 7.83-7.61 (m, 3H), 7.34 (br d, *J =* 7.9 Hz, 1H), 7.27 (d, *J =* 7.6 Hz, 1H); LCMS: [M+H]⁺ 215.2

### Step 3: 2-methyl-N- ((6- (trifluoromethyl) -1H-Benzo [d] imidazol-2-yl) methyl) prop-1-amine (Intermediate B6)

To a solution of 6- (trifluoromethyl) -1H-benzo [d] imidazol-2-carbaldehyde (4) (0.31g, 1.45mmol, 1 equivalent) and 2-methylpropan-1-amine (105mg, 1.45mmol, 143uL, 1 equivalent) in DCM (6.2mL) was added KOAc (170mg, 1.74mmol, 1.2 eq) at 25°C. The mixture was stirred at 25 °C for 0.5 hours, and then NaBH(OAc)₃ (398mg, 1.88mmol, 1.3 equivalents) was added to the above-mentioned mixture at 25 °C. The mixture was stirred at 25 °C for 15.5 hours. LC-MS (ET60224-77-P1A) showed that Cpd.4 was consumed and the desired mass was detected. The reaction mixture was diluted with water(10 mL) and extracted with DCM (2mL×3). The combined organic layers were washed with brine(10mL) and dried over MgSO₄, filtered and concentrated under reduced pressure to obtain the residue. The residue was purified by preparative TLC (petroleum ether/ethyl acetate=0/1) to obtain a colorless oily 2-methyl-N- ((6- (trifluoromethyl) -1H-Benzo [d] imidazol-2-yl) methyl) prop-1-amine (Intermediate B6) (100mg, 368umol, 25.4% yield, 100% purity). ¹H NMR (400 MHz, CHLOROFORM-d) 7.80 (s, 1H), 7.56 (d, *J* = 8.4 Hz, 1H), 7.43 (d, *J* = 8.5 Hz, 1H), 4.07 (s, 2H), 2.45 (d, *J =* 6.8 Hz, 2H), 1.82-1.68 (m, 1H), 0.89 (d, *J =* 6.6 Hz, 6H); LCMS: [M+H]⁺ 272.0

### General method: Synthesis of intermediate B7

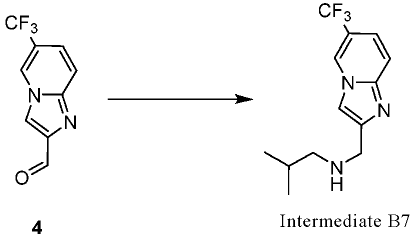

### Step: 2-Methyl-N-((6-(trifluoromethyl)imidazolo [1,2-a]pyridin-2-yl)methyl)propan-1-amine (Intermediate B7)

To a solution of 2-methylpropan-1-amine (68.3mg, 933umol, 92.8uL, 1 equivalent) and 6- (trifluoromethyl) imidazolo [1,2-a] pyridin-2-formaldehyde (0.2g, 933umol, 1 equivalent) in DCM (4mL) was added KOAc (109mg, 1.12mmol, 1.2 equivalents) at 25 °C. The mixture was stirred at 25 °C for 0.5 hours, and then NaBH (OAc)₃ (257mg, 1.21mmol, 1.3 equivalent) was added to the above-mentioned mixture at 25 °C. The mixture was stirred at 25 °C for 15.5 hours. LC-MS (ET60224-75-P1A) showed that Cpd.4 was consumed and the desired mass was detected. The reaction mixture was diluted with water(10 mL) and extracted with DCM (2mL×3) to remove impurities. The aqueous layer was alkalized with saturated Na₂CO₃ to pH=8, and then extracted with DCM (10mL * 3). The combined organic layer was dried with MgSO₄, filtered and concentrated under reduced pressure to obtain a colorless oily intermediate 2-Methyl-N-((6-(trifluoromethyl)imidazolo [1,2-a]pyridin-2-yl)methyl)propan-1-amine (Intermediate B7) (108mg, 398umol, 42.6% yield, 100% purity). ¹H NMR (400 MHz, CHLOROFORM-d) δ 8.49 (s, 1H), 7.70-7.62 (m, 2H), 7.33 (br d, *J* = 9.2 Hz, 1H), 4.01 (s, 2H), 2.54 (d, *J=* 6.7 Hz, 2H), 1.84 (quind, *J* = 6.6, 13.3 Hz, 1H), 0.97 (d, *J* = 6.6 Hz, 6H); LC-MS, [MH]⁺ 272.0.

### Synthesis of Example 4

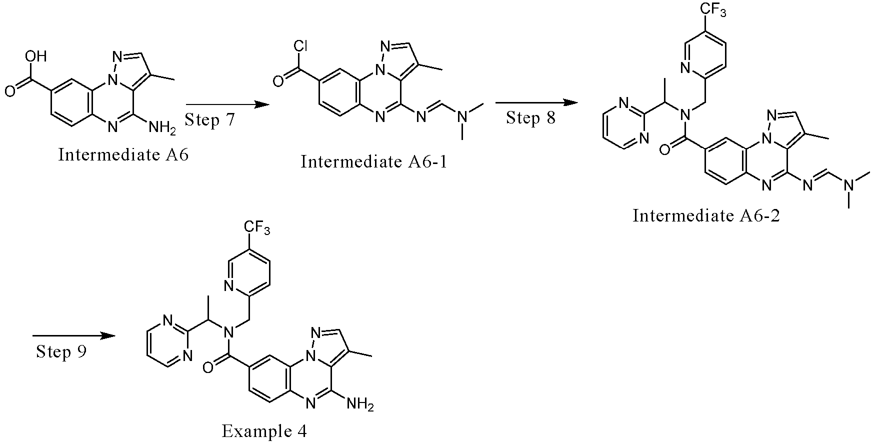

### Step 7: (E) -4- (((dimethylamino) methylene) amino) -3-methylpyrazolo [1,5-a] quinoxalin-8-carboxyl chloride (intermediate A6-1)

To a solution of 4-Amino-3-methylpyrazolo[1,5-a]quinoxalin-8-carboxylic acid (Intermediate A6)(50.0mg, 206µmol, 1 equivalent) in DCM(2 mL) was added HCl/dioxane(4M). The mixture was stirred at 25°C for 0.5 hours. Then the reaction mixture was concentrated, steamed with hexane (10mL × 3), and cooled to 0 °C. Oxalic dichloride (157mg, 1.24mmol, 108uL, 6 equivalents) and DMF (3.02mg, 41.3umol, 3.18uL, 0.2 equivalents) were added dropwise at 0 °C. The mixture was stirred at 0°C for 2 hours. The sample was taken out form MeOH. LC-MS showed that carboxylate of the desired mass was detected. The mixture was concentrated under reduced pressure to obtain the residue. The residue was ground with n-hexane (5mL) and then concentrated under reduced pressure to obtain (E) -4- (((dimethylamino) methylene) amino) -3-methylpyrazolo [1,5-a] quinoxalin-8-carboxyl chloride (Intermediate A6-1) as a yellow solid (50.0mg, 158umol, 76.7% yield). LC-MS, [MH]⁺ 243.1

### Step 8: (E) -4- (((dimethylamino) methylene) amino) -3-methyl-N - (1- (pyrimidin-2-yl) ethyl) - N - ((5- (trifluoromethyl) pyridin-2-yl) methyl) pyrazolo [1,5-a] quinoxalin-8-carboxamide (Intermediate A6-2)

To a solution of (E) -4- (((dimethylamino) methylene) amino) -3-methylpyrazolo [1,5-a] quinoxalin-8-carboxyl chloride (Intermediate A6-1)(50.0 mg, 158 umol, 1 eq) and DIEA (81.9 mg, 633 umol, 110 uL, 4 eq) in THF (2 mL) was added Int. 6 (44.7 mg, 158 umol, 1 equivalent) at 0 °C. The mixture was stirred at 0°C for 2 hours. LC-MS detected that the raw material was completely consumed and the desired mass was detected. The mixture was concentrated under reduced pressure to obtain (E) -4- (((dimethylamino) methylene) amino) -3-methyl-N - (1- (pyrimidin-2-yl) ethyl)-N- ((5- (trifluoromethyl) pyridin-2-yl) methyl) pyrazolo [1,5-a] quinoxalin-8-carboxamide (Intermediate A6-2) (61.0mg, 109umol, 68.6% yield) as a yellow solid. LC-MS, [MH]⁺ 562.2.

### Step 9: 4-Amino-3-methyl-N - (1- (pyrimidin-2-yl) ethyl)-N-((5- (trifluoromethyl) pyridin-2-yl) methyl) pyrazolo [1,5-a] quinoxalin-8-carboxamide (Example 4)

A solution of (E) -4- (((dimethylamino) methylene) amino) -3-methyl-N - (1-(pyrimidin-2-yl) ethyl) - N - ((5- (trifluoromethyl) pyridin-2-yl) methyl) pyrazolo [1,5-a] quinoxalin-8-carboxamide (Intermediate A6-2) (60.0mg, 107umol, 1eq) in NH₃/MeOH (2mL) was stirred at 70 °C for 2 hours. LC-MS detected that the raw material was completely consumed and the desired mass was detected. The reaction mixture was concentrated under reduced pressure to obtain a residue. The residue was purified by preparative HPLC (column: Waters Xbridge Prep OBD C18 150 * 40 mm * 10 um; mobile phase: [water (NH4HCO3) - ACN]); B%: 25% -65%, 8 minutes, UV 220&254 nm) to obtain 4-amino-3-methyl-N - (1- (pyrimidin-2-yl) ethyl)-N-((5- (trifluoromethyl) pyridin-2-yl) methyl) pyrazolo [1,5-a] quinoxalin-8-carboxamide (Example 4) (19.2 mg, 37.9 umol, 35.48% yield, 100% purity) as a white solid. ¹H NMR (400 MHz, DMSO-*d*₆) δ 8.84 (br s, 1H) 8.79 (br d, *J =* 4.63 Hz, 2H) 8.28 (br s, 1H) 8.13 (br d, *J =* 6.75 Hz, 1H) 7.80 - 8.01 (m, 1H) 7.49 - 7.72 (m, 3H) 7.29 - 7.47 (m, 1H) 6.99 (br s, 2H) 5.43 (br s, 1H) 4.92 (br d, *J =* 17.01 Hz, 1H) 4.52 (br d, *J =* 16.51 Hz, 1H) 2.50 (br s, 3H) 1.62 (br d, *J =* 7.00 Hz, 3H). LC-MS, [MH]⁺ 507.2

### Synthesis of Example 15

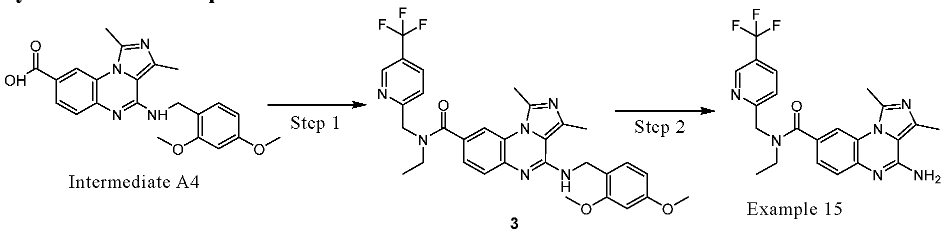

### Step 1: 4-(2,4-dimethoxybenzyl) amino) - N-ethyl-1,3-dimethyl-N - (5-(trifluoromethyl) pyridin-2-ylmethyl) imidazolo [1,5-a] quinoxalin-8-carboxamide (3)

4- ((2,4-dimethoxybenzyl) amino) -1,3-dimethylimidazolo [1,5-a] quinoxalin-8-carboxylic acid (h) (100 mg, 246.0 µmol, 1 equivalent) was dissolved in N, N-dimethylformamide (2.5 mL), and then N-(5- (trifluoromethyl) pyridin-2-ylmethyl) ethylamine (60.3 mg, 295.2 µmol, 1.2 equivalents), N, N, N, N-tetramethylchloroformamidine hexafluorophosphate (138 mg, 492.1 µmol, 2 equivalents), and N-methylimidazole (101 mg, 1.23 mmol, 98.1 µL, 5 equivalents) were added. The reaction solution was reacted at 50 °C for 16 hours. LCMS detected that the raw material was completely consumed and the target product was generated. The reaction solution was concentrated under reduced pressure to dryness and purified by column chromatography (ISCO^{®}; 4 g of SepaFlash ^{®} silica gel column using 0-2.3% methanol/dichloromethane system with a flow rate of 20 mL/min) to obtain 4-(2,4-dimethoxybenzyl) amino) - N-ethyl-1,3-dimethyl-N - (5- (trifluoromethyl) pyridin-2-ylmethyl) imidazolo [1,5-a] quinoxalin-8-carboxamide (3) (150 mg, 233.45 µmol, 94.88% yield, 92.23% purity) as a yellow oil. LCMS: ES19974-409-P1B1, (ESI) m/z= 593.2 [M+1] +; RT=0.82 min, purity: 92.23%

### Step 2: 4-Amino-N-ethyl-1,3-dimethyl-N- ((5-trifluoromethyl) pyridin-2-ylmethyl) imidazolo [1,5-a] quinoxalin-8-carboxamide (Example 15)

4- (2,4-dimethoxybenzyl) amino) - N-ethyl-1,3-dimethyl-N-(5- (trifluoromethyl) pyridin-2-ylmethyl) imidazolo [1,5-a] quinoxalin-8-carboxamide (3) (140 mg, 236.2 µmol, 1 equivalent) was dissolved in dichloromethane (0.5 mL) and trifluoroacetic acid (2.5 mL) was added. The reaction solution was reacted at 50 °C for 16 hours. LC-MS detected that the raw material was completely consumed and the target product was generated. The reaction solution was concentrated to dryness under reduced pressure, and 10% sodium carbonate (10 mL) was added. The mixture was extracted with dichloromethane (10 mL * 3), and the combined organic phases were washed with saturated saline (10 mL), dried over magnesium sulfate, filtered, concentrated to dryness, and purified by reverse phase preparative liquid chromatography (Boston Prime C18 column, 150 * 30 mm * 5 µ m; mobile phase: [water (ammonia)-acetonitrile]; B% gradient: 30%-50%, 9 min) to obtain 4-Amino-N-ethyl-1,3-dimethyl-N- ((5-trifluoromethyl) pyridin-2-ylmethyl) imidazolo [1,5-a] quinoxalin-8-carboxamide (Example 15) (30 mg, 67.81 µmol, 28.70% yield) as a white solid. LCMS: ES19974-414-P1B1, (ESI) m/z= 443.1 [M+1] +; RT=2.623 min, purity: 100.0%. HNMR: ES19974-414-P1A, 1H NMR (400MHz, DMSO-d6) Shift = 8.97 (br s, 1H), 8.20 (br d, J=6.9 Hz, 1H), 7.97 (br s, 1H), 7.61 (br d, J=8.1 Hz, 1H), 7.39 (br s, 2H), 6.72 (br s, 2H), 4.82 (br s, 2H), 3.45 (q, J=6.9 Hz, 2H), 3.10 - 2.60 (m, 3H), 2.56 (br s, 3H), 1.14 (t, J=7.0 Hz, 3H)

### Synthesis of Example 86

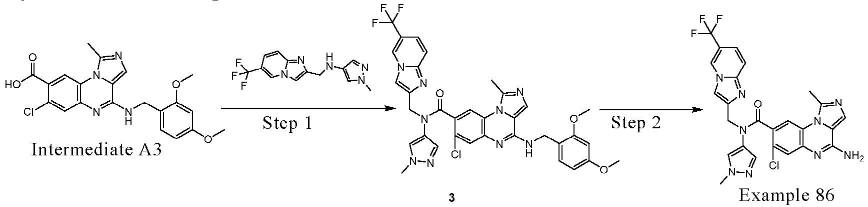

### Step 1: 7-Chloro-4- [(2,4-dimethoxyphenyl) methylamino] -1-methyl-N - (1-methylpyrazol-4-yl)-N-[[6- (trifluoromethyl) imidazolo [1,2-a] pyridin-2-yl] methyl] imidazolo [1,5-a] quinoxalin-8-carboxamide (3)

7-Chloro-4- (2,4-dimethoxybenzyl) amino) -1-methylimidazolo [1,5-a] quinoxalin-8-carboxylic acid (intermediate A3) (145 mg, 339 µmol, 1 equivalent) was added to a solution of 1-methyl-N-[[6- (trifluoromethyl) imidazolo [1,2-a] pyridin-2-yl] methyl] pyrazol-4-amine (intermediate B2) (100 mg, 339 µmol, 1 equivalent) in acetonitrile (2 mL). After dissolution, N, N, N,N-tetramethylchloroformamidinium hexafluorophosphate (285 mg, 1 mmol, 3 equivalents) and N-methylimidazole (139 mg, 1.7 mmol, 135 µL, 5 equivalents) were added sequentially. The reaction was stirred at 50°C for 16 hours. LC-MS detected that the raw material was completely consumed and the target product was generated. The reaction solution was filtered and concentrated under reduced pressure to obtain the crude product. The crude product was purified by column chromatography (silica, 10% methanol in dichloromethane) to obtain 7-Chloro-4- [(2,4-dimethoxyphenyl) methylamino] -1-methyl-N - (1-methylpyrazol-4-yl)-N-[[6- (trifluoromethyl) imidazolo [1,2-a] pyridin-2-yl] methyl] imidazolo [1,5-a] quinoxalin-8-carboxamide (3) (130 mg, 185 µmol, 54.5% yield) as a yellow oil.

### Step 2: 4-Amino-7-chloro-1-methyl-N - (1-methyl-1-hydro-pyrazol-4-yl) - N - ((6-(trifluoromethyl) imidazolo [1,2-a] pyridin-2-yl]) methyl) imidazolo [1,5-a] quinoxalin-8-carboxamide (Example 86)

7-Chloro-4- [(2,4-dimethoxyphenyl) methylamino] -1-methyl-N - (1-methylpyrazol-4-yl)-N-[[6- (trifluoromethyl) imidazolo [1,2-a] pyridin-2-yl] methyl] imidazolo [1,5-a] quinoxalin-8-carboxamide (3) (120 mg, 170 µmol, 1 equivalent) was added to a mixed solution of trifluoroacetic acid (0.4 mL) and dichloromethane (1 mL) and dissolved. The reaction was stirred at 50°C for 16 hours. LC-MS detected that the raw material was completely consumed and the target product was generated. The reaction was concentrated to dryness under reduced pressure, then purified by reverse phase preparative liquid chromatography (Boston Prime C18 column, 5-µm silica with a diameter of 30 mm, and a length of 150 mm, using a mixture of water and acetonitrile with decreasing polarity as eluent) to obtain 4-amino-7-chloro-1-methyl-N - (1-methyl-1-hydro-pyrazol-4-yl)-N-((6-(trifluoromethyl) imidazolo [1,2-a] pyridin-2-yl])methyl) imidazolo[1,5-a] quinoxalin-8-carboxamide (Example 86) as a white solid.H NMR: 1H NMR (400 MHz, DMSO-d6) δ 9.15-9.31 (m, 1H), 8.33 (s, 1H), 8.07-8.16 (m, 1H), 7.92-8.03 (m, 1H), 7.76-7.86 (m, 1H), 7.58-7.75 (m, 2H), 7.41-7.55 (m, 3H), 7.18-7.33 (m, 1H), 4.71-5.17 (m, 2H), 3.53-3.83 (m, 3H), 2.80-2.97 (m, 3H).LCMS: (ESI) m/z=554.2 (M+1)+, RT= 1.544 min, purity of 98.7 %.

### Synthesis of Example 143

Synthesis method:

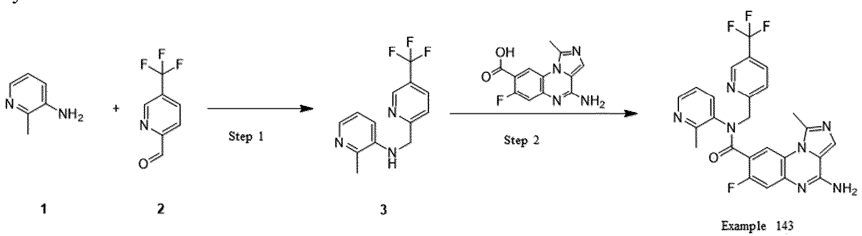

### Step 1: 2-Methyl-N-(5-trifluoromethyl) pyridin-2-ylmethyl) pyridin-3-amine(3)

5- (trifluoromethyl) pyridine carboxaldehyde (2) (300 mg, 1.71 mmol, 1.0 equivalent), 2-methylpyridine-3-amine (1) (216 mg, 2.00 mmol, 1.17 equivalent), and acetic acid (123 mg, 2.05 mmol, 117.25 µL, 1.2 equivalent) were dissolved in dichloromethane (10 mL) and stirred at room temperature for 4 hours. Sodium triacetoxyborohydride (1.09 g, 5.15 mmol, 3.01 equivalent) was added and stirred at room temperature for 12 hours. LCMS detected that the raw material was completely consumed and the target product was generated. The reaction solution was diluted with water and dichloromethane, and the pH of the reaction solution was adjusted to around 12 with a 2 mol potassium carbonate aqueous solution. After partition, the aqueous phase was extracted with dichloromethane (10 mL * 3), and the combined organic phases were dried with sodium sulfate, filtered, concentrated under reduced pressure to dryness, and purified by column chromatography (ISCO) ^{®}; 12 g of SepaFlash ^{®} silica gel column in a 0-25% ethyl acetate/petroleum ether system at a flow rate of 30 mL/min) to obtain 2-methyl-N-(5-trifluoromethyl) pyridin-2-ylmethyl) pyridin-3-amine(3) (205 mg, 44.7% yield) as a yellow slurry. LCMS ES15882-1182-P1A: (ESI) m/z= 268.1 [M+1]⁺; RT= 0.65 min

### Step 2: 4-Amino-7-fluoro-1-methyl-N - (2-methylpyridin-3-yl) - N - (5-(trifluoromethyl) pyridin-2-ylmethyl) imidazolo [1,5-a] quinoxalin-8-carboxamide (Example 143)

2-Methyl-N-(5-trifluoromethyl)pyridin-2-ylmethyl)pyridine-3-amine (3) (60 mg, 224.51 µmol, 1.0 equivalent), 4-amino-7-fluoro-1-methylimidazolo [1,5-a] quinoxalin-8-carboxylic acid (60 mg, 230.57 µmol, 1.03 equivalent), and N-ethyl-N-isopropyl-2-propylamine (119 mg, 925.11 µmol, 161.13 µL, 4.12 equivalents) were dissolved in 1-methylpyrrolidin-2-one (1.0 mL), and 2-chloro-1,3-dimethyl-4,5-dihydroimidazol-1-chloride (DMC) (57.82 mg, 342.02 µmol, 1.52 equivalents) was added. The reaction solution was reacted at 80 °C for 16 hours. LC-MS detection showed that the target product was generated. The reaction solution was purified by reverse phase preparative liquid chromatography (formic acid conditions; Boston Prime C18 column, 150 * 30 mm * 5 µm; Mobile phase: [water (formic acid) - acetonitrile]; Gradient: 15% -35% B, 10 minutes) to obtain 4-amino-7-fluoro-1-methyl-N-(2-methylpyridin-3-yl)-N-(5-(trifluoromethyl) pyridin-2-ylmethyl)imidazolo[1,5-a] quinoxalin-8-carboxamide (Example 143) (8 mg, 6.29% yield) as a yellow solid . LCMS ES15882-1205-P1B1: (ESI) m/z= 510.1 [M+1]⁺; RT= 0.996 min. 1H NMR (400 MHz, DMSO-d6) Shift 8.84-8.98 (m, 1H), 8.17-8.27 (m, 2H), 7.72-7.87 (m, 4H), 7.50 (s, 2H), 7.13 (dd, J=4.64, 7.91 Hz, 1H), 7.01 (d, J=11.29 Hz, 1H), 5.44 (d, J=15.81 Hz, 1H), 5.01 (d, J=15.81 Hz, 1H), 2.82 (s, 3H), 2.37 (s, 3H)
19F NMR (376.5 MHz, DMSO-d6) Shift -60.71, -60.85, -116.51, -119.18

### Synthesis of Example 145

### Synthesis method:

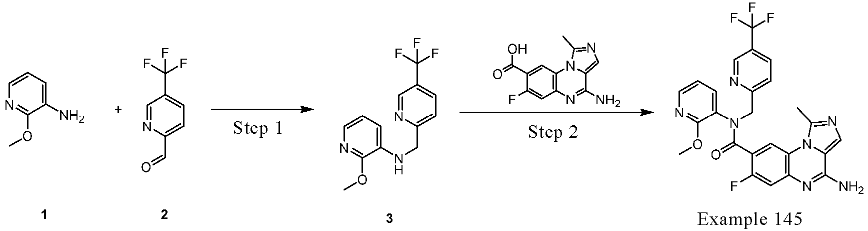

### Step 1: 2-Methoxy-N-(5-trifluoromethyl) pyridin-2-ylmethyl) pyridin-3-amine (3)

5- (trifluoromethyl) pyridine formaldehyde (2) (300 mg, 1.71 mmol, 1.0 equivalent), 2-methoxypyridin-3-amine (1) (248 mg, 2.00 mmol, 1.17 equivalent), and acetic acid (123 mg, 2.05 mmol, 1.2 equivalent) were dissolved in dichloromethane (10 mL) and stirred at room temperature for 4 hours. Sodium triacetoxyborohydride (1.09 g, 5.15 mmol, 3.01 equivalents) was added and the reaction solution was stirred at room temperature for 12 hours. LCMS detected that the raw material was completely consumed and the target product was generated. The reaction solution was diluted with water and dichloromethane, and the pH of the reaction solution was adjusted to around 12 with a 2 mol potassium carbonate aqueous solution. After partition, the aqueous phase was extracted with dichloromethane (10 mL * 3), and the combined organic phases were dried with sodium sulfate, filtered, concentrated under reduced pressure to dryness, and purified by column chromatography (ISCO) ^{®}; 12 g of SepaFlash ^{®} silica gel column in a 0-15% ethyl acetate/petroleum ether system at a flow rate of 30 mL/min) to obtain 2-methoxy-N-(5-trifluoromethyl) pyridin-2-ylmethyl) pyridin-3-amine (3) (430 mg, 1.52mmol, 88.61% yield) as a yellow slurry.
LCMS ES15882-1189-P1A: (ESI) m/z= 284.0 [M+1] +; RT= 0.82 min

### Step 2: 4-Amino-7-fluoro-N-(2-methoxypyridin-3-yl) -1-methyl-N- (5-trifluoromethyl) pyridin-2-methyl) imidazolo [1,5-a] quinoxalin-8-carboxamide (Example 145)

2-Methoxy-N-(5-trifluoromethyl) pyridin-2-ylmethyl) pyridin-3-amine (3) (64 mg, 226 µmol, 1.0 equivalent), 4-amino-7-fluoro-1-methylimidazolo [1,5-a] quinoxalin-8-carboxylic acid (61 mg, 234 µmol, 1.04 equivalent.), and N-ethyl-N-isopropyl-2-propylamine (119 mg, 919 µmol, 4.07 equivalents) were dissolved in 1-methylpyrrolidin-2-one (1.0 mL) and 2-chloro-1,3-dimethyl-4,5-dihydroimidazol-1-chloride (DMC) (58 mg, 344 µmol, 1.52 equivalent.) was added. The reaction solution was reacted at 80 °C for 16 hours. LC-MS detection showed that the target product was generated. The reaction solution was purified by reverse phase preparative liquid chromatography (formic acid conditions; Boston Prime C18 column, 150 * 30 mm * 5 µ m; Mobile phase: [water (formic acid) - acetonitrile]; Gradient: 23% -43% B, 10 minutes) to obtain 4-amino-7-fluoro-N-(2-methoxypyridin-3-yl) -1-methyl-N- (5-trifluoromethyl) pyridin-2-methyl) imidazolo [1,5-a] quinoxalin-8-carboxamide (Example 145) (12 mg, 8.79% yield, 87% purity) as a yellow solid. LCMS ES15882-1206-P1B1: (ESI) m/z= 526.1 [M+1]⁺; RT= 1.140 min

1H NMR (400 MHz, DMSO-d6) Shift 8.91 (s, 1H), 8.23-8.30 (m, 1H), 8.20 (s, 0.2H, HCOOH), 7.91-7.95 (m, 1H), 7.86 (d, J=6.53 Hz, 1H), 7.77-7.83 (m, 2H), 7.72-7.76 (m, 1H), 7.50 (br s, 2H), 7.03 (d, J=11.29 Hz, 1H), 6.89 (dd, J=5.02, 7.53 Hz, 1H), 5.34 (d, J=16.06 Hz, 1H), 5.04 (d, J=15.81 Hz, 1H), 3.74 (s, 3H), 2.80 (s, 3H)
19F NMR (376.5 MHz, DMSO-d6) Shift -60.72, -117.24

### Synthesis of Example 188

### Synthesis method:

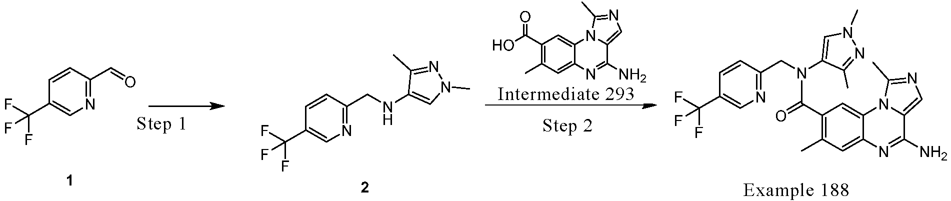

### Step 1: 1,3-Dimethyl-N-((5- (trifluoromethyl) pyridin-2-yl) methyl) -1H-pyrazol-4-amine (2)

5- (trifluoromethyl) pyridine formaldehyde (1) (300 mg, 1.71 mmol, 1 equivalent) was dissolved in dichloromethane (8 mL), and then 1,3-dimethyl-1H-pyrazol-4-amine (247 mg, 2.22 mmol, 1.3 equivalents) and glacial acetic acid (134 mg, 2.23 mmol, 127.74 µL, 1.3 equivalents) were added. The reaction was stirred at 15-20 °C for 4 hours. Sodium borohydride acetate (1.09 g, 5.15 mmol, 3.01 equivalents) was added and the mixture was stirred at 15-20 °C for 12 hours. LC-MS detected that the raw material was completely consumed and the target product was generated. The reaction solution was added with 10 mL of water and 10 mL of dichloromethane, and the pH of the reaction solution was alkalized to around 12 with a 2 mol potassium carbonate aqueous solution. After partition, the aqueous phase was extracted with dichloromethane (10 mL * 3), and the organic phase was dried with anhydrous sodium sulfate, filtered, concentrated under reduced pressure to dryness, and purified by column chromatography (12 g silica gel column using a 0-40% (ethyl acetate:ethanol=3:1)/petroleum ether, at a flow rate of 30 mL/min) to obtain 1,3-dimethyl-N-((5- (trifluoromethyl) pyridin-2-yl) methyl) -1H-pyrazol-4-amine (2) (311 mg, 1.15 mmol, 67.17% yield) as a yellow solid.
LCMS ES15882-1166-P1A: (ESI) m/z= 228.2 [M+1]⁺; RT= 0.601 min

### Step 2: 4-Amino-N-(1,3-dimethyl-1H-pyrazol-4-yl) -1,7-dimethyl-N-((5-(trifluoromethyl) pyridin-2-yl) methyl) imidazolo [1,5-a] quinoxalin-8-carboxamide (Example 188)

To a reaction solution of 4-amino-1,7-dimethylimidazolo [1,5-a] quinoxalin-8-carboxylic acid (c) (42 mg, 163.90 µmol, 1 equivalent), 1,3-dimethyl-N-((5-(trifluoromethyl) pyridin-2-yl) methyl) -1H-pyrazol-4-amine (b) (45.18 mg, 167.17 µmol, 1.02 equivalents), and N, N-diisopropylethylamine (84.73 mg, 655.59 µmol, 114.19 µL, 4 equivalents) in 1-methyl-2-pyrrolidone (1 mL) was added 2-chloro-1,3-dimethyl-4,5-dihydroimidazol-1-chloride (41.56 mg, 245.84 µmol, 1.5 equivalents). The reaction was stirred at 50°C for 3 hours. LC-MS detected that the raw material was completely consumed and the target product was generated. A yellow solid was obtained by reverse phase preparative liquid chromatography purification (Boston Prime C18 column, 5-µm silica with a diameter of 30 mm and a length of 150 mm, using a mixture of water (containing 0.05% formic acid) and acetonitrile (20% -40%) with a decreasing polarity as eluent). The crude product was further purified by reverse phase preparative liquid chromatography (Boston Prime C18 column, 5-µm silica with a diameter of 30 mm and a length of 150 mm, using a mixture of water (containing 0.05% formic acid) and acetonitrile (18% -38%) with a decreasing polarity as eluent) to obtain 4-amino-N-(1,3-dimethyl-1H-pyrazol-4-yl)-1,7-dimethyl-N-((5- (trifluoromethyl) pyridin-2-yl) methyl) imidazolo [1,5-a] quinoxalin-8-carboxamide (Example 188) (10 mg, 19.67 µmol, 12.00% yield) as a white solid. LCMS: ES13685-1063-P1D, (ESI) m/z= 509.1 [M+1] +; RT= 1.066 minNMR: ES13685-1063-P1A, 1H NMR (400 MHz, DMSO-d6) 8.95 (s, 1H), 8.25 (br d, J=8.60 Hz, 1H), 8.15 (s, 1H), 7.75 (s, 2H), 7.56-7.72 (m, 2H), 7.23 (br d, J=4.84 Hz, 3H), 5.14 (s, 2H), 3.51-3.70 (m, 3H), 2.79 (s, 3H), 2.38-2.44 (m, 3H), 1.83 (s, 3H) , 19F NMR (376 MHz, DMSO-d6) -60.68 (br s, 3F)

### Synthesis of Example 210

Synthesis method:

### Step 1: 1,4-Dimethyl-N-((5- (trifluoromethyl) pyridin-2-yl) methyl) -1H-imidazol-2-amine (2)

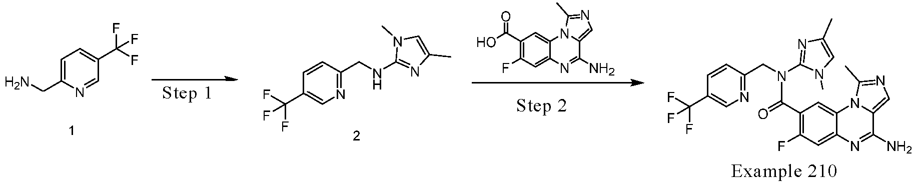

2-Bromo-1,4-dimethyl-1H-imidazole (210 mg, 1.20 mmol, 1 equivalent) was dissolved in tetrahydrofuran (12 mL), and (5- (trifluoromethyl) pyridin-2-yl) methylamine (1) (211 mg, 1.20 mmol, 1 equivalent), sodium trimethyl (oxide) silane (267 mg, 1.44 mmol, 1.2 equivalents), and bromo[dicyclohexyl[3-(1,1-dimethylethoxy)-6-methoxy-2',6'-bis(1-methylethyl)[1,1'-biphenyl]-2-yl-κC1']phosphine-κP][4-[[2-(trimethylsilyl)ethoxy]carbonyl]phenyl]-, (SP-4-2)-palladium (GPhos-Pd-G6) (50 milligrams, 59.99 µmol, 0.05 equivalents) were added under N₂ atmosphere. The reaction was reacted and stirred at 80 °C for 16 hours. LC-MS detected that the raw material was completely consumed and the target product was generated. After cooling to room temperature, the reaction solution was added with 10 mL of water, extracted with ethyl acetate (10 mL * 3); the organic phase was dried with anhydrous magnesium sulfate, and filtered; the filtrate was concentrated under reduced pressure to dryness, and purified by column chromatography (4 g+4 g silica gel column, using 0-40% ethyl acetate/petroleum ether as eluent at a flow rate of 20 mL/min) to obtain 1,4-dimethyl-N-((5- (trifluoromethyl) pyridin-2-yl) methyl) -1H-imidazol-2-amine (2) (50 mg, 185.01 µmol, 15.42% yield) as a yellow liquid.
LCMS: ES13685-1155-P1C, (ESI) m/z= 271.1[M+1] +; RT= 0.926min

### Step 2: 4-Amino-N-(1,4-dimethyl-1H-imidazol-2-yl) -7-fluoro-1-methyl-N - ((5-(trifluoromethyl) pyridin-2-yl) methyl) imidazolo [1,5-a] quinoxalin-8-carboxamide (Example 210)

To a reaction solution of 4-amino-7-fluoro-1-methylimidazolo [1,5-a] quinoxalin-8-carboxylic acid (40 mg, 153.71 µmol, 1 equivalent), 1,4-dimethyl-N - ((5- (trifluoromethyl) pyridin-2-yl) methyl) -1H-imidazol-2-amine (2) (41.54 mg, 153.71 µmol, 1 equivalent), and N, N-diisopropylethylamine (79.46 mg, 614.86 µmol, 107.09 µL, 4 equivalents) in 1-methyl-2-pyrrolidone (1 mL) was added 2-chloro-1,3-dimethyl-4,5-dihydroimidazol-1-chloride (38.98 mg, 230.5 µmol, 1.5 equivalents). The reaction solution was stirred at 60°C for 16 hours. LC-MS detection showed that there were a small amount of residual raw materials and the target product was generated. A yellow solid was obtained by reverse phase preparative liquid chromatography purification (Boston Prime C18 column, 5-µm silica with a diameter of 30 mm and a length of 150 mm, using a mixture of water (containing 0.05% formic acid) and acetonitrile (10% -30%) with a decreasing polarity as eluent). The crude product was further purified by reverse phase preparative liquid chromatography (Boston Prime C18 column, 5-µm silica with a diameter of 30 mm and a length of 150 mm, using a mixture of water (containing 0.05% ammonia) and acetonitrile (28% -48%) with a decreasing polarity as eluent) to obtain 4-amino-N-(1,4-dimethyl-1H-imidazol-2-yl) -7-fluoro-1-methyl-N-((5- (trifluoromethyl) pyridin-2-yl) methyl) imidazolo [1,5-a] quinoxalin-8-carboxamide (Example 210)(3 mg, 5.56 µmol, 3.62% yield) as a white solid. LCMS: ES13685-1158-P1C1, (ESI) m/z= 513.3 [M+1] +; RT= 0.759min
NMR: ES13685-1158-P1A, 1H NMR (400 MHz, DMSO-d6) 8.91 (br s, 1H), 8.23 (br d, J=8.13 Hz, 1H), 7.97 (br d, J=6.75 Hz, 1H), 7.89 (br s, 1H), 7.81 (s, 1H), 7.34 (br s, 2H), 7.13 (br d, J=11.26 Hz, 1H), 6.50 (s, 1H), 5.17 (br s, 2H), 3.23-3.28 (m, 3H), 2.87 (s, 3H), 1.96 (br s, 3H) . NMR: ES13685-1158-P1A, 19F NMR (376 MHz, DMSO-d6) -60.85 (s, 3F), -118.24 (br s, 1F)

### Synthesis of Example 213

### Synthesis method:

### Step 1: 1-Methyl-N - (1-trifluoromethyl) pyrazol-4-ylmethyl) -1H-pyrazol-5-amine (3)

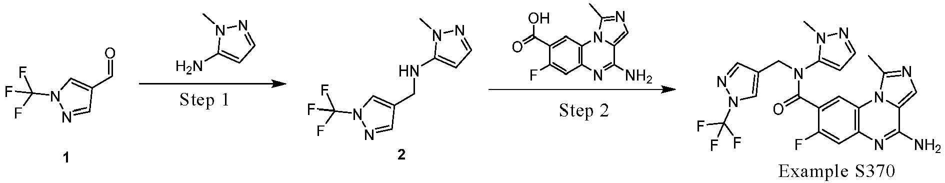

1-Methylpyrazol-5-amine (133 mg, 1.37 mmol, 1.5 equivalents) and 1-trifluoromethylpyrazol-4-carboxaldehyde (1) (150 mg, 914.16 µmol, 1 equivalent) were dissolved in methanol (5 mL), and acetic acid (164 mg, 2.74 mmol, 156.99 µL, 3 equivalents) was added. The reaction solution was stirred at room temperature for half an hour. Sodium cyanoborohydride (172.34 mg, 2.74 mmol, 3 equivalents) was added and the mixture was stirred at room temperature for 15.5 hours. LCMS detection showed that the reaction was complete and the target product was generated. The reaction solution was concentrated to dryness under reduced pressure, added with sodium bicarbonate (10 mL), and extracted with ethyl acetate (10 mL * 3); the combined organic phases were dried over anhydrous sodium sulfate, filtered, concentrated to dryness under reduced pressure, and purified by column chromatography (ISCO) ^{®}; 12 g SepaFlash ^{®} silica gel column, 0-60% ethyl acetate: ethanol (3:1)/petroleum ether system, at a flow rate of 35 mL/min) to obtain 1-methyl-N-(1-trifluoromethyl) pyrazol-4-ylmethyl) -1H-pyrazol-5-amine (3) (220 mg, 897.21 µmol, 86.86% yield) as a colorless oil. LCMS: ES13683-1961-P1C, (ESI) m/z= 246.1 (M+1) +, RT=0.786 min

### Step 2: 4-Amino-7-fluoro-1-methyl-1-methylpyrazol-5-yl)-N-(1-trifluoromethyl-1H-pyrazol-4-yl) methyl) imidazolo [1,5-a] quinoxalin-8-carboxamide (Example 213)

4-Amino-7-fluoro-1-methylimidazolo [1,5-a] quinoxalin-8-carboxylic acid (63.6 mg, 244.69 µmol, 1 equivalent) and 1-methyl-N - (1-trifluoromethyl) pyrazol-4-ylmethyl) -1H-pyrazol-5-amine (3) (60 mg, 244.69 µmol, 1 equivalent) were dissolved in 1-methylpyrrolidin-2-one (1.5 mL), and N-ethyl-N-isopropyl-2-propylamine (158 mg, 1.22 mmol, 213.11 µL, 5 equivalents) and 2-chloro-1,3-dimethyl-4,5-dihydroimidazol-1-chloride (DMC) (62.0 mg, 367.04 µmol, 1.5 equivalents) were added. The reaction solution was reacted at 50 °C for 16 hours. LC-MS detection showed that the raw material was completely consumed and the target product was generated. The reaction solution was diluted with acetonitrile (1 mL) and water (1 mL), filtered, and the filtrate was purified by reverse phase preparative liquid chromatography (Boston Prime C18 column 150 * 30 mm * 5 µ m; mobile phase: [water (formic acid) - acetonitrile]; Gradient: 18% -38%, 10 minutes) to obtain 4-amino-7-fluoro-1-methyl-1-methylpyrazol-5-yl)-N-(1-trifluoromethyl-1H-pyrazol-4-yl) methyl) imidazolo [1,5-a] quinoxalin-8-carboxamide (Example 213) (50 mg, 100.53 µmol, 41.08% yield, 98% purity) as a white solid. LCMS: ES13683-1966-P1C, (ESI) m/z= 488.3 [M+1] +, RT=0.767 min HNMR: ES13683-1966-P1A, 1H NMR (400 MHz, DMSO-d6) Shift 8.39 (s, 1H), 8.17 (s, 0.2H), 7.84-8.07 (m, 2H), 7.80 (s, 1H), 7.52 (s, 2H), 7.23 (br s, 1H), 7.06 (br d, J=11.01 Hz, 1H), 6.06 (br s, 1H), 4.49-5.28 (m, 2H), 3.48-3.74 (m, 3H), 2.87 (s, 3H). FNMR: 19F NMR (376 MHz, DMSO-d6) Shift -59.21 (s, 3F), -118.10 (br s, 1F)

### Synthesis of Example 220

### Synthesis method:

### Step 1: 4-fluoro-2-methyl-N - [[6- (trifluoromethyl) imidazolo [1,2-a] pyridin-2-yl] methyl] pyrazol-3-amine (3)

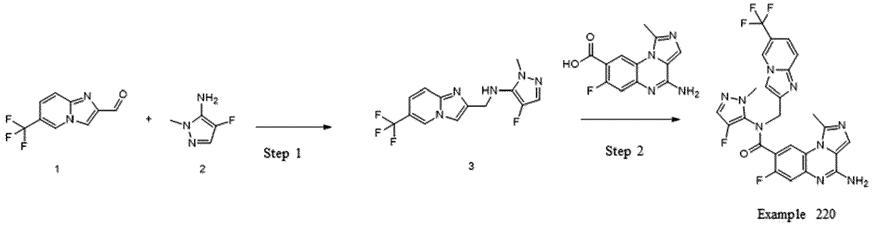

To a solution of 6- (trifluoromethyl) imidazolo [1,2-a] pyridin-2-carbaldehyde (1) (700 mg, 3.27 mmol, 1 equivalent) in methanol (14 mL) was added 4-fluoro-1-methyl-1H-pyrazol-5-amine (2) (402.61 mg, 3.50 mmol, 1.07 equivalents) and glacial acetic acid (255.18 mg, 4.25 mmol, 243.26 µL, 1.3 equivalents). The reaction was stirred at 25 °C for 1 hour. Sodium cyanoborohydride (616.24 mg, 9.81 mmol, 3 equivalents) was added and the mixture was stirred at 25 °C for 15 hours. LC-MS detected that the raw material was completely consumed and the target product was generated. The reaction solution was rotary concentrated to dryness, and added with 14 mL of 2 mol/L sodium carbonate aqueous solution and 14 mL of ethyl acetate. After partition, the aqueous phase was extracted with ethyl acetate(14 mL * 3), and the organic phase was dried over anhydrous magnesium sulfate, filtered, and the filtrate was concentrated under reduced pressure to dryness, and purified by column chromatography (12 g +4g silica gel column using a 0-30% ethyl acetate:ethanol 3:1/petroleum ether as eluent, at a flow rate of 30 mL/min), then concentrated under reduced pressure to obtain 4-fluoro-2-methyl-N - [[6- (trifluoromethyl) imidazolo [1,2-a] pyridin-2-yl] methyl] pyrazol-3-amine (3) (970 mg, 3.10 mmol, 94.73% yield) as a light yellow solid. LCMS: ES13685-1213-P1A, (ESI) m/z =314.0[M+1] +; RT= 1.537 min. NMR: ES13685-1213-R2A, 1H NMR (400 MHz, CHLOROFORM-d) 7.11 (d, J=4.38 Hz, 1H), 3.57 (s, 3H), 3.24 (br s, 2H). HNMR: ES13685-1213-R2A, 19F NMR (376 MHz, CHLOROFORM-d) -185.48 (s, 1F)

### Step 2: 4-Amino-7-fluoro-N-(4-fluoro-1-methyl-1H-pyrazol-5-yl) -1-methyl-N - ((6- (trifluoromethyl) imidazolo [1,2-a] pyridin) -2-yl) methyl) imidazolo [1,5-a] quinoxalin-8-carboxamide (Example 220)

To a reaction solution of 4-amino-7-fluoro-methyl-imidazolo [1,5-a] quinoxalin-8-carboxylic acid (500 mg, 1.92 mmol, 1 equivalent), 4-fluoro-2-methyl-N-[[6-(trifluoromethyl) imidazolo [1,2-a] pyridin-2-yl] methyl] pyrazol-3-amine (3) (601.89 mg, 1.92 mol, 1 equivalent), and N, N-diisopropylethylamine (993.30 mg, 7.69 mol, 1.34 mL, 4 equivalents) in 1-methyl-2-pyrrolidone (10 mL) was added 2-chloro-1,3-dimethyl-4,5-dihydroimidazol-1-chloride (487.23 mg, 2.88 mmol, 1.5 equivalents). The reaction was stirred at 50°C for 16 hours. LC-MS detection showed that 47% of the raw materials remained and 36% of the target product was generated. 2-Chloro-1,3-dimethyl-4,5-dihydroimidazol-1-chloride (74.71 mg, 441.93 µmol, 0.23 equivalents) was added. The reaction was stirred at 50°C for 16 hours. LC-MS detection showed that 49% of the raw materials remained and 38% of the target product was generated. A light yellow solid was obtained by reverse phase preparative liquid chromatography purification (Boston Prime C18 column, 5-µm silica with a diameter of 30 mm and a length of 150 mm, using a mixture of water (containing 0.05% formic acid) and acetonitrile (20% -40%) with a decreasing polarity as eluent). Partial formate was detected by nuclear magnetic resonance, and the crude product was purified by reverse phase preparative liquid chromatography (Boston Prime C18 column, 5 µm silica with a diameter of 30 mm, and a lenth of 150 mm, using a mixture of water (containing 0.05% ammonia) and acetonitrile (32% -52%) with a decreasing polarity as the eluent) to obtain 4-amino-7-fluoro-N-(4-fluoro-1-methyl-1H-pyrazol-5-yl) -1-methyl-N - ((6- (trifluoromethyl) imidazolo [1,2-a] pyridin) -2-yl) methyl) imidazolo [1,5-a] quinoxalin-8-carboxamide (Example 220) (155 mg, 276.26 µmol, 14.38% yield) as a white solid. LCMS: ES13685-1217-P1D1, (ESI) m/z= 556.1 [M+1] +; RT= 1.929minNMR: ES13685-1217-P1B, 1H NMR (400 MHz, DMSO-d6) 9.28 (br s, 1H), 8.10 (br s, 1H), 7.92 (br s, 1H), 7.82 (s, 1H), 7.73 (d, J=9.76 Hz, 1H), 7.55 (s, 2H), 7.47 (br d, J=8.63 Hz, 1H), 7.29 (br s, 1H), 7.11 (br d, J=10.38 Hz, 1H), 5.19 (br s, 1H), 5.04-5.14 (m, 1H), 3.59 (s, 3H), 2.86 (br s, 3H). NMR: ES13685-1217-P1B, 19F NMR (376 MHz, DMSO-d6) -60.47 (s, 3F), -118.15 (s, 1F), -173.54 (s, 1F)

### Synthesis of Example 287

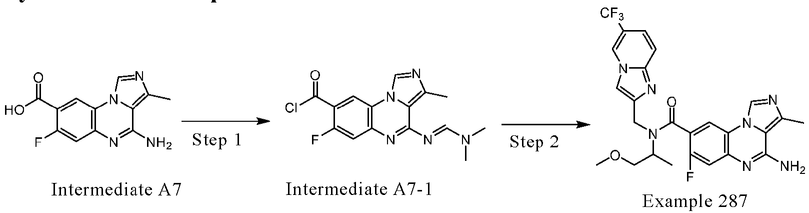

### Step 1: (E) -4- (((dimethylamino) methylene) amino) -7-fluoro-3-methylimidazolo [1,5-a] quinoxalin-8-carbonyl chloride (intermediate A7-1)

To a stirred solution of 4-amino-7-fluoro-3-methylimidazolo [1,5-a] quinoxalin-8-carboxylic acid (intermediate A7) (270mg, 1.04mmol, 1 equivalent) in DCM (3.00mL) was added HCl/dioxane (4M, 778uL, 3 equivalents). The mixture was stirred at 25°C for 0.5 hours. Then the reaction mixture was concentrated, evaporated and dried with toluene (10mL x 3), and the crude substance was dissolved in DCM (3.00mL) and cooled to 0 °C. Oxaloyl chloride (790mg, 6.23mmol, 544uL, 6 equivalents added dropwise at 0 °C) and DMF (75.8 mg, 1.04 mmol, 79.8uL, 1 equivalent) were added. The mixture was stirred at 25°C for 12 hours. LCMS indicated that the starting material has been consumed and a main peak with the desired mass has been detected. The reaction mixture was concentrated, steamed with n-hexane (10mL × 3) and dried under reduced pressure to obtain (E) -4-(((dimethylamino) methylene) amino) -7-fluoro-3-methylimidazolo [1,5-a] quinoxalin-8-carbonyl chloride (intermediate A7-1) as a yellow solid (340mg, crude). LC-MS (ESI) m/z = 330.0 [M+H]⁺

### Step 2: 4-Amino-7-fluoro-N-(1-methoxyprop-2-yl) -3-methyl-N-((6-(trifluoromethyl) imidazolo [1,2-a] pyridin-2-yl) methyl) imidazolo [1,5-a] quinoxalin-8-carboxamide(example 287)

To a solution of 1-methoxy-N - ((6- (trifluoromethyl) imidazolo [1,2-a] pyridin-2-yl) methyl) propan-2-amine (50.0 mg, 174 umol, 1 eq) and DIEA (89.9 mg, 696 umol, 121 uL, 4 eq) in THF (1.00 mL) was added Int.24_COC1 (63.9 mg, 191 umol, 1.1 eq) at 0 °C. The mixture was stirred at 25°C for 3 hours. The reaction mixture was quenched by adding MeOH (1.00 mL) at 25 °C and concentrated under reduced pressure to obtain a residue. The residue was dissolved in MeOH (1mL) and NH₃/MeOH (7M, 1mL). The mixture was stirred at 70°C for 2 hours. LCMS indicated that the starting material has been consumed and a main peak with the desired mass has been detected. The reaction mixture was concentrated under reduced pressure. The residue was purified by preparative HPLC (column: Waters Xbridge BEH C18 100 * 30 mm * 10 um; mobile phase: [water (NH4HCO3) - ACN]); B%: 30% -60%, 8 minutes, UV 220 nm & 254 nm) to obtain 4-amino-7-fluoro-N - (1-methoxyprop-2-yl) -3-methyl-N-((6- (trifluoromethyl) imidazolo [1,2-a] pyridin-2-yl) methyl) imidazolo [1,5-a] quinoxalin-8-carboxamide(example 287) as a yellow solid (58.1 mg, 109 umol, 64.2% yield). ¹H NMR (400MHz, DMSO-*d*₆) δ 9.29-8.54 (m, 2H), 8.18-7.78 (m, 2H), 7.66 (br s, 1H), 7.38 (br d, *J* = 7.9 Hz, 1H), 7.13 (br s, 1H), 6.72 (br s, 2H), 4.95-4.39 (m, 2H), 4.22-3.82 (m, 1H), 3.52 (br s, 2H), 3.31-3.10 (m, 3H), 2.63 (s, 3H), 1.21 (br s, 3H) LC-MS (ESI) m/z = 530.2 [M+H]⁺

The following compounds are synthesized using general methods, and the product structures are shown in Tables 1 and 2. The characterization of product is as follows:

**Table 3**

| Exam ple No. | Characterization data | Exa mpl e No. | Characterization data |
|---|---|---|---|
| 1 | 1H NMR (400MHz, DMSO-d6) Shift = 8.89 - 8.73 (m, 3H), 8.34 (br s, 1H), 8.13 (br s, 2H), 7.72 - 7.49 (m, 4H), 7.40 (t, J=4.9 Hz, 1H), 7.19 (br s, 1H), 5.49 - 5.35 (m, 1H), 4.93 (d, J=17.1 Hz, 1H), 4.52 (br d, J=15.4 Hz, 1H), 1.63 (br d, J=7.0 Hz, 3H). | 2 | 1H NMR (400 MHz, MeOD) δ 9.06 (d, *J =* 4.8 Hz, 2H), 8.74 (s, 1H), 8.32 (s, 1H), 8.12 (s, 1H), 8.02 - 7.76 (m, 3H), 7.65 (s, 1H), 4.87 (s, 2H), 3.32 (d, *J =* 8.8 Hz, 2H), 2.44 - 2.20 (m, 1H), 2.00 (d, *J* = 8.0 Hz, 3H), 1.59 (s, 3H). LC-MS: Rt =0.995 min, (ESI) *m*/*z.* |
| | LC-MS: Rt = 2.600 min, (ESI) *m*/*z.* [M+H]⁺ 493.2; C₂₄H₁₉F₃N₈O | | [M+H]⁺ 507.1; C₂₅H₂₁F₃N₈O |
| 3 | 1H NMR (400MHz, DMSO-d6) Shift 8.90 - 8.70 (m, 3H), 8.33 - 8.01 (m, 2H), 7.63 - 7.27 (m, 4H), 6.73 (br s, 2H), 5.54 (br s, 1H), 4.87 (br d, J=15.3 Hz, 1H), 4.57 (br s, 1H), 2.93 - 2.62 (m, 3H), 2.57 (s, 3H), 1.59 (d, J=7.0 Hz, 3H). LC-MS: Rt =2.792 min, (ESI) *m*/*z.* [M+H]⁺ 521.2; | 4 | 1H NMR (400 MHz, DMSO-d6) δ ppm 8.84 (br s, 1 H) 8.79 (br d, J=4.63 Hz, 2 H) 8.28 (br s, 1 H) 8.13 (br d, J=6.75 Hz, 1 H) 7.80 - 8.01 (m, 1 H) 7.49 - 7.72 (m, 3 H) 7.29 - 7.47 (m, 1 H) 6.99 (br s, 2 H) 5.43 (br s, 1 H) 4.92 (br d, J=17.01 Hz, 1 H) 4.52 (br d, J=16.51 Hz, 1 H) 2.50 (br s, 3 H) 1.62 (br d, J=7.00 Hz, 3 H) LC-MS: Rt = 2.693 min , (ESI) *m*/*z* = 507.2 [M+H]+; |
| | C₂₆H₂₃F₃N₈O | | |
| | | | C₂₅H₂₁F₃N₈O |
| 5 | NT | 6 | 1H NMR (400MHz, DMSO-d6) Shift 8.85 (br s, 1H), 8.80 (d, J=4.8 Hz, 2H), 8.29 - 7.96 (m, 2H), 7.76 - 7.24 (m, 4H), 6.96 (br s, 2H), 5.74 - 5.30 (m, 1H), 5.03 - 4.79 (m, 1H), 4.54 (br d, J=16.8 Hz, 1H), 2.45 - 2.32 (m, 6H), 1.63 (d, J=7.0 Hz, 3H). LC-MS: Rt =3.277 min, (ESI) *m*/*z.* [M+H]⁺ 521.2; |
| | | | C₂₆H₂₃F₃N₈O |
| 9 | 1H NMR (400 MHz, DMSO-d6) Shift 8.93 (br d, J=19.59 Hz, 1H), 8.67 (s, 1H), 8.49 (s, 1H), 8.15-8.27 (m, 2H), 7.87 (d, J=12.98 Hz, 1H), 7.57-7.68 (m, 3H), 5.02 (s, 1H), 4.88 (s, 1H), 3.63 (br d, J=7.26 Hz, 1H), 3.51-3.56 (m, 1H), 2.97 (s, 2H), 2.88 (s, 1H), 1.18 (td, J=6.99, 14.20 Hz, 3H). LC-MS: Rt =3.430 min, (ESI) *m*/*z.* [M+H]⁺ 430.1; | 10 | NT |
| | C₂₀H₁₈F₃N₇O | | |
| 11 | 1H NMR (400 MHz, DMSO-d6) Shift 10.76 (br s, 1H), 8.78 (br t, J=4.84 Hz, 3H), 8.10 (br s, 1H), 7.45-7.68 (m, 1H), 7.39 (br s, 1H), 7.03 (br d, J=14.53 Hz, 1H), 6.89 (br s, 2H), 5.32-5.71 (m, 1H), 4.79-4.91 (m, 1H), 4.53 (br d, J=16.29 Hz, 1H), 4.16 (br s, 1H), 3.96 (br s, 1H), 3.37-3.71 (m, 4H), 2.59-2.88 (m, 1H), 1.52-1.61 (m, 3H). LC-MS: Rt =1.669 min, (ESI) *m*/*z.* [M+H]⁺ 513.2; | 12 | 1H NMR (400 MHz, CD₃OD) Shift 7.91 (s, 1H), 7.70 (d, J=8.76 Hz, 1H), 7.53 (d, J=8.76 Hz, 1H), 7.09 (t, JH-F = 9.76 Hz, 2H), 4.66-4.84 (m, 2H), 4.03 (s, 3H), 3.73-3.87 (m, 2H), 3.49-3.73 (m, 3H), 3.14-3.28 (m, 2H), 2.94 (s, 3H), 2.83-2.92 (m, 2H), 2.81 (s, 3H), 2.02-2.22 (m, 4H). ¹⁹F NMR (376 MHz, CD₃OD) Shift-145.43. LC-MS: Rt =3.170 min, (ESI) *m*/*z.* [M+H]⁺ 506.2; |
| | C₂₅H₂₃F₃N₆O₃ | | C₂₆H₂₂F₃N₇O |
| 13 | NT | 14 | NT |
| 15 | 1H NMR (400MHz, DMSO-d6) Shift 8.97 (br s, 1H), 8.20 (br d, J=6.9 Hz, 1H), 7.97 (br s, 1H), 7.61 (br d, J=8.1 Hz, 1H), 7.39 (br s, 2H), 6.72 (br s, 2H), 4.82 (br s, 2H), 3.45 (q, J=6.9 Hz, 2H), 3.10 - 2.60 (m, 3H), 2.56 (br s, 3H), 1.14 (t, J=7.0 Hz, 3H). LC-MS: Rt =2.623 min, (ESI) *m*/*z.* [M+H]⁺ 443.1; | 16 | 1H NMR (400 MHz, DMSO-d6) Shift 8.85 (s, 1H), 8.77 (d, J=4.88 Hz, 2H), 8.68 (s, 1H), 8.43 (s, 1H), 8.27 (s, 1H), 8.17 (br d, J=8.50 Hz, 1H), 8.12 (s, 1H), 8.03 (br d, J=8.88 Hz, 1H), 7.88 (s, 1H), 7.85 (s, 1H), 7.53-7.71 (m, 3H), 7.35-7.42 (m, 1H), 5.91-5.99 (m, 1H), 5.81 (br d, J=7.25 Hz, 1H), 5.33 (br d, J=17.76 Hz, 1H), 5.26-5.39 (m, 1H), 4.97-5.09 (m, 1H), 4.51 (br d, J=17.01 Hz, 1H), 3.37-3.45 (m, 1H), 3.28 (br s, 1H), 2.93 (s, 2H), 2.88 (s, 1H), 2.53-2.58 (m, 1H), 1.67 (d, J=6.88 Hz, 2H), 1.60 (br d, J=7.13 Hz, 1H), 1.24 (s, 1H). LC-MS: RT =1.542 min, (ESI) *m*/*z.* [M+H]⁺ 508.1; |
| | C₂₂H₂₁F₃N₆O | | |
| | | | C₂₄H₂₀F₃N₉O |
| 17 | 1H NMR (400 MHz, DMSO-d6) Shift 8.66 (d, J=6.88 Hz, 1H), 8.12 (s, 1H), 7.78 (s, 1H), 7.66 (d, J=8.88 Hz, 1H), 7.47 (s, 2H), 7.30 (br s, 2H), 7.17-7.25 (m, 1H), 6.89 (br t, J=6.69 Hz, 1H), 6.59 (s, 1H), 4.77 (br s, 2H), 3.41-3.57 (m, 2H), 2.54-3.11 (m, 3H), 1.17 (t, J=7.00 Hz, 3H). LC-MS: Rt =1.262 min, (ESI) *m*/*z.* [M+H]⁺ 400.1; C₂₂H₂₁N₇O | 18 | 1H NMR (400 MHz, DMSO-d6) Shift 8.95 (br s, 1H), 8.09-8.36 (m, 1H), 7.72-8.08 (m, 2H), 7.34-7.67 (m, 3H), 7.02-7.33 (m, 1H), 4.52-5.03 (m, 2H), 3.50 (br s, 2H), 2.95 (s, 2H), 2.65 (s, 1H), 0.95-1.20 (m, 3H) |
| | | | 19F NMR (376 MHz, DMSO-d6) Shift -60.76 (br d, J=50.35 Hz, 3F), -119.58 (br d, J=123.60 Hz, 1F). LC-MS: Rt =1.653 min, (ESI) *m*/*z.* [M+H]⁺ 447.2; |
| | | | C₂₁H₁₈F₄N₆O |
| 19 | 1H NMR (400MHz, DMSO-d6) Shift 8.97 (br s, 1H), 8.21 (br d, J=7.9 Hz, 1H), 8.11 (br s, 1H), 7.73 - 7.27 (m, 3H), 6.93 (br s, 2H), 4.84 (br s, 2H), 3.46 (br s, 2H), 2.46 - 2.17 (m, 6H), 1.14 (br s, 3H) . LC-MS: Rt =3.237 min, (ESI) *m*/*z.* [M+H]⁺ 443.2; C₂₂H₂₁F₃N₆O | 20 | 1H NMR (400 MHz, D₂O) Shift 7.56-7.65 (m, 4H), 7.47-7.55 (m, 2H), 7.30 (dd, *J*=2.76, 10.54 Hz, 1H), 7.10-7.16 (m, 1H), 4.45-4.58 (m, 2H), 3.95-4.04 (m, 1H), 3.82-3.94 (m, 3H), 3.64-3.70 (m, 2H), 3.48-3.59 (m, 2H), 3.30-3.40 (m, 1H), 2.84 (s, 3H), 2.78 (s, 3H), 2.34-2.47 (m, 1H), 2.17-2.31 (m, 3H), 2.07-2.17 (m, 1H), 1.82-1.99 (m, 1H). LC-MS: Rt =3.077 min, (ESI) *m*/*z.* [M+H]⁺ 428.2; |
| | | | C₂₂H₂₀F₃N₅O |
| 21 | 1H NMR (400MHz, DMSO-d6) Shift 9.26 (s, 1H), 8.53 (d, J=5.3 Hz, 1H), 8.20 (d, J=5.3 Hz, 1H), 8.07 (br s, 1H), 7.81 (br s, 1H), 7.47 (br s, 1H), 7.44 - 7.39 (m, 1H), 7.34 (br s, 2H), 5.13 (s, 2H), 3.41 (br d, J=7.3 Hz, 2H), 3.33 (br s, 1H), 2.92 (br s, 2H), 2.13 - 1.91 (m, 1H), 0.76 (br s, 6H). LC-MS: Rt =0.723 min, (ESI) *m*/*z.* [M+H]⁺ 446.3; | 22 | NT |
| | C₂₃H₂₃N₇OS | | |
| 23 | 1H NMR (400MHz, DMSO-d6) Shift 9.10 - 8.60 (m, 3H), 8.52 - 8.27 (m, 1H), 8.13 (s, 1H), 8.05 - 7.88 (m, 2H), 7.65 (br s, 2H), 7.52 (br s, 1H), 5.10 - 4.72 (m, 2H), 3.45 - 3.18 (m, 2H), 3.14 - 2.84 (m, 3H), 2.68 (s, 3H), 2.16 - 1.88 (m, 1H), 1.08 - 0.62 (m, 6H). LC-MS: Rt =1.623 min, (ESI) *m*/*z.* [M+H]⁺ 442.2; | 24 | 1H NMR (400 MHz, DMSO-d6) Shift 8.50 (br d, J=6.60 Hz, 2H), 7.91 (br s, 1H), 7.79 (s, 1H), 7.53 (d, J=8.80 Hz, 1H), 7.41 (s, 2H), 7.23-7.30 (m, 2H), 6.89 (t, J=6.71 Hz, 1H), 5.03 (br d, J=6.82 Hz, 1H), 3.45-3.53 (m, 2H), 2.93 (s, 3H), 1.58 (br d, J=6.82 Hz, 3H), 0.72-1.15 (m, 3H). LC-MS: Rt =3.743 min, (ESI) *m*/*z.* [M+H]⁺ 432.3; |
| | C₂₅H₂₇N₇O | | |
| 25 | 1H NMR (400MHz, DMSO-d6) Shift 9.10 - 8.60 (m, 3H), 8.52 - 8.27 (m, 1H), 8.13 (s, 1H), 8.05 - 7.88 (m, 2H), 7.65 (br s, 2H), 7.52 (br s, 1H), 5.10 - 4.72 (m, 2H), 3.45 - 3.18 (m, 2H), 3.14 - 2.84 (m, 3H), 2.68 (s, 3H), 2.16 - 1.88 (m, 1H), 1.08 - 0.62 (m, 6H). LC-MS: Rt =1.623 min, (ESI) *m*/*z.* [M+H]⁺ | 26 | 1H NMR (400 MHz, DMSO-d6) Shift 8.45-8.60 (m, 1H), 7.82-8.31 (m, 2H), 7.42-7.82 (m, 5H), 7.13-7.31 (m, 1H), 6.83-6.92 (m, 1H), 4.30-5.09 (m, 2H), 3.11-3.33 (m, 2H), 2.55 (br s, 3H), 1.87-2.23 (m, 1H), 0.59-1.04 (m, 6H). LC-MS: Rt =1.568 min, (ESI) *m*/*z.* [M+H]⁺ 429.2; |
| | 442.2; | | C₂₃H₂₄N₈O |
| | C₂₅H₂₇N₇O | | |
| 27 | 1H NMR (400 MHz, DMSO-d6) Shift 8.42-8.65 (m, 1H), 7.93-8.31 (m, 1H), 7.85-7.93 (m, 1H), 7.69-7.85 (m, 1H), 7.35-7.62 (m, 3H), 7.11-7.34 (m, 2H), 6.88 (t, J=6.71 Hz, 1H), 4.50-4.93 (m, 2H), 3.44-3.68 (m, 4H), 3.10-3.32 (m, 3H), 2.73-2.97 (m, 3H). 19F NMR (376.5 MHz, DMSO-d6) Shift - 119.21, -119.78. LC-MS: Rt =1.289 min, (ESI) *m*/*z.* [M+H]⁺ 448.3; | 28 | 1H NMR (400 MHz, DMSO-d6) Shift 9.00 (s, 1H), 8.49 (s, 1H), 8.01-8.11 (m, 1H), 7.39-7.52 (m, 3H), 7.28-7.39 (m, 2H), 6.76 (br s, 2H), 5.49-6.49 (m, 1H), 4.61-4.80 (m, 2H), 2.87-2.95 (m, 3H), 2.64-2.70 (m, 3H), 2.58 (s, 3H). 19F NMR (376 MHz, DMSO-d6) Shift -59.29 (s, 3F). LC-MS: Rt =1.840 min, (ESI) *m*/*z.* [M+H]⁺ 522.2; |
| | C₂₃H₂₂FN₇O₂ | | C₂₆H₂₂F₃N₇O₂ |
| 29 | 1H NMR (400 MHz, DMSO-d6) Shift 8.97 (s, 1H), 8.44-8.55 (m, 2H), 7.96 (s, 1H), 7.51-7.60 (m, 2H), 7.38 (d, J=8.14 Hz, 1H), 7.16-7.26 (m, 1H), 6.88 (t, J=6.27 Hz, 1H), 6.82 (br s, 2H), 5.01-5.39 (m, 1H), 4.53-4.71 (m, 1H), 4.11-4.48 (m, 1H), 3.84 (dd, J=3.85, 11.77 Hz, 1H), 3.62-3.77 (m, 2H), 2.61 (s, 3H), 0.94 (d, J=7.04 Hz, 3H). LC-MS: Rt =2.003 min, (ESI) *m*/*z.* | 30 | 1H NMR (400 MHz, DMSO-d6) Shift 8.41 - 8.63 (m, 1 H),7.92 - 8.15 (m, 1 H), 7.71 - 7.91 (m, 2 H),7.33 - 7.56 (m, 3 H), 7.14 - 7.27 (m, 2 H), 6.88 (t, J=6.75 Hz, 1 H), 4.34 - 5.02 (m, 2 H), 2.82 - 3.18 (m, 2 H), 2.52 - 2.81 (m, 2 H), 1.93 - 2.26 (m, 3 H), 1.56 - 1.87 (m, 3 H). LC-MS: Rt =1.462 min, (ESI) *m*/*z.* [M+H]⁺ 456.2; |
| | [M+H]⁺ 442.2; | | C₂₅H₂₂FN₇O |
| | C₂₄H₂₃N₇O₂ | | |
| 31 | 1H NMR (400 MHz, DMSO-d6) Shift 9.10-9.39 (m, 1H), 9.00 (s, 1H), 8.16-8.74 (m, 1H), 8.06 (s, 1H), 7.80 (d, J=7.53 Hz, 1H), 7.48 (d, J=9.54 Hz, 1H), 6.74-7.36 (m, 3H), 4.16-5.89 (m, 2H), 3.49-3.96 (m, 4H), 2.55-2.68 (m, 3H), 0.92 (d, J=6.53 Hz, 3H). 19F NMR (376.5 MHz, DMSO-d6) Shift -60.43, - 118.60, -119.72. LC-MS: Rt =1.583 min, (ESI) *m*/*z.* [M+H]⁺ 528.2; | 32 | 1H NMR (400 MHz, DMSO-d6) Shift 8.47-8.58 (m, 1H), 7.85-8.46 (m, 2H), 7.75-7.85 (s, 1H), 7.50-7.58 (m, 1H), 7.43 (br s, 2H), 7.20-7.32 (m, 2H), 6.89 (t, J=6.71 Hz, 1H), 4.85-5.92 (m, 1H), 3.39-3.62 (m, 3H), 3.20 (s, 3H), 3.05-3.28 (m, 1H) 2.92 (s, 3H), 1.50-1.75 (m, 3H). 19F NMR (376.5 MHz, DMSO-d6) Shift -118.53, - 119.65, -120.69. LC-MS: Rt =1.383 min, (ESI) *m*/*z.* [M+H]⁺ |
| | C₂₅H₂₁F₄N₇O₂ | | 462.3; |
| | | | C₂₄H₂₄FN₇O₂ |
| 33 | 1H NMR (400 MHz, DMSO-d6) Shift 8.22-8.61 (m, 2H), 7.87-8.07 (m, 1H), 7.78 (s, 1H), 7.47-7.55 (m, 1H), 7.41 (br s, 2H), 7.11-7.30 (m, 2H), 6.88 (t, J=6.65 Hz, 1H), 4.12-5.23 (m, 2H), 3.51-3.92 (m, 3H), 2.92 (s, 3H), 0.88 (d, J=6.78 Hz, 3H). 19F NMR (376.5 MHz, DMSO-d6) Shift -119.26, -120.20. LC-MS: Rt =2.304 min, (ESI) *m*/*z.* [M+H]⁺ 460.2; | 34 | 1H NMR (400 MHz, DMSO-d6) Shift 8.89 (s, 1H), 8.30 (d, J=9.26 Hz, 1H), 7.94 (s, 1H), 7.82 (s, 1H), 7.48 (br s, 2H), 7.23 (d, J=10.13 Hz, 1H), 5.75 (br s, 1H), 4.78 (d, J=10.88 Hz, 1H), 3.55-4.13 (m, 4H), 2.90 (s, 3H), 0.91 (d, J=5.63 Hz, 3H). 19F NMR (376.5 MHz, DMSO-d6) Shift -60.49, -119.37, -120.53. LC-MS: Rt =1.701 min, (ESI) *m*/*z.* [M+H]⁺ 507.3; |
| | C₂₄H₂₂FN₇O₂ | | C₂₃H₁₉F₅N₆O₂ |
| 35 | 1H NMR (400 MHz, DMSO-d6) Shift 9.17-9.31 (m, 1H), 7.94-8.30 (m, 2H), 7.77-7.87 (m, 1H), 7.69-7.76 (m, 1H), 7.38-7.56 (m, 3H), 7.23 (d, J=11.00 Hz, 1H), 4.54-4.89 (m, 2H), 2.78-2.98 (m, 3H). 19F NMR (376.5 MHz, DMSO-d6) Shift -60.38, -60.44, -118.63, - 119.29. LC-MS: Rt =1.503 min, (ESI) *m*/*z.* [M+H]⁺ 475.2; | 36 | 1H NMR (400 MHz, DMSO-d6) Shift 8.86-9.15 (m, 1H), 8.17-8.80 (m, 2H), 7.92 (br s, 1H), 7.57 (d, J=7.78 Hz, 1H), 6.72-7.34 (m, 5H), 5.41-5.85 (m, 0.2H), 4.28-5.16 (m, 1.8H), 3.53-3.92 (m, 4H), 2.60 (s, 3H), 0.94 (d, J=6.53 Hz, 3H). 19F NMR (376.5 MHz, DMSO-d6) Shift -119.70, - 120.05. LC-MS: Rt =2.166 min, (ESI) *m*/*z.* [M+H]⁺ 460.2; |
| | C₂₂H₁₄D₃F₄N₇O | | C₂₄H₂₂FN₇O₂ |
| 37 | 1H NMR (400 MHz, DMSO-d6) Shift 8.40-8.64 (m, 1H), 7.95-8.32 (m, 1H), 7.69-7.94 (m, 2H), 7.40-7.61 (m, 3H), 7.15-7.36 (m, 2H), 6.89 (t, J=6.60 Hz, 1H), 6.11-6.50 (m, 1H), 4.53-5.04 (m, 2H), 3.63-4.10 (m, 2H), 2.72-2.98 (m, 3H). 19F NMR (376.5 MHz, DMSO-d6) Shift -119.26, -119.63, -120.56, - 121.90. LC-MS: Rt =1.400 min, (ESI) *m*/*z.* [M+H]⁺ 452.2; | 38 | 1H NMR (400 MHz, DMSO-d6) Shift 8.22-8.61 (m, 2H), 7.87-8.07 (m, 1H), 7.78 (s, 1H), 7.47-7.55 (m, 1H), 7.41 (br s, 2H), 7.11-7.30 (m, 2H), 6.88 (t, J=6.65 Hz, 1H), 4.12-5.23 (m, 2H), 3.51-3.92 (m, 3H), 2.92 (s, 3H), 0.88 (d, J=6.78 Hz, 3H). 19F NMR (376.5 MHz, DMSO-d6) Shift -119.26, - 120.20. LC-MS: Rt =2.304 min, (ESI) *m*/*z.* [M+H]⁺ 460.2; |
| | C₂₂H₁₈F₃N₇O | | C₂₄H₂₂FN₇O₂ |
| 39 | 1H NMR (400 MHz, DMSO-d6) Shift 8.34-8.54 (m, 1H), 7.94-8.11 (m, 1H), 7.71-7.94 (m, 2H), 7.32-7.68 (m, 3H), 7.10-7.30 (m, 2H), 6.77-6.93 (m, 1H), 3.78-4.09 (m, 0.7H), 3.49-3.74 (m, 4H), 3.02-3.08 (m, 0.3H), 2.85-2.99 (m, 3H), 2.04-2.42 (m, 2H). 19F NMR (376.5 MHz, DMSO-d6) Shift -118.69, - 118.71. LC-MS: Rt =1.321 min, (ESI) *m*/*z.* [M+H]⁺ 430.2; | 40 | 1H NMR (400 MHz, DMSO-d6) Shift 7.96-8.18 (m, 1H), 7.83 (s, 1H), 7.48 (s, 2H), 7.05-7.38 (m, 4H), 5.42-6.43 (m, 1H), 4.54-4.85 (m, 2H), 2.95 (br d, J=9.76 Hz, 3H), 2.55-2.68 (m, 3H). 19F NMR (376 MHz, DMSO-d6) Shift - 119.37 (br s, 1F). LC-MS: Rt =1.813 min, (ESI) *m*/*z.* [M+H]⁺ 470.1; |
| | C₂₃H₂₀FN₇O | | C₂₁H₁₇BrFN₅O₂ |
| 41 | 1H NMR (400 MHz, DMSO-d6) Shift 8.41-8.58 (m, 1H), 8.21 (s, 0.21H, HCOOH), 7.91-8.15 (m, 1H), 7.65-7.90 (m, 2H), 7.33-7.59 (m, 3H), 7.13-7.32 (m, 2H), 6.87 (t, J=6.53 Hz, 1H), 4.57-4.98 (m, 2H), 4.05-4.57 (m, 1H), 3.71-4.01 (m, 1H), 3.44-3.52 (m, 3H), 2.91-3.16 (m, 3.9H), 2.58-2.65 (m, 0.6H), 1.91-2.03 (m, 2.5H). 19F NMR (376.5 MHz, DMSO-d6) Shift - 119.18, -119.36, -119.98. LC-MS: Rt =1.349 min, (ESI) *m*/*z.* [M+H]⁺ 474.3; | 42 | 1H NMR (400 MHz, DMSO-d6) Shift 9.15-9.31 (m, 1H), 7.94-8.28 (m, 2H), 7.66-7.88 (m, 2H), 7.34-7.55 (m, 3H), 7.16-7.29 (m, 1H), 4.52-4.91 (m, 2H), 3.43-3.52 (m, 2H), 2.78-3.00 (m, 3H), 1.03-1.19 (m, 3H). 19F NMR (376.5 MHz, DMSO-d6) Shift -60.37, -60.44, - 119.21, -119.92. LC-MS: Rt =1.580 min, (ESI) *m*/*z.* [M+H]⁺ 486.3; |
| | | | C₂₃H₁₉F₄N₇O |
| | C₂₄H₂₃F₆N₇OS | | |
| 43 | 1H NMR (400 MHz, DMSO-d6) Shift 8.41-8.61 (m, 1H), 7.93-8.13 (m, 1H), 7.69-7.89 (m, 2H), 7.34-7.58 (m, 3H), 7.12-7.31 (m, 2H), 6.87 (t, J=6.71 Hz, 1H), 4.11-5.02 (m, 3H), 2.57-3.02 (m, 3H), 1.78-2.31 (m, 4H), 1.30-1.70 (m, 2H). 19F NMR (376.5 MHz, DMSO-d6) Shift -119.35, -119.94. LC-MS: Rt =1.452 min, (ESI) *m*/*z.* [M+H]⁺ 444.3; | 44 | 1H NMR (400 MHz, DMSO-d6) Shift 8.40-8.61 (m, 1H), 7.97-8.28 (m, 1H), 7.69-7.92 (m, 2H), 7.40-7.62 (m, 3H), 7.19-7.34 (m, 2H), 6.88 (t, J=6.49 Hz, 1H), 4.59-5.05 (m, 2H), 4.10-4.59 (m, 2H), 2.71-2.96 (m, 3H). 19F NMR (376.5 MHz, DMSO-d6) Shift -68.10, - 68.99, -119.46, -120.09. LC-MS: Rt =1.337 min, (ESI) *m*/*z.* [M+H]⁺ 444.3; |
| | C₂₄H₂₂FN₇O | | C₂₂H₁₇F₄N₇O |
| 45 | 1H NMR (400 MHz, DMSO-d6) Shift 9.13 - 9.28 (m, 1 H) 7.98 (br s, 2 H) 7.54 - 7.88 (m, 3 H) 7.36 - 7.53 (m, 3 H) 7.12 - 7.32 (m, 1 H) 7.02 (br d, J=10.88 Hz, 1 H) 4.90 - 5.20 (m, 2 H) 3.54 - 3.82 (m, 3 H) 2.73 - 2.94 (m, 3 H). 19F NMR (376 MHz, DMSO-d6) Shift -60.66 (s, 1 F) -119.61 - -117.01 (m, 1 F). LC-MS: Rt =1.496 min, (ESI) *m*/*z.* [M+H]⁺ 538.2; | 46 | 1H NMR (400 MHz, DMSO-d6) Shift 8.99 (br s, 1H), 8.47 (br s, 1H), 8.05 (br s, 1H), 7.83 (s, 1H), 7.48 (s, 2H), 7.19-7.43 (m, 4H), 5.43-6.46 (m, 1H), 4.50-4.91 (m, 2H), 2.97 (br d, J=13.20 Hz, 3H), 2.57 (br d, J=6.16 Hz, 3H). 19F NMR (376 MHz, DMSO-d6) Shift -59.30 (br s, 3F), -119.40 (br s, 1F). LC-MS: Rt =1.836 min, (ESI) *m*/*z.* [M+H]⁺ 526.2; |
| | C₂₅H₁₉F₄N₉O | | C₂₅H₁₉F₄N₇O₂ |
| 47 | 1H NMR (400 MHz, DMSO-d6) Shift 9.12-9.33 (m, 1H), 8.05-8.17 (m, 1H), 7.90-8.02 (m, 1H), 7.67-7.88 (m, 2H), 7.36-7.49 (m, 3H), 7.23 (d, J=10.78 Hz, 1H), 4.47-4.96 (m, 2H), 3.40 (br s, 2H), 2.71-2.96 (m, 3H), 1.90-2.20 (m, 1H), 0.66-0.99 (m, 6H). 19F NMR (376 MHz, DMSO-d6) Shift -84.67--83.60 (m, 3F), -113.70--112.30 (m, 2F), - 120.55--118.62 (m, 1F). LC-MS: Rt =1.887 min, (ESI) *m*/*z.* [M+H]⁺ | 48 | 1H NMR (400 MHz, DMSO-d6) Shift 9.13 - 9.28 (m, 1 H) 7.98 (br s, 2 H) 7.54 - 7.88 (m, 3 H) 7.36 - 7.53 (m, 3 H) 7.12 - 7.32 (m, 1 H) 7.02 (br d, J=10.88 Hz, 1 H) 4.90 - 5.20 (m, 2 H) 3.54 - 3.82 (m, 3 H) 2.73 - 2.94 (m, 3 H). 19F NMR (376 MHz, DMSO-d6) Shift - 60.66 (s, 1 F) -119.61 - -117.01 (m, 1 F). LC-MS: Rt =1.496 min, (ESI) *m*/*z.* [M+H]⁺ 538.2; |
| | 564.3; | | C₂₅H₁₉F₄N₉O |
| | C₂₆H₂₃F₆N₇O | | |
| 51 | (400MHz, DMSO-d6) Shift 8.77 - 8.46 (m, 3H), 7.98 (s, 1H), 7.88 - 7.65 (m, 3H), 7.55 (br d, J=9.0 Hz, 1H), 7.28 (br s, 1H), 6.91 (br t, J=6.5 Hz, 1H), 4.63 (br s, 2H), 3.73 - 3.51 (m, 2H), 3.03 - 2.62 (m, 3H), 1.25 - 1.06 (m, 3H). LC-MS: Rt =2.973 min, (ESI) *m*/*z.* [M+H]⁺ 401.2; | 52 | 1H NMR (400 MHz, DMSO-d6) Shift 8.43-8.63 (m, 1H), 7.98-8.29 (m, 1H), 7.85-7.94 (m, 1H), 7.74-7.84 (m, 1H), 7.35-7.62 (m, 3H), 7.15-7.32 (m, 2H), 6.89 (t, J=6.65 Hz, 1H), 4.55-4.94 (m, 2H), 3.57-3.85 (m, 2H), 2.70-2.97 (m, 5H). 19F NMR (376.5 MHz, DMSO-d6) Shift -119.41. LC-MS: Rt =1.241 min, (ESI) *m*/*z.* [M+H]⁺ |
| | C₂₁H₂₀N₈O | | 443.2; |
| | | | C₂₃H₁₉FN₈O |
| 53 | 1H NMR (400MHz, DMSO-d6) Shift 8.37 - 7.75 (m, 2H), 8.39 - 7.73 (m, 1H), 7.58 - 7.39 (m, 3H), 7.33 - 7.19 (m, 2H), 7.07 - 6.93 (m, 1H), 6.95 - 6.42 (m, 1H), 4.90 - 4.47 (m, 2H), 3.27 - 3.03 (m, 1H), 3.33 - 3.03 (m, 1H), 2.89 (s, 3H), 2.23 - 1.95 (m, 1H), 1.00 - 0.66 (m, 6H). 19F NMR (376MHz, DMSO-d6) Shift -119.073, -120.192; 158.442, -159.220. LC-MS: Rt =1.97 min, (ESI) *m*/*z.* [M+H]⁺ 464.1; | 54 | 1H NMR (400 MHz, DMSO-d6) Shift 9.01 (s, 0.7H), 8.66 (s, 0.3H), 8.53 (d, J=6.60 Hz, 0.7H), 8.31 (d, J=6.60 Hz, 0.3H), 8.04-8.20 (m, 1H), 7.35-7.86 (m, 2H), 7.08-7.26 (m, 2H), 6.91-7.08 (m, 2H), 6.71-6.91 (m, 1H), 4.19-5.11 (m, 3H), 2.75-2.86 (m, 1H), 2.63-2.70 (m, 1H), 2.56-2.63 (m, 3H), 2.34-2.46 (m, 1H), 2.15-2.27 (m, 3H), 1.92-2.15 (m, 2H). 19F NMR (376.5 MHz, DMSO-d6) Shift - 118.93, -119.11, -119.89. LC-MS: Rt =1.824 min, (ESI) *m*/*z.* [M+H]⁺ 572.3; |
| | C₂₄H₂₃F₂N₇O | | |
| | | | C₂₄H₂₃F₆N₇OS |
| 55 | 1H NMR (400 MHz, DMSO-d6) Shift 7.95-8.03 (m, 1H), 7.80-7.84 (m, 1H), 7.40-7.52 (m, 4H), 7.18-7.24 (m, 1H), 7.04-7.10 (m, 1H), 6.97-7.03 (m, 1H), 3.56-3.65 (m, 1H), 3.41-3.47 (m, 4H), 3.21-3.31 (m, 2H), 3.21-3.30 (m, 1H), 2.88-2.97 (m, 3H), 1.76-1.92 (m, 2H), 1.59-1.74 (m, 2H), 1.52-1.59 (m, 2H). 19F NMR (376.5 MHz, DMSO-d6) Shift -59.31, -59.34, - 119.07, -119.10. | 56 | 1H NMR (400 MHz, CHLOROFORM-d) Shift 8.28-8.68 (m, 1H), 7.57-8.19 (m, 3H), 7.07-7.57 (m, 5H), 6.75-6.98 (m, 1H), 5.49-5.86 (m, 1H), 4.44-5.01 (m, 2H), 3.74-4.41 (m, 2H), 2.93 (s, 3H), 1.18-1.96 (m, 6H). 19F NMR (376.5 MHz, DMSO-d6) Shift -118.56, -120.12. LC-MS: Rt =1.365 min, (ESI) *m*/*z.* [M+H]⁺ 474.3; |
| | LC-MS: Rt =1.959 min, (ESI) *m*/*z.* [M+H]⁺ 549.3; | | C₂₅H₂₄FN₇O₂ |
| | C₂₇H₂₆F₄N₆O | | |
| 57 | 1H NMR (400 MHz, DMSO-d6) Shift 8.65 (d, J=6.16 Hz, 1H), 8.49 (br d, J=6.60 Hz, 1H), 8.30 (s, 1H), 7.84-7.93 (m, 2H), 7.58 (br d, J=17.83 Hz, 2H), 7.47-7.53 (m, 1H), 7.20-7.26 (m, 1H), 6.88 (q, J=7.41 Hz, 1H), 4.78 (br d, J=6.16 Hz, 2H), 3.46-3.62 (m, 2H), 2.88-3.00 (m, 3H), 1.15-1.25 (m, 3H) LC-MS: Rt =1.640 min, (ESI) *m*/*z.* [M+H]⁺ 401.2; | 58 | 1H NMR (400 MHz, DMSO-d6) Shift 8.43-8.60 (m, 1H), 7.93-8.23 (m, 1H), 7.84-7.93 (m, 1H), 7.75-7.84 (m, 1H), 7.35-7.56 (m, 4H), 7.18-7.29 (m, 1H), 6.82-6.92 (m, 1H), 3.84-5.07 (m, 2H), 3.01-3.32 (m, 2H), 2.78-2.96 (m, 3H), 1.00-1.23 (m, 3H). LC-MS: Rt =1.390 min, (ESI) *m*/*z.* [M+H]⁺ 434.2; |
| | | | C₂₂H₂₀ClN₇O |
| | C₂₁H₂₀N₈O | | |
| 59 | 1H NMR (400MHz, DMSO-d6) Shift 9.18 (s, 1H), 8.47 (br s, 1H), 8.33 - 8.14 (m, 1H), 8.08 (s, 1H), 7.87 - 7.68 (m, 5H), 7.47 (br d, J=8.4 Hz, 1H), 5.09 (s, 2H), 3.68 (br s, 3H), 2.71 (s, 3H). 19F NMR (376MHz, DMSO-d6) Shift - 60.427. LC-MS: Rt =3.535 min, (ESI) *m*/*z.* [M+H]⁺ 521.3; | 60 | 1H NMR (400MHz, DMSO-d6) Shift 8.37 - 7.75 (m, 2H), 8.39 - 7.73 (m, 1H), 7.58 - 7.39 (m, 3H), 7.33 - 7.19 (m, 2H), 7.07 - 6.93 (m, 1H), 6.95 - 6.42 (m, 1H), 4.90 - 4.47 (m, 2H), 3.27 - 3.03 (m, 1H), 3.33 - 3.03 (m, 1H), 2.89 (s, 3H), 2.23 - 1.95 (m, 1H), 1.00 - 0.66 (m, 6H). 19F NMR (376MHz, DMSO-d6) Shift - 119.073, -120.192; 158.442, - 159.220. LC-MS: Rt =1.97 min, (ESI) *m*/*z.* [M+H]⁺ 464.1; |
| | C₂₄H₁₉F₃N₁₀O | | |
| | | | C₂₄H₂₃F₂N₇O |
| 61 | 1H NMR (400 MHz, DMSO-d6) Shift 8.82 (br d, J=5.72 Hz, 1H), 8.06 (br s, 1H), 7.97 (br d, J=5.72 Hz, 1H), 7.65-7.84 (m, 3H), 7.47 (br s, 2H), 7.25 (br s, 1H), 7.04 (br d, J=5.94 Hz, 2H), 5.10 (br s, 2H), 3.60 (br s, 3H), 2.78-3.06 (m, 3H). LC-MS: Rt =1.453 min, (ESI) *m*/*z.* [M+H]⁺ 538.2; | 62 | 1H NMR (400 MHz, DMSO-d6) Shift 8.99 (d, J=6.38 Hz, 1H), 8.13-8.31 (m, 1H), 7.31-7.55 (m, 2H), 7.15 (dd, J=5.72, 11.00 Hz, 1H), 6.90-7.11 (m, 4H), 3.48-3.77 (m, 3H), 3.12-3.37 (m, 5H), 2.61 (m, 3H), 1.76-1.93 (m, 2H), 1.59-1.72 (m, 2H), 1.50-1.58 (m, 2H) 19F NMR (376.5 MHz, DMSO-d6) Shift -59.35, -118.93, -119.08. LC-MS: Rt =1.988 min, (ESI) *m*/*z.* [M+H]⁺ 527.3; |
| | C₂₅H₁₉F₄N₉O | | |
| | | | C₂₇H₂₆F₄N₆O |
| 63 | 1H NMR (400 MHz, DMSO-d6) Shift 9.33 (br s, 1H), 7.88-8.42 (m, 2H), 7.55-7.85 (m, 3H), 7.37-7.55 (m, 3H), 6.91-7.36 (m, 2H), 4.92-5.14 (m, 2H), 3.48-3.85 (m, 3H), 2.74-2.93 (m, 3H). 19F NMR (376 MHz, DMSO-d6) Shift -120.51-115.64 (m, 1F). LC-MS: Rt =1.204 min, (ESI) *m*/*z.* [M+H]⁺ 495.3; | 64 | 1H NMR (400 MHz, DMSO-d6) Shift 7.95-8.11 (m, 1H), 7.82 (d, J=5.52 Hz, 1H), 7.34-7.58 (m, 4H), 7.17-7.26 (m, 1H), 6.38-6.63 (m, 2H), 3.67-4.03 (m, 5H), 3.42-3.65 (m, 3H), 2.94 (s, 3H), 2.09-2.28 (m, 2H). 19F NMR (376.5 MHz, DMSO-d6) Shift -59.03, - 59.05, -118.69, -118.86. LC-MS: Rt =1.905 min, (ESI) *m*/*z.* [M+H]⁺ |
| | C₂₅H₁₉FN₁₀O | | 499.3; |
| | | | C₂₅H₂₂F₄N₆O |
| 65 | 1H NMR (400 MHz, DMSO-d6) Shift 8.33-8.61 (m, 1H), 8.20 (s, 0.6H, HCOOH), 7.63-8.05 (m, 3H), 7.33-7.61 (m, 3H), 7.09-7.30 (m, 2H), 6.72-6.96 (m, 1H), 4.34-5.03 (m, 3H), 2.63-3.02 (m, 6H), 1.92-2.35 (m, 6H). 19F NMR (376.5 MHz, DMSO-d6) Shift -119.22, - 122.68. LC-MS: Rt =1.344 min, (ESI) *m*/*z.* [M+H]⁺ 473.3; | 66 | 1H NMR (400 MHz, DMSO-d6) Shift 9.23 (br s, 1H), 8.66 (br s, 1H), 8.45 (br s, 1H), 7.97-8.15 (m, 2H), 7.84-7.88 (m, 1H), 7.70 (br d, J=14.30 Hz, 2H), 7.57 (br s, 2H), 7.45 (br s, 1H), 7.11 (br s, 1H), 5.05-5.30 (m, 2H), 3.56-3.88 (m, 3H), 2.91 (s, 3H). LC-MS: Rt =1.484 min, (ESI) *m*/*z.* [M+H]⁺ 521.3; |
| | C₂₅H₂₅FN₈O | | C₂₄H₁₉F₃N₁₀O |
| 67 | 1H NMR (400 MHz, DMSO-d6) Shift 8.41 - 8.56 (m, 2 H) 8.21 - 8.32 (m, 1 H) 7.99 - 8.14 (m, 1 H) 7.87 - 7.95 (m, 1 H) 7.76 - 7.86 (m, 2 H) 7.44 - 7.56 (m, 3 H) 7.16 (s, 2 H) 6.81 - 7.03 (m, 2 H) 5.15 - 5.26 (m, 2 H) 2.88 (s, 3 H). 19F NMR (376 MHz, DMSO-d6) Shift - 117.24 - -115.16 (m, 1 F). LC-MS: Rt =1.271 min, (ESI) *m*/*z.* [M+H]⁺ 467.2; | 68 | 1H NMR (400 MHz, DMSO-d6) Shift 8.02-8.43 (m, 2H), 7.62-8.02 (m, 3H), 7.19-7.62 (m, 4H), 6.83-7.19 (m, 2H), 4.75-5.19 (m, 1H), 4.75-5.19 (m, 1H), 3.51-3.83 (m, 6H), 2.68-2.97 (m, 3H) LC-MS: Rt =1.319 min, (ESI) *m*/*z.* [M+H]⁺ 500.3 |
| | | | C₂₅H₂₂FN₉O₂ |
| | C₂₅H₁₉FN₈O | | |
| 69 | 1H NMR (400MHz, DMSO-d6) Shift 8.53 - 8.36 (m, 1H), 8.28 - 7.92 (m, 1H), 7.88 - 7.75 (m, 2H), 7.55 - 7.37 (m, 3H), 7.29 - 7.16 (m, 2H), 4.87 - 4.43 (m, 2H), 3.58 (br t, J=4.2 Hz, 4H), 3.52 - 3.36 (m, 4H), 3.00 - 2.76 (m, 3H), 2.45 - 2.29 (m, 4H), 1.19 - 1.05 (m, 3H). 19F NMR (376MHz, DMSO-d6) Shift - 119.304, -120.046. LC-MS: Rt =3.633 min, (ESI) *m*/*z.* [M+H]⁺ 517.3 | 70 | NT |
| | C₂₇H₂₉FN₈O₂ | | |
| 71 | 1H NMR (400 MHz, DMSO-d6) Shift 8.55-8.69 (m, 1H), 8.17 (br s, 1H), 7.79 (s, 1H), 7.64 (d, J=8.80 Hz, 1H), 7.44 (br s, 2H), 7.10-7.31 (m, 2H), 6.86 (t, J=6.60 Hz, 1H)124, 6.61 (br s, 1H), 4.61-5.85 (m, 2H), 3.50-4.04 (m, 3H), 3.28-3.36 (m, 1H), 2.68-3.06 (m, 3H), 0.80-1.01 (m, 3H). 19F NMR (376.5 MHz, DMSO-d6) Shift - 119.63 | 72 | 1H NMR (400 MHz, DMSO-d6) Shift 8.31-8.45 (m, 1H), 7.92-8.17 (m, 1H), 7.72-7.85 (m, 2H), 7.58-7.69 (m, 1H), 7.44 (br s, 3H), 7.14-7.32 (m, 2H), 7.02 (br d, J=11.13 Hz, 1H), 4.83-5.08 (m, 2H), 3.53-3.86 (m, 3H), 3.39-3.48 (m, 2H), 2.64-2.95 (m, 3H), 2.16 (br s, 6H). 19F NMR (376 MHz, DMSO-d6) Shift -120.02--116.80 (m, 1F).LC-MS: Rt =1.342 (ESI) *m*/*z.* [M+H]⁺ 527.4; |
| | LC-MS: Rt =1.479 min, (ESI) *m*/*z.* [M+H]⁺ 460.3; | | |
| | C₂₄H₂₂FN₇O₂ | | C₂₇H₂₇FN₁₀O |
| 73 | 1H NMR (400 MHz, DMSO-d6) Shift 9.16-9.45 (m, 1H), 8.38-8.63 (m, 2H), 7.71-8.04 (m, 3H), 7.38-7.53 (m, 2H), 7.18-7.31 (m, 1H), 4.85-5.12 (m, 2H), 2.54-2.93 (m, 5H), 1.08-1.25 (m, 3H) 19F NMR (376.5 MHz, DMSO-d6) Shift -66.23, -119.4, -119.5. LC-MS: Rt =1.625 min, (ESI) *m*/*z.* [M+H]⁺ 530.3; | 74 | 1H NMR (400 MHz, DMSO-d6) Shift 8.14-8.38 (m, 2H), 7.75-8.01 (m, 1H), 7.35-7.55 (m, 3H), 7.18-7.31 (m, 2H), 6.85 (br d, J=7.04 Hz, 1H), 5.53-5.67 (m, 1H), 4.45-4.94 (m, 2H), 3.44 (br s, 2H), 2.77-2.97 (m, 3H), 1.48-1.70 (m, 6H), 0.98-1.26 (m, 3H). 19F NMR (376 MHz, DMSO-d6) Shift - 121.18--118.30 (m, 1F). LC-MS: Rt =1.370 min, (ESI) *m*/*z.* [M+H]⁺ |
| | C₂₄H₁₉F₄N₇OS | | 476.3; |
| | | | C₂₅H₂₆FN₇O₂ |
| 75 | 1H NMR (400 MHz, DMSO-d6) Shift 9.00 (br s, 1H), 8.48 (br s, 1H), 8.02 (br s, 1H), 7.83 (s, 1H), 7.19-7.56 (m, 6H), 5.51 (br s, 1H), 4.62 (br s, 2H), 3.05-3.26 (m, 2H), 2.97 (br s, 3H), 0.97 (br s, 3H). LC-MS: Rt =1.752 min, (ESI) *m*/*z.* [M+H]⁺ 540.2; | 76 | 1H NMR (400 MHz, DMSO-d6) Shift 8.14-8.38 (m, 2H), 7.75-8.01 (m, 1H), 7.35-7.55 (m, 3H), 7.18-7.31 (m, 2H), 6.85 (br d, J=7.04 Hz, 1H), 5.53-5.67 (m, 1H), 4.45-4.94 (m, 2H), 3.44 (br s, 2H), 2.77-2.97 (m, 3H), 1.48-1.70 (m, 6H), 0.98-1.26 (m, 3H). 19F NMR (376 MHz, DMSO-d6) Shift - 121.18--118.30 (m, 1F). LC-MS: Rt =1.370 min, (ESI) *m*/*z.* [M+H]⁺ 476.3; |
| | C₂₆H₂₁F₄N₇O₂ | | |
| | | | C₂₅H₂₆FN₇O₂ |
| 77 | 1H NMR (400MHz, DMSO-d6) Shift 9.06 - 8.84 (m, 1H), 8.31 (d, J=6.6 Hz, 1H), 8.24 - 8.11 (m, 1H), 7.52 (br t, J=7.5 Hz, 1H), 7.33 - 6.89 (m, 5H), 4.94 - 4.45 (m, 2H), 3.60 (br s, 2H), 2.61 (d, J=2.4 Hz, 3H), 1.22 - 1.07 (m, 3H).19F NMR (376MHz, DMSO-d6) Shift - 119.164, -119.681; -157.688, - 158.649. C-MS: Rt =3.824 (ESI) *m*/*z.* [M+H]⁺ 436.3 | 78 | 1H NMR (400MHz, DMSO-d6) Shift 8.50 - 8.26 (m, 1H), 8.21 - 7.93 (m, 1H), 7.81 (br d, J=5.9 Hz, 2H), 7.63 (br s, 1H), 7.44 (s, 3H), 7.35 - 7.13 (m, 2H), 7.02 (br d, J=10.3 Hz, 1H), 5.12 - 4.86 (m, 2H), 3.86 - 3.46 (m, 9H), 2.96 - 2.69 (m, 3H), 2.44 - 2.30 (m, 4H). 19F NMR (376MHz, DMSO-d6) Shift -117.195, -119.505. LC-MS: Rt =1.673 min, (ESI) *m*/*z.* [M+H]⁺ 569.2; |
| | C₂₂H₁₉F₂N₇O | | C₂₉H₂₉FN₁₀O₂ |
| 79 | 1H NMR (400 MHz, DMSO-d6) Shift 9.25 - 9.27 (m, 1 H) 9.26 (s, 1 H) 9.14 - 9.19 (m, 1 H) 8.87 - 9.01 (m, 1 H) 8.06 (s, 1 H) 6.96 - 7.21 (m, 3 H) 4.62 - 4.95 (m, 2 H) 3.47 - 3.60 (m, 2 H) 2.58 - 2.63 (m, 3 H) 1.05 - 1.20 (m, 3 H). 19F NMR (376 MHz, DMSO-d6) Shift -65.75 - - 65.59 (m, 1 F) -119.33 - -119.19 (m, 1 F). LC-MS: Rt =1.520 min, (ESI) *m*/*z.* [M+H]⁺ 487.3; | 80 | 1H NMR (400 MHz, DMSO-d6) Shift 8.83-9.14 (m, 1H), 7.69-8.26 (m, 4H), 7.39-7.62 (m, 2H), 7.12-7.38 (m, 2H), 4.72-5.15 (m, 2H), 3.48-3.69 (m, 1H), 3.48-3.69 (m, 2H), 2.56-3.02 (m, 3H), 2.50-2.51 (m, 3H), 1.05-1.30 (m, 3H). 19F NMR (376.5 MHz, DMSO-d6) Shift -119.36, -119.49. LC-MS: Rt =0.975 min, (ESI) *m*/*z.* [M+H]⁺ 476.2; |
| | C₂₂H₁₃F₄N₈O | | C₂₄H₂₂FN₇O_{S} |
| 81 | 1H NMR (400MHz, DMSO-d6) Shift 8.41 - 8.17 (m, 1H), 8.12 - 7.94 (m, 1H), 7.92 - 7.75 (m, 2H), 7.56 - 7.38 (m, 2H), 7.39 - 7.36 (m, 1H), 7.33 - 7.14 (m, 1H), 5.13 (s, 1H), 5.00 - 4.85 (m, 1H), 3.54 (br s, 1H), 3.01 - 2.65 (m, 3H), 1.25 - 1.05 (m, 3H). 19F NMR (376MHz, DMSO-d6) Shift -64.74 - -66.99 (m, 1F), -118.68 - -120.65 (m, 1F). LC-MS: Rt =1.521 min, (ESI) *m*/*z.* [M+H]⁺ 448.2; | 82 | 1H NMR (400MHz, DMSO-d6) Shift 9.06 - 8.86 (m, 1H), 8.62 - 8.03 (m, 2H), 7.78 - 6.78 (m, 8H), 5.10 - 4.95 (m, 2H), 3.50 (br s, 3H), 2.62 - 2.59 (m, 3H). F NMR 376MHz, DMSO-d6) Shift - 116.957; -157.804, -157.907.LC-MS: Rt =3.475 (ESI) *m*/*z.* [M+H]⁺ 488.3 |
| | | | C₂₄H₁₉F₂N₉O |
| | C₂₀H₁₇F₄N₇O | | |
| 83 | 1H NMR (400 MHz, DMSO-d6) Shift 8.01-8.14 (m, 1H), 7.06-7.54 (m, 2H), 6.96 (s, 1H), 6.60-6.92 (m, 3H), 6.14-6.59 (m, 1H), 4.43 (s, 2H), 3.12 (s, 3H), 1.72-2.17 (m, 3H). 19F NMR (376.5 MHz, DMSO-d6) Shift -60.63, -60.74, - 117.34, -119.02. LC-MS: Rt =1.666 min, (ESI) *m*/*z.* [M+H]⁺ 500.3; | 84 | 1H NMR (400 MHz, DMSO-d6) Shift 12.55-12.90 (m, 1H), 8.89-9.00 (m, 1H), 8.12-8.30 (m, 1H), 7.95-8.10 (m, 1H), 7.76-7.90 (m, 1H), 7.52-7.73 (m, 2H), 7.46 (s, 2H), 7.16-7.37 (m, 1H), 6.97-7.10 (m, 1H), 5.10-5.25 (s, 2H), 2.60-2.98 (m, 3H). 19F NMR (376.5 MHz, DMSO-d6) Shift -60.70, - 60.82, -117.24, -119.11. LC-MS: Rt =1.490 min, (ESI) *m*/*z.* [M+H]⁺ 485.3; |
| | C₂₂H₁₇F₄N₉O | | |
| | | | C₂₂H₁₆F₄N₈O |
| 85 | 1H NMR (400 MHz, DMSO-d6) Shift 0.61 - 0.73 (m, 2 H) 0.76 - 0.85 (m, 2 H) 1.01 - 1.14 (m, 3 H) 2.71 - 2.79 (m, 2 H) 2.88 - 3.01 (m, 1 H) 3.39 - 4.00 (m, 2 H) 4.46 - 4.79 (m, 2 H) 7.17 - 7.27 (m, 2 H) 7.31 - 7.60 (m, 4 H) 7.76 - 7.86 (m, 1 H) 7.93 - 8.03 (m, 1 H) 8.30 - 8.36 (m, 1 H). LC-MS: Rt =0.934 min, (ESI) *m*/*z.* [M+H]⁺ 435.3; | 86 | 1H NMR (400 MHz, DMSO-d6) Shift 9.15-9.31 (m, 1H), 8.33 (s, 1H), 8.07-8.16 (m, 1H), 7.92-8.03 (m, 1H), 7.76-7.86 (m, 1H), 7.58-7.75 (m, 2H), 7.41-7.55 (m, 3H), 7.18-7.33 (m, 1H), 4.71-5.17 (m, 2H), 3.53-3.83 (m, 3H), 2.80-2.97 (m, 3H). LC-MS: Rt =1.544 min, (ESI) *m*/*z.* [M+H]⁺ 554.2; |
| | C₂₃H₂₃FN₆O₂ | | C₂₅H₁₉ClF₃N₉O |
| 87 | 1H NMR (400 MHz, DMSO-d6) Shift 7.94-8.26 (m, 1H), 7.82 (s, 1H), 7.46 (br s, 3H), 7.19-7.31 (m, 2H), 7.03 (br d, J=8.36 Hz, 2H), 4.64 (br d, J=11.66 Hz, 1H), 3.62-3.92 (m, 4H), 3.58 (br d, J=5.94 Hz, 1H), 2.93 (br s, 3H), 1.17-1.41 (m, 1H), 0.66-0.90 (m, 5H), 0.63 (br s, 2H). 19F NMR (376 MHz, DMSO-d6) Shift -120.00 (br s, 1F). LC-MS: Rt =1.751 min, (ESI) *m*/*z.* [M+H]⁺ 476.3; | 88 | 1H NMR (400 MHz, DMSO-d6) Shift 8.72 - 9.11 (m, 1 H) 7.94 - 8.33 (m, 2 H) 7.75 - 7.88 (m, 1 H) 7.52 - 7.73 (m, 2 H) 7.37 - 7.52 (m, 2 H) 6.94 - 7.33 (m, 2 H) 4.99 - 5.32 (m, 2 H) 3.53 - 3.84 (m, 3 H) 2.90 (br s, 3 H). 19F NMR (376 MHz, DMSO-d6) Shift -61.16 - - 60.44 (m, 1 F) -119.57 - -116.77 (m, 1 F). LC-MS: Rt =1.543 min, (ESI) *m*/*z.* [M+H]⁺ 499.3; |
| | C₂₆H₂₆FN₅O₃ | | C₂₃H₁₈F₄N₈O |
| 89 | 1H NMR (400 MHz, DMSO-d6) Shift 8.10 - 8.33 (m, 1 H) 8.02 (br d, J=5.02 Hz, 1 H) 7.83 (s, 1 H) 7.51 (br s, 2 H) 7.27 (br d, J=10.79 Hz, 1 H) 5.68 (br d, J=1.51 Hz, 1 H) 4.64 (br d, J=1.51 Hz, 1 H) 3.63 - 4.04 (m, 4 H) 2.95 (br s, 3 H) 0.83 (br s, 3 H). 19F NMR (376 MHz, DMSO-d6) Shift -59.89 (br s, 1 F) -119.98 (br d, J=52.64 Hz, 1 F) | 90 | 1H NMR (400 MHz, DMSO-d6) Shift 8.72 - 9.11 (m, 1 H) 7.94 - 8.33 (m, 2 H) 7.75 - 7.88 (m, 1 H) 7.52 - 7.73 (m, 2 H) 7.37 - 7.52 (m, 2 H) 6.94 - 7.33 (m, 2 H) 4.99 - 5.32 (m, 2 H) 3.53 - 3.84 (m, 3 H) 2.90 (br s, 3 H). 19F NMR (376 MHz, DMSO-d6) Shift -61.16 - - 60.44 (m, 1 F) -119.57 - -116.77 (m, 1 F). LC-MS: Rt =1.543 min, (ESI) *m*/*z.* [M+H]⁺ 499.3; |
| | LC-MS: Rt =0.980 min, (ESI) *m*/*z.* [M+H]⁺ 495.3; | | |
| | C₂₁H₁₈F₄N₆O₂S | | C₂₃H₁₈F₄N₈O |
| 91 | 1H NMR (400 MHz, DMSO-d6) Shift 8.50 (br s, 1H), 7.74-8.27 (m, 2H), 7.62 (br s, 1H), 7.29-7.52 (m, 3H), 6.91-7.29 (m, 2H), 4.74-5.27 (m, 6H), 3.59 (br s, 3H), 2.62-2.98 (m, 3H). LC-MS: Rt =1.982 min, (ESI) *m*/*z.* [M+H]⁺ 473.3; | 92 | 1H NMR (400 MHz, DMSO-d6) Shift 7.35 - 8.50 (m, 7 H) 6.96 - 7.35 (m, 1 H) 4.53 - 5.22 (m, 2 H) 2.60 - 2.98 (m, 3 H) 1.05 - 1.27 (m, 3 H). 19F NMR (376 MHz, DMSO-d6) Shift -58.95 - -58.53 (m, 1 F) -120.17 - -118.91 (m, 1 F). LC-MS: Rt =1.613 min, (ESI) |
| | C₂₄H₂₁FN₈O₂ | | *m*/*z.* [M+H]⁺ 486.2; |
| | | | C₂₃H₁₉F₄N₇O |
| 93 | 1H NMR (400 MHz, DMSO-d6) Shift 8.83 - 9.07 (m, 1 H) 8.34 - 8.50 (m, 2 H) 8.15 - 8.29 (m, 1 H) 7.62 - 7.72 (m, 1 H) 7.09 - 7.24 (m, 1 H) 6.96 - 7.07 (m, 2 H) 4.61 - 4.95 (m, 2 H) 3.51 - 3.61 (m, 2 H) 2.58 - 2.64 (m, 3 H) 1.05 - 1.19 (m, 3 H). 19F NMR (376 MHz, DMSO-d6) Shift -65.19 - -64.80 (m, 1 F) - 119.50 (br d, J=20.60 Hz, 1 F). LC-MS: Rt =1.633 min, (ESI) *m*/*z.* | 94 | 1H NMR (400 MHz, DMSO-d6) Shift 9.06 (s, 1H), 8.25-8.43 (m, 1H), 8.11 (d, J=6.02 Hz, 1H), 7.62-7.92 (m, 2H), 7.10-7.57 (m, 3H), 4.19-5.92 (m, 2H), 3.42-4.07 (m, 4H), 2.96 (s, 3H), 0.75 (d, J=6.78 Hz, 3H). 19F NMR (376.5 MHz, DMSO-d6) Shift -119.78. LC-MS: Rt =0.567 min, (ESI) *m*/*z.* [M+H]⁺ 446.1; |
| | [M+H]⁺ 487.3; | | C₂₃H₂₀FN₇O₂ |
| | C₂₂H₁₈F₄N₈O | | |
| 95 | 1H NMR (400MHz, DMSO-d6) Shift 8.08 - 7.94 (m, 1H), 7.81 (br d, J=19.6 Hz, 1H), 7.59 (br d, J=9.0 Hz, 1H), 7.52 - 7.39 (m, 2H), 7.32 - 7.09 (m, 2H), 4.91 (s, 1H), 4.66 (s, 1H), 4.57 - 4.39 (m, 2H), 3.32 (s, 2H), 2.99 - 2.75 (m, 3H), 1.43 - 1.32 (m, 3H), 1.19 - 1.02 (m, 3H). 19F NMR (376MHz, DMSO-d6) Shift - 119.94 (br d, J=183.1 Hz, 1F) LC-MS: Rt =1.457 min, (ESI) *m*/*z.* [M+H]⁺ 424.2; | 96 | 1H NMR (400 MHz, DMSO-d6) Shift 8.15 (s, 0.16H, HCOOH), 7.76-7.99 (m, 2H), 7.35-7.52 (m, 2H), 6.85-7.27 (m, 3H), 6.62-6.78 (m, 1H), 4.30-4.74 (m, 4H), 3.01-3.21 (m, 4H), 2.71-2.96 (m, 3H), 0.93-1.21 (m, 3H). 19F NMR (376.5 MHz, DMSO-d6) Shift - 119.74, -119.89. LC-MS: Rt =1.623 min, (ESI) *m*/*z.* [M+H]⁺ 420.3; |
| | C₂₁H₂₂FN₇O₂ | | C₂₃H₂₂FN₅O₂ |
| 97 | 1H NMR (400 MHz, DMSO-d6) Shift 8.99 (s, 1H), 8.06-8.47 (m, 1H), 7.57 (br d, J=7.75 Hz, 1H), 7.33 (br d, J=7.63 Hz, 1H), 7.24 (s, 1H), 7.18 (br d, J=10.76 Hz, 1H), 7.05 (br s, 2H), 5.49-6.15 (m, 1H), 4.50-5.06 (m, 2H), 3.17 (br dd, J=7.75, 14.88 Hz, 2H), 2.63 (s, 3H), 0.83-1.04 (m, 3H) | 98 | 1H NMR (400 MHz, DMSO-d6) Shift 8.15 (s, 0.16H, HCOOH), 7.76-7.99 (m, 2H), 7.35-7.52 (m, 2H), 6.85-7.27 (m, 3H), 6.62-6.78 (m, 1H), 4.30-4.74 (m, 4H), 3.01-3.21 (m, 4H), 2.71-2.96 (m, 3H), 0.93-1.21 (m, 3H). 19F NMR (376.5 MHz, DMSO-d6) Shift - 119.74, -119.89. LC-MS: Rt =1.623 min, (ESI) *m*/*z.* [M+H]⁺ 420.3; |
| | LC-MS: Rt =0.78 min, (ESI) *m*/*z.* [M+H]⁺ 474.3; | | |
| | C₂₃H₁₉F₄N₅O₂ | | C₂₃H₂₂FN₅O₂ |
| 99 | 1 H NMR (400 MHz, DMSO-d6) Shift 8.15 (d, J=2.45 Hz, 1H), 7.96 (br dd, J=6.24, 16.75 Hz, 1H), 7.81 (br d, J=17.73 Hz, 1H), 7.43 (br d, J=19.20 Hz, 2H), 7.10-7.35 (m, 3H), 5.21-5.45 (m, 1H), 4.93 (td, J=6.71, 13.60 Hz, 2H), 4.74 (br s, 1H), 4.44-4.63 (m, 3H), 3.44 (br s, 1H), 3.29 (br d, J=11.00 Hz, 1H), 2.94 (s, 1H), 2.74 (s, 2H), 0.97-1.20 (m, 3H). LC-MS: Rt =0.61 min, (ESI) *m*/*z.* [M+H]⁺ 451.3; | 100 | 1H NMR (400 MHz, DMSO-d6) Shift 9.13-9.40 (m, 1H), 8.86-9.09 (m, 1H), 8.11-8.31 (m, 1H), 7.71-7.98 (m, 1H), 7.42 (br dd, J=9.35, 19.50 Hz, 1H), 7.16 (br dd, J=10.94, 19.62 Hz, 1H), 7.01 (br d, J=8.93 Hz, 2H), 6.56-6.83 (m, 1H), 4.54-5.07 (m, 2H), 3.53 (br d, J=5.38 Hz, 1H), 3.29 (br s, 1H), 2.61 (br d, J=9.54 Hz, 3H), 1.02-1.19 (m, 3H). LC-MS: Rt =0.899 min, (ESI) *m*/*z.* [M+H]⁺ 486.3; |
| | C₂₃H₂₃FN₆O₃ | | C₂₃H₁₉F₄N₇O |
| 101 | 1 H NMR (400 MHz, DMSO-d6) Shift 8.15 (d, J=2.45 Hz, 1H), 7.96 (br dd, J=6.24, 16.75 Hz, 1H), 7.81 (br d, J=17.73 Hz, 1H), 7.43 (br d, J=19.20 Hz, 2H), 7.10-7.35 (m, 3H), 5.21-5.45 (m, 1H), 4.93 (td, J=6.71, 13.60 Hz, 2H), 4.74 (br s, 1H), 4.44-4.63 (m, 3H), 3.44 (br s, 1H), 3.29 (br d, J=11.00 Hz, 1H), 2.94 (s, 1H), 2.74 (s, 2H), 0.97-1.20 (m, 3H). LC-MS: Rt =1.572 min, (ESI) m/z. [M+H]+ 471.2; | 102 | 1H NMR (400MHz, DMSO-d6) Shift 8.08 - 7.94 (m, 1H), 7.81 (br d, J=19.6 Hz, 1H), 7.59 (br d, J=9.0 Hz, 1H), 7.52 - 7.39 (m, 2H), 7.32 - 7.09 (m, 2H), 4.91 (s, 1H), 4.66 (s, 1H), 4.57 - 4.39 (m, 2H), 3.32 (s, 2H), 2.99 - 2.75 (m, 3H), 1.43 - 1.32 (m, 3H), 1.19 - 1.02 (m, 3H). 19F NMR (376MHz, DMSO-d6) Shift - 119.94 (br d, J=183.1 Hz, 1F) LC-MS: Rt =1.457 min, (ESI) *m*/*z.* [M+H]⁺ 424.2; |
| | C23H21F3N6O2 | | C₂₁H₂₂FN₇O₂ |
| 103 | 1H NMR (400 MHz, DMSO-d6) Shift 9.03 (br s, 1H), 8.46 (br s, 1H), 8.33 (br s, 1H), 7.67 (br s, 1H), 7.36-7.55 (m, 1H), 6.90-7.31 (m, 3H), 5.57 (br s, 1H), 4.68-5.29 (m, 1H), 4.33-5.75 (m, 1H), 3.49-3.79 (m, 6H), 3.43 (br s, 2H), 2.81 (br d, J=6.24 Hz, 2H), 2.61 (s, 3H), 0.99-1.11 (m, 3H), 0.80 (br d, J=6.97 Hz, 3H). LC-MS: Rt =1.593 min, (ESI) *m*/*z.* [M+H]⁺ 493.3; | 104 | 1H NMR (400 MHz, DMSO-d6) Shift 8.75-9.25 (m, 1H), 8.14-8.49 (m, 2H), 7.64-7.86 (m, 1H), 7.46 (br d, J=7.82 Hz, 1H), 7.12-7.24 (m, 2H), 7.02 (br s, 2H), 4.31-4.93 (m, 2H), 3.49-3.65 (m, 4H), 3.00-3.32 (m, 3H), 2.54-2.77 (m, 6H), 2.43 (br s, 4H), 1.04-1.18 (m, 3H), ~90% of purity. LC-MS: Rt =0.774 min, (ESI) *m*/*z.* [M+H]⁺ 531.3; |
| | C₂₆H₂₉FN₆O₃ | | C₂₈H₃₁FN₈O₂ |
| 105 | 1H NMR (400 MHz, DMSO-d6) Shift 8.94 - 9.12 (m, 1 H) 8.20 - 8.42 (m, 2 H) 7.31 - 7.55 (m, 2 H) 6.93 - 7.31 (m, 3 H) 4.48 (br s, 3 H) 4.17 (br s, 2 H) 3.61 - 3.72 (m, 4 H) 3.31 (br s, 4 H) 2.56 - 2.65 (m, 3 H) 0.64 - 0.87 (m, 3 H). 19F NMR (376 MHz, DMSO-d6) Shift -119.96 - - 119.49 (m, 1 F). LC-MS: Rt =0.866 min, (ESI) *m*/*z.* [M+H]⁺ 495.3; | 106 | 1H NMR (400 MHz, DMSO-d6) Shift 8.81-9.21 (m, 1H), 8.33-8.63 (m, 1H), 7.74-8.31 (m, 3H), 7.40-7.68 (m, 1H), 6.91-7.28 (m, 3H), 5.37-5.90 (m, 1H), 4.71-5.05 (m, 1H), 4.38-4.70 (m, 1H), 3.80-3.93 (m, 3H), 3.22 (br d, J=6.75 Hz, 2H), 2.61 (s, 3H), 0.69-1.12 (m, 3H). LC-MS: Rt =2.220 min, (ESI) *m*/*z.* [M+H]⁺ 487.3; |
| | C₂₅H₂₇FN₆O₄ | | C₂₅H₂₃FN₈O₂ |
| 107 | 1H NMR (400 MHz, DMSO-d6) Shift 7.88-8.15 (m, 1H), 7.81 (s, 1H), 7.41-7.60 (m, 3H), 7.14-7.28 (m, 1H), 6.84-7.13 (m, 3H), 6.62-6.77 (m, 1H), 4.87 (s, 2H), 4.38-4.61 (m, 2H), 3.51-3.91 (m, 3H), 3.00-3.22 (m, 2H), 2.64-2.97 (m, 3H). 19F NMR (376.5 MHz, DMSO-d6) Shift -117.53, -119.22. LC-MS: Rt =1.720 min, (ESI) *m*/*z.* [M+H]⁺ 472.1; | 108 | 1H NMR (400 MHz, DMSO-d6) Shift 8.79 (s, 1H), 7.91-8.24 (m, 2H), 7.82 (s, 1H), 7.71 (s, 1H), 7.46 (s, 2H), 6.97-7.37 (m, 2H), 4.45-5.90 (m, 2H), 3.47-4.42 (m, 4H), 2.94 (s, 3H), 0.77 (d, J=6.88 Hz, 3H). 19F NMR (376.5 MHz, DMSO-d6) Shift -111.03, - 119.80. LC-MS: Rt =1.559 min, (ESI) *m*/*z.* [M+H]⁺ 471.3; |
| | C₂₅H₂₂FN₇O₂ | | C₂₃H₂₁F₃N₆O₂ |
| 109 | 1H NMR (400 MHz, DMSO-d6) Shift 8.94 (s, 1H), 8.26 (br d, J=6.82 Hz, 1H), 7.91 (d, J=6.60 Hz, 1H), 7.80 (s, 1H), 7.70 (d, J=8.36 Hz, 1H), 7.65 (s, 1H), 7.47 (s, 2H), 7.07 (d, J=11.00 Hz, 1H), 5.10 (s, 2H), 3.54 (s, 3H), 2.84 (s, 3H), 1.84 (s, 3H). 19F NMR (376 MHz, DMSO-d6) Shift -61.36--60.34 (m, 1F), -117.28 (br s, 1F). LC-MS: Rt =1.582 min, (ESI) *m*/*z.* [M+H]⁺ | 110 | 1H NMR (400 MHz, DMSO-d6) Shift 8.79 (s, 1H), 7.91-8.24 (m, 2H), 7.82 (s, 1H), 7.71 (s, 1H), 7.46 (s, 2H), 6.97-7.37 (m, 2H), 4.45-5.90 (m, 2H), 3.47-4.42 (m, 4H), 2.94 (s, 3H), 0.77 (d, J=6.88 Hz, 3H). 19F NMR (376.5 MHz, DMSO-d6) Shift -111.03, - 119.80. LC-MS: Rt =1.559 min, (ESI) *m*/*z.* [M+H]⁺ 471.3; |
| | 513.3; | | C₂₃H₂₁F₃N₆O₂ |
| | C₂₄H₂₀F₄N₈O | | |
| 111 | 1H NMR (400 MHz, DMSO-d6) Shift 8.94 (s, 1H), 8.26 (br d, J=6.82 Hz, 1H), 7.91 (d, J=6.60 Hz, 1H), 7.80 (s, 1H), 7.70 (d, J=8.36 Hz, 1H), 7.65 (s, 1H), 7.47 (s, 2H), 7.07 (d, J=11.00 Hz, 1H), 5.10 (s, 2H), 3.54 (s, 3H), 2.84 (s, 3H), 1.84 (s, 3H). 19F NMR (376 MHz, DMSO-d6) Shift -61.36--60.34 (m, 1F), -117.28 (br s, 1F). LC-MS: Rt =1.582 min, (ESI) *m*/*z.* [M+H]⁺ 513.3; | 112 | 1H NMR (400 MHz, DMSO-d6) Shift 9.02 (br s, 1H), 8.51 (br s, 1H), 8.14-8.38 (m, 1H), 7.71 (br s, 1H), 7.46 (br s, 1H), 7.22 (br s, 1H), 7.03 (br s, 2H), 4.20-5.71 (m, 3H), 3.94 (br s, 3H), 3.68 (br s, 4H), 2.84 (br s, 1H), 2.62 (br s, 3H), 1.71 (br s, 4H), 0.81 (br d, J=6.88 Hz, 3H). 19F NMR (376 MHz, DMSO-d6) Shift -119.62 (br s, 1F) |
| | | | LC-MS: Rt =1.501 min, (ESI) *m*/*z.* [M+H]⁺ 505.3; |
| | C₂₄H₂₀F₄N₈O | | C₂₇H₂₉FN₆O₃ |
| 113 | 1H NMR (400MHz, DMSO-d6) Shift 9.00 - 8.77 (m, 1H), 7.72 (br d, J=9.5 Hz, 1H), 7.65 - 7.53 (m, 1H), 7.47 (br d, J=9.9 Hz, 2H), 7.38 - 7.01 (m, 2H), 6.79 (br s, 2H), 5.33 - 5.20 (m, 2H), 5.14 - 4.86 (m, 4H), 3.82 - 3.53 (m, 3H). 19F NMR (376MHz, DMSO-d6) Shift - 60.560, -61.047, -61.162; -113.827, -115.669; -154.764. | 114 | 1H NMR (400 MHz, DMSO-d6) Shift 7.92-8.19 (m, 1H), 7.83 (s, 1H), 7.57 (br s, 1H), 7.47 (s, 2H), 7.31 (br d, J=7.83 Hz, 1H), 7.13-7.28 (m, 2H), 5.48-6.23 (m, 1H), 4.49-5.02 (m, 2H), 3.18 (br d, J=8.44 Hz, 2H), 2.96 (br s, 3H), 0.81-1.11 (m, 3H). LC-MS: Rt =0.78 min, (ESI) *m*/*z.* [M+H]⁺ 474.3; |
| | LC-MS: Rt =4.270 min, (ESI) *m*/*z.* [M+H]⁺ 544.2; | | C₂₃H₁₉F₄N₅O₂ |
| | C₂₅H₁₈F₅N₇O₂ | | |
| 115 | 1H NMR (400 MHz, DMSO-d6) Shift 8.94 (s, 1 H) 8.22 - 8.29 (m, 2 H) 8.04 (d, J=6.63 Hz, 1 H) 7.81 (s, 1 H) 7.68 - 7.72 (m, 1 H) 7.51 -7.56 (m, 2 H) 7.07 - 7.13 (m, 1 H) 5.24 - 5.32 (m, 2 H) 3.61 - 3.65 (m, 3 H) 2.81 (s, 3 H). 19F NMR (376 MHz, DMSO-d6) Shift -60.73 - -60.67 (m, 1 F) -117.75 - -117.69 (m, 1 F). LC-MS: Rt =1.545 min, (ESI) *m*/*z.* [M+H]⁺ 500.2; | 116 | 1H NMR (400 MHz, DMSO-d6) Shift 8.30 - 8.41 (m, 1 H) 7.94 - 8.15 (m, 1 H) 7.77 - 7.84 (m, 1 H) 7.49 - 7.63 (m, 2 H) 7.29 - 7.49 (m, 3 H) 6.98 - 7.29 (m, 2 H) 4.77 - 5.05 (m, 4 H) 3.53 - 3.82 (m, 3 H) 2.66 - 2.94 (m, 3 H). 19F NMR (376 MHz, DMSO-d6) Shift - 72.47 (br s, 1 F) -119.73 - -117.01 (m, 1 F). LC-MS: Rt =1.560 min, (ESI) *m*/*z.* [M+H]⁺ 529.3; |
| | C₂₂H₁₇F₄N₉O | | C₂₄H₂₀F₄N₈O₂ |
| 117 | 1H NMR (400 MHz, DMSO-d6) Shift 8.93 (br s, 1H), 8.24 (br s, 1H), 7.97 (br d, J=6.38 Hz, 1H), 7.80 (s, 1H), 7.71 (br s, 1H), 7.50 (s, 3H), 7.11 (br d, J=10.56 Hz, 1H), 5.86 (br s, 1H), 5.25 (br s, 2H), 4.70 (br s, 1H), 3.90 (br s, 2H), 3.42 (br d, J=8.58 Hz, 2H), 2.65-2.92 (m, 3H). 19F NMR (376 MHz, DMSO-d6) Shift -60.70 (br s, 1F), -117.80 (br s, 1F). LC-MS: Rt =0.909 min, (ESI) *m*/*z.* [M+H]⁺ 529.3; | 118 | 1H NMR (400MHz, DMSO-d6) Shift 8.95 (s, 1H), 8.26 (br d, J=8.4 Hz, 1H), 7.92 (d, J=6.4 Hz, 1H), 7.80 (s, 1H), 7.71 (d, J=8.1 Hz, 1H), 7.47 (s, 2H), 7.28 (s, 1H), 7.06 (d, J=11.0 Hz, 1H), 5.11 (s, 2H), 3.48 (s, 3H), 2.87 (s, 3H), 1.97 (s, 3H). 19F NMR (376MHz, DMSO-d6) Shift -59.68 - -61.93 (m, 1F), -117.63 (br s, 1F). LC-MS: Rt =1.567 min, (ESI) *m*/*z.* [M+H]⁺ 513.2; |
| | C₂₄H₂₀F₄N₈O₂ | | C₂₄H₂₀F₄N₈O |
| 119 | 1H NMR (400MHz, DMSO-d6) Shift 8.33 (br d, J=8.6 Hz, 1H), 8.11 - 7.98 (m, 2H), 7.81 (s, 1H), 7.70 (s, 1H), 7.49 (s, 2H), 7.26 (s, 1H), 7.05 (br d, J=10.8 Hz, 1H), 5.39 (s, 2H), 3.61 (s, 3H), 2.90 (s, 3H). 19F NMR (376MHz, DMSO-d6) Shift -64.18 - -67.55 (m, 1F), -117.26 (br s, 1F). LC-MS: Rt =1.413 min, (ESI) *m*/*z.* [M+H]⁺ 500.3; | 120 | 1H NMR (400 MHz, DMSO-d6) Shift 8.87 (s, 1H), 8.04-8.38 (m, 1H), 7.94 (br d, J=6.25 Hz, 1H), 7.80 (s, 1H), 7.64 (s, 1H), 7.46 (s, 2H), 7.20 (s, 1H), 7.04 (br d, J=10.88 Hz, 1H), 5.20 (s, 2H), 3.55-3.88 (m, 3H), 2.60-2.91 (m, 3H). 19F NMR (376 MHz, DMSO-d6) Shift -61.38--60.23 (m, 3F), -117.45 (br s, 1F), - 124.61--123.30 (m, 1F). LC-MS: Rt =1.595 min, (ESI) *m*/*z.* [M+H]⁺ 517.3; |
| | C₂₂H₁₇F₄N₉O | | |
| | | | C₂₃H₁₇F₅N₈O |
| 121 | 1H NMR (400 MHz, DMSO-d6) Shift 8.93 (s, 1H), 8.13 (br d, J=6.16 Hz, 1H), 7.47 (br d, J=7.26 Hz, 1H), 7.29 (s, 1H), 7.22 (t, J=7.70 Hz, 1H), 6.86-7.13 (m, 4H), 6.81 (s, 1H), 6.72 (d, J=7.92 Hz, 1H), 6.56 (br d, J=6.16 Hz, 1H), 4.77 (br t, J=9.35 Hz, 1H), 4.49 (br dd, J=3.30, 10.34 Hz, 1H), 2.60 (s, 3H). 19F NMR (376 MHz, DMSO-d6) Shift -117.76 (br s, 1F). LC-MS: Rt =0.802 min, (ESI) *m*/*z.* [M+H]⁺ 458.3; | 122 | 1H NMR (400 MHz, DMSO-d6) Shift 10.11 (br s, 1H), 8.79 (s, 1H), 8.35 (d, J=6.82 Hz, 1H), 8.18 (s, 1H), 8.07 (br d, J=8.14 Hz, 1H), 7.86 (s, 1H), 7.65 (br s, 2H), 7.38 (d, J=8.14 Hz, 1H), 7.24 (d, J=12.10 Hz, 1H), 3.87 (s, 2H), 3.79 (s, 3H), 3.58 (s, 3H), 2.95 (s, 3H). 19F NMR (376 MHz, DMSO-d6) Shift -60.67 (br s, 1F), -116.08 (br s, 1F). LC-MS: Rt =1.038 min, (ESI) *m*/*z.* [M+H]⁺ 529.3; |
| | C₂₄H₂₀FN₇O₂ | | C₂₄H₂₀F₄N₈O₂ |
| 123 | 1H NMR (400 MHz, DMSO-d6) Shift 9.00 (s, 1H), 8.89 (br d, J=16.51 Hz, 1H), 8.57 (br d, J=3.67 Hz, 1H), 8.15-8.48 (m, 1H), 7.95-8.15 (m, 1H), 7.41-7.66 (m, 2H), 6.83-7.41 (m, 5H), 5.45-6.29 (m, 1H), 4.61 (br d, J=10.15 Hz, 2H), 3.15-3.29 (m, 2H), 2.63 (s, 3H), 0.78-1.12 (m, 3H). LC-MS: Rt =0.807 min, (ESI) *m*/*z.* [M+H]⁺ 483.3; | 124 | 1H NMR (400MHz, DMSO-d6) Shift 8.96 (s, 1H), 8.26 (br d, J=7.7 Hz, 1H), 8.05 (br d, J=6.6 Hz, 2H), 7.81 (s, 1H), 7.75 (br d, J=7.9 Hz, 2H), 7.50 (s, 2H), 6.99 (br d, J=10.8 Hz, 1H), 6.71 (br d, J=8.8 Hz, 1H), 5.27 (s, 2H), 3.69 (s, 3H), 2.91 (s, 3H). 19F NMR (376MHz, DMSO-d6) Shift - 59.12 - -62.21 (m, 3F), -116.43 (br s, 1F). LC-MS: Rt =1.779 min, (ESI) *m*/*z.* [M+H]⁺ 526.2; |
| | C₂₇H₂₃FN₆O₂ | | C₂₅H₁₉F₄N₇O₂ |
| 125 | 1H NMR (400 MHz, DMSO-d6) Shift 8.93 (s, 1H), 8.13 (br d, J=6.16 Hz, 1H), 7.47 (br d, J=7.26 Hz, 1H), 7.29 (s, 1H), 7.22 (t, J=7.70 Hz, 1H), 6.86-7.13 (m, 4H), 6.81 (s, 1H), 6.72 (d, J=7.92 Hz, 1H), 6.56 (br d, J=6.16 Hz, 1H), 4.77 (br t, J=9.35 Hz, 1H), 4.49 (br dd, J=3.30, 10.34 Hz, 1H), 2.60 (s, 3H). 19F NMR (376 MHz, DMSO-d6) Shift -117.76 (br s, 1F). LC-MS: Rt =0.802 min, (ESI) *m*/*z.* | 126 | 1H NMR (400 MHz, DMSO-d6) Shift 8.82 (br d, J=4.62 Hz, 2H), 8.09-8.21 (m, 1H), 7.99 (br d, J=5.94 Hz, 1H), 7.73-7.85 (m, 2H), 7.51 (br s, 2H), 7.42 (br t, J=4.73 Hz, 1H), 7.01 (br d, J=11.00 Hz, 1H), 6.69 (br d, J=8.80 Hz, 1H), 5.25 (br s, 2H), 3.67 (s, 3H), 2.87 (s, 3H). LC-MS: Rt =1.347 min, (ESI) *m*/*z.* [M+H]⁺ 459.3; |
| | [M+H]⁺ 458.3; | | C₂₃H₁₉FN₈O₂ |
| | C₂₄H₂₀FN₇O₂ | | |
| 127 | 1H NMR (400 MHz, DMSO-d6) Shift 8.33 (d, J=8.80 Hz, 1H), 8.08 (d, J=8.80 Hz, 1H), 7.93 (d, J=6.60 Hz, 1H), 7.81 (s, 1H), 7.70 (s, 1H), 7.50 (s, 2H), 7.08 (d, J=11.22 Hz, 1H), 5.33 (s, 2H), 3.56 (s, 3H), 2.84 (s, 3H), 1.85 (s, 3H). 19F NMR (376 MHz, DMSO-d6) Shift -65.59 (br s, 1F), -117.21 (br s, 1F). LC-MS: Rt =0.741 min, (ESI) *m*/*z.* [M+H]⁺ 514.3; | 128 | 1H NMR (400 MHz, DMSO-d6) Shift 9.00 (s, 1H), 8.25 (br d, J=6.38 Hz, 1H), 7.14-7.51 (m, 3H), 6.85-7.13 (m, 4H), 5.40-6.21 (m, 1H), 4.36-4.88 (m, 2H), 2.99-3.20 (m, 1H), 3.07 (br d, J=6.82 Hz, 1H), 2.63 (s, 3H), 0.55-1.09 (m, 3H). 19F NMR (376 MHz, DMSO-d6) Shift -119.88 (br d, J=16.02 Hz, 1F). LC-MS: Rt =0.842 min, (ESI) *m*/*z.* [M+H]⁺ 406.2; |
| | C₂₃H₁₉F₄N₉O | | C₂₂H₂₀FN₅O₂ |
| 129 | 1H NMR (400 MHz, DMSO-d6) Shift 8.94 (br s, 1H), 8.07-8.29 (m, 1H), 7.86-8.05 (m, 1H), 7.80 (s, 1H), 7.69 (d, J=8.13 Hz, 1H), 7.56-7.66 (m, 1H), 7.47 (s, 2H), 7.04-7.28 (m, 1H), 4.85-5.29 (m, 2H), 3.77-4.01 (m, 2H), 2.73-2.93 (s, 3H), 1.78-2.13 (m, 3H), 0.98-1.34 (m, 3H). 19F NMR (376.5 MHz, DMSO-d6) Shift -60.73, -60.82, - 117.12, -119.40. LC-MS: Rt =1.609 min, (ESI) *m*/*z.* [M+H]⁺ 527.3; | 130 | 1H NMR (400 MHz, DMSO-d6) Shift 9.00 (s, 1H), 8.06-8.52 (m, 2H), 7.86 (d, J=7.95 Hz, 1H), 6.83-7.48 (m, 6H), 5.14-6.64 (m, 1H), 4.39-4.93 (m, 2H), 3.85 (d, J=5.01 Hz, 3H), 2.56-2.70 (m, 6H). LC-MS: Rt =0.761 min, (ESI) *m*/*z.* [M+H]⁺ 472.2; |
| | | | C₂₅H₂₂FN₇O₂ |
| | C₂₅H₂₂F₄N₈O | | |
| 131 | 1H NMR (400 MHz, DMSO-d6) 7.85-7.99 (m, 1H), 7.80 (br s, 1H), 7.68 (br d, J=8.51 Hz, 1H), 7.61 (br s, 1H), 7.46 (br s, 2H), 7.28 (br d, J=8.50 Hz, 1H), 7.06 (br d, J=10.63 Hz, 1H), 5.11 (br s, 2H), 4.02 (br s, 3H), 3.55 (br s, 3H), 2.85 (br s, 3H), 1.76 (br s, 3H). 19F NMR (376 MHz, DMSO-d6) -117.38 (s, 1F). LC-MS: Rt =0.868 min, (ESI) *m*/*z.* [M+H]⁺ 476.1; | 132 | H NMR (400 MHz, DMSO-d6) Shift 8.99 (s, 1H), 8.08-8.54 (m, 2H), 7.62 (d, J=1.63 Hz, 1H), 7.16 (d, J=10.88 Hz, 1H), 7.04 (br s, 2H), 5.42-5.78 (m, 1H), 4.75-5.07 (m, 1H), 4.54-4.75 (m, 1H), 3.28 (br d, J=7.38 Hz, 1H), 3.18 (br d, J=7.50 Hz, 1H), 2.61 (d, J=3.13 Hz, 3H), 0.87-1.04 (m, 3H). 19F NMR (376 MHz, DMSO-d6) Shift -120.15-118.26 (m, 1F). LC-MS: Rt =0.847 min, (ESI) *m*/*z.* [M+H]⁺ |
| | C₂₃H₂₂FN₉O₂ | | 485.4; |
| | | | C₂₁H₁₈BrFN₆O₂ |
| 133 | 1H NMR (400 MHz, DMSO-d6) Shift 8.94 (s, 1H), 8.21-8.32 (m, 1H), 7.95 (d, J=6.38 Hz, 1H), 7.79 (s, 1H), 7.71 (d, J=8.36 Hz, 1H), 7.59 (s, 1H), 7.45 (s, 2H), 7.05 (d, J=11.00 Hz, 1H), 5.16 (br s, 3H), 4.29 (s, 2H), 3.55 (s, 3H), 2.86 (s, 3H). LC-MS: Rt =0.986 min, (ESI) *m*/*z.* [M+H]⁺ 529.2; | 134 | 1H NMR (400 MHz, DMSO-d6) Shift 8.98 (s, 1H), 8.94 (s, 1H), 8.26 (dd, J=1.88, 8.41 Hz, 1H), 8.19 (d, J=6.52 Hz, 1H), 7.71 (d, J=8.28 Hz, 1H), 7.59 (s, 1H), 7.32-7.43 (m, 1H), 7.01 (br s, 2H), 6.95 (d, J=11.04 Hz, 1H), 5.08 (s, 2H), 3.51 (s, 3H), 2.60 (s, 3H), 1.85 (s, 3H). 19F NMR (376.5 MHz, DMSO-d6) Shift -60.71, - 60.79, -116.76, -118.94. LC-MS: Rt =1.552 min, (ESI) *m*/*z.* [M+H]⁺ 513.3; |
| | C₂₄H₂₀F₄N₈O₂ | | |
| | | | C₂₄H₂₀F₄N₈O |
| 135 | 1H NMR (400 MHz, DMSO-d6) Shift 8.60 (d, J=7.04 Hz, 1H), 7.89 (d, J=6.38 Hz, 1H), 7.81 (s, 1H), 7.57-7.72 (m, 2H), 7.48 (s, 2H), 7.15-7.34 (m, 1H), 7.07 (d, J=11.22 Hz, 1H), 6.85 (t, J=6.71 Hz, 1H), 6.58 (s, 1H), 5.07 (s, 2H), 3.54 (s, 3H), 2.85 (s, 3H), 1.77 (s, 3H). 19F NMR (376 MHz, DMSO-d6) Shift - 117.27 (br s, 1F). LC-MS: Rt =0.719 min, (ESI) *m*/*z.* [M+H]⁺ 484.4; | 136 | 1H NMR (400 MHz, DMSO-d6) Shift 8.19 (s, 1H), 7.87 (d, J=5.77 Hz, 1H), 7.67-7.81 (m, 1H), 7.55 (s, 1H), 7.21-7.51 (m, 4H), 6.99-7.21 (m, 1H), 4.65-4.94(m, 2H), 4.09 (q, J=6.78 Hz, 2H), 3.45-3.73 (m, 3H), 2.62-2.89 (m, 3H), 1.62-2.06 (m, 3H), 1.34 (t, J=6.90 Hz, 3H). 19F NMR (376.5 MHz, DMSO-d6) Shift -117.41, - 119.66. LC-MS: Rt =1.411 min, (ESI) *m*/*z.* [M+H]⁺ 489.4; |
| | C₂₅H₂₂FN₉O | | |
| 137 | 1H NMR (400 MHz, DMSO-d6) 8.52 (br s, 1H), 8.03 (br s, 2H), 7.91 (br s, 1H), 7.82 (br s, 1H), 7.75 (br s, 1H), 7.49 (br s, 3H), 7.22 (br s, 1H), 6.97 (br s, 1H), 6.87 (br s, 1H), 6.68 (br s, 1H), 5.17 (br s, 2H), 3.63-3.82 (m, 3H), 2.92 (br s, 3H). 19F NMR (376 MHz, DMSO-d6) - 116.39 (s, 1F) LC-MS: Rt =0.800 min, (ESI) *m*/*z.* [M+H]⁺ 497.1; | 138 | 1H NMR (400 MHz, DMSO-d6) Shift 8.93 (s, 1H), 8.22-8.31 (m, 1H), 8.17 (s, 1H), 7.92 (d, J=6.53 Hz, 1H), 7.81 (s, 1H), 7.69 (d, J=8.28 Hz, 1H), 7.55 (s, 1H), 7.38-7.52 (m, 2H), 7.08 (d, J=11.04 Hz, 1H), 5.06 (s, 2H), 3.57 (s, 3H), 3.45 (s, 4H), 2.86 (s, 3H). 19F NMR (376.5 MHz, DMSO-d6) Shift -60.73, -117.12. LC-MS: Rt =1.079 min, (ESI) *m*/*z.* [M+H]⁺ 529.3; |
| | C₂₆H₂₁FN₈O₂ | | |
| | | | C₂₄H₂₀F₄N₈O₂ |
| 139 | 1H NMR (400 MHz, DMSO-d6) Shift 8.94 (s, 1H), 8.32 (s, 1H), 8.24 (d, J=7.53 Hz, 1H), 8.05 (d, J=6.53 Hz, 1H), 7.80 (s, 1H), 7.73 (d, J=7.28 Hz, 1H), 7.68 (dd, J=2.01, 8.28 Hz, 1H), 7.49 (br s, 2H), 7.13 (d, J=8.28 Hz, 1H), 6.98 (d, J=10.79 Hz, 1H), 5.30 (s, 2H), 2.87 (s, 3H), 2.30 (s, 3H). 19F NMR (376.5 MHz, DMSO-d6) Shift -60.73, - 116.52. LC-MS: Rt =1.614 min, (ESI) *m*/*z.* [M+H]⁺ 510.3; | 140 | 1H NMR (400 MHz, DMSO-d6) Shift 9.30 (s, 2H), 7.91 (d, J=6.38 Hz, 1H), 7.80 (s, 1H), 7.71 (s, 1H), 7.47 (s, 2H), 7.09 (d, J=11.13 Hz, 1H), 5.20 (s, 2H), 3.56 (s, 3H), 2.83 (s, 3H), 1.94 (s, 3H). LC-MS: Rt =0.872 min, (ESI) *m*/*z.* [M+H]⁺ 514.3; |
| | | | C₂₃H₁₉F₄N₉O |
| | C₂₅H₁₉F₄N₇O | | |
| 143 | 1H NMR (400 MHz, DMSO-d6) Shift 8.84-8.98 (m, 1H), 8.17-8.27 (m, 2H), 7.72-7.87 (m, 4H), 7.50 (s, 2H), 7.13 (dd, J=4.64, 7.91 Hz, 1H), 7.01 (d, J=11.29 Hz, 1H), 5.44 (d, J=15.81 Hz, 1H), 5.01 (d, J=15.81 Hz, 1H), 2.82 (s, 3H), 2.37 (s, 3H). 19F NMR (376.5 MHz, DMSO-d6) Shift -60.71, -60.85, - 116.51, -119.18. LC-MS: Rt =0.996 min, (ESI) *m*/*z.* [M+H]⁺ 510.1; | 144 | 1H NMR (400 MHz, DMSO) Shift Shift 8.29 (br d, J=2.20 Hz, 1H), 7.88 (br d, J=6.60 Hz, 1H), 7.80 (br s, 1H), 7.50-7.62 (m, 2H), 7.46 (s, 2H), 7.39 (d, J=8.80 Hz, 1H), 7.05 (d, J=11.22 Hz, 1H), 4.94 (s, 2H), 3.94 (td, J=3.03, 5.83 Hz, 1H), 3.53 (s, 3H), 2.83 (s, 3H), 1.62-1.82 (m, 3H), 0.55-0.89 (m, 4H). LC-MS: Rt =0.96 min, (ESI) *m*/*z.* [M+H]⁺ 501.2; |
| | C₂₅H₁₉F₄N₇O | | C₂₆H₂₅FN₈O₂ |
| 145 | H NMR (400 MHz, DMSO-d6) Shift 8.91 (s, 1H), 8.23-8.30 (m, 1H), 8.20 (s, 0.2H, HCOOH), 7.91-7.95 (m, 1H), 7.86 (d, J=6.53 Hz, 1H), 7.77-7.83 (m, 2H), 7.72-7.76 (m, 1H), 7.50 (br s, 2H), 7.03 (d, J=11.29 Hz, 1H), 6.89 (dd, J=5.02, 7.53 Hz, 1H), 5.34 (d, J=16.06 Hz, 1H), 5.04 (d, J=15.81 Hz, 1H), 3.74 (s, 3H), 2.80 (s, 3H). 19F NMR (376.5 MHz, DMSO-d6) Shift - 60.72, -117.24. LC-MS: Rt=1.14 min, (ESI) *m*/*z.* [M+H]⁺ 526.1; | 146 | 1H NMR (400 MHz, DMSO-d6) Shift 8.96 (br s, 1H), 8.63 (br s, 2H), 8.24 (br d, J=6.25 Hz, 1H), 8.10 (br d, J=6.13 Hz, 1H), 7.82 (s, 1H), 7.74 (br d, J=6.88 Hz, 1H), 7.52 (br s, 2H), 6.98 (br d, J=10.76 Hz, 1H), 5.30 (br s, 2H), 3.79 (br s, 3H), 2.93 (br s, 3H). 19F NMR (376 MHz, DMSO-d6) Shift -60.74 (br s, 3F), -116.07 (br s, 1F). LC-MS: Rt =1.640 min, (ESI) *m*/*z.* [M+H]⁺ 527.3; |
| | | | C₂₄H₁₈F₄N₈O₂ |
| | C₂₅H₁₉F₄N₇O₂ | | |
| 147 | 1H NMR (400 MHz, DMSO-d6) Shift 8.87-9.41 (m, 1H), 7.64-8.23 (m, 4H), 7.35-7.59 (m, 3H), 7.07-7.32 (m, 1H), 4.36-5.16 (m, 3H), 3.47-4.08 (m, 4H), 2.60-3.08 (m, 3H), 1.97-2.30 (m, 2H). 19F NMR (376 MHz, DMSO-d6) Shift -60.42 (br d, J=43.49 Hz, 3F), -122.45-117.86 (m, 1F). LC-MS: Rt =1.009 min, (ESI) *m*/*z.* [M+H]⁺ 528.3; | 148 | 1H NMR (400 MHz, DMSO-d6) Shift 8.96 (br s, 1H), 8.63 (br s, 2H), 8.24 (br d, J=6.25 Hz, 1H), 8.10 (br d, J=6.13 Hz, 1H), 7.82 (s, 1H), 7.74 (br d, J=6.88 Hz, 1H), 7.52 (br s, 2H), 6.98 (br d, J=10.76 Hz, 1H), 5.30 (br s, 2H), 3.79 (br s, 3H), 2.93 (br s, 3H). 19F NMR (376 MHz, DMSO-d6) Shift -60.74 (br s, 3F), -116.07 (br s, 1F). LC-MS: Rt =1.640 min, (ESI) *m*/*z.* [M+H]⁺ 527.3; |
| | C₂₅H₂₁F₄N₇O₂ | | |
| | | | C₂₄H₁₈F₄N₈O₂ |
| 149 | 1H NMR (400 MHz, DMSO-d6) Shift 8.83-8.96 (m, 1H), 7.64-8.32 (m, 3.5H, 0.5 HCOOH), 7.36-7.62 (m, 3H), 7.07-7.33 (m, 1H), 4.59-5.00 (m, 3H), 3.72-3.93 (m, 2H), 3.05-3.20 (m, 2H), 2.62-2.98 (m, 3H), 1.55-1.85 (m, 4H). 19F NMR (376.5 MHz, DMSO-d6) Shift - 60.66, -60.86, -119.75, -120.17. LC-MS: Rt =1.047 min, (ESI) *m*/*z.* [M+H]⁺ 503.2; | 150 | 1H NMR (400 MHz, DMSO-d6) Shift 8.94 (br s, 1H), 8.07-8.29 (m, 1H), 7.86-8.05 (m, 1H), 7.80 (s, 1H), 7.69 (d, J=8.13 Hz, 1H), 7.56-7.66 (m, 1H), 7.47 (s, 2H), 7.04-7.28 (m, 1H), 4.85-5.29 (m, 2H), 3.77-4.01 (m, 2H), 2.73-2.93 (s, 3H), 1.78-2.13 (m, 3H), 0.98-1.34 (m, 3H). 19F NMR (376.5 MHz, DMSO-d6) Shift -60.73, - 60.82, -117.12, -119.40. LC-MS: Rt =1.609 min, (ESI) *m*/*z.* [M+H]⁺ |
| | C₂₄H₂₂F₄N₆O₂ | | 527.3; |
| | | | C₂₅H₂₂F₄N₈O |
| 151 | 1H NMR (400 MHz, DMSO-d6) Shift 11.90-12.90 (m, 1H), 8.94 (s, 1H), 8.25 (dd, J=1.98, 8.36 Hz, 1H), 7.90 (d, J=6.38 Hz, 1H), 7.80 (s, 1H), 7.70 (d, J=8.36 Hz, 1H), 7.16-7.60 (m, 3H), 7.05 (d, J=11.00 Hz, 1H), 5.13 (s, 2H), 2.85 (s, 3H), 1.90 (s, 3H). LC-MS: Rt =0.906 min, (ESI) *m*/*z.* [M+H]⁺ 499.2; | 152 | 1H NMR (400 MHz, DMSO-d6) Shift 8.19-8.31 (m, 1H), 7.87 (d, J=6.27 Hz, 1H), 7.79 (s, 1H), 7.55 (s, 1H), 7.46 (s, 2H), 7.39-7.44 (m, 1H), 7.34-7.39 (m, 1H), 7.05 (d, J=11.04 Hz, 1H), 4.67-5.00 (m, 2H), 4.05-4.21 (m, 2H), 3.60-3.72 (m, 2H), 3.52 (s, 3H), 3.30 (s, 3H), 2.83 (s, 3H), 1.70-2.05 (m, 3H). 19F NMR (376.5 MHz, DMSO-d6) Shift -117.38, -119.61. LC-MS: Rt =0.878 min, (ESI) *m*/*z.* [M+H]⁺ 519.2; |
| | C₂₃H₁₈F₄N₈O | | |
| | | | C26H27FN8O3 |
| 153 | 1H NMR (400 MHz, DMSO-d6) Shift 9.04 (s, 2H), 8.11-8.44 (m, 2H), 7.63-7.83 (m, 1H), 6.91-7.33 (m, 3H), 4.30-5.91 (m, 2H), 3.41-4.01 (m, 4H), 2.62 (s, 3H), 0.77 (d, J=6.78 Hz, 3H). 19F NMR (376.5 MHz, DMSO-d6) Shift -60.69, - 119.60. LC-MS: Rt =1.076 min, (ESI) *m*/*z.* [M+H]⁺ 489.3; | 154 | 1H NMR (400 MHz, DMSO-d6) Shift 8.60 (br s, 1H), 8.23 (s, 1H), 7.70-8.10 (m, 2H), 7.38-7.70 (m, 3H), 7.04-7.38 (m, 2H), 6.84 (br s, 1H), 6.27-6.60 (m, 1H), 4.36-5.18 (m, 3H), 3.79-4.04 (m, 2H), 3.45 (br s, 2H), 2.97 (br s, 2H), 2.38-2.50 (m, 1H), 2.11 (br s, 2H). LC-MS: Rt =1.081 min, (ESI) *m*/*z.* [M+H]⁺ 460.4; |
| | C₂₃H₂₀F₄N₆O₂ | | |
| | | | C₂₄H₂₂FN₇O₂ |
| 155 | 1H NMR (400 MHz, DMSO-d6) Shift 9.03 (s, 2H), 8.01-8.61 (m, 2H), 7.64-7.88 (m, 1H), 6.95-7.34 (m, 2H), 3.40-5.90 (m, 6H), 2.62 (s, 3H), 0.77 (d, J=6.78 Hz, 3H). 19F NMR (376 MHz, DMSO-d6) Shift - 60.69, -119.63. LC-MS: Rt =1.695 min, (ESI) *m*/*z.* [M+H]⁺ 489.3; | 156 | 1H NMR (400 MHz, DMSO-d6) Shift 8.94 (s, 1H), 8.01-8.32 (m, 1H), 7.69-8.01 (m, 2H), 7.34-7.67 (m, 3H), 7.12-7.28 (m, 1H), 5.06-5.23 (m, 2H), 4.78-4.97 (m, 1H), 4.56-4.76 (m, 4H), 2.59-2.96 (m, 3H). 19F NMR (376.5 MHz, DMSO-d6) Shift -60.67,-60.85, - 119.33. LC-MS: Rt =1.004 min, (ESI) *m*/*z.* [M+H]⁺ 475.0; |
| | C₂₃H₂₀F₄N₆O₂ | | |
| | | | C₂₂H₁₈F₄N₆O₂ |
| 157 | 1H NMR (400 MHz, DMSO-d6) 8.63 (br s, 1H), 7.95 (br d, J=8.88 Hz, 1H), 7.90 (br d, J=6.38 Hz, 1H), 7.80 (s, 1H), 7.58-7.68 (m, 2H), 7.48 (s, 2H), 7.07 (d, J=11.13 Hz, 1H), 5.05 (s, 2H), 3.51-3.59 (m, 3H), 2.84 (s, 3H), 1.80 (s, 3H). 19F NMR (376 MHz, DMSO-d6) - 57.38--57.11 (m, 3F), -119.93-116.78 (m, 1F). LC-MS: Rt =1.079 min, (ESI) *m*/*z.* [M+H]⁺ 529.1; | 158 | 1H NMR (400 MHz, DMSO-d6) Shift 8.94 (s, 1H), 8.01-8.32 (m, 1H), 7.69-8.01 (m, 2H), 7.34-7.67 (m, 3H), 7.12-7.28 (m, 1H), 5.06-5.23 (m, 2H), 4.78-4.97 (m, 1H), 4.56-4.76 (m, 4H), 2.59-2.96 (m, 3H). 19F NMR (376.5 MHz, DMSO-d6) Shift -60.67,-60.85, - 119.33. LC-MS: Rt =1.004 min, (ESI) *m*/*z.* [M+H]⁺ 475.0; |
| | C₂₄H₂₀F₄N₈O₂ | | C₂₂H₁₈F₄N₆O₂ |
| 159 | 1H NMR (400 MHz, DMSO-d6) Shift 8.84-9.01 (m, 1H), 8.11-8.27 (m, 1H), 8.03 (t, J=6.60 Hz, 1H), 7.83 (d, J=5.28 Hz, 1H), 7.57-7.74 (m, 1H), 7.49 (br d, J=3.96 Hz, 2H), 7.22 (dd, J=7.59, 11.11 Hz, 1H), 3.51-4.11 (m, 5H), 2.93 (d, J=9.46 Hz, 3H), 2.04-2.47 (m, 2H). 19F NMR (376 MHz, DMSO-d6) Shift - 60.72 (br d, J=25.18 Hz, 3F), - 118.69 (br d, J=18.31 Hz, 1F). LC-MS: Rt =1.054 min, (ESI) *m*/*z.* [M+H]⁺ 459.3; | 160 | 1H NMR (400 MHz, DMSO-d6) Shift 8.50 (br d, J=3.38 Hz, 1H), 8.18 (s, 1H), 8.08 (d, J=8.63 Hz, 1H), 7.90 (d, J=6.38 Hz, 1H), 7.79 (s, 1H), 7.65 (s, 1H), 7.46 (s, 2H), 7.22 (dd, J=4.44, 9.19 Hz, 1H), 7.05 (d, J=11.13 Hz, 1H), 5.04 (br s, 2H), 3.47-3.71 (m, 3H), 2.85 (s, 3H), 1.79 (s, 3H). 19F NMR (376 MHz, DMSO-d6) Shift -120.22-116.68 (m, 1F). LC-MS: Rt =1.24 min, (ESI) *m*/*z.* [M+H]⁺ 485.3; |
| | C₂₂H₁₈F₄N₆O | | C₂₄H₂₁FN₁₀O |
| 161 | 1H NMR (400 MHz, DMSO-d6) Shift 8.99 (s, 1H), 8.28 (d, J=2.75 Hz, 1H), 8.18 (d, J=6.50 Hz, 1H), 7.29-7.62 (m, 3H), 6.77-7.17 (m, 3H), 4.91 (s, 2H), 3.80-4.03 (m, 1H), 3.51 (s, 3H), 2.59 (s, 3H), 1.76 (s, 3H), 0.74-0.89 (m, 2H), 0.57-0.74 (m, 2H). 19F NMR (376 MHz, DMSO). LC-MS: Rt =0.824 min, (ESI) *m*/*z.* [M+H]⁺ 501.2; | 162 | NT |
| | C₂₆H₂₅FN₃O₂ | | |
| 163 | 1H NMR (400 MHz, DMSO-d6) Shift 9.25 (s, 1H), 8.97 (s, 1H), 8.15 (d, J=6.38 Hz, 1H), 8.04 (s, 1H), 7.73 (d, J=9.46 Hz, 1H), 7.57 (s, 1H), 7.47 (dd, J=1.54, 9.46 Hz, 1H), 6.63-7.24 (m, 3H), 5.01 (s, 2H), 3.52 (s, 3H), 2.60 (s, 3H), 1.79 (s, 3H). 19F NMR (376 MHz, DMSO-d6) Shift -60.40 (br s, 3F), - 116.73 (br s, 1F). LC-MS: Rt =0.994 min, (ESI) *m*/*z.* [M+H]⁺ 522.3; | 164 | NT |
| | C₂₆H₂₁F₄N₉O | | |
| 165 | 1H NMR (400 MHz, DMSO-d6) Shift 8.89-9.01 (m, 2H), 8.25 (dd, J=1.81, 8.32 Hz, 1H), 8.16 (d, J=6.38 Hz, 1H), 7.71 (d, J=8.13 Hz, 1H), 7.62 (s, 1H), 6.89-7.09 (m, 3H), 5.08 (s, 2H), 4.65 (br t, J=5.00 Hz, 1H), 3.80 (t, J=5.82 Hz, 2H), 3.42 (br d, J=5.13 Hz, 2H), 2.60 (s, 3H), 1.85 (s, 3H). 19F NMR (376 MHz, DMSO-d6) Shift -61.84-59.73 (m, 3F), -116.72 (br s, 1F). LC-MS: Rt =1.460 min, (ESI) *m*/*z.* [M+H]⁺ 543.3; | 166 | 1H NMR (400 MHz, DMSO-d6) Shift 9.25 (s, 1H), 8.04 (s, 1H), 7.88 (br d, J=6.38 Hz, 1H), 7.81 (s, 1H), 7.73 (d, J=9.46 Hz, 1H), 7.62 (s, 1H), 7.37-7.56 (m, 3H), 7.07 (d, J=11.22 Hz, 1H), 5.04 (br s, 2H), 3.55 (s, 3H), 2.84 (s, 3H), 1.79 (s, 3H). 19F NMR (376 MHz, DMSO-d6) Shift -60.40, -117.07. LC-MS: Rt =1.017 min, (ESI) *m*/*z.* [M+H]⁺ 522.3; |
| | | | C₂₆H₂₁F₄N₉O |
| | C₂₅H₂₂F₄N₈O₂ | | |
| 167 | 1H NMR (400 MHz, DMSO) Shift 9.10 (s, 1H), 8.88 (s, 1H), 8.09 (d, J=6.38 Hz, 1H), 7.82 (d, J=9.24 Hz, 1H), 7.57 (s, 1H), 7.38 (d, J=9.24 Hz, 1H), 6.94 (d, J=10.78 Hz, 1H), 6.65-6.88 (m, 3H), 5.03 (br s, 2H), 3.46 (s, 3H), 2.56 (s, 3H), 1.73 (s, 3H). LC-MS: Rt =1.113 min, (ESI) *m*/*z.* [M+H]⁺ 552.1; | 168 | 1H NMR (400 MHz, DMSO-d6) Shift 8.99 (s, 1H), 8.03-8.38 (m, 2H), 7.49 (s, 1H), 7.38 (s, 2H), 6.81-7.13 (m, 3H), 4.62-5.03 (m, 2H), 3.95-4.15 (m, 2H), 3.48-3.70 (m, 3H), 2.59 (s, 3H), 1.63-2.07 (m, 3H), 1.25-1.39 (m, 3H). 19F NMR (376.5 MHz, DMSO-d6) Shift -116.92, -118.98. LC-MS: Rt =2.382 min, (ESI) *m*/*z.* [M+H]⁺ 489.2; |
| | C₂₆H₂₁F₄N₉O | | |
| | | | C₂₅H₂₅FN₈O₂ |
| 169 | 1H NMR (400 MHz, DMSO-d6) Shift 8.98 (s, 1H), 8.95 (s, 1H), 8.26 (dd, J=2.01, 8.28 Hz, 1H), 8.16 (d, J=6.27 Hz, 1H), 7.71 (d, J=8.28 Hz, 1H), 7.62 (s, 1H), 7.01 (br s, 2H), 6.94 (d, J=11.04 Hz, 1H), 4.89-5.10 (m, 2H), 3.73-4.00 (m, 2H), 2.60 (s, 3H), 1.83-2.10 (m, 3H), 0.95-1.30 (m, 3H). 19F NMR (376.5 MHz, DMSO-d6) Shift -60.72, -60.80, - 116.74, -118.95. LC-MS: Rt =1.609 min, (ESI) *m*/*z.* [M+H]⁺ 527.3; | 170 | 1H NMR (400 MHz, DMSO-d6) 8.99 (s, 1H), 8.64 (d, J=2.13 Hz, 1H), 8.19 (d, J=6.38 Hz, 1H), 7.95 (br d, J=7.00 Hz, 1H), 7.63 (d, J=8.63 Hz, 1H), 7.59 (s, 1H), 7.03 (br s, 2H), 6.95 (d, J=11.01 Hz, 1H), 5.02 (s, 2H), 3.49-3.58 (m, 3H), 2.57-2.65 (m, 3H), 1.80 (s, 3H). 19F NMR (376 MHz, DMSO-d6) -57.23 (s, 3F), -116.82 (br s, 1F). LC-MS: Rt =1.069 min, (ESI) *m*/*z.* [M+H]⁺ 529.1; |
| | C₂₅H₂₂F₄N₈O | | C₂₄H₂₀F₄N₈O₂ |
| 171 | 1H NMR (400 MHz, DMSO-d6) 8.29 (d, J=2.63 Hz, 1H), 7.87 (d, J=6.50 Hz, 1H), 7.80 (s, 1H), 7.57 (s, 1H), 7.53 (dd, J=2.81, 8.57 Hz, 1H), 7.45 (s, 2H), 7.39 (d, J=8.63 Hz, 1H), 7.05 (d, J=11.13 Hz, 1H), 4.96 (s, 2H), 3.95 (td, J=3.02, 5.85 Hz, 1H), 3.81 (q, J=7.25 Hz, 2H), 2.85 (s, 3H), 1.78 (s, 3H), 1.03 (t, J=7.19 Hz, 3H), 0.75-0.85 (m, 2H), 0.61-0.73 (m, 2H). 19F NMR (376 MHz, DMSO-d6) -117.25 (br s, 1F), -119.64 (br s, 1F). LC-MS: Rt =1.006 min, (ESI) *m*/*z.* [M+H]⁺ 515.2; | 172 | 1H NMR (400 MHz, DMSO) Shift 9.26 (s, 1H), 7.84-7.96 (m, 2H), 7.80 (s, 1H), 7.64 (s, 1H), 7.48 (s, 2H), 7.42 (dd, J=1.21, 9.35 Hz, 1H), 7.06 (d, J=11.22 Hz, 1H), 6.78 (s, 1H), 5.10 (s, 2H), 3.53 (s, 3H), 2.84 (s, 3H), 1.77 (s, 3H). LC-MS: Rt =1.113 min, (ESI) *m*/*z.* [M+H]⁺ 552.1; |
| | | | C₂₆H₂₁F₄N₉O |
| | C₂₇H₂₇FN₈O₂ | | |
| 173 | 1H NMR (400 MHz, DMSO-d6) 8.94 (s, 1H), 8.24 (br d, J=6.60 Hz, 1H), 7.87 (d, J=6.38 Hz, 1H), 7.79 (s, 1H), 7.69 (d, J=8.36 Hz, 1H), 7.48 (s, 1H), 7.44 (br s, 2H), 7.04 (d, J=11.22 Hz, 1H), 5.99 (br t, J=54.91 Hz, 1H), 5.15 (s, 2H), 4.25-4.36 (m, 2H), 2.84 (s, 3H), 1.92 (s, 3H). 19F NMR (376 MHz, DMSO-d6) -60.75 (s, 3F), -117.50 (s, 1F), - 123.23 (s, 2F). LC-MS: Rt =1.114 min, (ESI) *m*/*z.* [M+H]⁺ 563.2; | 174 | 1H NMR (400 MHz, DMSO-d6) Shift 10.05 (s, 1H), 8.80 (s, 1H), 8.37 (d, J=7.04 Hz, 1H), 8.07 (br d, J=6.38 Hz, 1H), 7.86 (s, 1H), 7.64 (s, 2H), 7.38 (d, J=8.36 Hz, 1H), 7.24 (d, J=11.88 Hz, 1H), 3.95 (s, 2H), 3.62 (s, 3H), 2.95 (s, 3H), 2.06 (s, 3H). LC-MS: Rt =1.019 min, (ESI) *m*/*z.* [M+H]⁺ 513.2; |
| | | | C₂₄H₂₀F₄N₈O |
| | C25H20F6N8O | | |
| 175 | 1H NMR (400 MHz, DMSO-d6) Shift 8.94 (s, 1H), 8.27 (d, J=7.78 Hz, 1H), 7.86-8.02 (m, 2H), 7.81 (s, 1H), 7.73 (d, J=8.28 Hz, 1H), 7.50 (s, 2H), 7.08 (d, J=11.04 Hz, 1H), 5.11 (s, 2H), 3.62 (s, 3H), 2.86 (s, 3H). 19F NMR (376.5 MHz, DMSO-d6) Shift -60.71, -116.80 LC-MS: Rt =1.451 min, (ESI) *m*/*z.* [M+H]⁺ 533.1; | 176 | 1H NMR (400 MHz, DMSO-d6) 8.93 (s, 1H), 8.21 (br d, J=6.75 Hz, 1H), 7.62-7.76 (m, 2H), 7.43-7.57 (m, 2H), 7.38 (br d, J=8.63 Hz, 1H), 6.70 (s, 2H), 5.19 (br s, 2H), 5.08 (br s, 2H), 4.98 (br s, 2H), 4.22 (td, J=6.50, 13.26 Hz, 1H), 1.74 (br s, 3H), 1.21 (br d, J=6.63 Hz, 6H). 19F NMR (376 MHz, DMSO-d6) -60.71 (s, 3F). LC-MS: Rt =0.74 min, (ESI) *m*/*z.* [M+H]⁺ 511.3; |
| | C₂₃H₁₇ClF₄N₈O | | |
| | | | C₂₆H₂₅F₃N₆O₂ |
| 177 | 1H NMR (400 MHz, DMSO-d6) 8.99 (s, 1H), 8.29 (d, J=2.63 Hz, 1H), 8.15 (d, J=6.13 Hz, 1H), 7.48-7.58 (m, 2H), 7.41 (d, J=8.63 Hz, 1H), 6.89-7.19 (m, 3H), 4.93 (s, 2H), 3.91-4.02 (m, 1H), 3.79 (q, J=7.13 Hz, 2H), 2.60 (s, 3H), 1.76 (s, 3H), 1.03 (t, J=7.19 Hz, 3H), 0.73-0.89 (m, 2H), 0.69 (br s, 2H). 19F NMR (376 MHz, DMSO-d6) - 116.89 (br s, 1F), -118.95 (br s, 1F), -119.08--118.85 (m, 1F). LC-MS: | 178 | 1H NMR (400 MHz, DMSO-d6) Shift 8.94 (s, 1H), 8.18-8.32 (m, 1H), 8.09 (s, 1H), 7.87-7.96 (m, 1H), 7.81 (s, 1H), 7.72 (d, J=8.28 Hz, 1H), 7.50 (s, 2H), 7.06 (d, J=11.04 Hz, 1H), 4.45-5.60 (m, 2H), 3.69-3.89 (m, 3H), 2.71-2.92 (m, 3H). 19F NMR (376.5 MHz, DMSO-d6) Shift -60.00, -60.65, - 60.75, -80.81, -116.77, -119.19. LC-MS: Rt =1.568 min, (ESI) *m*/*z.* [M+H]⁺ 567.1; |
| | Rt =1.002 min, (ESI) *m*/*z.* [M+H]⁺ 515.2; | | C₂₄H₁₇F₇N₈O |
| | C₂₇H₂₇FN₈O₂ | | |
| 179 | 1H NMR (400 MHz, DMSO-d6) Shift 8.92 (s, 1H), 8.28 (br d, J=7.04 Hz, 1H), 7.73-7.95 (m, 3H), 7.53 (s, 2H), 7.26 (s, 1H), 7.14 (d, J=11.44 Hz, 1H), 5.17 (s, 2H), 3.62 (s, 3H), 2.78 (s, 3H), 1.59 (s, 3H). 19F NMR (376 MHz, DMSO-d6) Shift -60.71 (br s, 3F), -118.03 (br s, 1F). LC-MS: Rt =1.136 min, (ESI) *m*/*z.* [M+H]⁺ 513.3; | 180 | 1H NMR (400 MHz, DMSO-d6) 8.95 (br s, 1H), 8.26 (br d, J=7.75 Hz, 1H), 7.92 (br d, J=6.25 Hz, 1H), 7.80 (s, 1H), 7.62-7.75 (m, 2H), 7.46 (br s, 2H), 7.05 (br d, J=11.01 Hz, 1H), 5.88 (br s, 1H), 5.13 (br s, 2H), 4.31 (br t, J=13.63 Hz, 2H), 2.87 (s, 3H), 1.92 (s, 3H). LC-MS: Rt =1.114 min, (ESI) *m*/*z.* [M+H]⁺ 563.1; |
| | C₂₄H₂₀F₄N₈O | | C₂₅H₂₀F₆N₈O |
| 181 | 1H NMR (400 MHz, DMSO-d6) 8.95 (s, 1H), 8.25 (br d, J=8.60 Hz, 1H), 8.15 (s, 1H), 7.75 (s, 2H), 7.56-7.72 (m, 2H), 7.23 (br d, J=4.84 Hz, 3H), 5.14 (s, 2H), 3.51-3.70 (m, 3H), 2.79 (s, 3H), 2.38-2.44 (m, 3H), 1.83 (s, 3H). 19F NMR (376 MHz, DMSO-d6) -60.68 (br s, 3F). LC-MS: Rt =1.066 min, (ESI) *m*/*z.* [M+H]⁺ 509.1; | 182 | 1H NMR (400 MHz, DMSO-d6) Shift 8.71 (s, 1H), 8.03 (br d, J=7.53 Hz, 1H), 7.93 (d, J=8.03 Hz, 1H), 7.90 (d, J=6.53 Hz, 1H), 7.79 (s, 1H), 7.65 (s, 1H), 7.47 (s, 2H), 7.05 (d, J=11.04 Hz, 1H), 5.05 (s, 2H), 3.54 (s, 3H), 2.84 (s, 3H), 1.74 (s, 3H). 19F NMR (376.5 MHz, DMSO-d6) Shift - 66.24, -117.39. LC-MS: Rt=1.336 min, (ESI) *m*/*z.* [M+H]⁺ 513.1; |
| | C₂₅H₂₃F₃N₈O | | C₂₄H₂₀F₄N₈O |
| 183 | 1H NMR (400 MHz, DMSO-d6) Shift 8.93 (s, 1H), 8.47 (s, 1H), 8.13-8.29 (m, 1H), 7.97 (s, 1H), 7.84 (s, 1H), 7.72 (br d, J=8.25 Hz, 1H), 7.59 (br d, J=6.63 Hz, 3H), 5.10 (s, 2H), 3.54 (s, 3H), 2.86 (s, 3H), 1.81 (s, 3H). 19F NMR (376 MHz, DMSO-d6) Shift -60.70 (s, 3F). LC-MS: Rt =0.996 min, (ESI) *m*/*z.* [M+H]⁺ 496.1; | 184 | 1H NMR (400 MHz, DMSO-d6) Shift 8.91-9.07 (m, 2H), 8.13-8.33 (m, 2H), 7.61-7.80 (m, 2H), 6.86-7.23 (m, 3H), 5.07 (br s, 2H), 3.51-3.76 (m, 3H), 2.60 (s, 2.8H), 2.22 (q, J=7.48 Hz, 2H), 1.85 (s, 0.2H), 0.87 (t, J=7.59 Hz, 3H). 19F NMR (376 MHz, DMSO-d6) Shift -60.72 (br s, 1F), -116.75 (br s, 1F). LC-MS: Rt =1.022 min, (ESI) *m*/*z.* [M+H]⁺ 527.4; |
| | C₂₃H₂₀F₃N₉O | | C₂₅H₂₂F₄N₈O |
| 187 | 1H NMR (400 MHz, DMSO-d6) Shift 8.78-9.15 (m, 1H), 7.99-8.36 (m, 1H), 7.44-7.95 (m, 6H), 6.88-7.37 (m, 2H), 4.79-5.56 (m, 2H), 2.84 (br s, 3H), 2.10-2.47 (m, 6H). LC-MS: Rt =1.117 min, (ESI) *m*/*z.* [M+H]⁺ 524.3; | 188 | 1H NMR (400 MHz, DMSO-d6) 8.95 (s, 1H), 8.25 (br d, J=8.60 Hz, 1H), 8.15 (s, 1H), 7.75 (s, 2H), 7.56-7.72 (m, 2H), 7.23 (br d, J=4.84 Hz, 3H), 5.14 (s, 2H), 3.51-3.70 (m, 3H), 2.79 (s, 3H), 2.38-2.44 (m, 3H), 1.83 (s, 3H). 19F NMR (376 MHz, DMSO-d6) -60.68 (br s, 3F). LC-MS: Rt =1.066 min, (ESI) *m*/*z.* [M+H]⁺ 509.1; |
| | C₂₆H₂₁F₄N₇O | | |
| | | | C₂₅H₂₃F₃N₈O |
| 189 | 1H NMR (400 MHz, DMSO) Shift 8.45 (s, 1H), 8.28 (br d, J=2.25 Hz, 1H), 7.93 (s, 1H), 7.84 (s, 1H), 7.56 (br s, 2H), 7.23-7.53 (m, 3H), 4.94 (s, 2H), 3.94 (br s, 1H), 3.54 (s, 3H), 2.86 (s, 3H), 1.73 (s, 3H), 0.80 (br d, J=5.75 Hz, 2H), 0.68 (br s, 2H). LC-MS: Rt =2.187 min, (ESI) *m*/*z.* [M+H]⁺ 484.2; | 190 | 1H NMR (400 MHz, DMSO-d6) Shift 8.92 (s, 1H), 8.20 (dd, J=1.87, 8.25 Hz, 1H), 7.92 (s, 1H), 7.65-7.77 (m, 2H), 7.43-7.51 (m, 1H), 7.29 (d, J=8.14 Hz, 1H), 6.81 (br s, 2H), 5.10 (s, 2H), 3.59-3.74 (m, 3H), 2.63 (s, 3H), 2.55 (s, 3H), 1.73 (br s, 3H). LC-MS: Rt =1.062 min, (ESI) *m*/*z.* [M+H]⁺ 509.3; |
| | C₂₅H₂₅N₉O | | C₂₅H₂₃F₃N₈O |
| 191 | 1H NMR (400 MHz, DMSO-d6) Shift 12.20-12.38 (m, 1H), 8.75-8.97 (m, 1H), 8.14-8.65 (m, 1H), 7.56-7.67 (m, 2H), 7.35-7.54 (m, 1H), 7.12-7.22 (m, 2H), 7.03 (br s, 2H), 6.95 (d, J=11.04 Hz, 1H), 4.82-5.13 (m, 2H), 3.51-3.70 (m, 3H), 2.59-2.70 (m, 3H), 1.75-2.06 (m, 3H). 19F NMR (376.5 MHz, DMSO-d6) Shift -116.39, -118.62 LC-MS: Rt =0.790 min, (ESI) *m*/*z.* [M+H]⁺ 484.1; | 192 | 1H NMR (400 MHz, DMSO) Shift 8.93 (br s, 1H), 8.46 (s, 1H), 8.24 (br d, J=7.50 Hz, 1H), 7.95 (s, 1H), 7.84 (s, 1H), 7.71 (br d, J=8.13 Hz, 1H), 7.49-7.67 (m, 3H), 5.11 (br s, 2H), 3.71-4.12 (m, 2H), 2.86 (s, 3H), 1.77-2.12 (m, 3H), 0.98-1.36 (m, 3H). LC-MS: Rt =1.034 min, (ESI) *m*/*z.* [M+H]⁺ 510.1; |
| | | | C₂₄H₂₂F₃N₉O |
| | C₂₅H₂₂FN₉O | | |
| 193 | 1H NMR (400 MHz, DMSO-d6) Shift 8.97 (s, 1H), 8.33 (d, J=8.76 Hz, 1H), 8.14-8.23 (m, 1H), 8.06 (d, J=8.75 Hz, 1H), 7.63 (s, 1H), 7.03 (br s, 2H), 6.96 (d, J=11.01 Hz, 1H), 5.31 (s, 2H), 3.53 (s, 3H), 2.60 (s, 3H), 1.86 (s, 3H). 19F NMR (376 MHz, DMSO-d6) Shift 65.59 (s, 3F), 116.72 (s, 1F). LC-MS: Rt =2.674 min, (ESI) *m*/*z.* [M+H]⁺ 514.2; | 194 | 1H NMR (400 MHz, DMSO-d6) Shift 8.84-9.07 (m, 1H), 8.17-8.30 (m, 2H), 7.72-8.16 (m, 3H), 7.49 (s, 2H), 6.90-7.30 (m, 2H), 5.14-5.52 (m, 1H), 4.87 (d, J=15.63 Hz, 1H), 2.84 (s, 3H), 2.18-2.41 (m, 6H). 19F NMR (376.5 MHz, DMSO-d6) Shift -60.71, -60.83, - 116.80, -119.12. LC-MS: Rt =1.248 min, (ESI) *m*/*z.* [M+H]⁺ 524.1; |
| | C₂₃H₁₉F₄N₉O | | C₂₆H₂₁F₄N₇O |
| 195 | 1H NMR (400 MHz, DMSO-d6) Shift 8.98 (s, 1H), 8.17 (d, J=6.38 Hz, 1H), 8.11 (d, J=2.88 Hz, 1H), 7.50 (s, 1H), 7.39 (d, J=8.63 Hz, 1H), 7.17-7.29 (m, 1H), 6.96-7.15 (m, 2H), 6.93 (d, J=11.01 Hz, 1H), 5.36 (t, J=5.44 Hz, 1H), 4.74-5.05 (m, 4H), 4.56 (dd, J=5.00, 7.13 Hz, 2H), 3.51 (s, 3H), 2.59 (s, 3H), 1.76 (s, 3H). LC-MS: Rt =2.404 min, (ESI) *m*/*z.* [M+H]⁺ 517.2; | 196 | 1H NMR (400 MHz, DMSO-d6) Shift 8.90-9.03 (m, 1H), 8.22-8.28 (m, 1H), 8.01-8.11 (m, 1H), 7.57-7.84 (m, 4H), 7.51 (s, 2H), 7.02-7.35 (m, 1H), 4.89-5.37 (m, 2H), 2.75-2.85 (m, 3H), 2.20-2.27 (m, 3H), 2.15 (s, 3H). LC-MS: Rt =1.670 min, (ESI) *m*/*z.* [M+H]⁺ 524.3; |
| | | | C₂₆H₂₁F₄N₇O |
| | C₂₆H₂₅FN₈O₃ | | |
| 197 | 1H NMR (400 MHz, DMSO-d6) Shift 8.96 (s, 1H), 8.18 (d, J=6.50 Hz, 1H), 7.67 (d, J=9.13 Hz, 1H), 7.50-7.58 (m, 1H), 7.17-7.32 (m, 1H), 7.02 (br s, 2H), 6.94 (d, J=11.01 Hz, 1H), 4.86-5.18 (m, 2H), 3.96-4.04 (m, 3H), 3.52-3.69 (m, 3H), 2.59 (s, 3H), 1.74-1.89 (m, 3H). 19F NMR (376.5 MHz, DMSO-d6) Shift -116.94, -118.89. LC-MS: Rt =0.863 min, (ESI) *m*/*z.* [M+H]⁺ 476.2; | 198 | 1H NMR (400 MHz, DMSO-d6) Shift 10.10 (s, 1H), 9.05 (s, 1H), 8.81 (s, 1H), 8.45 (d, J=7.04 Hz, 1H), 8.18 (s, 1H), 8.07 (dd, J=1.98, 8.14 Hz, 1H), 7.37 (d, J=8.14 Hz, 1H), 7.18 (br d, J=11.88 Hz, 2H), 3.94 (s, 2H), 3.62 (s, 3H), 2.62 (s, 3H), 2.02 (s, 3H). LC-MS: Rt =1.014 min, (ESI) *m*/*z.* [M+H]⁺ 513.3; |
| | | | C₂₄H₂₀F₄N₈O |
| | C₂₃H₂₂FN₉₀ | | |
| 199 | 1H NMR (400 MHz, DMSO) Shift 8.45 (s, 1H), 8.28 (br d, J=2.42 Hz, 1H), 7.91 (s, 1H), 7.83 (s, 1H), 7.17-7.70 (m, 5H), 4.95 (s, 2H), 3.68-4.09 (m, 3H), 2.86 (s, 3H), 2.54 (s, 1H), 1.75 (s, 3H), 0.95-1.34 (m, 3H), 0.72-0.87 (m, 2H), 0.52-0.72 (m, 2H). LC-MS: Rt =2.229 min, (ESI) *m*/*z.* [M+H]⁺ 498.2; | 200 | 1H NMR (400 MHz, DMSO-d6) Shift 8.97 (br s, 1H), 8.08-8.34 (m, 1H), 7.95 (br d, J=6.13 Hz, 1H), 7.81 (s, 1H), 7.71 (br d, J=8.00 Hz, 1H), 7.53 (s, 2H), 7.21 (br s, 1H), 7.09 (br d, J=11.13 Hz, 1H), 6.15 (br s, 1H), 4.94-5.48 (m, 2H), 3.63 (s, 3H), 2.86 (s, 3H). 19F NMR (376 MHz, DMSO-d6) Shift -60.74, -117.78. LC-MS: Rt =2.652 min, (ESI) *m*/*z.* [M+H]⁺ 499.2; |
| | C₂₆H₂₇N₉O₂ | | |
| | | | C₂₃H₁₈F₄N₈O |
| 201 | 1H NMR (400 MHz, DMSO-d6) 8.96 (s, 1H), 8.29 (br d, J=6.88 Hz, 1H), 7.94 (br d, J=6.13 Hz, 1H), 7.82 (s, 1H), 7.75 (br d, J=8.25 Hz, 1H), 7.58 (s, 2H), 7.31 (br d, J=4.00 Hz, 1H), 7.12 (br d, J=11.26 Hz, 1H), 5.40 (br d, J=15.63 Hz, 1H), 5.03 (br d, J=16.01 Hz, 1H), 3.72 (s, 3H), 2.85 (s, 3H). 19F NMR (376 MHz, DMSO-d6) -60.76 (s, 3F), - 118.22 (s, 1F), -173.29 (s, 1F). LC-MS: Rt =1.117 min, (ESI) *m*/*z.* [M+H]⁺ 517.2; | 202 | 1H NMR (400 MHz, DMSO-d6) Shift 8.31 (d, J=8.80 Hz, 1H), 8.07 (d, J=8.80 Hz, 1H), 7.91 (d, J=6.38 Hz, 1H), 7.80 (s, 1H), 7.69 (s, 1H), 7.47 (s, 2H), 7.06 (d, J=11.22 Hz, 1H), 5.33 (s, 2H), 3.82 (q, J=7.12 Hz, 2H), 2.84 (s, 3H), 1.86 (s, 3H), 1.05 (t, J=7.15 Hz, 3H) 19F NMR (376 MHz, DMSO-d6) -60.73 (s, 3F), - 117.41 (s, 1F), -123.65 (s, 2F). LC-MS: Rt =0.879 min, (ESI) *m*/*z.* [M+H]⁺ 528.3; |
| | C₂₃H₁₇F₅N₈O | | C₂₄H₂₁F₄N₉O |
| 203 | 1H NMR (400 MHz, DMSO-d6) Shift 8.40 (s, 1H), 7.89 (d, J=6.38 Hz, 1H), 7.80 (s, 1H), 7.75 (s, 1H), 7.52 (s, 2H), 7.07 (d, J=11.22 Hz, 1H), 5.23 (s, 2H), 3.58 (s, 3H), 2.82 (s, 3H), 1.78 (s, 3H) 19F NMR (376.5 MHz, DMSO-d6) Shift -52.94, -117.27. LC-MS: Rt =2.469 min, (ESI) *m*/*z.* [M+H]+ 519.0; | 204 | 1H NMR (400 MHz, DMSO-d6) 8.91 (br s, 1H), 8.23 (br d, J=8.13 Hz, 1H), 7.97 (br d, J=6.75 Hz, 1H), 7.89 (br s, 1H), 7.81 (s, 1H), 7.34 (br s, 2H), 7.13 (br d, J=11.26 Hz, 1H), 6.50 (s, 1H), 5.17 (br s, 2H), 3.23-3.28 (m, 3H), 2.87 (s, 3H), 1.96 (br s, 3H) 19F NMR (376 MHz, DMSO-d6) -60.85 (s, 3F), -118.24 (br s, 1F). LC-MS: Rt =0.759 min, (ESI) *m*/*z.* [M+H]⁺ 513.3; |
| | C₂₂H₁₈F₄N₈OS | | |
| | | | C₂₄H₂₀F₄N₈O |
| 205 | 1H NMR (400 MHz, DMSO-d6) δ 8.31 (br s, 1H), 7.92 (br d, J=5.13 Hz, 1H), 7.80 (s, 1H), 7.50 (s, 3H), 7.39 (br d, J=8.13 Hz, 1H), 7.19 (br s, 1H), 7.08 (br d, J=10.88 Hz, 1H), 6.09 (br s, 1H), 4.98 (br s, 2H), 3.94 (br s, 1H), 3.50 (br s, 3H), 2.85 (s, 3H), 0.80 (br d, J=5.88 Hz, 2H), 0.67 (br s, 2H). FNMR: 19F NMR (376 MHz, DMSO-d6) -117.84, - 119.28. LC-MS: Rt =2.478 min, (ESI) *m*/*z.* [M+H]⁺ 487.1; | 206 | 1H NMR (400 MHz, DMSO-d6) δ 8.97 (br s, 1H), 8.25 (br d, J=7.70 Hz, 1H), 7.91-8.02 (m, 1H), 7.80 (s, 1H), 7.72 (br d, J=7.70 Hz, 1H), 7.52 (s, 2H), 7.28 (s, 1H), 7.10 (br d, J=11.22 Hz, 1H), 6.24 (br s, 1H), 4.98-5.38 (m, 2H), 3.62-4.12 (m, 2H), 2.86 (s, 3H), 1.08 (br t, J=6.82 Hz, 3H). LC-MS: Rt =0.849 min, (ESI) *m*/*z.* [M+H]⁺ 513.3; |
| | C₂₅H₂₃FN₈O₂ | | C₂₄H₂₀F₄N₈O |
| 207 | 1H NMR (400 MHz, DMSO-d6) 8.56 (br s, 1H), 7.94 (br s, 1H), 7.81 (br s, 1H), 7.76 (br s, 1H), 7.51 (br s, 3H), 7.20 (br s, 1H), 7.09 (br d, J=11.76 Hz, 1H), 6.10 (br s, 1H), 5.03 (br s, 2H), 3.52-3.55 (m, 3H), 2.86 (br s, 3H) 19F NMR (376 MHz, DMSO-d6) - 117.87 (br s, 1F), -129.22 (br s, 1F). LC-MS: Rt =0.944 min, (ESI) *m*/*z.* [M+H]⁺ 449.1; | 208 | 1H NMR (400 MHz, DMSO-d6) δ 8.83 (s, 1H), 8.13 (br d, J=9.02 Hz, 1H), 7.87 (d, J=7.04 Hz, 1H), 7.77 (s, 1H), 7.65 (d, J=8.14 Hz, 1H), 7.37 (s, 1H), 7.26 (s, 2H), 7.12 (d, J=11.22 Hz, 1H), 4.26 (s, 2H), 4.19 (s, 2H), 3.58 (s, 3H), 2.79 (s, 3H), 2.08 (s, 3H). LC-MS: Rt =0.884 min, (ESI) *m*/*z.* [M+H]⁺ 499.4; |
| | C₂₂H₁₈F₂N8O | | C₂₄H₂₂F₄N₈ |
| 209 | 1H NMR (400 MHz, DMSO-d6) 8.93 (br s, 1H), 8.22 (br s, 1H), 8.03 (br s, 1H), 7.82 (s, 1H), 7.66 (br s, 1H), 7.53 (br s, 2H), 7.13 (br s, 1H), 5.28 (br s, 2H), 3.97 (br s, 3H), 2.51 (br s, 3H) 19F NMR (376 MHz, DMSO-d6) - 60.76 (br s, 3F), -117.36 (br s, 1F). LC-MS: Rt =1.093 min, (ESI) *m*/*z.* [M+H]⁺ 500.2; | 210 | 1H NMR (400 MHz, DMSO-d6) 8.91 (br s, 1H), 8.23 (br d, J=8.13 Hz, 1H), 7.97 (br d, J=6.75 Hz, 1H), 7.89 (br s, 1H), 7.81 (s, 1H), 7.34 (br s, 2H), 7.13 (br d, J=11.26 Hz, 1H), 6.50 (s, 1H), 5.17 (br s, 2H), 3.23-3.28 (m, 3H), 2.87 (s, 3H), 1.96 (br s, 3H) 19F NMR (376 MHz, DMSO-d6) -60.85 (s, 3F), -118.24 (br s, 1F). LC-MS: Rt =0.759 min, (ESI) *m*/*z.* [M+H]⁺ 513.3; |
| | C₂₂H₁₇F₄N₉O | | |
| | | | C₂₄H₂₀F₄N₈O |
| 211 | 1H NMR (400 MHz, DMSO-d6) δ 8.95 (br s, 1H), 8.33 (s, 1H), 8.23 (br s, 2H), 7.86 (s, 1H), 7.72 (br d, J=7.78 Hz, 1H), 7.67 (s, 2H), 7.18 (br s, 1H), 5.97 (br s, 1H), 4.91-5.46 (m, 2H), 3.68 (s, 3H), 2.93 (s, 3H). 19F NMR (376 MHz, DMSO-d6) - 60.73. LC-MS: Rt =0.791 min, (ESI) *m*/*z.* [M+H]⁺ 482.3; | 212 | 1H NMR (400 MHz, DMSO-d6) Shift 8.22 (s, 1H), 7.87-7.97 (m, 1H), 7.80 (s, 1H), 7.50 (s, 2H), 6.98-7.44 (m, 4H), 5.91-6.13 (m, 1H), 4.70-5.30 (m, 2H), 4.08 (q, J=6.75 Hz, 2H), 3.45-3.73 (m, 3H), 2.85 (s, 3H), 1.33 (t, J=6.94 Hz, 3H). 19F NMR (376.5 MHz, DMSO-d6) Shift -117.889, - 119.349. LC-MS: Rt =1.573 min, (ESI) *m*/*z.* [M+H]⁺ 475.3; |
| | C₂₂H₁₈F₃N₉O | | |
| | | | C₂₄H₂₃FN₈O₂ |
| 213 | 1H NMR (400 MHz, DMSO-d6) Shift 8.39 (s, 1H), 8.17 (s, 0.2H), 7.84-8.07 (m, 2H), 7.80 (s, 1H), 7.52 (s, 2H), 7.23 (br s, 1H), 7.06 (br d, J=11.01 Hz, 1H), 6.06 (br s, 1H), 4.49-5.28 (m, 2H), 3.48-3.74 (m, 3H), 2.87 (s, 3H) 19F NMR (376 MHz, DMSO-d6) Shift -59.21 (s, 3F), -118.10 (br s, 1F). LC-MS: Rt =0.767 min, (ESI) *m*/*z.* [M+H]⁺ 488.3; | 214 | 1H NMR (400 MHz, DMSO-d6) 8.39 (br s, 1H), 7.93 (br s, 1H), 7.81 (s, 1H), 7.62 (br s, 1H), 7.51 (s, 2H), 7.35 (br d, J=7.63 Hz, 1H), 7.19 (br s, 1H), 7.09 (br d, J=11.76 Hz, 1H), 6.09 (br s, 1H), 4.71-5.26 (m, 2H), 3.53 (br s, 3H), 2.86 (s, 3H), 2.27-2.32 (m, 3H). 19F NMR (376 MHz, DMSO-d6) -117.93 (br s, 1F). LC-MS: Rt =0.858 min, (ESI) *m*/*z.* [M+H]⁺ 445.2; |
| | C₂₁H₁₇F₄N₉O | | C₂₃H₂₁FN₈O |
| 215 | 1H NMR (400 MHz, DMSO-d6) δ 7.95 (br s, 1H), 7.80 (s, 1H), 7.70 (br d, J=7.88 Hz, 1H), 7.52 (s, 2H), 7.28 (br d, J=8.13 Hz, 1H), 7.21 (br s, 1H), 7.08 (br d, J=11.13 Hz, 1H), 6.09 (s, 1H), 5.06 (br s, 2H), 4.39 (td, J=3.08, 5.97 Hz, 1H), 3.58 (br s, 3H), 2.86 (s, 3H), 0.80-0.86 (m, 2H), 0.73 (br s, 2H). LC-MS: Rt =0.701 min, (ESI) *m*/*z.* [M+H]⁺ 488.3; | 216 | 1H NMR (400 MHz, DMSO-d6) δ 9.23 (br s, 1H), 7.87-8.56 (m, 2H), 7.68-7.84 (m, 2H), 7.43-7.55 (m, 3H), 7.01-7.37 (m, 2H), 6.17 (br s, 1H), 4.84-5.28 (m, 2H), 3.45-3.83 (m, 3H), 2.86 (br s, 3H). LC-MS: Rt =0.811 min, (ESI) *m*/*z.* [M+H]⁺ 538.3; |
| | | | C₂₅H₁₉F₄N₉O |
| | C₂₄H₂₂FN₉O₂ | | |
| 217 | 1H NMR (400 MHz, DMSO-d6) Shift 8.15 (br s, 1H), 7.91 (br d, J=5.25 Hz, 1H), 7.80 (s, 1H), 7.51 (s, 2H), 7.37 (br d, J=8.00 Hz, 1H), 7.14-7.29 (m, 2H), 7.08 (br d, J=10.88 Hz, 1H), 6.08 (br s, 1H), 5.34 (br d, J=4.75 Hz, 1H), 4.93 (br t, J=6.57 Hz, 4H), 4.45-4.63 (m, 2H), 3.50 (br s, 3H), 2.85 (s, 3H). 19F NMR (376 MHz, DMSO-d6) Shift -117.87 (br s, 1F). LC-MS: Rt =1.33 min, (ESI) *m*/*z.* [M+H]⁺ 503.1; | 218 | 1H NMR (400 MHz, DMSO-d6) δ 9.24 (br s, 1H), 8.22 (s, 1H), 7.95-8.16 (m, 2H), 7.74 (br d, J=9.02 Hz, 1H), 7.47 (br d, J=9.90 Hz, 1H), 6.92-7.38 (m, 4H), 6.19 (br s, 1H), 5.14 (br s, 2H), 4.29 (br s, 3H), 3.48 (br s, 3H).. LC-MS: Rt =0.786 min, (ESI) *m*/*z.* [M+H]⁺ 538.3; |
| | | | C₂₅H₁₉F₄N₉O |
| | C₂₅H₂₃FN₈O | | |
| 219 | 1H NMR (400 MHz, DMSO-d6) Shift 7.68-8.07 (m, 3H), 7.50 (s, 2H), 6.84-7.38 (m, 4H), 6.08 (br s, 1H), 4.64-5.18 (m, 2H), 4.35 (br t, J=12.32 Hz, 4H), 3.46-3.67 (m, 3H), 2.75-2.99 (m, 3H). 19F NMR (376 MHz, DMSO-d6) Shift -98.30 (s, 2F), -117.91 (br s, 1F). LC-MS: Rt =0.759 min, (ESI) *m*/*z.* [M+H]⁺ 522.3; | 220 | 1H NMR (400 MHz, DMSO-d6) 9.28 (br s, 1H), 8.10 (br s, 1H), 7.92 (br s, 1H), 7.82 (s, 1H), 7.73 (d, J=9.76 Hz, 1H), 7.55 (s, 2H), 7.47 (br d, J=8.63 Hz, 1H), 7.29 (br s, 1H), 7.11 (br d, J=10.38 Hz, 1H), 5.19 (br s, 1H), 5.04-5.14 (m, 1H), 3.59 (s, 3H), 2.86 (br s, 3H). 19F NMR (376 MHz, DMSO-d6) -60.47 (s, 3F), -118.15 (s, 1F), - 173.54 (s, 1F). LC-MS: Rt =1.961 min, (ESI) *m*/*z.* [M+H]⁺ 556.1; |
| | C₂₅H₂₂F₃N₉O | | |
| | | | C₂₅H₁₈F₅N₉O |
| 221 | 1H NMR (400 MHz, DMSO-d6) Shift 7.88 (br d, J=4.88 Hz, 1H), 7.80 (s, 1H), 7.53-7.64 (m, 1H), 7.48 (br s, 2H), 7.18-7.37 (m, 2H), 7.06 (br d, J=10.63 Hz, 1H), 6.11 (br s, 1H), 4.82 (br s, 2H), 4.21 (br t, J=5.00 Hz, 2H), 3.62 (br t, J=5.07 Hz, 2H), 3.40-3.48 (m, 3H), 3.19 (s, 3H), 2.86 (s, 3H). 19F NMR (376 MHz, DMSO-d6) Shift -117.97 (br s, 1F). LC-MS: Rt =0.613 min, (ESI) *m*/*z.* [M+H]⁺ 478.4; | 222 | 1H NMR (400 MHz, DMSO-d6) δ 9.23 (br s, 1H), 7.86-8.62 (m, 2H), 7.71-7.84 (m, 2H), 7.40-7.64 (m, 3H), 7.28 (br s, 1H), 7.09 (br d, J=10.38 Hz, 1H), 6.27 (br s, 1H), 4.76-5.34 (m, 2H), 3.47-4.17 (m, 2H), 2.85 (br s, 3H), 0.90-1.26 (m, 3H). LC-MS: Rt =0.801 min, (ESI) *m*/*z.* [M+H]⁺ 552.3; |
| | C₂₃H₂₄FN₉O₂ | | C₂₆H₂₁F₄N₉O |
| 223 | 1H NMR (400 MHz, DMSO-d6) Shift 8.24-8.47 (m, 1H), 8.05-8.19 (m, 1H), 7.98 (br s, 1H), 7.81 (s, 1H), 7.51 (s, 2H), 7.24 (br s, 1H), 7.10 (br d, J=11.26 Hz, 1H), 6.17 (s, 1H), 5.33 (br s, 2H), 3.68 (br s, 3H), 2.86 (s, 3H), 2.08 (s, 1H).19F NMR (376 MHz, DMSO-d6) Shift -65.62 (br s, 1F), -117.70 (br s, 1F). LC-MS: Rt =0.721 min, (ESI) *m*/*z.* [M+H]⁺ 500.1; | 224 | 1H NMR (400 MHz, DMSO-d6) Shift 8.52 (br d, J=4.63 Hz, 1H), 7.62-8.03 (m, 3H), 7.44-7.59 (m, 3H), 7.02-7.39 (m, 3H), 6.72-6.99 (m, 1H), 6.18 (br s, 1H), 4.67-5.25 (m, 2H), 3.44-3.79 (m, 3H), 2.87 (br s, 3H). 19F NMR (376 MHz, DMSO-d6) Shift -120.00--116.92 (m, 1F). LC-MS: Rt =0.645 min, (ESI) *m*/*z.* [M+H]⁺ 470.3; |
| | C₂₂H₁₇F₄N₉O | | C₂₄H₂₀FN₉O |
| 225 | 1H NMR (400 MHz, DMSO-d6) δ 8.43 (br s, 1H), 7.76-7.97 (m, 1H), 7.72-8.01 (m, 2H), 7.39-7.66 (m, 3H), 7.03-7.36 (m, 3H), 6.17 (br s, 1H), 4.77-5.16 (m, 2H), 3.57 (br t, J=4.29 Hz, 4H), 3.37-3.50 (m, 6H), 2.86 (br s, 2H), 2.31-2.39 (m, 4H). LC-MS: Rt =0.614 min, (ESI) *m*/*z.* [M+H]⁺ 569.4; | 226 | 1H NMR (400 MHz, DMSO-d6) 7.77-7.93 (m, 2H), 7.49 (br s, 4H), 7.20 (br s, 1H), 7.03 (br d, J=11.38 Hz, 1H), 6.15 (s, 1H), 4.83 (br s, 2H), 3.60 (br s, 3H), 3.41-3.45 (m, 3H), 2.78-2.93 (m, 3H). 19F NMR (376 MHz, DMSO-d6) -117.85 (br s, 1F). LC-MS: Rt =1.155 min, (ESI) *m*/*z.* [M+H]+ 434.1; |
| | C₂₉H₂₉FN₁₀O₂ | | C₂₁H₂₀FN₉O |
| 227 | 1H NMR (400 MHz, DMSO-d6) δ 7.88 (br d, J=5.94 Hz, 1H), 7.79 (s, 1H), 7.64 (br s, 1H), 7.49 (s, 2H), 7.33 (br s, 1H), 7.23 (br s, 1H), 7.05 (br d, J=10.12 Hz, 1H), 6.10 (br s, 1H), 4.82 (br s, 2H), 4.35 (br s, 1H), 3.92 (br d, J=11.66 Hz, 2H), 3.35-3.48 (m, 6H), 2.85 (s, 3H), 1.88 (br s, 3H). LC-MS: Rt =0.746 min, (ESI) *m*/*z.* [M+H]⁺ 504.2; | 228 | 1H NMR (400 MHz, DMSO-d6) Shift 8.89 (s, 1H), 8.27-8.41 (m, 1H), 7.92 (br d, J=6.38 Hz, 1H), 7.80 (s, 1H), 7.52 (s, 2H), 7.23 (s, 1H), 7.09 (br d, J=11.26 Hz, 1H), 6.19 (s, 1H), 5.02-5.53 (m, 2H), 3.66 (s, 3H), 2.85 (s, 3H).19F NMR (376 MHz, DMSO-d6) Shift -60.46 (s, 3F), -117.87 (br s, 1F), -123.90 (br s, 1F). LC-MS: Rt =1.630 min, (ESI) *m*/*z.* [M+H]⁺ 517.1; |
| | C₂₅H₂₆FN₉O₂ | | |
| | | | C₂₃H₁₇F₅N₈O |
| 229 | 1H NMR (400 MHz, DMSO-d6) δ 7.89-7.96 (m, 3H), 7.80 (s, 1H), 7.57 (br d, J=8.14 Hz, 2H), 7.52 (s, 2H), 7.20 (s, 1H), 7.07 (br d, J=11.00 Hz, 1H), 6.05 (s, 1H), 4.73-5.47 (m, 2H), 3.55 (s, 3H), 2.86 (s, 3H). LC-MS: Rt =0.903 min, (ESI) *m*/*z.* [M+H]⁺ 556.2; | 230 | 1H NMR (400 MHz, DMSO-d6) 9.10 (s, 1H), 8.39 (s, 2H), 7.75 (s, 1H), 7.59 (br d, J=7.00 Hz, 1H), 7.33 (s, 2H), 7.17 (d, J=11.51 Hz, 1H), 6.01 (s, 2H), 2.53 (br s, 3H), 2.47 (s, 3H). 19F NMR (376 MHz, DMSO-d6) -61.07 (s, 3F), -121.32 (s, 1F). LC-MS: Rt =1.532 min, (ESI) *m*/*z.* [M+H]⁺ 457.2; |
| | C₂₃H₁₉F₆N₇OS | | C₂₁H₁₆F₄N₈ |
| 231 | 1H NMR (400 MHz, DMSO-d6) 7.90 (br s, 1H), 7.80 (br s, 2H), 7.49 (br s, 3H), 7.23 (br s, 1H), 7.05 (br d, J=10.26 Hz, 1H), 6.09 (br s, 1H), 5.54 (br d, J=6.13 Hz, 1H), 4.72-4.95 (m, 6H), 3.43 (br s, 3H), 2.86 (s, 3H) 19F NMR (376 MHz, DMSO-d6) - 117.94 (s, 1F). LC-MS: Rt =1.194 min, (ESI) *m*/*z.* [M+H]⁺ 476.2; | 232 | 1H NMR (400 MHz, DMSO-d6) 9.20 (s, 1H), 8.82 (br d, J=5.13 Hz, 1H), 7.97 (br d, J=5.63 Hz, 1H), 7.81 (s, 1H), 7.62 (br d, J=4.38 Hz, 1H), 7.54 (s, 2H), 7.23 (s, 1H), 7.10 (br d, J=11.63 Hz, 1H), 6.19 (s, 1H), 5.23 (br s, 2H), 3.68 (s, 3H), 2.87 (s, 3H). 19F NMR (376 MHz, DMSO-d6) - 117.82 (s, 1F). LC-MS: Rt =1.14 min, (ESI) *m*/*z.* [M+H]⁺ 432.2; |
| | C₂₃H₂₂FN₉O₂ | | |
| | | | C₂₁H₁₈FN₉O |
| 233 | 1H NMR (400 MHz, DMSO-d6) Shift 7.74-7.86 (m, 2H), 7.47 (s, 2H), 7.26 (d, J=1.25 Hz, 1H), 7.05 (d, J=11.01 Hz, 1H), 6.22 (s, 1H), 4.09 (br s, 1H), 3.64 (s, 3H), 2.85 (s, 3H), 2.03 (br s, 2H), 1.63-1.98 (m, 6H), 1.30 (br d, J=10.88 Hz, 2H).(ES15393-1398-P1A_HNMR) 19F NMR (376 MHz, DMSO-d6) Shift -116.50 (s, 1F), -117.82 (br s, 2F). LC-MS: Rt =2.68 min, (ESI) *m*/*z.* [M+H]⁺ 472.2; | 234 | 1H NMR (400 MHz, DMSO-d6) Shift 8.53 (br s, 1H), 8.46 (br d, J=3.79 Hz, 1H), 7.96-8.03 (m, 1H), 7.91 (br t, J=7.89 Hz, 2H), 7.79 (s, 1H), 7.75 (s, 1H), 7.50 (s, 2H), 7.35 (dd, J=5.01, 6.36 Hz, 1H), 7.22 (br s, 1H), 7.05 (br d, J=11.13 Hz, 1H), 6.06 (br s, 1H), 4.69-5.17 (m, 2H), 3.54 (s, 3H), 2.86 (s, 3H).19F NMR (376 MHz, DMSO-d6) Shift -118.10 (br s, 1F). LC-MS: Rt =1.48 min, (ESI) *m*/*z.* [M+H]⁺ 497.2; |
| | C₂₃H₂₄F₃N₇O | | C₂₅H₂₁FN₁₀O |
| 235 | 1H NMR (400 MHz, DMSO-d6) δ 7.88 (br d, J=5.94 Hz, 1H), 7.79 (s, 1H), 7.64 (br s, 1H), 7.49 (s, 2H), 7.33 (br s, 1H), 7.23 (br s, 1H), 7.05 (br d, J=10.12 Hz, 1H), 6.10 (br s, 1H), 4.82 (br s, 2H), 4.35 (br s, 1H), 3.92 (br d, J=11.66 Hz, 2H), 3.35-3.48 (m, 6H), 2.85 (s, 3H), 1.88 (br s, 3H).19F NMR (376 MHz, DMSO-d6) Shift -117.82 (br s, 1F). LC-MS: Rt =0.65 min, (ESI) *m*/*z.* [M+H]⁺ 424.4; | 236 | 1H NMR (400 MHz, DMSO-d6) δ 12.99 (br s, 1H), 7.87-8.05 (m, 2H), 7.69-7.84 (m, 2H), 7.48-7.60 (m, 3H), 7.22 (br s, 1H), 6.97-7.13 (m, 1H), 6.22 (br s, 1H), 5.25 (br s, 2H), 3.54-3.66 (m, 3H), 2.87 (br s, 3H). LC-MS: Rt =0.774 min, (ESI) *m*/*z.* [M+H]⁺ 538.2; |
| | | | C₂₅H₁₉F₄N₉O |
| | C₂₁H₂₂FN₇O₂ | | |
| 237 | 1H NMR (400 MHz, DMSO-d6) δ 8.84 (s, 1H), 8.11 (s, 1H), 8.00 - 7.85 (m, 2H), 7.72 - 7.45 (m, 3H), 7.28 (s, 1H), 7.17 - 6.90 (m, 3H), 5.26 (s, 1H), 4.93 - 4.79(m, 1H), 4.48 (d, *J* = 16.0 Hz, 1H), 2.50 - 2.41 (m, 3H), 1.57 (s, 3H). LC-MS: Rt =1.233 min, (ESI) m/z. 485.2 [M+H]⁺. C₂₄H₂₀F₄N₆O | 238 | 1H NMR (400 MHz, DMSO-d6) δ 9.15-9.28 (m, 1H), 8.05 (br s, 2H), 7.68-7.89 (m, 3H), 7.37-7.62 (m, 4H), 7.05 (br s, 1H), 6.42 (br s, 1H), 4.92-5.33 (m, 2H), 2.90 (br s, 3H). LC-MS: Rt =0.814 min, (ESI) *m*/*z.* [M+H]⁺ 574.3; C₂₅H₁₇F₆N₉O |
| 239 | 1H NMR (400 MHz, DMSO-d6) δ ppm 8.41 (br s, 1 H) 8.17 (s, 1 H) 7.76 (s, 3 H) 7.57 - 7.64 (m, 1 H) 7.50 - 7.56 (m, 1 H) 7.36 - 7.43 (m, 1 H) 7.19 - 7.32 (m, 2 H) 5.63 (br s, 1 H) 4.73 (br d, J=12.51 Hz, 1 H) 4.10 (br s, 1 H) 3.90 (dd, J=12.63, 3.75 Hz, 1 H) 3.75 (br dd, J=11.51, 2.88 Hz, 1 H) 3.60 (br d, J=11.26 Hz, 1 H) 2.85 (s, 3 H) 0.80 (d, J=7.00 Hz, 3 H) | 240 | 1H NMR (400 MHz, DMSO-d6) δ = 9.36 - 9.10 (m, 1H), 8.05 - 7.89 (m, 2H), 7.88 - 7.63 (m, 2H), 7.53 - 7.33 (m, 3H), 7.30 - 7.14 (m, 1H), 4.95 - 4.62 (m, 2H), 4.13 - 4.02 (m, 1H), 3.24 - 3.10 (m, 1H), 2.96 (br d, J = 17.5 Hz, 3H), 2.69 - 2.51 (m, 3H), 2.03 - 1.61 (m, 3H), 1.50 - 1.12 (m, 1H) LC-MS: Rt = 2.526 min, (ESI) *m*/*z.* [M+H]⁺ 542.2; C₂₆H₂₃F₄N₇O₂ |
| | LC-MS: Rt = 2.839 min, (ESI) *m*/*z* = 470.2 [M+H]+; C₂₄H₂₂F₃N₅O₂ | | |
| 241 | ¹H NMR (400 MHz, DMSO-d₆) Shift 8.84-8.73 (m, 1H), 8.33-8.10 (m, 2H), 8.00-7.86 (m, 1H), 7.84-7.74 (m, 2H), 7.58-7.48 (m, 2H), 7.48-7.38 (m, 2H), 7.29-7.17 (m, 1H), 4.87-4.49 (m, 2H), 3.89 (s, 3H), 3.58-3.35 (m, 2H), 2.99-2.85 (m, 3H), 1.18-1.07 (m, 3H). LC-MS: Rt = 2.109 min, (ESI) *m*/*z.* [M+H]⁺498.1; C₂₆H₂₄FN₉O | 242 | 1H NMR (400 MHz, DMSO-d6) Shift 9.58-9.37 (m, 1H), 9.35-9.17 (m, 2H), 9.00-8.73 (m, 2H), 8.73-8.53 (m, 1H), 8.52-8.33 (m, 1H), 8.12-7.73 (m, 2H), 7.48 (br d, J = 9.7 Hz, 1H), 5.13-4.75 (m, 2H), 3.43 (br d, J = 2.4 Hz, 2H), 3.40 (br s, 1H), 3.13-2.98 (m, 4H), 2.73 (s, 3H), 2.12-1.95 (m, 4H), 1.16 (br s, 3H). LC-MS: Rt = 1.343 min, (ESI) *m*/*z.* [M+H]⁺ 501.3; C₂₇H₃₀ClFN₈O |
| 243 | 1H NMR (400 MHz, DMSO-d6) δ = 8.36 (br d, J = 6.5 Hz, 1H), 7.91 (br d, J = 6.4 Hz, 1H), 7.78 (s, 1H), 7.67 (s, 1H), 7.44 (d, J = 9.0 Hz, 1H), 7.25 - 7.13 (m, 4H), 6.78 (t, J = 6.8 Hz, 1H), 5.41 - 5.06 (m, 1H), 4.94 - 4.71 (m, 1H), 4.68 - 4.55 (m, 1H), 2.74 (br s, 3H), 1.50 (d, J = 7.1 Hz, 3H) LC-MS: Rt = 2.364 min, (ESI) *m*/*z.* [M+H]⁺ 486.2; C₂₃H₁₉F₄N₇O | 244 | **1H** NMR (400 MHz, DMSO-d6) δ = 9.05 - 8.91 (m, 1H), 8.90 - 8.79 (m, 1H), 8.43 - 8.14 (m, 1H), 8.04 - 7.86 (m, 1H), 7.66 (t, J = 8.2 Hz, 1H), 7.37 (br dd, J = 2.6, 9.3 Hz, 1H), 7.28 - 7.07 (m, 2H), 7.05 - 6.92 (m, 2H), 4.82 (s, 1H), 4.55 (s, 1H), 3.62 - 3.45 (m, 2H), 2.61 (d, J = 5.5 Hz, 3H), 1.19 - 1.04 (m, 3H) LC-MS: Rt = 2.312 min, (ESI) *m*/*z* = 468.0 [M+H]+; C₂₃H₂₀F₃N₇O |
| 245 | 1H NMR (400 MHz, DMSO-d6) δ = 9.04 - 8.82 (m, 1H), 8.55 - 8.15 (m, 2H), 7.94 - 7.74 (m, 1H), 7.53 (br t, J = 9.5 Hz, 1H), 7.32 - 7.11 (m, 2H), 7.02 (br s, 2H), 4.80 (s, 1H), 4.52 (s, 1H), 3.75 - 3.38 (m, 8H), 2.70 - 2.51 (m, 7H), 1.20 - 1.03 (m, 3H) LC-MS: Rt = 2.142 min, (ESI) *m*/*z.* [M+H]⁺ 517.3; C₂₇H₂₉FN₈O₂ | 246 | 1H NMR (400 MHz, DMSO-d6) δ = 9.00 (s, 1H), 8.24 (s, 1H), 8.21 (dd, J = 1.9, 8.4 Hz, 1H), 7.80 (d, J = 8.4 Hz, 1H), 7.59 (s, 2H), 7.39 (s, 1H), 7.26 (br s, 2H), 5.47 (br s, 1H), 4.97 (br d, J = 11.9 Hz, 1H), 4.22 (br s, 1H), 3.85 (dd, J = 3.9, 12.1 Hz, 1H), 3.74 (br dd, J = 2.6, 11.2 Hz, 1H), 3.59 (br d, J = 11.3 Hz, 1H), 2.85 (s, 3H), 0.80 (d, J = 7.0 Hz, 3H) LC-MS: Rt = 2.605 min, (ESI) *m*/*z.* [M+H]⁺ 471.1; C₂₃H₂₁F₃N₆O₂ |
| 247 | 1H NMR (400 MHz, DMSO-d6) δ = 9.07 - 8.88 (m, 1H), 8.55 - 8.40 (m, 2H), 7.90 - 7.77 (m, 1H), 7.48 - 7.39 (m, 1H), 7.35 - 7.26 (m, 1H), 7.16 (t, J = 10.7 Hz, 1H), 7.00 (br s, 2H), 5.22 - 5.18 (m, 1H), 4.78 (s, 1H), 4.50 (s, 1H), 3.74 (s, 1H), 3.51 (br d, J = 5.8 Hz, 1H), 2.61 (d, J = 4.9 Hz, 3H), 1.46 (t, J = 5.7 Hz, 6H), 1.16 - 1.04 (m, 3H) LC-MS: Rt = 2.138 min, (ESI) *m*/*z.* [M+H]⁺ 476.2; C₂₅H₂₆FN₇O₂ | 248 | 1H NMR (400 MHz, DMSO-d6) δ ppm 8.56 (d, J=7.00 Hz, 2 H) 7.98 (d, J=6.38 Hz, 1 H) 7.81 (s, 1 H) 7.62 (br d, J=8.88 Hz, 1 H) 7.13 - 7.26 (m, 4 H) 6.77 - 6.90 (m, 1 H) 6.51 (br s, 1 H) 4.76 (br s, 2 H) 3.48 (br s, 2 H) 2.67 - 2.93 (m, 3 H) 1.15 (br s, 3 H) |
| | | | LC-MS: Rt = 2.357 min, (ESI) *m*/*z* = 418.2 [M+H]+ ; C₂₂H₂₀FN₇O |
| 249 | 1H NMR (400 MHz, DMSO-d6) δ = 9.35 - 9.22 (m, 1H), 8.99 - 8.81 (m, 1H), 8.22 - 8.10 (m, 1H), 8.01 - 7.84 (m, 1H), 7.73 - 7.66 (m, 1H), 7.58 (s, 1H), 7.50 (br dd, J = 1.2, 9.3 Hz, 1H), 7.17 (s, 1H), 7.01 (br s, 2H), 6.94 (br d, J = 10.9 Hz, 1H), 5.08 (s, 2H), 3.58 (s, 3H), 2.60 (s, 3H) LC-MS: Rt = 2.092 min, (ESI) *m*/*z.* [M+H]⁺ 495.2; C₂₅H₁₉FN₁₀O | 250 | 1H NMR (400 MHz, DMSO-d6) δ = 8.63 - 8.43 (m, 1H), 8.39 - 8.07 (m, 1H), 7.95 - 7.68 (m, 2H), 7.59 - 7.36 (m, 3H), 7.33 - 7.18 (m, 2H), 6.90 (t, J = 6.6 Hz, 1H), 6.49 - 6.15 (m, 1H), 4.64 (br s, 2H), 3.89 (br s, 2H), 2.95 - 2.73 (m, 3H) LC-MS: Rt = 2.178 min, (ESI) *m*/*z.* [M+H]⁺ 454.1; C₂₂H₁₈F₃N₇O |
| 251 | 1H NMR (400 MHz, DMSO-d6) δ = 8.96 (s, 1H), 8.53 (d, J = 6.8 Hz, 1H), 8.15 (d, J = 6.5 Hz, 1H), 7.87 (s, 1H), 7.50 (d, J = 9.0 Hz, 1H), 7.26 - 7.17 (m, 2H), 7.03 - 6.84 (m, 4H), 4.97 (s, 2H), 3.44 (s, 3H), 2.59 (s, 3H), 1.85 (s, 3H) LC-MS: Rt = 2.088 min, (ESI) *m*/*z.* [M+H]⁺ 484.2; C₂₅H₂₂FN₉O | 252 | **1H** NMR (400 MHz, DMSO-d6) δ = 9.04 - 8.80 (m, 1H), 8.46 - 8.31 (m, 1H), 8.23 - 8.09 (m, 1H), 7.83 (s, 1H), 7.70 - 7.55 (m, 1H), 7.47 (br d, J = 9.1 Hz, 1H), 7.25 - 7.12 (m, 2H), 7.08 - 6.89 (m, 3H), 5.06 - 4.93 (m, 2H), 3.58 (br s, 9H), 2.60 (s, 3H), 2.39 (br s, 4H) LC-MS: Rt = 2.046 min, (ESI) *m*/*z.* [M+H]⁺ 569.3; C₂₉H₂₉FN₁₀O₂ |
| 253 | 1H NMR (400 MHz, DMSO-d6) δ = 9.04 - 8.78 (m, 1H), 8.62 - 8.40 (m, 1H), 8.26 - 8.07 (m, 1H), 7.91 - 7.74 (m, 1H), 7.71 - 7.56 (m, 1H), 7.51 (br d, J = 8.5 Hz, 1H), 7.29 - 7.12 (m, 2H), 7.11 - 6.78 (m, 4H), 5.14 - 4.93 (m, 2H), 3.85 - 3.53 (m, 3H), 2.60 (br s, 3H) LC-MS: Rt = 2.060 min, (ESI) *m*/*z.* [M+H]⁺ 470.2; C₂₄H₂₀FN₉O | 254 | 1H NMR (400 MHz, DMSO-d6) δ = 9.03 - 8.87 (m, 1H), 8.52 - 8.14 (m, 1H), 7.70 (br d, J = 5.5 Hz, 1H), 7.56 (s, 1H), 7.39 - 7.26 (m, 1H), 7.20 - 7.05 (m, 2H), 7.05 - 6.94 (m, 2H), 5.46 - 5.07 (m, 1H), 4.74 (s, 1H), 4.61 - 4.37 (m, 2H), 3.57 - 3.39 (m, 2H), 2.85 (br dd, J = 4.8, 19.3 Hz, 2H), 2.61 (br d, J = 2.3 Hz, 3H), 1.23 - 1.15 (m, 6H), 1.14 - 1.04 (m, 3H) LC-MS: Rt = 2.119 min, (ESI) *m*/*z.* [M+H]⁺ 505.2; C₂₆H₂₉FN₈O₂ |
| 255 | 1H NMR (400 MHz, DMSO-d6) δ = 9.29 (br s, 2H), 8.81 - 8.32 (m, 3H), 8.30 - 8.08 (m, 1H), 7.84 (br d, J = 4.5 Hz, 1H), 7.73 - 7.52 (m, 2H), 7.43 - 7.12 (m, 2H), 5.13 (br s, 2H), 3.64 (br s, 3H), 2.70 (s, 3H) LC-MS: Rt = 1.695 min, (ESI) *m*/*z.* [M+H]⁺ 538.2; C₂₅H₁₉F₄N₉O | 256 | H NMR (400 MHz, DMSO-d₆) Shift 9.15-8.91 (m, 1H), 8.90-8.72 (m, 1H), 8.55-8.23 (m, 1H), 8.20 (d, *J =* 11.0 Hz, 1H), 7.97-7.74 (m, 2H), 7.66-7.58 (m, 1H), 7.56-7.50 (m, 1H), 7.23 (dd, *J* = 10.9, 13.2 Hz, 1H), 7.06 (br s, 2H), 4.93-4.51 (m, 2H), 3.94 (d, *J =* 3.9 Hz, 3H), 3.41 (br s, 2H), 2.67 (d, *J =* 8.4 Hz, 3H), 1.30-1.03 (m, 3H). LC-MS: Rt = 2.166 min, (ESI) *m*/*z.* [M+H]⁺ 498.2; C₂₆H₂₄FN₉O |
| 257 | 1H NMR (400 MHz, DMSO-d6) δ = 9.08 - 8.71 (m, 1H), 8.59 - 8.39 (m, 1H), 8.29 - 8.09 (m, 1H), 7.85 - 7.66 (m, 1H), 7.58 - 7.39 (m, 1H), 7.30 - 7.14 (m, 2H), 7.12 - 6.94 (m, 2H), 6.91 - 6.78 (m, 1H), 5.53 - 5.30 (m, 1H), 4.90 - 4.79 (m, 1H), 4.67 - 4.38 (m, 1H), 4.30 - 4.15 (m, 1H), 3.89 (br dd, J = 1.9, 3.7 Hz, 1H), 2.64 - 2.57 (m, 3H), 1.90 - 1.78 (m, 1H), 1.70 - 1.40 (m, 2H), 1.28 - 1.12 (m, 1H) LC-MS: Rt = 1.996 min, (ESI) *m*/*z.* [M+H]⁺ 460.1; C₂₄H₂₂FN₇O₂ | 258 | 1H NMR (400 MHz, DMSO-d6) δ = 9.07 - 8.85 (m, 1H), 8.48 - 8.14 (m, 1H), 8.12 - 7.95 (m, 1H), 7.80 - 7.59 (m, 1H), 7.44 (br d, J = 9.6 Hz, 1H), 7.24 (br d, J = 9.1 Hz, 1H), 7.16 (dt, J = 3.1, 10.7 Hz, 1H), 7.00 (br s, 2H), 4.80 - 4.44 (m, 2H), 3.57 - 3.41 (m, 2H), 3.18 (br d, J = 3.3 Hz, 4H), 3.09 - 2.90 (m, 4H), 2.61 (br s, 6H), 1.18 - 1.03 (m, 3H) LC-MS: Rt = 2.158 min, (ESI) *m*/*z.* [M+H]⁺ 516.3; C₂₇H₃₀FN₉O |
| 259 | 1H NMR (400 MHz, DMSO-d6) δ = 9.05 - 8.85 (m, 1H), 8.44 - 8.25 (m, 1H), 8.21 - 8.13 (m, 1H), 7.82 - 7.67 (m, 1H), 7.44 (d, J = 9.8 Hz, 1H), 7.16 (t, J = 11.3 Hz, 1H), 7.01 (br dd, J = 2.3, 9.8 Hz, 3H), 4.76 (s, 1H), 4.49 (s, 1H), 3.76 (d, J = 14.4 Hz, 3H), 3.51 (br d, J = 6.6 Hz, 1H), 3.30 (br s, 1H), 2.61 (d, J = 3.9 Hz, 3H), 1.19 - 1.03 (m, 3H) LC-MS: Rt = 2.228 min, (ESI) m/z. [M+H]+ 448.2; C₂₃H₂₂FN₇O₂ | 260 | 1H NMR (400 MHz, DMSO-d6) δ = 9.13 - 8.79 (m, 1H), 8.60 - 8.21 (m, 2H), 7.79 (br s, 1H), 7.59 - 7.16 (m, 2H), 7.15 - 6.92 (m, 3H), 6.85 (br t, J = 6.7 Hz, 1H), 5.17 (s, 2H), 4.11 - 3.84 (m, 3H), 2.60 (s, 3H) LC-MS: Rt = 2.170 min, (ESI) *m*/*z.* [M+H]⁺ 471.2; C₂₃H₁₉FN₁₀O |
| 261 | 1H NMR (400 MHz, DMSO-d6) δ = 9.07 - 8.82 (m, 1H), 8.52 - 8.46 (m, 1 H), 8.36 (br d, J = 6.6 Hz, 1H), 8.26 - 8.17 (m, 1H), 8.14 - 8.05 (m, 2H), 7.26 - 7.15 (m, 2H), 7.00 (br d, J = 6.4 Hz, 1H), 4.91 - 4.50 (m, 2H), 3.54 (br d, J = 5.9 Hz, 2H), 2.61 (d, J = 6.3 Hz, 3H), 1.17 - 1.06 (m, 3H) LC-MS: Rt = 2.131 min, (ESI) *m*/*z.* [M+H]⁺ 419.2; C₂₁H₁₉FN₈O | 262 | 1H NMR (400 MHz, DMSO-d6) δ = 9.00 - 8.80 (m, 1H), 8.62 - 8.12 (m, 2H), 7.97 - 7.73 (m, 1H), 7.54 (br d, J = 9.0 Hz, 1H), 7.28 - 7.14 (m, 2H), 7.03 (br s, 2H), 6.93 - 6.82 (m, 1H), 4.92 - 4.57 (m, 2H), 3.81 - 3.62 (m, 2H), 2.93 - 2.84 (m, 2H), 2.61 (s, 3H) LC-MS: Rt = 1.308 min, (ESI) *m*/*z.* [M+H]⁺ 443.2; C₂₃H₁₉FN₈O |
| 263 | 1H NMR (400 MHz, DMSO-d6) δ = 9.06 - 8.85 (m, 1H), 8.81 - 8.66 (m, 1H), 8.41 - 8.16 (m, 1H), 7.93 - 7.75 (m, 1H), 7.67 - 7.55 (m, 1H), 7.37 - 7.28 (m, 1H), 7.16 (dd, J = 10.9, 14.9 Hz, 1H), 7.01 (br d, J = 1.1 Hz, 2H), 4.79 (s, 1H), 4.52 (s, 1H), 3.52 (br d, J = 6.1 Hz, 2H), 2.61 (d, J = 4.6 Hz, 3H), 1.20 - 1.03 (m, 3H) LC-MS: Rt = 2.244 min, (ESI) m/z. [M+H]+ 436.2; C₂₂H₁₉F₂N₇O | 264 | 1H NMR (400MHz, DMSO-d6) Shift = 9.19 (s, 1H), 8.16 (br s, 1H), 8.05 (s, 1H), 7.73 (d, J=9.5 Hz, 2H), 7.57 - 7.40 (m, 3H), 7.35 (s, 1H), 7.29 (br s, 2H), 7.31 - 7.09 (m, 1H), 5.09 (s, 2H), 3.66 (br s, 3H), 2.72 - 2.58 (m, 3H). |
| | | | LC-MS: Rt = 1.711 min, (ESI) *m*/*z.* [M+H]⁺ 520.1; C₂₅H₂₀F₃N₉O |
| 265 | 1H NMR (400 MHz, DMSO-d6) δ = 8.99 - 8.68 (m, 1H), 8.27 - 8.08 (m, 1H), 8.00 - 7.40 (m, 4H), 7.18 - 7.09 (m, 1H), 7.07 - 6.94 (m, 2H), 4.99 - 4.71 (m, 2H), 2.62 (s, 1H), 2.58 (s, 1H), 2.50 (br s, 3H), 0.95 (d, J = 6.5 Hz, 3H), 0.88 - 0.80 (m, 1H), 0.69 (d, J = 6.5 Hz, 3H) LC-MS: Rt = 1.617 min, (ESI) *m*/*z.* [M+H]⁺ 514.3; C₂₅H₂₃F₄N₇O | 266 | 1H NMR (400 MHz, DMSO-d6) δ = 8.47 (br d, J = 5.0 Hz, 1H), 8.03 - 7.94 (m, 1H), 7.84 (br s, 1H), 7.77 (s, 1H), 7.48 (br d, J = 8.9 Hz, 1H), 7.33 (s, 1H), 7.28 - 7.19 (m, 1H), 6.98 (br s, 2H), 6.87 (t, J = 7.1 Hz, 1H), 4.77 - 4.28 (m, 2H), 3.63 - 3.34 (m, 2H), 2.83 (br s, 3H), 2.32 (s, 3H), 1.22 - 1.11 (m, 3H) LC-MS: Rt = 2.057 min, (ESI) *m*/*z.* [M+H]⁺ 414.2; C23H23N7O |
| 267 | 1H NMR (400 MHz, DMSO-d6) Shift 9.04 (s, 1H), 9.10-8.76 (m, 1H), 8.61-8.39 (m, 1H), 8.31-8.14 (m, 1H), 8.34-8.11 (m, 1H), 7.91-7.72 (m, 1H), 7.53-7.45 (m, 1H), 7.56-7.42 (m, 1H), 7.24-7.06 (m, 2H), 7.05-6.90 (m, 2H), 6.89-6.76 (m, 1H), 4.83-4.52 (m, 2H), 4.00-3.58 (m, 1H), 3.52-3.42 (m, 1H), 3.27-3.05 (m, 4H), 2.64-2.57 (m, 3H), 1.23-1.13 (m, 3H). LC-MS: Rt = 1.104 min, (ESI) *m*/*z.* [M+H]⁺ 462.3; C₂₄H₂₄FN₇O₂ | 268 | 1H NMR (400MHz, DMSO-d6) δ 9.06 - 8.86 (m, 2H), 8.56 - 8.50 (m, 1H), 8.48 - 8.18 (m, 1H), 7.89 - 7.73 (m, 1H), 7.26 - 7.11 (m, 1H), 7.10 - 6.95 (m, 3H), 4.87 - 4.54 (m, 2H), 3.62 - 3.34 (m, 2H), 2.62 (d, J=6.5 Hz, 3H), 1.21 - 1.06 (m, 3H) ; LC-MS: Rt = 1.889 min, (ESI) m/z. [M+H]+ 419.1; C₂₁H₁₉FN₈O |
| 269 | 1H NMR (400 MHz, DMSO-d6) δ = 9.05 - 8.75 (m, 1H), 8.53 (br d, J = 7.4 Hz, 1H), 8.16 (br d, J = 6.9 Hz, 1H), 7.82 - 7.69 (m, 1H), 7.53 (br d, J = 9.4 Hz, 1H), 7.26 - 7.14 (m, 2H), 7.04 (br d, J = 1.9 Hz, 2H), 6.88 (s, 1H), 4.83 (br s, 2H), 3.89 (br dd, J = 3.8, 8.3 Hz, 1H), 3.41 - 3.35 (m, 1H), 3.21 - 2.87 (m, 4H), 2.65 - 2.56 (m, 4H), 2.18 - 1.97 (m, 2H) LC-MS: Rt = 2.127 min, (ESI) *m*/*z.* [M+H]⁺ 474.2; C₂₅H₂₄FN₇O₂ | 270 | 1H NMR (400 MHz, DMSO-d6) δ = 9.04 - 8.85 (m, 1H), 8.52 - 8.18 (m, 1H), 7.81 - 7.59 (m, 1H), 7.54 - 7.41 (m, 1H), 7.34 - 7.14 (m, 2H), 7.04 (br s, 2H), 6.89 - 6.73 (m, 1H), 4.85 (s, 1H), 4.54 (s, 1H), 3.53 (br dd, J = 1.9, 14.5 Hz, 2H), 2.64 - 2.53 (m, 6H), 1.24 - 1.05 (m, 3H) LC-MS: Rt = 2.265 min, (ESI) m/z. [M+H]+ 432.2; C₂₃H₂₂FN₇O |
| 271 | 1H NMR (400MHz, DMSO-d6) Shift = 8.14 (br s, 1H), 7.81 (s, 1H), 7.76 - 7.53 (m, 1H), 7.46 (br s, 2H), 7.33 - 7.07 (m, 2H), 6.04 - 4.59 (m, 2H), 4.04 (s, 3H), 3.96 - 3.48 (m, 4H), 2.96 (s, 3H), 0.75 (br d, J=5.8 Hz, 3H). | 272 | 1H NMR (400 MHz, DMSO-d6) δ = 9.04 - 8.87 (m, 1H), 8.60 - 8.46 (m, 1H), 8.45 - 8.17 (m, 1H), 7.94 - 7.79 (m, 1H), 7.57 - 7.49 (m, 1H), 7.30 - 7.11 (m, 2H), 7.02 (br s, 2H), 6.95 - 6.84 (m, 1H), 4.82 - 4.51 (m, 2H), 3.57 - 3.35 (m, 2H), 2.64 - 2.59 (m, 3H), 1.17 - 1.05 (m, 3H) LC-MS: Rt = 1.051 min, (ESI) *m*/*z.* [M+H]⁺ 518.2; C₂₂H₂₀FN₇O |
| | LC-MS: Rt = 2.181 min, (ESI) *m*/*z.* [M+H]⁺ 452.2; C₂₂H₂₂FN₇O₃ | | |
| 273 | 1H NMR (400 MHz, DMSO-d6) δ = 8.99 (br s, 1H), 8.28 - 8.19 (m, 1H), 8.09 (d, J = 6.4 Hz, 1H), 7.81 (s, 1H), 7.79 - 7.73 (m, 1H), 7.45 (br s, 2H), 7.32 - 7.20 (m, 1H), 5.28 - 4.80 (m, 1H), 3.85 - 3.77 (m, 1H), 3.75 - 3.47 (m, 2H), 3.33 - 3.31 (m, 2H), 2.93 (br s, 3H), 0.77 (br d, J = 6.9 Hz, 3H) LC-MS: Rt = 2.411 min, (ESI) *m*/*z.* [M+H]⁺ 489.2; C₂₃H₂₀F₄N₆O₂ | 274 | 1H NMR (400 MHz, DMSO-d6) δ = 12.73 - 12.43 (m, 1H), 8.02 (br d, J = 6.4 Hz, 1H), 7.90 - 7.82 (m, 1H), 7.78 (br s, 1H), 7.73 - 7.64 (m, 1H), 7.47 (br d, J = 8.5 Hz, 1H), 7.20 (d, J = 10.9 Hz, 1H), 7.17 (br s, 2H), 4.95 - 4.69 (m, 2H), 3.46 - 3.22 (m, 2H), 2.91 - 2.57 (m, 3H), 2.16 - 2.03 (m, 1H), 0.99 - 0.78 (m, 6H) LC-MS: Rt = 2.498 min, (ESI) *m*/*z.* [M+H]⁺ 514.2; C₂₅H₂₃F₄N₇O |
| 275 | 1H NMR (400MHz, DMSO-d6) Shift = 8.02 (br d, J=6.4 Hz, 1H), 7.87 - 7.65 (m, 5H), 7.46 (s, 2H), 7.26 (d, J=10.9 Hz, 1H), 5.99 - 5.38 (m, 1H), 4.74 (br d, J=12.4 Hz, 1H), 4.05 - 3.48 (m, 4H), 3.03 - 2.83 (m, 3H), 0.76 (br s, 3H). | 276 | 1H NMR (400 MHz, DMSO-d6) δ = 8.97 - 8.71 (m, 1H), 8.54 - 8.39 (m, 1H), 8.15 (br s, 1H), 7.79 (br s, 1H), 7.47 (br d, J = 9.1 Hz, 1H), 7.25 - 7.06 (m, 2H), 6.83 (br t, J = 6.4 Hz, 1H), 6.72 (br s, 2H), 4.80 - 4.52 (m, 2H), 4.26 - 3.82 (m, 2H), 3.13 (br s, 3H), 2.63 (s, 3H), 1.87 - 1.81 (m, 2H), 1.73 - 1.33 (m, 4H) LC-MS: Rt = 2.317 min, (ESI) *m*/*z.* [M+H]⁺ 488.2; C₂₆H₂₆FN₇O₂ |
| | LC-MS: Rt = 2.809 min, (ESI) *m*/*z.* [M+H]⁺ 488.2; C₂₄H₂₁F₄N₅O₂ | | |
| 277 | ¹H NMR (400 MHz, DMSO-d₆) δ = 9.10 (s, 1H), 8.84 (s, 1H), 8.23 (s, 1H), 7.90 (br s, 1H), 7.71 (d, *J* = 9.5 Hz, 1H), 7.44 - 7.41 (m, 1H), 7.40 - 7.36 (m, 2H), 6.55 (br s, 2H), 4.93 (br s, 2H), 4.62 (br s, 1H), 3.54 (s, 2H), 2.64 (s, 3H), 1.19 (br s, 6H) LC-MS: Rt = 2.316 min, (ESI) *m*/*z.* [M+H]⁺ 512.2; C₂₅H₂₄F₃N₇O₂ | 278 | 1H NMR (400MHz, DMSO-d6) Shift = 9.07 - 8.93 (m, 1H), 9.00 (br s, 1H), 8.31 (br d, J=6.0 Hz, 1H), 7.64 (br s, 1H), 7.36 - 7.10 (m, 2H), 7.02 (br s, 2H), 5.14 (br s, 1H), 4.04 (br s, 3H), 3.87 (s, 1H), 3.80 - 3.65 (m, 4H), 2.62 (s, 3H), 0.75 (br s, 3H). |
| | | | LC-MS: Rt = 2.187 min, (ESI) *m*/*z.* [M+H]⁺ 452.2; C₂₂H₂₂FN₇O₃ |
| 279 | 1H NMR (400 MHz, DMSO-d6) δ ppm 8.75 - 8.99 (m, 1 H) 8.15 - 8.56 (m, 2 H) 7.74 (br s, 1 H) 7.50 (br d, J=9.06 Hz, 1 H) 7.09 - 7.26 (m, 2 H) 6.84 (br s, 1 H) 6.73 (br s, 2 H) 4.31 - 4.94 (m, 2 H) 3.37 (br s, 2 H) 2.63 (s, 3 H) 2.15 (br s, 1 H) 0.84 - 0.85 (m, 1 H) 0.61 - 1.06 (m, 5 H) LC-MS: Rt = 2.387 min, (ESI) *m*/*z* = 446.2 [M+H]+; C₂₄H₂₄FN₇O | 280 | 1H NMR (400 MHz, DMSO-d6) δ = 8.99 (br s, 1H), 8.28 - 8.19 (m, 1H), 8.09 (d, J = 6.4 Hz, 1H), 7.81 (s, 1H), 7.79 - 7.73 (m, 1H), 7.45 (br s, 2H), 7.32 - 7.20 (m, 1H), 5.28 - 4.80 (m, 1H), 3.85 - 3.77 (m, 1H), 3.75 - 3.47 (m, 2H), 3.33 - 3.31 (m, 2H), 2.93 (br s, 3H), 0.77 (br d, J = 6.9 Hz, 3H) LC-MS: Rt = 2.411 min, (ESI) *m*/*z.* [M+H]⁺ 489.2; C₂₃H₂₀F₄N₆O₂ |
| 281 | 1H NMR (400MHz, DMSO-d6) Shift = 8.52 (br d, J=6.6 Hz, 1H), 8.46 - 8.11 (m, 1H), 7.95 (s, 1H), 7.66 - 7.46 (m, 3H), 7.40 - 7.12 (m, 4H), 6.90 (br t, J=6.6 Hz, 1H), 4.62 (br s, 2H), 3.45 (br d, J=6.4 Hz, 2H), 2.98 - 2.55 (m, 3H), 1.15 (br t, J=6.9 Hz, 3H). | 282 | 1H NMR (400 MHz, DMSO-d6) δ = 9.13 - 8.93 (m, 2H), 8.32 (br d, J = 6.5 Hz, 2H), 7.75 (br d, J = 7.5 Hz, 1H), 7.30 - 6.94 (m, 3H), 5.87 - 5.02 (m, 1H), 5.01 - 4.31 (m, 1H), 3.92 - 3.45 (m, 4H), 2.62 (s, 3H), 0.77 (br d, J = 6.9 Hz, 3H) LC-MS: Rt = 2.575 min, (ESI) *m*/*z.* [M+H]⁺ 489.1; C₂₃H₂₀F₄N₆O₂ |
| | LC-MS: Rt = 1.454 min, (ESI) *m*/*z.* [M+H]⁺ 400.2; C₂₂H₂₁N₇O | | |
| 283 | ¹H NMR (400 MHz, DMSO-d₆) δ = 9.09 - 8.94 (m, 1H), 8.72 (br dd, *J =* 2.3, 3.5 Hz, 1H), 8.09 (d, *J* = 6.4 Hz, 1H), 7.83 - 7.60 (m, 2H), 7.45 - 7.31 (m, 1H), 7.11 (d, *J =* 11.1 Hz, 1H), 6.71 (br s, 2H), 4.80 (br s, 2H), 4.49 (d, *J= 2.1* Hz, 1H), 3.60 (br s, 2H), 2.62 (s, 3H), 1.40 - 1.13 (m, 6H) | 284 | 1H NMR (400 MHz, DMSO-d6) δ = 9.32 - 9.07 (m, 1H), 9.00 - 8.73 (m, 1H), 8.15 (br s, 1H), 8.02 - 7.82 (m, 1H), 7.78 - 7.63 (m, 1H), 7.50 - 7.39 (m, 1H), 7.19 (d, J = 10.8 Hz, 1H), 7.09 - 6.93 (m, 2H), 5.40 - 5.26 (m, 1H), 4.98 - 4.55 (m, 2H), 4.29 - 4.12 (m, 1H), 4.00 - 3.82 (m, 1H), 2.63 (s, 3H), 1.90 - 1.79 (m, 1H), 1.69 - 1.52 (m, 2H), 1.28 - 1.11 (m, 1H) LC-MS: Rt = 2.344 min, (ESI) *m*/*z.* [M+H]⁺ 527.1; C₂₅H₂₁F₄N₇O₂ |
| | LC-MS: Rt = 2.356 min, (ESI) *m*/*z.* [M+H]⁺ 530.2; C₂₅H₂₃F₄N₇O₂ | | |
| 285 | 1H NMR (400 MHz, DMSO-d6) δ = 9.30 - 9.13 (m, 1H), 9.04 - 8.86 (m, 1H), 8.36 - 8.16 (m, 1H), 8.07 - 7.89 (m, 1H), 7.74 (t, J = 8.8 Hz, 1H), 7.51 - 7.43 (m, 1H), 7.17 (dd, J = 10.9, 17.5 Hz, 1H), 7.05 - 6.96 (m, 2H), 4.87 - 4.56 (m, 2H), 3.40 - 3.33 (m, 2H), 2.64 - 2.60 (m, 3H), 1.20 - 1.06 (m, 3H) LC-MS: Rt = 2.497 min, (ESI) *m*/*z.* [M+H]⁺ 486.2; C₂₃H₁₉F₄N₇O | 286 | 1H NMR (400 MHz, DMSO-d6) δ = 9.32 - 9.09 (m, 1H), 9.05 - 8.85 (m, 1H), 8.31 - 8.13 (m, 1H), 8.11 - 7.83 (m, 1H), 7.81 - 7.67 (m, 1H), 7.55 - 7.40 (m, 1H), 7.21 - 7.11 (m, 1H), 7.09 - 6.87 (m, 2H), 4.57 (s, 2H), 3.29 - 3.11 (m, 2H), 2.64 - 2.60 (m, 3H), 2.18 - 1.97 (m, 1H), 0.97 - 0.69 (m, 6H) LC-MS: Rt = 1.965 min, (ESI) *m*/*z.* [M+H]⁺ 514.2; C₂₅H₂₃F₄N₇O |
| 287 | 1H NMR (400MHz, DMSO-d6) Shift = 9.29 - 8.54 (m, 2H), 8.18 - 7.78 (m, 2H), 7.66 (br s, 1H), 7.38 (br d, J=7.9 Hz, 1H), 7.13 (br s, 1H), 6.72 (br s, 2H), 4.95 - 4.39 (m, 2H), 4.22 - 3.82 (m, 1H), 3.52 (br s, 2H), 3.31 - 3.10 (m, 3H), 2.63 (s, 3H), 1.21 (br s, 3H). LC-MS: Rt = 2.517 min, (ESI) *m*/*z.* [M+H]⁺ 530.2; C₂₅H₂₃F₄N₇O₂ | 288 | ¹H NMR (400 MHz, DMSO-d6) δ = 9.33 - 9.07 (m, 1H), 9.05 - 8.75 (m, 1H), 8.22 - 8.09 (m, 1H), 8.05 - 7.92 (m, 1H), 7.88 - 7.70 (m, 1H), 7.69 - 7.27 (m, 2H), 7.25 - 7.08 (m, 2H), 4.92 - 4.60 (m, 2H), 4.32 - 3.91 (m, 2H), 3.18 - 2.96 (m, 3H), 2.64 - 2.58 (m, 3H), 2.14 - 1.69 (m, 3H), 1.51 - 1.14 (m, 1H) LC-MS: Rt = 1.843 min, (ESI) *m*/*z.* [M+H]⁺ 542.2; C₂₆H₂₃F₄N₇O₂ |
| 289 | 1H NMR (400 MHz, DMSO-d6) δ = 9.15 (s, 1H), 8.82 (s, 1H), 8.19 (d, J = 1.5 Hz, 1H), 8.01 (s, 1H), 7.73 (d, J = 9.3 Hz, 1H), 7.45 (dd, J = 1.8, 9.5 Hz, 1H), 7.37 - 7.24 (m, 2H), 6.79 (br s, 2H), 4.70 (s, 2H), 3.71 (s, 2H), 3.32 (s, 3H), 2.58 (s, 3H), 1.45 (s, 6H) LC-MS: Rt = 2.611 min, (ESI) *m*/*z.* [M+H]⁺ 526.2; C₂₆H₂₆F₃N₇O₂ | 290 | 1H NMR (400 MHz, DMSO-d6) δ = 9.22 (br s, 1H), 9.01 - 8.75 (m, 1H), 8.22 (br d, J = 2.0 Hz, 1H), 8.00 (br s, 1H), 7.73 (d, J = 9.4 Hz, 1H), 7.46 (dd, J = 1.6, 9.5 Hz, 1H), 7.42 - 7.27 (m, 2H), 6.89 (br s, 2H), 4.85 (br d, J = 3.9 Hz, 2H), 4.12 (br dd, J = 1.4, 7.7 Hz, 2H), 3.06 (s, 3H), 2.62 (s, 3H), 1.98 - 1.66 (m, 3H), 1.38 - 1.08 (m, 1H) LC-MS: Rt = 2.518 min, (ESI) *m*/*z.* [M+H]⁺ 524.2; C₂₆H₂₄F₃N₇O₂ |
| 291 | 1H NMR (400 MHz, DMSO-d6) δ = 9.08 - 8.87 (m, 1H), 8.71 - 8.55 (m, 1H), 8.52 (d, J = 6.8 Hz, 1H), 7.94 (s, 1H), 7.65 - 7.48 (m, 1H), 7.39 (br d, J = 5.0 Hz, 1H), 7.30 - 7.21 (m, 1H), 6.90 (br t, J = 6.6 Hz, 1H), 6.85 (br s, 2H), 4.82 - 4.57 (m, 2H), 3.52 - 3.40 (m, 2H), 2.62 (s, 3H), 1.13 (t, J = 7.0 Hz, 3H) LC-MS: Rt = 2.093 min, (ESI) *m*/*z.* [M+H]⁺ 400.2; C₂₂H₂₁N₇O | 292 | ¹H NMR (400 MHz, DMSO-d6) δ = 9.28 - 8.80 (m, 2H), 8.39 - 7.94 (m, 2H), 7.73 (br d, J = 9.3 Hz, 1H), 7.54 - 7.26 (m, 3H), 6.85 (br s, 2H), 4.87 - 4.57 (m, 2H), 4.16 (br d, J = 1.4 Hz, 1H), 3.50 (br s, 1H), 3.31 - 3.02 (m, 4H), 2.67 - 2.56 (m, 3H), 1.16 (br s, 3H) LC-MS: Rt = 2.461 min, (ESI) *m*/*z.* [M+H]⁺ 512.2; C₂₅H₂₄F₃N₇O₂ |
| 293 | 1H NMR (400 MHz, DMSO-d6) Shift 9.23-9.10 (m, 1H), 8.97 (s, 1H), 8.46-8.14 (m, 1H), 8.05-7.84 (m, 1H), 7.75 (d, J = 9.5 Hz, 1H), 7.48 (dd, J = 1.8, 9.5 Hz, 1H), 7.44 (br s, 2H), 6.90 (br s, 2H), 5.15 - 4.81 (m, 3H), 4.77 (br s, 2H), 4.60 (br t, J = 6.8 Hz, 2H), 2.63 (s, 3H). LC-MS: Rt = 2.285 min, (ESI) *m*/*z.* [M+H]⁺ 496.2; C₂₄H₂₀F₃N₇O₂ | 294 | 1H NMR (400MHz, DMSO-d6) Shift = 9.97 - 9.55 (m, 1H), 9.29 (br d, J=4.0 Hz, 1H), 9.04 (br s, 1H), 8.74 - 8.55 (m, 1H), 8.27 - 7.88 (m, 2H), 7.49 - 7.23 (m, 2H), 6.87 (br s, 2H), 5.11 - 4.84 (m, 2H), 3.36 (br s, 2H), 2.67 - 2.57 (m, 3H), 2.14 - 1.84 (m, 1H), 1.04 - 0.58 (m, 6H). |
| | | | LC-MS: Rt = 2.264 min, (ESI) *m*/*z.* [M+H]⁺ 473.2; C₂₄H₂₄N₈OS |
| 295 | 1H NMR (400 MHz, DMSO-d6) δ ppm 9.18 (br s, 1 H) 8.07 - 8.25 (m, 1 H) 7.81 - 8.06 (m, 2 H) 7.74 (br d, J=8.13 Hz, 1 H) 7.28 - 7.65 (m, 3 H) 6.97 (br s, 2 H) 4.50 - 4.99 (m, 2 H) 3.30 (br s, 2 H) 2.50 (br s, 3 H) 1.91 - 2.19 (m, 1 H) 0.64 - 1.00 (m, 1 H) 0.64 - 1.00 (m, 5 H) LC-MS: Rt = 2.838 min , (ESI) *m*/*z* = 496.2 [M+H]+; C₂₅H₂₄F₃N₇O | 296 | NT |
| 297 | 1H NMR (400 MHz, DMSO-d6) δ = 9.13 - 8.80 (m, 1H), 8.65 (br d, J = 6.6 Hz, 1H), 8.41 - 8.12 (m, 1H), 7.65 (d, J = 8.9 Hz, 1H), 7.39 (br s, 2H), 7.24 - 7.17 (m, 1H), 6.92 - 6.78 (m, 3H), 6.59 (br s, 1H), 4.98 - 4.52 (m, 2H), 3.56 - 3.40 (m, 2H), 2.62 (s, 3H), 1.13 (br s, 3H) LC-MS: Rt = 2.283 min , (ESI) *m*/*z* = 400.2 [M+H]+; C₂₂H₂₁N₇O | 298 | 1H NMR (400 MHz, DMSO-d6) δ = 9.15 (s, 1H), 8.93 (s, 1H), 8.10 (br d, J = 1.6 Hz, 1H), 7.99 (s, 1H), 7.78 (d, J = 9.5 Hz, 1H), 7.47 (dd, J = 1.6, 9.5 Hz, 1H), 7.40 (s, 1H), 7.29 - 7.14 (m, 2H), 6.98 (s, 1H), 6.84 (br s, 2H), 6.27 - 6.05 (m, 1H), 3.56 (s, 3H), 2.61 (s, 3H), 1.51 (d, J = 7.0 Hz, 3H) LC-MS: Rt = 2.371 min, (ESI) *m*/*z.* [M+H]⁺ 534.2; C₂₆H₂₂F₃N₉O |
| 299 | 1H NMR (400 MHz, DMSO-d6) δ = 9.09 (s, 1H), 8.62 (br d, J = 4.4 Hz, 1H), 8.25 (s, 2H), 8.08 (d, J = 7.9 Hz, 1H), 7.92 (dt, J = 1.6, 7.7 Hz, 1H), 7.55 - 7.30 (m, 3H), 6.87 (br s, 2H), 5.55 - 5.33 (m, 1H), 3.38 (br d, J = 6.9 Hz, 2H), 2.62 (s, 4H), 1.92 - 1.77 (m, 3H), 0.96 - 0.56 (m, 6H) LC-MS: Rt = 1.855 min , (ESI) *m*/*z* = 486.2 [M+H]+; C₂₆H₂₇N₇OS | 300 | 1H NMR (400 MHz, DMSO-d6) δ = 9.22 (br s, 1H), 8.95 (br s, 1H), 8.21 (br d, J = 5.0 Hz, 1H), 8.05 (s, 1H), 7.73 (d, J = 9.4 Hz, 1H), 7.53 - 7.31 (m, 3H), 6.87 (br s, 2H), 4.71 (br s, 2H), 4.19 - 4.06 (m, 1H), 4.04 - 3.84 (m, 1H), 3.11 (s, 3H), 2.62 (s, 3H), 2.00 - 1.72 (m, 3H), 1.68 - 1.22 (m, 3H) LC-MS: Rt = 2.581 min, (ESI) *m*/*z.* [M+H]⁺ 538.2; C₂₇H₂₆F₃N₇O₂ |
| 301 | 1H NMR (400 MHz, DMSO-d6) δ = 9.13 - 8.90 (m, 1H), 8.16 (s, 1H), 7.57 - 7.17 (m, 2H), 6.86 (br s, 2H), 6.22 (br s, 1H), 4.93 - 4.61 (m, 2H), 3.78 - 3.62 (m, 4H), 3.30 - 3.25 (m, 2H), 3.19 - 3.05 (m, 4H), 2.62 (s, 3H), 2.18 - 1.82 (m, 1H), 0.98 - 0.61 (m, 6H) LC-MS: Rt = 2.459 min , (ESI) *m*/*z* = 480.2 [M+H]+; C₂₄H₂₉N₇O₂S | 302 | 1H NMR (400 MHz, DMSO-d6) Shift 9.24 (br d, *J =* 17.9 Hz, 2H), 9.09-8.57 (m, 2H), 8.35 (br s, 1H), 8.08 (br d, *J =* 10.5 Hz, 1H), 7.79 (br d, *J =* 9.4 Hz, 1H), 7.62 (br s, 1H), 7.55 (br d, *J* = 7.4 Hz, 1H), 7.51-7.30 (m, 2H), 7.18 (br s, 1H), 5.09 (br s, 2H), 3.62 (br s, 3H), 2.71-2.68 (m, 3H). LC-MS: Rt = 1.707 min, (ESI) *m*/*z.* [M+H]⁺ 520.1; C₂₅H₂₀F₃N₉O |
| 303 | 1H NMR (400 MHz, DMSO-d6) δ ppm 9.02 (br s, 1 H) 8.15 (s, 1 H) 7.20 - 7.54 (m, 3 H) 6.87 (br s, 2 H) 6.58 (br s, 1 H) 4.91 (br s, 2 H) 4.22 (br s, 2 H) 3.79 (br s, 2 H) 3.11 - 3.31 (m, 2 H) 2.55 - 2.71 (m, 3 H) 2.22 - 2.46 (m, 2 H) 1.69 - 2.17 (m, 1 H) 0.63 - 1.03 (m, 6 H) LC-MS: Rt = 2.533 min , (ESI) *m*/*z* = 477.2 [M+H]+; C₂₅H₂₈N₆O₂S | 304 | 1H NMR (400 MHz, DMSO-d6) δ = 8.65 (d, J = 6.9 Hz, 1H), 8.33 - 8.04 (m, 1H), 7.93 (br s, 1H), 7.66 (d, J = 8.8 Hz, 1H), 7.57 - 7.40 (m, 2H), 7.29 - 7.16 (m, 1H), 7.09 - 6.78 (m, 3H), 6.57 (s, 1H), 4.91 - 4.60 (m, 2H), 3.61 - 3.33 (m, 2H), 2.53 - 2.51 (m, 3H), 1.29 (d, J = 12.2 Hz, 1H), 1.14 (br t, J = 6.3 Hz, 3H) LC-MS: Rt = 2.468 min , (ESI) *m*/*z* = 400.1 [M+H]+; C₂₂H₂₁N₇O |
| 305 | 1H NMR (400MHz, DMSO-d6) Shift = 9.12 (br s, 1H), 8.91 (br d, J=4.5 Hz, 2H), 8.71 - 8.01 (m, 2H), 7.59 - 7.20 (m, 3H), 6.87 (br s, 2H), 5.21 - 4.69 (m, 2H), 3.49 - 3.34 (m, 2H), 2.62 (s, 3H), 2.12 - 1.85 (m, 1H), 1.09 - 0.50 (m, 6H). LC-MS: Rt = 2.312 min, (ESI) *m*/*z.* [M+H]⁺ 473.2; C₂₄H₂₄N₈OS | 306 | 1H NMR (400 MHz, DMSO-d6) δ = 9.16 (br s, 1H), 9.07 (s, 1H), 8.36 (br s, 1H), 7.97 (br s, 1H), 7.77 (d, J = 9.5 Hz, 1H), 7.59 - 7.39 (m, 3H), 6.85 (br s, 2H), 5.24 (br d, J = 4.4 Hz, 1H), 3.26 (dd, J = 7.2, 13.6 Hz, 1H), 2.91 - 2.75 (m, 1H), 2.63 (s, 3H), 2.07 - 1.87 (m, 1H), 1.64 (br s, 3H), 1.01 - 0.55 (m, 6H) LC-MS: Rt = 2.726 min , |
| | | | (ESI) *m*/*z* = 510.2 [M+H]+; C₂₆H₂₆F₃N₇O |
| 307 | 1H NMR (400 MHz, DMSO-d6) δ = 9.10 - 8.96 (m, 1H), 8.61 (br d, J = 3.8 Hz, 1H), 8.32 - 8.14 (m, 2H), 8.11 - 7.84 (m, 2H), 7.45 - 7.28 (m, 3H), 6.87 (br s, 2H), 5.11 - 4.89 (m, 2H), 3.39 - 3.33 (m, 2H), 2.61 (s, 3H), 2.07 - 1.88 (m, 1H), 1.01 - 0.65 (m, 6H) LC-MS: Rt = 2.514 min, (ESI) *m*/*z.* [M+H]⁺ 472.1; C₂₅H₂₅N₇OS | 308 | 1H NMR (400 MHz, DMSO-d6) δ = 12.50 - 11.98 (m, 1H), 8.92 (s, 1H), 7.85 (d, J = 7.7 Hz, 1H), 7.75 (t, J = 7.7 Hz, 1H), 7.49 - 7.40 (m, 2H), 7.22 (d, J = 7.5 Hz, 1H), 7.09 (s, 1H), 6.58 - 6.53 (m, 2H), 4.53 (br s, 2H), 3.30 (br d, J = 6.8 Hz, 2H), 2.64 (s, 4H), 2.57 (s, 3H), 0.92 - 0.81 (m, 7H) LC-MS: Rt = 2.380 min , |
| | | | (ESI) *m*/*z* = 469.2 [M+H]+; C₂₆H₂₈N₈O |
| 309 | 1H NMR (400 MHz, DMSO-d6) Shift 8.99 (br s, 1H), 8.61-8.42 (m, 2H), 7.88 (br s, 1H), 8.00-7.79 (m, 1H), 7.61-7.48 (m, 2H), 7.39 (br d, J = 7.9 Hz, 1H), 7.25 (br t, J = 7.7 Hz, 1H), 6.89 (dt, J = 0.9, 6.8 Hz, 1H), 6.85 (br s, 2H), 4.62 (br s, 2H), 3.30-3.18 (m, 2H), 2.63 (s, 3H), 2.14-1.93 (m, 1H), 0.99-0.61 (m, 6H). LC-MS: Rt = 2.316 min, (ESI) *m*/*z.* [M+H]⁺ 428.3; C₂₄H₂₅N₇O | 310 | 1H NMR (400 MHz, DMSO-d6) δ ppm 9.01 (br s, 1 H) 8.14 (s, 1 H) 7.13 - 7.47 (m, 3 H) 6.86 (br s, 2 H) 4.89 (br s, 2 H) 3.89 (br s, 2 H) 3.41 (br s, 3 H) 2.91 (br s, 1 H) 2.62 (s, 3 H) 1.33 - 2.15 (m, 6 H) 0.50 - 1.04 (m, 6 H) |
| | | | LC-MS: Rt = 2.473 min , (ESI) *m*/*z* = 479.2 [M+H]+; C₂₅H₃₀N₆O₂S |
| 311 | 1H NMR (400 MHz, DMSO-d6) Shift 9.18 (br s, 1H), 9.13-9.03 (m, 1H), 8.47 (br s, 1H), 8.21-8.11 (m, 1H), 7.98 (br s, 1H), 7.77 (br d, J = 9.4 Hz, 1H), 7.56-7.42 (m, 4H), 4.85-4.64 (m, 3H), 3.27-3.15 (m, 2H), 2.65 (s, 3H), 2.15-2.02 (m, 1H), 0.93 (br s, 4H), 0.70 (br s, 2H). LC-MS: Rt = 2.642 min, (ESI) *m*/*z.* [M+H]⁺ 496.3; C₂₅H₂₄F₃N₇O | 312 | 1H NMR (400MHz, DMSO-d6) Shift = 9.05 (br s, 1H), 8.29 - 8.11 (m, 2H), 7.77 (br s, 2H), 7.52 - 7.30 (m, 2H), 7.22 (d, J=7.9 Hz, 1H), 6.88 (br s, 2H), 4.99 (br s, 2H), 3.42 - 3.34 (m, 2H), 2.62 (s, 3H), 2.52 (br s, 3H), 2.15 - 1.87 (m, 1H), 1.98 (br s, 1H), 1.00 - 0.58 (m, 6H). |
| | | | LC-MS: Rt = 2.702 min, (ESI) *m*/*z.* [M+H]⁺ 486.2; C₂₆H₂₇N₇OS |
| 313 | NT | 314 | 1H NMR (400 MHz, DMSO-d6) δ ppm 9.02 (br s, 1 H) 8.39 - 8.53 (m, 2 H) 7.91 (br s, 1 H) 7.49 - 7.62 (m, 2 H) 7.41 (d, J=8.25 Hz, 1 H) 7.21 - 7.28 (m, 1 H) 6.74 - 7.01 (m, 3 H) 5.18 (br s, 1 H) 3.02 - 3.28 (m, 2 H) 2.62 (s, 3 H) 1.61 (br s, 3 H) 0.84 - 1.20 (m, 3 H) LC-MS: Rt = 2.178 min , (ESI) *m*/*z* = 414.2 [M+H]+; C₂₃H₂₃N₇O |
| 315 | 1H NMR (400 MHz, DMSO-d6) Shift 9.02 (br s, 1H), 8.85 (br s, 1H), 8.79 (d, J = 5.0 Hz, 2H), 8.31 (br s, 1H), 8.11 (br d, J = 7.6 Hz, 1H), 7.59 (br d, J = 7.9 Hz, 1H), 7.52 (br d, J = 5.0 Hz, 1H), 7.46-7.37 (m, 2H), 6.93-6.79 (m, 2H), 5.43 (td, J = 1.7, 3.6 Hz, 1H), 4.93 (br d, J = 17.1 Hz, 1H), 4.57 (br d, J = 16.6 Hz, 1H), 2.63 (br s, 3H), 1.63 (br d, J = 7.0 Hz, 3H). LC-MS: Rt = 2.472 min, (ESI) *m*/*z.* [M+H]⁺ 507.2; C₂₅H₂₁F₃N₈O | 316 | 1H NMR (400 MHz, DMSO-d6) Shift 8.80 (s, 1H), 8.46 (d, J=7.00 Hz, 1H), 7.96 (dd, J=2.13, 8.38 Hz, 1H), 7.85 (s, 1H), 7.74 (s, 2H), 7.53 (d, J=8.25 Hz, 1H), 7.16 (d, J=12.38 Hz, 1H), 7.11 (d, J=1.63 Hz, 1H), 6.42 (t, J=5.94 Hz, 1H), 5.22 (d, J=1.63 Hz, 1H), 4.62 (t, J=5.25 Hz, 2H), 4.38 (br d, J=4.63 Hz, 4H), 2.86 (s, 3H). LC-MS: RT =0.764 min, (ESI) *m*/*z.* [M+H]⁺ 529.3; |
| | | | C₂₄H₂₀N₈O₂F₄ |
| 317 | 1H NMR (400 MHz, DMSO-d6) δ 7.88 (br d, J=5.72 Hz, 1H), 7.79 (s, 1H), 7.64 (br s, 1H), 7.49 (br s, 2H), 7.17-7.33 (m, 2H), 7.05 (br d, J=10.56 Hz, 1H), 6.07 (br s, 1H), 4.78 (br s, 2H), 3.68 (br s, 1H), 3.38-3.44 (m, 3H), 2.85 (s, 3H), 0.89-0.98 (m, 4H) . LC-MS: Rt =0.645 min, (ESI) *m*/*z.* [M+H]+ 460.4; | 318 | 1H NMR (400 MHz, DMSO-d6) δ 7.88 (br d, J=5.72 Hz, 1H), 7.79 (s, 1H), 7.64 (br s, 1H), 7.49 (br s, 2H), 7.17-7.33 (m, 2H), 7.05 (br d, J=10.56 Hz, 1H), 6.07 (br s, 1H), 4.78 (br s, 2H), 3.68 (br s, 1H), 3.38-3.44 (m, 3H), 2.85 (s, 3H), 0.89-0.98 (m, 4H) . LC-MS: Rt =0.645 min, (ESI) *m*/*z.* [M+H]+ 460.4; |
| | C₂₃H₂₂FN₉₀ | | C₂₃H₂₂FN₉₀ |
| 319 | 1H NMR (400 MHz, DMSO-d6) Shift 7.88 (br d, J=4.63 Hz, 1H), 7.73-7.83 (m, 2H), 7.38-7.55 (m, 3H), 7.16-7.29 (m, 1H), 7.05 (br d, J=11.38 Hz, 1H), 6.06 (br s, 1H), 5.10 (q, J=9.05 Hz, 2H), 4.84 (br s, 2H), 3.40 (br s, 3H), 2.85 (s, 3H) 19F NMR (376 MHz, DMSO-d6) Shift -70.40 (s, 3F), -118.09 (br s, 1F) LC-MS: (ESI) m/z=502.1 [M+1]+; RT=1.544 min | 320 | 1H NMR (400 MHz, DMSO-d6) δ 7.74-8.35 (m, 3H), 7.49 (s, 2H), 6.72-7.40 (m, 4H), 6.06 (br s, 1H), 4.53-5.15 (m, 2H), 3.41-3.75 (m, 7H), 3.23 (s, 3H), 2.92 (s, 3H), 2.85 (s, 3H). LC-MS: (ESI) m/z=518.4 [M+1] +, RT= 0.704 min |
| 321 | 1H NMR (400 MHz, DMSO-d6) 9.24 (s, 1H), 8.09 (s, 1H), 7.99 (br s, 1H), 7.80 (s, 1H), 7.72 (br d, J=9.38 Hz, 1H), 7.33-7.50 (m, 6H), 5.03-5.19 (m, 2H), 3.55 (s, 3H), 2.84 (s, 3H) 19F NMR (376 MHz, DMSO-d6) - 60.48 (s, 1F), -174.29 (br s, 1F) LC-MS: (ESI) m/z=538.1 [M+1] +; RT=2.897 min | 322 | 1H NMR (400 MHz, DMSO-d6) δ 9.39 (br s, 1H), 9.16 (s, 1H), 8.24-8.43 (m, 1H), 7.78-7.99 (m, 2H), 7.55 (s, 2H), 7.31 (br s, 1H), 7.10 (br d, J=11.29 Hz, 1H), 5.08-5.39 (m, 2H), 3.61 (s, 3H), 2.85 (br s, 3H). LC-MS: (ESI) m/z=557.3 [M+1] +, RT= 0.749 min |
| 323 | 1H NMR (400 MHz, DMSO-d6) δ 8.66 (s, 1H), 7.75-8.02 (m, 3H), 7.44-7.55 (m, 3H), 7.19 (br s, 1H), 7.09 (br d, J=11.66 Hz, 1H), 6.11 (br s, 1H), 4.83-5.43 (m, 2H), 4.44 (s, 1H), 3.57 (br s, 3H), 2.85 (s, 3H). LC-MS: (ESI) m/z=455.1 [M+1] +, RT= 0.800 min | 324 | 1H NMR (400 MHz, DMSO-d6) δ 9.25 (br s, 1H), 8.30 (br d, J=3.38 Hz, 2H), 8.09 (s, 1H), 7.87 (s, 1H), 7.67-7.74 (m, 3H), 7.41-7.49 (m, 1H), 7.26 (br d, J=4.00 Hz, 1H), 4.94-5.26 (m, 2H), 3.56 (s, 3H), 2.96 (s, 3H). LC-MS: (ESI) m/z = 539.3 [M+1] +, RT = 0.755 min 19F NMR (376 MHz, DMSO-d6) δ -60.46 (3F), -175.89 (1F) |
| 325 | 1H NMR (400 MHz, DMSO-d6) Shift 8.93 (br s, 1H), 8.00-8.15 (m, 1H), 7.92 (br d, J=4.88 Hz, 1H), 7.81 (s, 1H), 7.65 (d, J=9.38 Hz, 1H), 7.54 (s, 2H), 7.37 (br d, J=9.51 Hz, 1H), 6.90-7.32 (m, 3H), 4.94-5.30 (m, 2H), 3.57 (s, 3H), 2.86 (br s, 3H), 2.08 (s, 1H) 19F NMR (376 MHz, DMSO-d6) Shift -109.29 (br s, 1F), -118.19 (br s, 1F), -173.53 (br s, 1F) LC-MS: (ESI) m/z= 538.3 [M+1] +, RT=0.723 min | 326 | 1H NMR (400 MHz, DMSO-d6) δ 8.06-8.20 (m, 1H), 7.90 (br d, J=6.16 Hz, 1H), 7.73-7.87 (m, 3H), 7.52 (s, 2H), 7.21 (br s, 1H), 7.09 (br d, J=11.22 Hz, 1H), 6.09-6.38 (m, 1H), 4.87-5.53 (m, 2H), 3.65 (s, 3H), 2.84 (s, 3H) LC-MS: (ESI) m/z=499.4 [M+1] +, RT= 0.776 min |

### Biological Test Example 2 Proliferation Inhibition of HCT116 and HCT116 MTAP-KO cells in vitro

### Experimental Materials:

HCT116 cell line was purchased from the Chinese Academy of Sciences Cell Bank, and the MTAP gene was knocked out by CRISPR/Cas9 technology to obtain HCT116-MTAP-KO cell line.

McCoy's 5A medium (Gibco, catalog No.16600082), fetal bovine serum (Gibco, catalog No.10099141C), penicillin-streptomycin double antibody (Gibco, catalog No.15140122), pancreatic enzyme (Gibco, catalog No.25200056), CellTiter Glo assay kit (Promega, catalog No.G7572), 384-well transparent flat-bottomed black walled cell culture plate (Corning, catalog No.3764), ultra micro sampler (Tecan, catalog No.D300e), Multimode reader (Biotek, catalog No. SynergyHTX)

### Experimental method:

1. Cell culture: HCT116 and HCT116-MTAP-KO cells were cultured in McCoy'5A medium + 10% fetal bovine serum + 1% penicillin-streptomycin double antibody; to ensure that they were always in the logarithmic growth phase and the cell viability greater than 95%.
2. Compound concentration gradient preparation: The compound to be tested was added to a 384-well plate using an ultra micro sampler, starting from 30 µM (HCT116 cells) or 3 µM (HCT116-MTAP-KO cells), and diluted with DMSO at 3 times for a total of 9 concentrations and set up three duplicate wells.
3. Treatment of cells with compounds: Trypsin-digested HCT116 or HCT116-MTAP-KO cell suspension was added to the 384-well plates spotted with the compounds to be tested at 40 µL per well, i.e., 100 cells per well, and the final concentration of DMSO was 0.4%. The cell culture plate was incubated at 37°C in a 5% CO₂ incubator for 6 days.
4. Detection: 20 µL of CellTiter Glo reagent was added to each well of the cell culture plate and incubated at room temperature for 30 minutes. A multimode reader was used to detect the luminescence signal at 578 nm.
5. Data analysis:
   The data was fitted with GraphPad Prism 8.0 software using a four parameter inhibitor-reaction model to obtain the IC₅₀ value (half inhibitory concentration) of the test compounds.

The biological activities of some of the compounds were measured by experimental methods. "A" represents IC₅₀ (nm)<100, "B" represents 100<IC₅₀ (nm)<1000, "C" represents 1000<IC₅₀ (nm)<10000, as shown in Table 4.

Among them, the first column represents cell proliferation inhibition rate HCT116 MTAP WT IC₅₀ (nm), and the second column represents cell proliferation inhibition rate HCT116-MTAP null IC50 (nm):
The corresponding structures of the tested products are shown in Tables 1 and 2, and the activity test results are as follows:

**Table 4**

| Example No. | Cell proliferation inhibition rate HCT116 MTAP WT IC₅₀ (nm) | Cell proliferation inhibition rate HCT116 MTAP null IC₅₀ (nm) | Example No. | Cell proliferation inhibition rate HCT116 MTAP WT IC₅₀ (nm) | Cell proliferation inhibition rate HCT116 MTAP null IC₅₀ (nm) |
|---|---|---|---|---|---|
| 3 | C | A | 2 | C | B |
| 9 | C | A | 12 | C | B |
| 13 | C | B | 14 | C | A |
| 15 | C | B | 16 | C | B |
| 17 | C | B | 18 | C | A |
| 21 | C | B | 24 | C | B |
| 23 | C | B | 28 | C | B |
| 27 | B | A | 30 | B | A |
| 29 | C | B | 32 | C | B |
| 31 | B | A | 34 | C | A |
| 33 | B | A | 36 | A | B |
| 35 | C | B | 38 | C | B |
| 37 | C | B | 40 | C | B |
| 41 | C | B | 42 | C | B |
| 43 | C | A | 44 | C | B |
| 45 | C | B | 46 | B | C |
| 47 | B | A | 48 | C | A |
| 49 | C | B | 50 | C | A |
| 53 | C | A | 54 | C | A |
| 61 | C | B | 56 | C | B |
| 63 | C | C | 58 | C | B |
| 65 | C | C | 60 | C | A |
| 67 | C | B | 70 | C | B |
| 71 | C | A | 80 | C | B |
| 73 | C | B | 84 | C | A |
| 75 | C | B | 86 | C | B |
| 77 | C | A | 88 | C | B |
| 79 | C | A | 90 | C | A |
| 81 | C | B | 92 | C | B |
| 83 | C | A | 94 | C | A |
| 85 | C | A | 96 | C | B |
| 87 | C | A | 98 | C | B |
| 89 | C | A | 100 | C | A |
| 91 | C | B | 102 | C | A |
| 93 | C | B | 104 | C | B |
| 95 | C | B | 108 | C | A |
| 97 | C | A | 110 | B | A |
| 99 | C | B | 112 | C | A |
| 101 | B | A | 114 | C | A |
| 103 | C | A | 116 | C | B |
| 105 | C | A | 118 | C | A |
| 107 | C | B | 120 | C | A |
| 109 | C | A | 122 | C | B |
| 111 | C | A | 124 | C | A |
| 113 | C | A | 128 | C | B |
| 115 | C | B | 130 | C | B |
| 117 | C | B | 132 | C | B |
| 119 | C | B | 134 | A | A |
| 121 | C | A | 136 | C | A |
| 123 | C | B | 138 | C | A |
| 125 | C | A | 140 | C | B |
| 127 | C | B | 144 | C | A |
| 129 | C | A | 146 | C | A |
| 131 | C | A | 148 | C | B |
| 133 | C | B | 150 | B | A |
| 135 | C | A | 152 | C | B |
| 137 | C | A | 154 | C | B |
| 139 | C | A | 156 | C | B |
| 143 | B | A | 158 | C | B |
| 145 | B | A | 160 | C | B |
| 147 | C | B | 166 | C | A |
| 149 | C | B | 168 | A | A |
| 151 | C | B | 170 | A | A |
| 153 | B | A | 172 | C | A |
| 155 | C | A | 176 | C | A |
| 157 | C | A | 178 | C | A |
| 159 | C | B | 180 | C | A |
| 161 | A | A | 182 | C | A |
| 163 | C | A | 184 | A | A |
| 165 | C | A | 188 | C | A |
| 167 | A | A | 190 | C | B |
| 169 | B | A | 192 | C | A |
| 171 | C | A | 194 | C | A |
| 173 | C | A | 196 | C | A |
| 175 | C | A | 200 | B | A |
| 177 | B | A | 202 | C | B |
| 179 | C | A | 204 | C | B |
| 181 | C | A | 206 | B | A |
| 183 | C | A | 208 | C | A |
| 187 | C | A | 210 | C | A |
| 189 | B | A | 212 | C | A |
| 191 | C | B | 214 | C | A |
| 193 | B | A | 216 | C | A |
| 195 | B | A | 218 | C | A |
| 197 | B | A | 220 | B | A |
| 199 | C | A | 222 | B | A |
| 201 | B | A | 224 | C | A |
| 203 | C | A | 228 | B | A |
| 205 | B | A | 232 | C | B |
| 207 | C | A | 234 | C | A |
| 209 | C | A | 236 | C | A |
| 211 | C | A | 238 | C | A |
| 213 | C | A | 240 | C | A |
| 215 | C | A | 244 | C | A |
| 217 | C | A | 248 | C | A |
| 219 | C | A | 250 | C | B |
| 221 | C | B | 252 | C | B |
| 223 | C | A | 256 | C | B |
| 225 | C | B | 260 | C | A |
| 227 | C | B | 262 | C | B |
| 229 | B | A | 266 | C | B |
| 231 | C | B | 270 | C | B |
| 241 | C | B | 272 | C | B |
| 243 | C | B | 274 | C | B |
| 245 | C | B | 276 | C | A |
| 249 | C | B | 278 | B | B |
| 251 | C | B | 280 | B | A |
| 253 | C | B | 282 | C | A |
| 255 | C | A | 284 | C | B |
| 257 | C | B | 286 | C | A |
| 259 | C | B | 288 | B | A |
| 263 | C | A | 290 | C | A |
| 265 | C | B | 292 | C | A |
| 267 | C | A | 294 | C | B |
| 269 | C | A | 298 | C | B |
| 271 | C | A | 300 | C | A |
| 273 | C | A | 302 | C | B |
| 275 | C | A | 306 | C | B |
| 279 | C | A | 308 | C | B |
| 281 | C | B | 310 | C | A |
| 285 | C | A | 312 | C | A |
| 287 | B | A | 314 | C | B |
| 295 | C | A | 318 | C | B |
| 299 | C | A | 320 | C | B |
| 301 | C | B | 324 | B | A |
| 303 | C | A | 326 | C | A |
| 305 | C | A | 411 | B | A |
| 307 | C | A | 413 | B | A |
| 309 | C | A | 419 | C | B |
| 311 | C | A | 421 | C | A |
| 315 | A | A | 412 | C | B |
| 317 | C | A | 414 | C | B |
| 319 | C | B | 416 | C | B |
| 321 | C | A | 418 | C | B |
| 325 | B | A | | | |

All documents mentioned in the present invention are cited as references in this application, just as each document is individually cited as a reference. In addition, it should be understood that, after reading the above teaching content of the present invention, those skilled in the art can make various changes or modifications to the present invention, and these equivalent forms also fall within the scope defined by the appended claims of the present application.

## Claims

1. A compound of formula I or formula II, or a pharmaceutically acceptable stereoisomer, a salt or a deuterated product thereof: 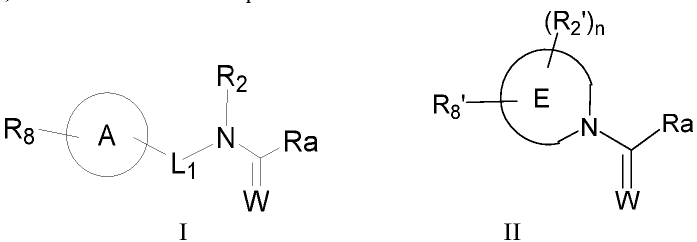 wherein,
Ra is 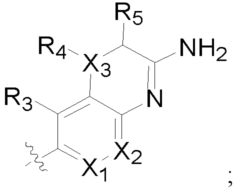
W is O or S;
X₁, and X₂ are each independently selected from the group consisting of: CR and N; X₃ is N;
L₁ is selected from the group consisting of: chemical bond, -CHR-, and -C(R)R-;
ring A is selected from the group consisting of: substituted or unsubstituted 7-12 membered bridged ring (including carbocycle or heterocycle), substituted or unsubstituted 7-12 membered spirocyclic ring (including carbocycle or heterocycle), substituted or unsubstituted 8-12 membered fused bicyclic heterocyclyl (including carbocycle or heterocycle, preferably five-membered fused six-membered ring), substituted or unsubstituted 7-10 membered fused bicyclic heteroaryl (preferably five-membered fused six-membered ring), or ring A is substituted or unsubstituted 3-7 membered carbocycle or heterocycle, and substituted or unsubstituted 5-6 membered aromatic ring or heteroaromatic ring;
ring E is selected from the group consisting of: substituted or unsubstituted 3-7 membered monocyclic heterocycle, substituted or unsubstituted 7-12 membered bridged heterocycle, substituted or unsubstituted 7-12 membered spiral heterocycle, substituted or unsubstituted 8-12 membered fused polycyclic heterocycle (such as fused bicyclic ring);
R₈ is selected from the group consisting of: H, deuterium, halogen, cyano, C₂-C₆ alkynyl, -SF₅, amino, nitro, hydroxyl, thiol, aldehyde group, carboxyl, substituted or unsubstituted or halogenated C₁-C₆ alkyl, and substituted or unsubstituted or halogenated C₁-C₆ alkoxyl, or R₈ is 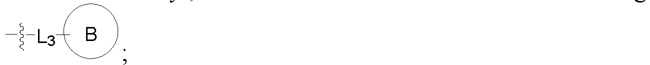
R₈' is selected from the group consisting of: H, deuterium, halogen, cyano, amino, nitro, hydroxyl, thiol, aldehyde group, carboxyl, unsubstituted or halogenated C₁-C₆ alkyl, substituted or unsubstituted benzene ring, substituted or unsubstituted 5-12 membered heteroaromatic ring, substituted or unsubstituted C₃-C₁₀ carbocycle (including saturated or partially unsaturated situations substituted or unsubstituted 3-12 membered heterocycle (including saturated or partially unsaturated situations), or R₈' is 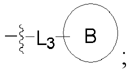
L₃ is selected from the group consisting of: chemical bonds, -O-, -CHR-, -C(R)R-, carbonyl, S, and -NH-;
ring B is selected from the group consisting of: substituted or unsubstituted benzene ring, substituted or unsubstituted 5-6-membered heteroaromatic ring, substituted or unsubstituted C₃-C₆ carbocycle (including saturated and partially unsaturated situations), substituted or unsubstituted 3-7-membered heterocycle (including saturated and partially unsaturated situations);
R₂ and R₂' are independently selected from the group consisting of: R₇, and -L₂R₇; wherein, L₂ is selected from the group consisting of: -O-, -CHR-, and -C(R)R-; wherein, R₇ is selected from the group consisting of: H, none, substituted or unsubstituted C₁-C₆ alkyl, substituted or unsubstituted C₆₋₁₀ aromatic ring, substituted or unsubstituted 5-12 membered (preferably 5-6 membered or 8-10 membered) heteroaromatic ring, substituted or unsubstituted C₃-C₁₀ carbocycle (including saturated and partially unsaturated situations, including single ring, fused ring, spirocyclic ring and bridged ring), substituted or unsubstituted 3-10 membered heterocycle (including saturated and partially unsaturated situations, including single ring, fused ring, spirocyclic ring and bridged ring); n is 0, 1, 2 or 3;
R₃ is selected from the group consisting of H, deuterium, halogen, cyano and substituted or unsubstituted C₁-C₆ alkyl;
R₄ and R₅ together with the attached ring atoms form a 5-12 membered saturated or unsaturated ring, and the ring can be substituted or unsubstituted;
R is H, deuterium, halogen, substituted or unsubstituted C₁-C₄ alkyl, substituted or unsubstituted C₁-C₄ alkoxyl, or substituted or unsubstituted C₃-C₆ cycloalkyl;
unless otherwise specified, in each of the above formulas, the "substituted" refers to that hydrogen atoms on the corresponding group is substituted by one or more substituents selected from the group consisting of: deuterium, tritium, halogen, hydroxyl, carboxyl, thiol, benzyl, C₁-C₁₂ alkoxycarbonyl, C₁-C₆ aldehyde group, amino, C₁-C₆ amide group, nitro, cyano, unsubstituted or halogenated C₁-C₆ alkyl, unsubstituted or halogenated C₁-C₆ alkyl-O-C₁-C₆ alkyl -, unsubstituted or halogenated C₁-C₆ alkyl-O-C₁-C₆ alkyl-O-, unsubstituted or halogenated C₁-C₆ alkylene-OH, unsubstituted or halogenated C₃-C₈ cycloalkyl, C₂-C₁₀ alkenyl, unsubstituted or halogenated C₁-C₆ alkoxyl, C₁-C₆ alkyl-amino, C₆-C₁₀ aryl, five-membered or six-membered heteroaryl, five-membered or six-membered non-aromatic heterocyclyl, -O-(C₆-C₁₀ aryl), -O- (five-membered or six-membered heteroaryl), C₁-C₁₂ alkylamino carbonyl, unsubstituted or halogenated C₂-C₁₀ acyl, sulfonyl (-SO₂-OH), phosphoryl-(-PO₃-OH), unsubstituted or halogenated C₁-C₄ alkyl-S(O)₂-, unsubstituted or halogenated C₁-C₄ alkyl-SO-, and -SF₅.

2. The compound according to claim 1, or a pharmaceutically acceptable stereoisomer, a salt or a deuterated product thereof, wherein, Ra is selected from the group consisting of: 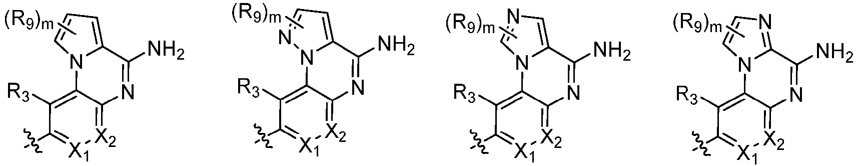 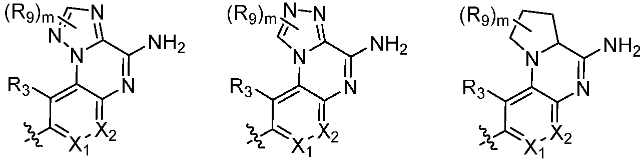 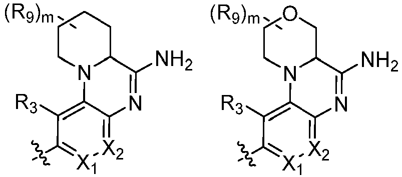 Wherein, R₉ is selected from the group consisting of: deuterium, tritium, halogen, hydroxyl, carboxyl, unsubstituted or halogenated C₁-C₆ alkyl, unsubstituted or halogenated C₁-C₆ alkoxyl, unsubstituted or substituted C₁-C₆ alkyl-OH, - NH (unsubstituted or halogenated C₁-C₆ alkyl), - N(unsubstituted or halogenated C₁-Cs alkyl)₂; m is selected from 0, 1, 2, and 3; preferably, R₉ is selected from the group consisting of: deuterium, tritium, halogen, and unsubstituted or halogenated C₁-C₆ alkyl.

3. The compound according to claim 1, or a pharmaceutically acceptable stereoisomer, a salt or a deuterated product thereof, wherein, L₁ is -CHR- or -C(R)R-; ring A is selected from the group consisting of: substituted or unsubstituted 8-12 membered fused bicyclic heterocyclyl, and substituted or unsubstituted 7-10 membered fused bicyclic heteroaryl; R₈ is selected from the group consisting of: H, halogen, cyano, amino, C₂-C₆ alkynyl, SF₅, hydroxyl, thiol, aldehyde group, carboxyl, unsubstituted or halogenated C₁-C₆ alkyl, and 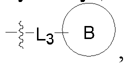 and ring B is selected from the group consisting of: substituted or unsubstituted benzene ring, substituted or unsubstituted 5-6 membered heteroaromatic ring, substituted or unsubstituted C₃-C₆ carbocycle, and substituted or unsubstituted 3-6 membered heterocycle; L₃ is selected from the group consisting of: chemical bond, -O-, -CHR-, carbonyl, S, and -NH-.

4. The compound according to claim 1, or a pharmaceutically acceptable stereoisomer, a salt or a deuterated product thereof, wherein, R₂ is selected from the group consisting of: R₇, and -L₂R₇; wherein, L₂ is selected from the group consisting of: -O-, -CHR-, carbonyl, S, and -NH-; wherein, R₇ is selected from the group consisting of: substituted or unsubstituted C₁-C₆ alkyl, substituted or unsubstituted C₆₋₁₀ aromatic ring, substituted or unsubstituted 5-12 membered heteroaromatic ring.

5. The compound according to claim 1, or a pharmaceutically acceptable stereoisomer, a salt or a deuterated product thereof, wherein, R₂ is ortho-substituted 5-membered or 6-membered heteroaromatic ring, as shown below: 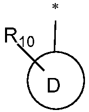
wherein, R₁₀ is a substituent located adjacent to the connecting site, and is selected from the group consisting of: hydrogen, deuterium, halogen, halogenated or unhalogenated C₁-C₃ alkyl, and halogenated or unhalogenated C₁-C₃ alkoxyl;
ring D is selected from the group consisting of: substituted or unsubstituted benzene ring, substituted or unsubstituted 5-6 membered heteroaromatic ring, preferably, ring D is selected from the group consisting of:

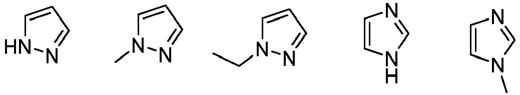

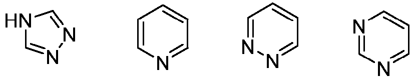

6. The compound according to claim 1, or a pharmaceutically acceptable stereoisomer, a salt or a deuterated product thereof, wherein, L₁ is -CH₂-, and -CH(CH₃)-; ring A is selected from the group consisting of: 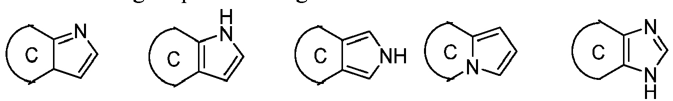 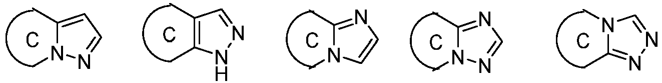 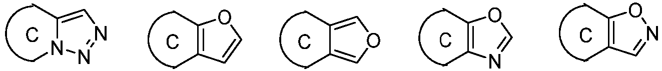 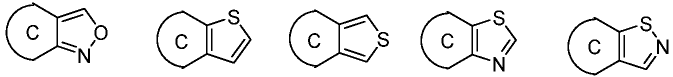 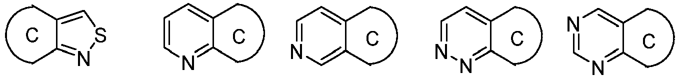 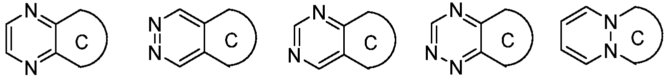
wherein, ring C is selected from the group consisting of: substituted or unsubstituted benzene ring, substituted or unsubstituted 5-6-membered heteroaromatic ring, substituted or unsubstituted C₃-C₆ carbocycle (including saturated and partially unsaturated situations), and substituted or unsubstituted 3-6-membered heterocycle (including saturated and partially unsaturated situations);
or, ring A is selected from the group consisting of: 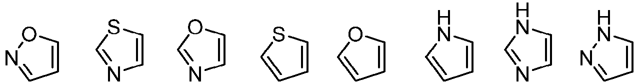 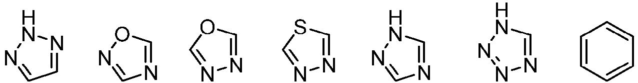 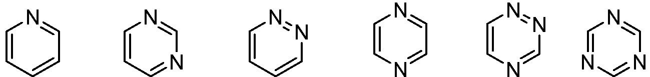
R₈ is halogenated or unhalogenated C₁-C₆ alkyl, or 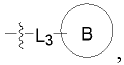
and ring B is selected form the group consisting of: substituted or unsubstituted benzene ring, substituted or unsubstituted 5-6 membered heteroaromatic ring, substituted or unsubstituted C₃-C₆ carbocycle, and substituted or unsubstituted 3-6 membered heterocycle; L₃ is selected from the group consisting of: chemical bond, -O-, -CHR-, carbonyl, S, and -NH-.

7. The compound according to claim 1, or a pharmaceutically acceptable stereoisomer, a salt or a deuterated product thereof, wherein, R₂ is selected from the group consisting of: R₇, and -(CHR)R₇; wherein, R₇ is selected from the group consisting of: hydrogen and none, substituted or unsubstituted C₁-C₆ alkyl, substituted or unsubstituted C₆₋₁₀ aromatic ring, substituted or unsubstituted 5-12 membered heteroaromatic ring, substituted or unsubstituted C₃-C₈ carbocycle (including saturated or partially unsaturated, including monocyclic, fused, spirocyclic or bridged rings), and substituted or unsubstituted 3-8-membered heterocycle (including saturated or partially unsaturated, including monocyclic, fused, spirocyclic or bridged rings).

8. The compound according to claim 1, or a pharmaceutically acceptable stereoisomer, a salt or a deuterated product thereof, wherein, the compound has a structure as shown in the following formulas: 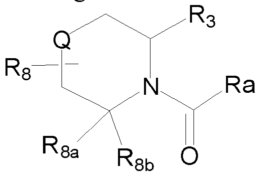
wherein, Q is O, NH, CH₂, or a chemical bond (i.e., 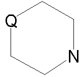
is a five membered ring);
R₈ is as described above;
R₈ₐ and R_{8b} are independently selected from H; or R₈ₐ and R_{8b} together with the attached carbon atom form a 4-7 membered carbocycle or heterocycle;
and when R₈ₐ and R_{8b} are independently H; R₈ₐ or R_{8b} may optionally substituted by R₈; when R₈ₐ and R_{8b} together with the attached carbon atom form a 4-7 membered carbocycle or heterocycle, R₈ may located on the carbocycle or heterocycle.

9. The compound according to claim 1, or a pharmaceutically acceptable stereoisomer, a salt or a deuterated product thereof, wherein, R₃ s selected from the group consisting of: H, deuterium, halogen, cyano, and substituted or unsubstituted C₁-C₆ alkyl.

10. The compound according to claim 1, or a pharmaceutically acceptable stereoisomer, a salt or a deuterated product thereof, wherein, R₂ is substituted or unsubstituted 5-7 membered heteroaromatic ring; and ring A is selected from the group consisting of: substituted or unsubstituted 5-6-membered aromatic ring or heteroaromatic ring, and substituted or unsubstituted 7-10-membered fused bicyclic heteroaryl; and R₈ is CF₃.

11. The compound according to claim 1, or a pharmaceutically acceptable stereoisomer, a salt or a deuterated product thereof, wherein, the compound is selected from the group consisting of:
**Table 1**
| No. | Structure | No. | Structure |
|---|---|---|---|
| 1 | 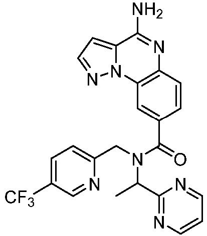 | 2 | 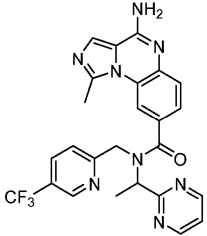 |
| 3 | 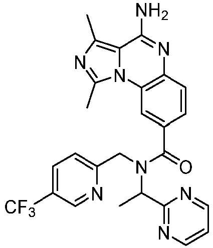 | 4 | 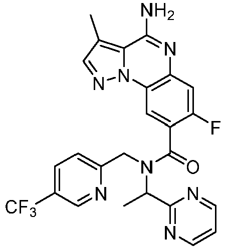 |
| 5 | 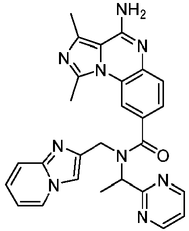 | 6 | 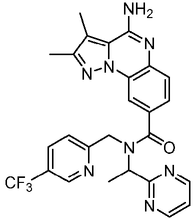 |
| 7 | 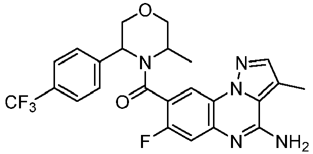 | 8 | 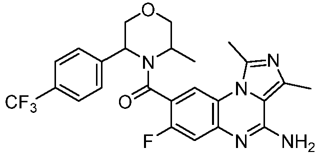 |
| 9 | 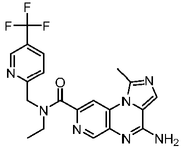 | 10 | 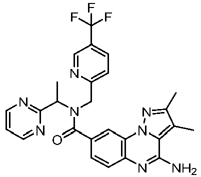 |
| 11 | 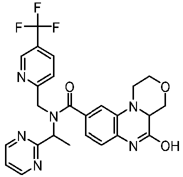 | 12 | 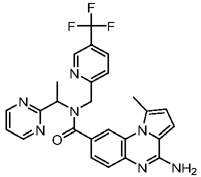 |
| 13 | 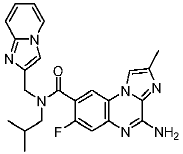 | 14 | 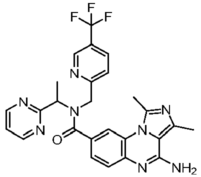 |
| 15 | 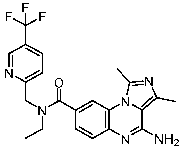 | 16 | 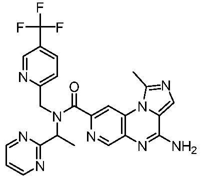 |
| 17 | 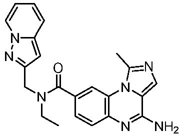 | 18 | 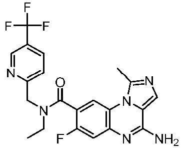 |
| 19 | 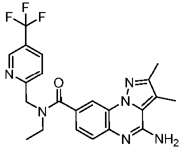 | 20 | 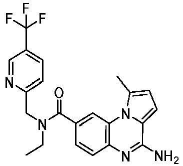 |
| 21 | 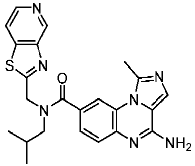 | 22 | 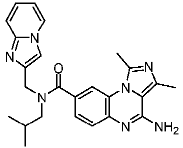 |
| 23 | 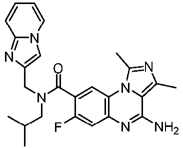 | 24 | 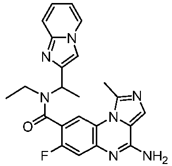 |
| 25 | 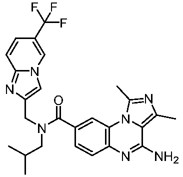 | 26 | 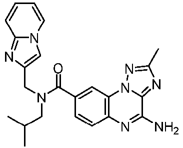 |
| 27 | 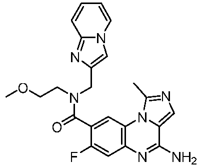 | 28 | 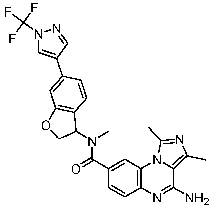 |
| 29 | 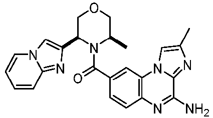 | 30 | 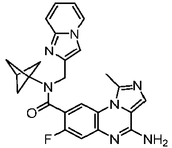 |
| 31 | 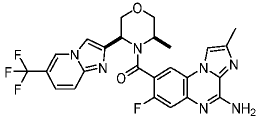 | 32 | 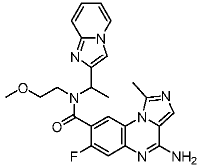 |
| 33 | 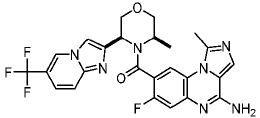 | 34 | 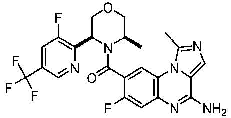 |
| 35 | 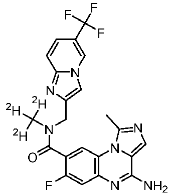 | 36 | 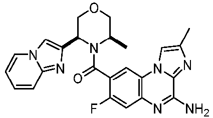 |
| 37 | 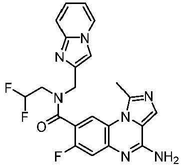 | 38 | 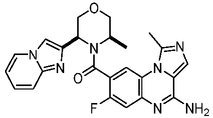 |
| 39 | 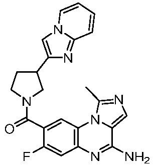 | 40 | 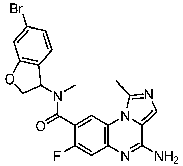 |
| 41 | 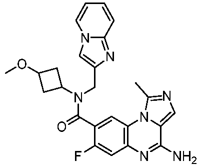 | 42 | 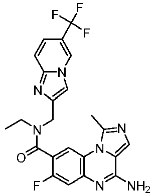 |
| 43 | 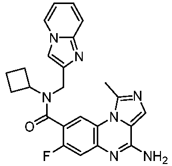 | 44 | 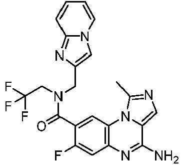 |
| 45 | 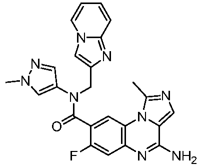 | 46 | 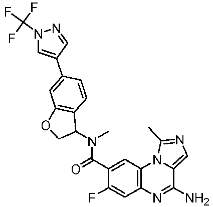 |
| 47 | 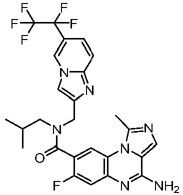 | 48 | 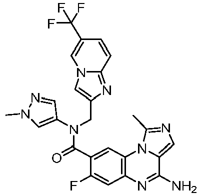 |
| 49 | 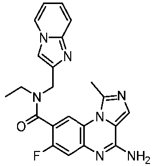 | 50 | 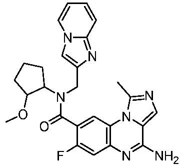 |
| 51 | 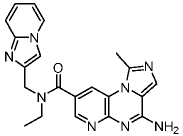 | 52 | 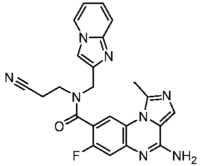 |
| 53 | 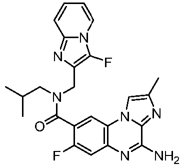 | 54 | 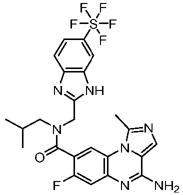 |
| 55 | 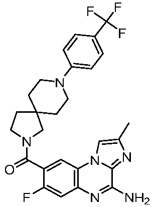 | 56 | 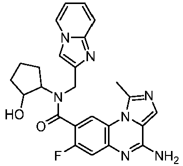 |
| 57 | 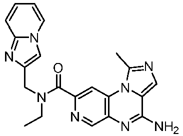 | 58 | 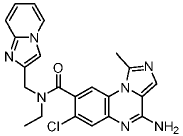 |
| 59 | 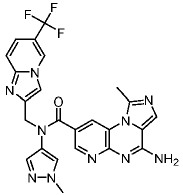 | 60 | 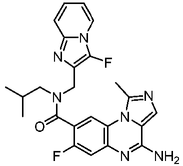 |
| 61 | 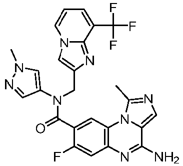 | 62 | 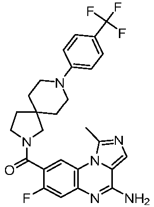 |
| 63 | 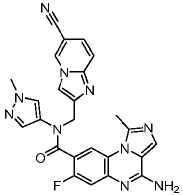 | 64 | 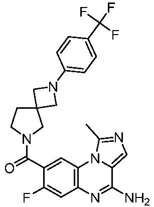 |
| 65 | 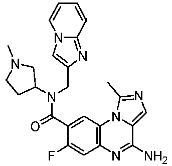 | 66 | 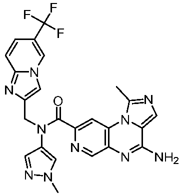 |
| 67 | 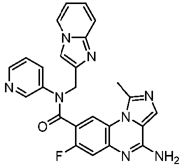 | 68 | 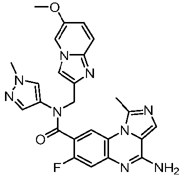 |
| 69 | 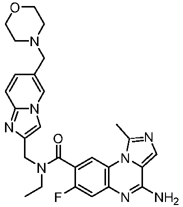 | 70 | 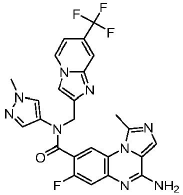 |
| 71 | 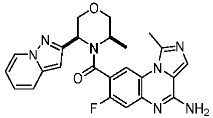 | 72 | 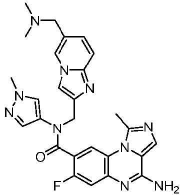 |
| 73 | 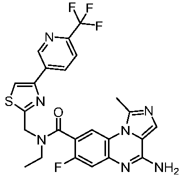 | 74 | 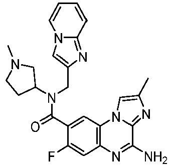 |
| 75 | 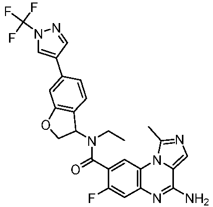 | 76 | 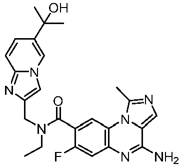 |
| 77 | 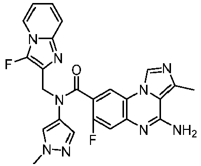 | 78 | 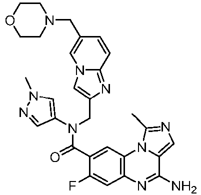 |
| 79 | 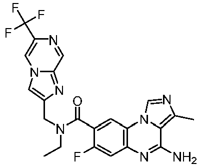 | 80 | 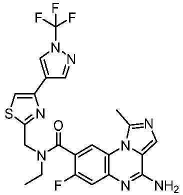 |
| 81 | 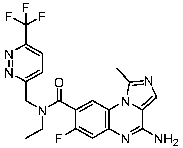 | 82 | 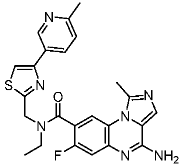 |
| 83 | 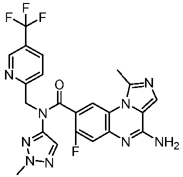 | 84 | 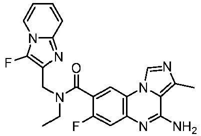 |
| 85 | 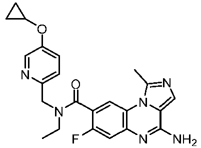 | 86 | 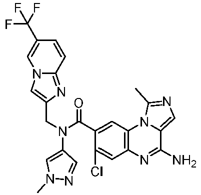 |
| 87 | 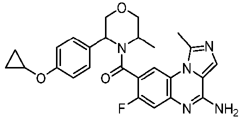 | 88 | 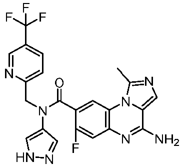 |
| 89 | 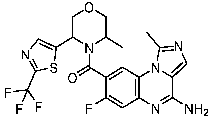 | 90 | 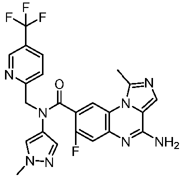 |
| 91 | 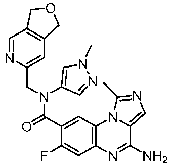 | 92 | 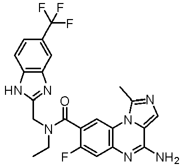 |
| 93 | 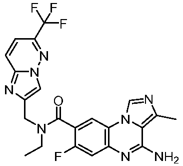 | 94 | 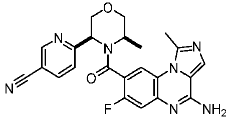 |
| 95 | 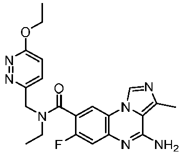 | 96 | 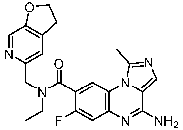 |
| 97 | 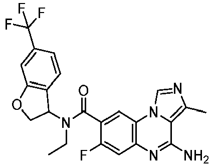 | 98 | 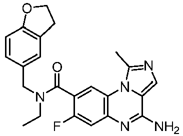 |
| 99 | 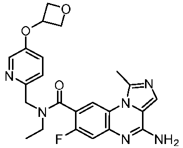 | 100 | 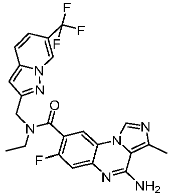 |
| 101 | 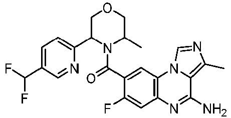 | 102 | 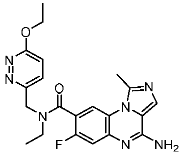 |
| 103 | 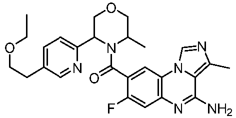 | 104 | 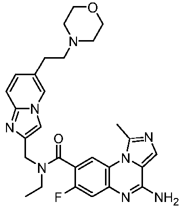 |
| 105 | 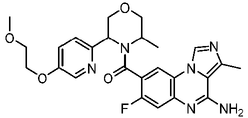 | 106 | 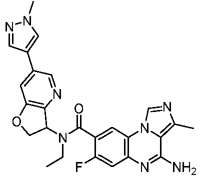 |
| 107 | 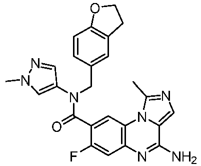 | 108 | 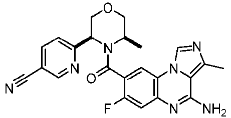 |
| 109 | 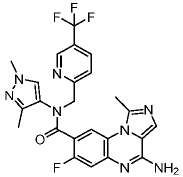 | 110 | 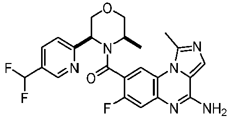 |
| 111 | 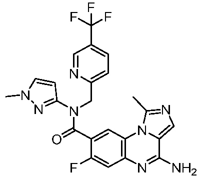 | 112 | 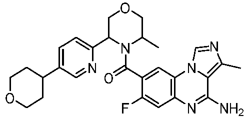 |
| 113 | 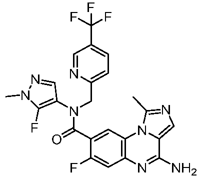 | 114 | 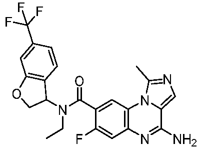 |
| 115 | 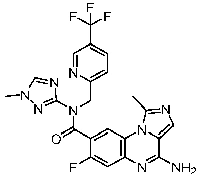 | 116 | 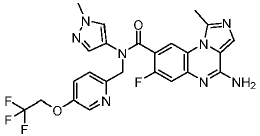 |
| 117 | 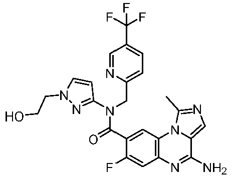 | 118 | 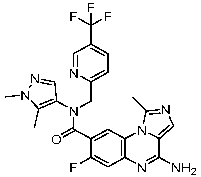 |
| 119 | 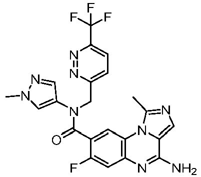 | 120 | 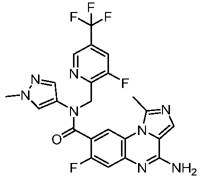 |
| 121 | 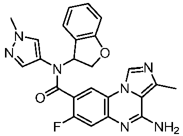 | 122 | 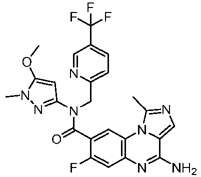 |
| 123 | 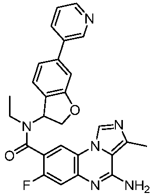 | 124 | 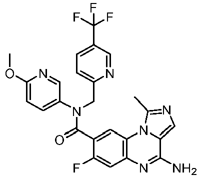 |
| 125 | 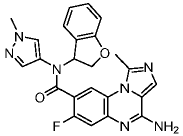 | 126 | 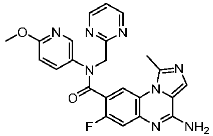 |
| 127 | 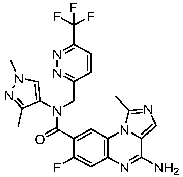 | 128 | 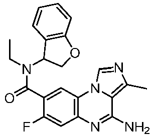 |
| 129 | 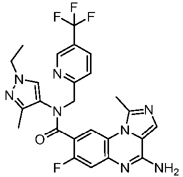 | 130 | 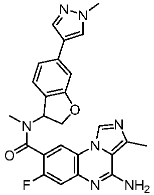 |
| 131 | 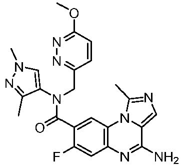 | 132 | 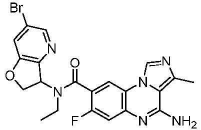 |
| 133 | 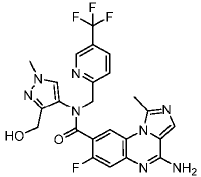 | 134 | 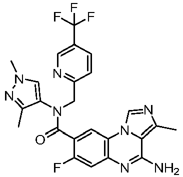 |
| 135 | 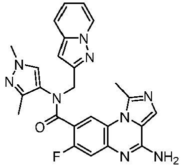 | 136 | 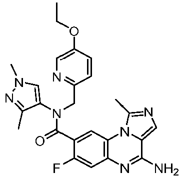 |
| 137 | 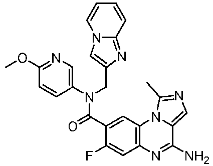 | 138 | 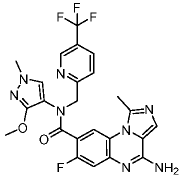 |
| 139 | 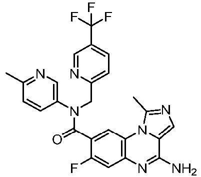 | 140 | 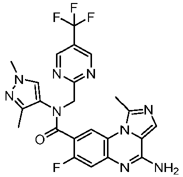 |
| 143 | 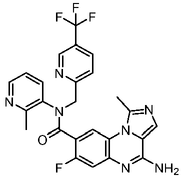 | 144 | 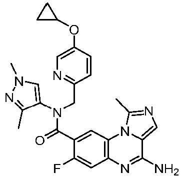 |
| 145 | 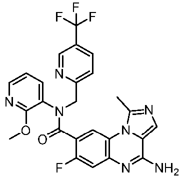 | 146 | 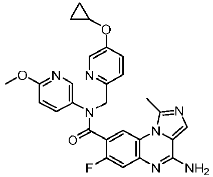 |
| 147 | 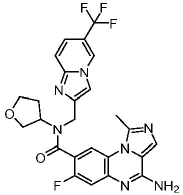 | 148 | 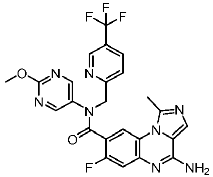 |
| 149 | 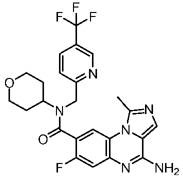 | 150 | 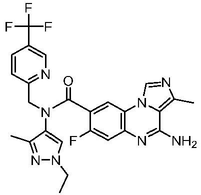 |
| 151 | 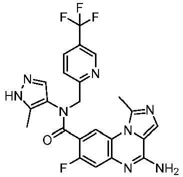 | 152 | 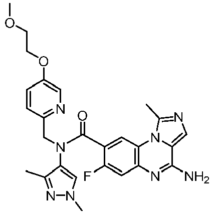 |
| 153 | 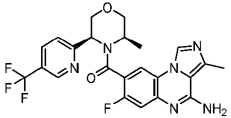 | 154 | 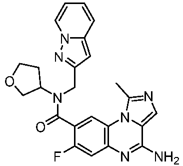 |
| 155 | 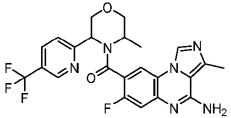 | 156 | 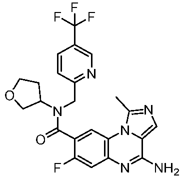 |
| 157 | 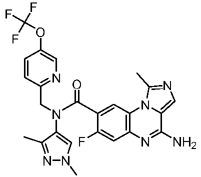 | 158 | 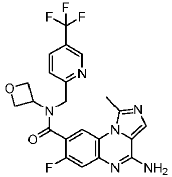 |
| 159 | 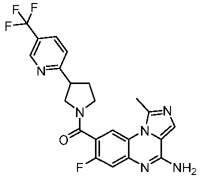 | 160 | 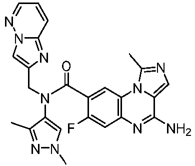 |
| 161 | 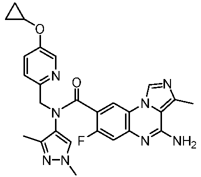 | 162 | 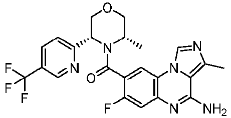 |
| 163 | 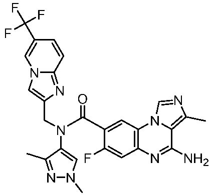 | 164 | 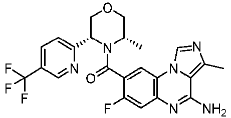 |
| 165 | 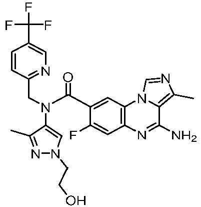 | 166 | 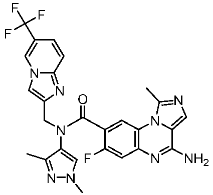 |
| 167 | 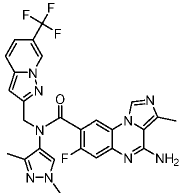 | 168 | 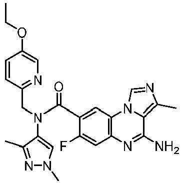 |
| 169 | 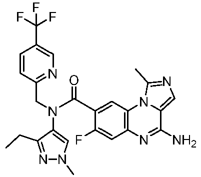 | 170 | 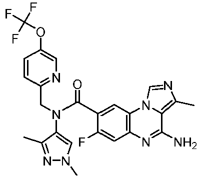 |
| 171 | 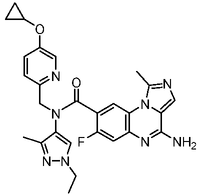 | 172 | 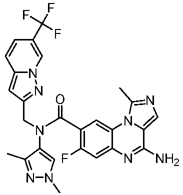 |
| 173 | 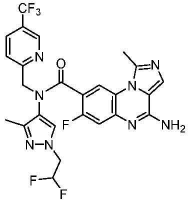 | 174 | 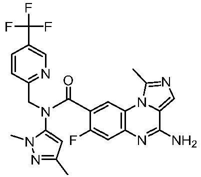 |
| 175 | 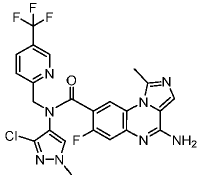 | 176 | 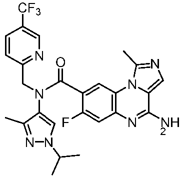 |
| 177 | 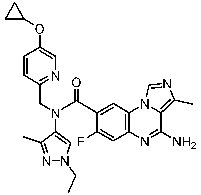 | 178 | 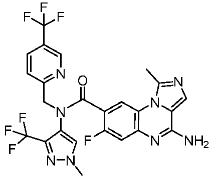 |
| 179 | 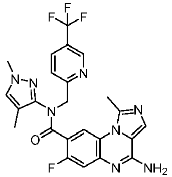 | 180 | 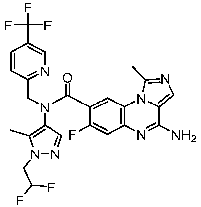 |
| 181 | 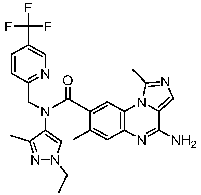 | 182 | 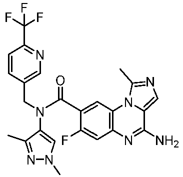 |
| 183 | 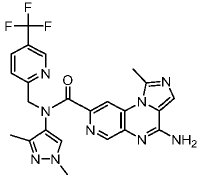 | 184 | 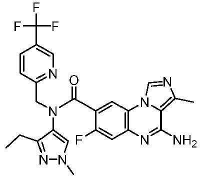 |
| 187 | 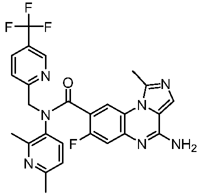 | 188 | 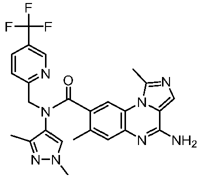 |
| 189 | 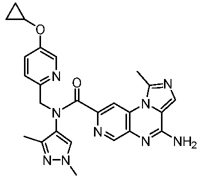 | 190 | 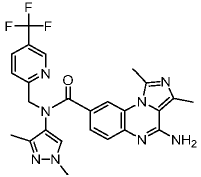 |
| 191 | 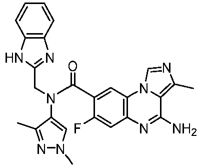 | 192 | 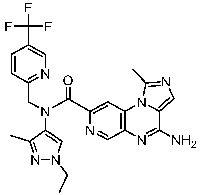 |
| 193 | 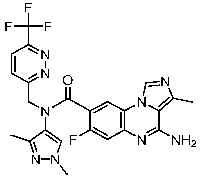 | 194 | 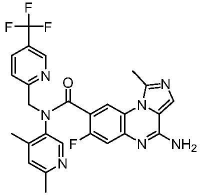 |
| 195 | 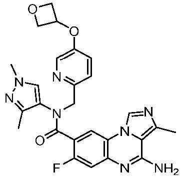 | 196 | 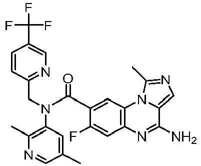 |
| 197 | 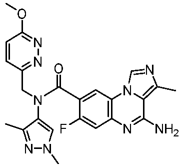 | 198 | 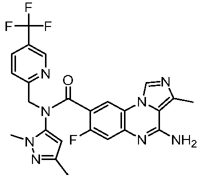 |
| 199 | 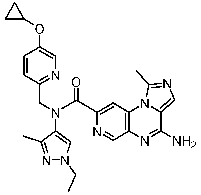 | 200 | 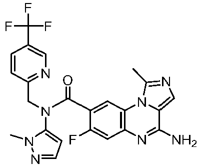 |
| 201 | 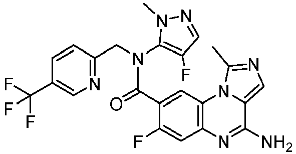 | 202 | 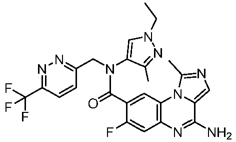 |
| 203 | 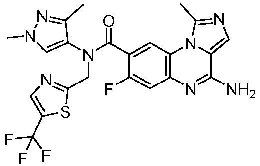 | 204 | 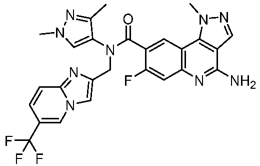 |
| 205 | 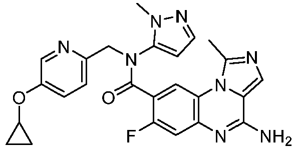 | 206 | 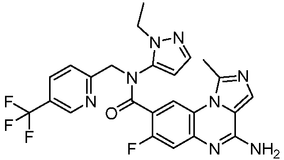 |
| 207 | 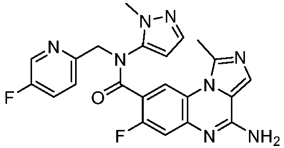 | 208 | 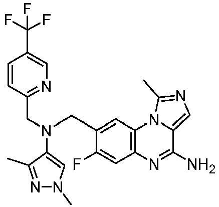 |
| 209 | 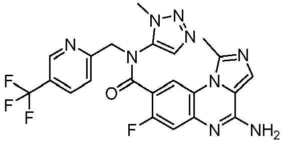 | 210 | 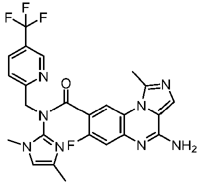 |
| 211 | 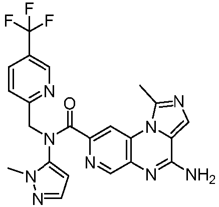 | 212 | 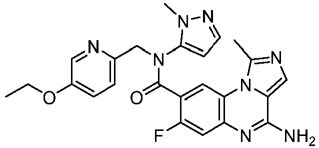 |
| 213 | 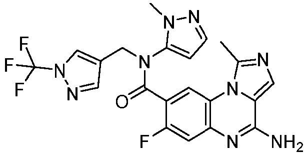 | 214 | 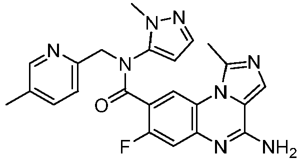 |
| 215 | 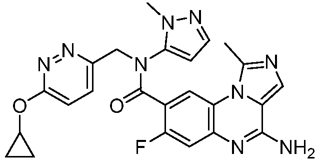 | 216 | 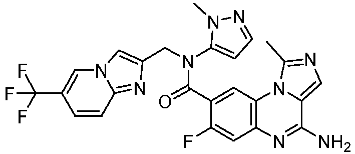 |
| 217 | 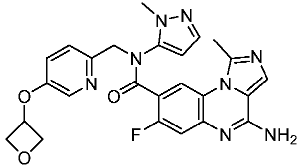 | 218 | 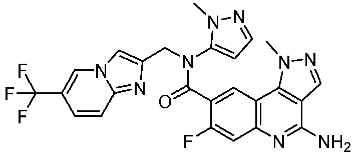 |
| 219 | 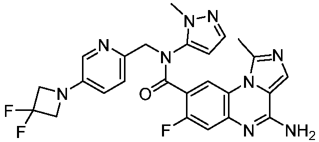 | 220 | 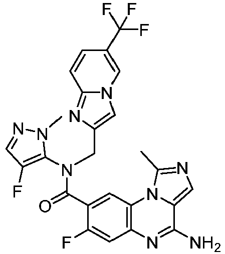 |
| 221 | 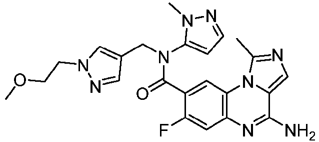 | 222 | 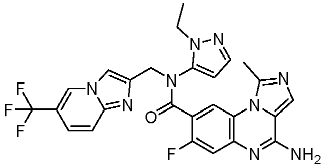 |
| 223 | 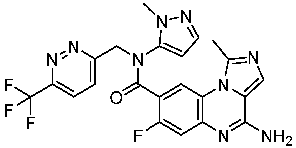 | 224 | 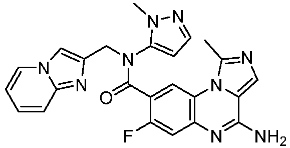 |
| 225 | 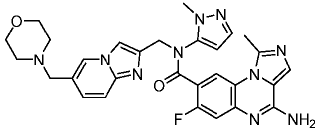 | 226 | 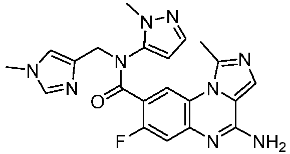 |
| 227 | 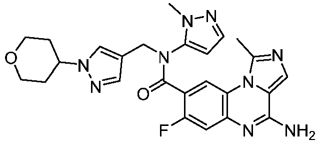 | 228 | 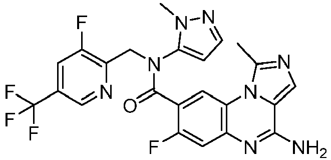 |
| 229 | 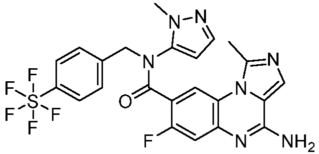 | 230 | 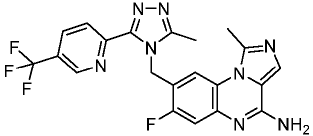 |
| 231 | 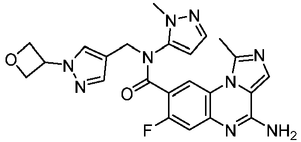 | 232 | 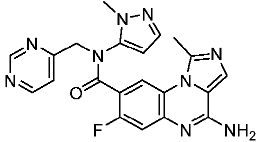 |
| 233 | 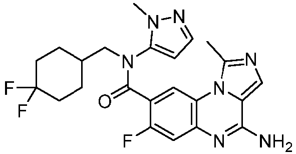 | 234 | 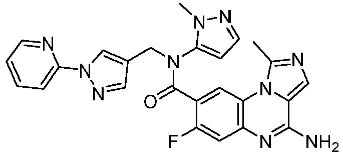 |
| 235 | 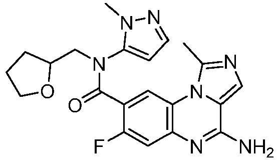 | 236 | 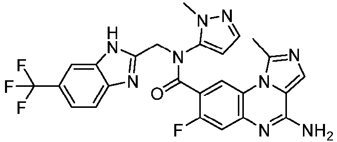 |
| 237 | 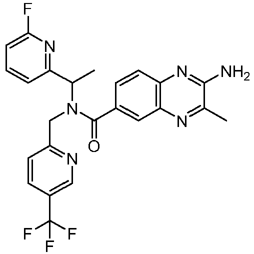 | 238 | 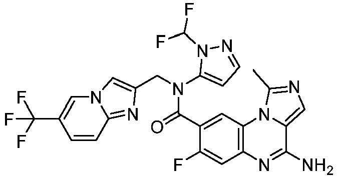 |
| 239 | 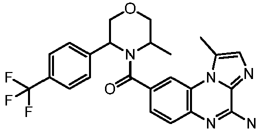 | 240 | 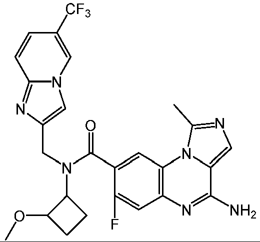 |
| 241 | 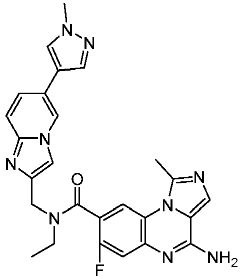 | 242 | 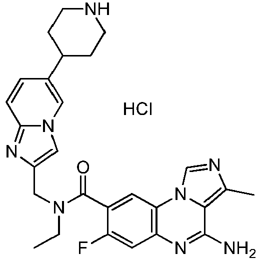 |
| 243 | 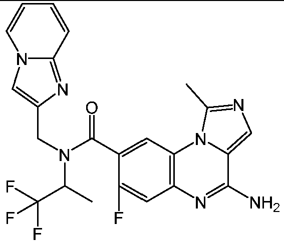 | 244 | 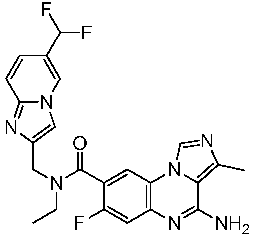 |
| 245 | 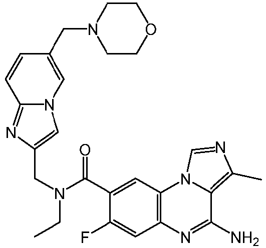 | 246 | 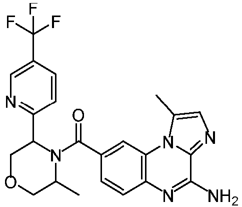 |
| 247 | 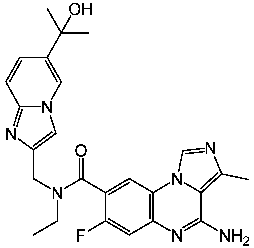 | 248 | 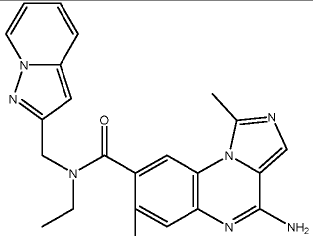 |
| 249 | 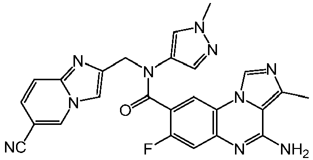 | 250 | 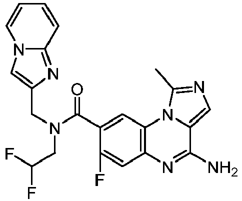 |
| 251 | 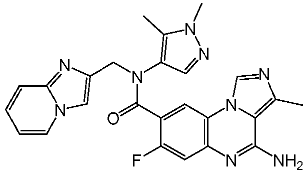 | 252 | 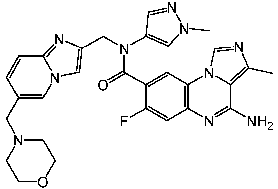 |
| 253 | 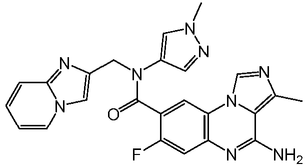 | 254 | 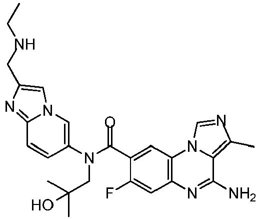 |
| 255 | 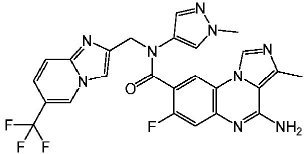 | 256 | 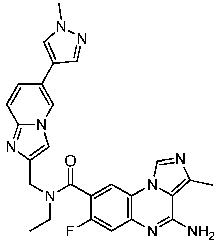 |
| 257 | 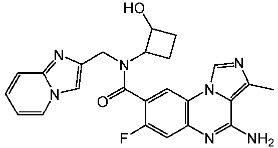 | 258 | 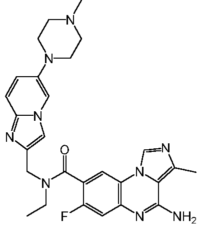 |
| 259 | 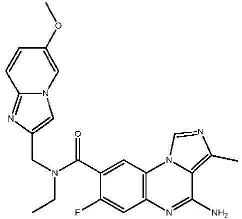 | 260 | 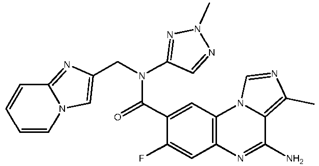 |
| 261 | 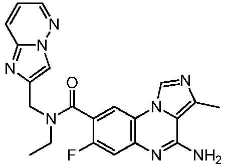 | 262 | 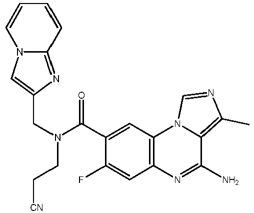 |
| 263 | 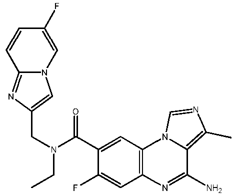 | 264 | 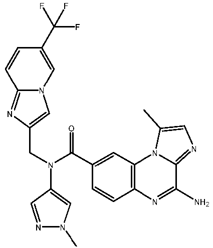 |
| 265 | 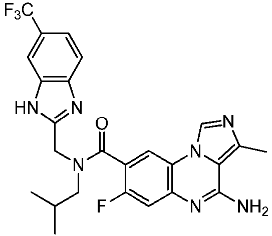 | 266 | 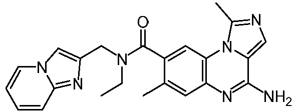 |
| 267 | 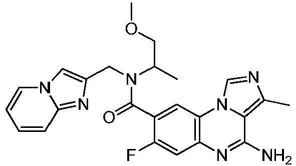 | 268 | 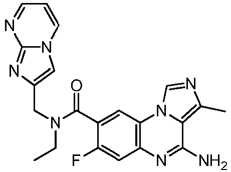 |
| 269 | 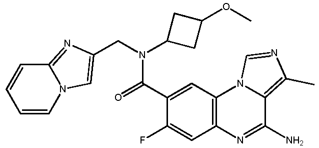 | 270 | 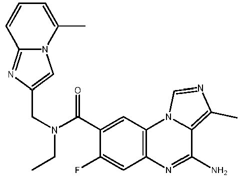 |
| 271 | 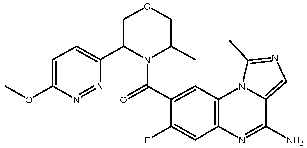 | 272 | 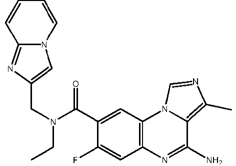 |
| 273 | 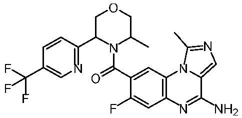 | 274 | 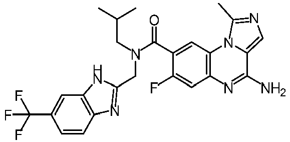 |
| 275 | 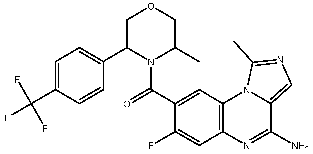 | 276 | 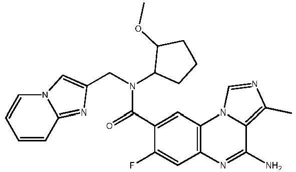 |
| 277 | 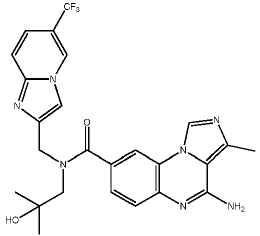 | 278 | 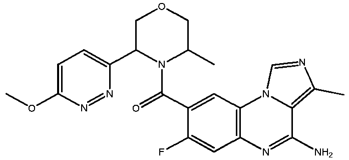 |
| 279 | 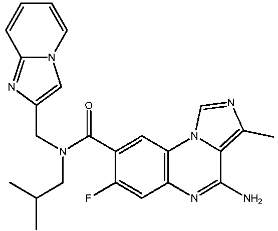 | 280 | 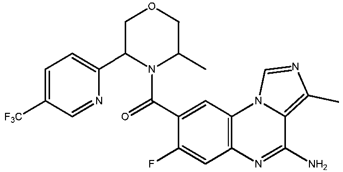 |
| 281 | 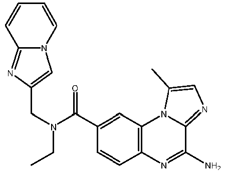 | 282 | 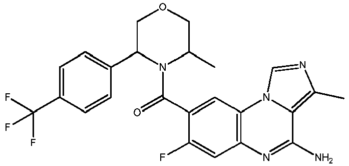 |
| 283 | 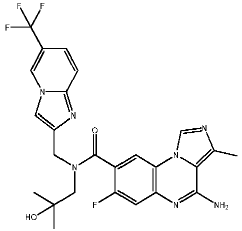 | 284 | 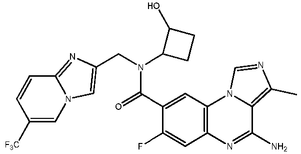 |
| 285 | 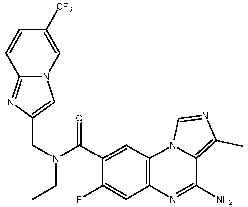 | 286 | 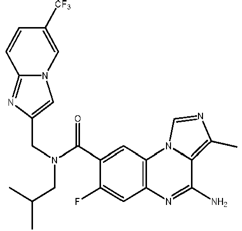 |
| 287 | 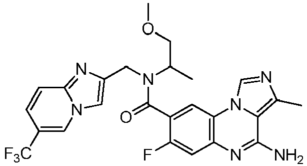 | 288 | 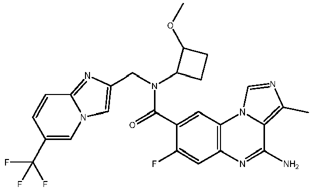 |
| 289 | 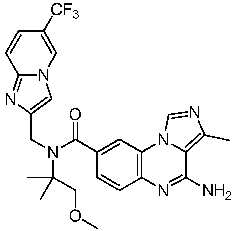 | 290 | 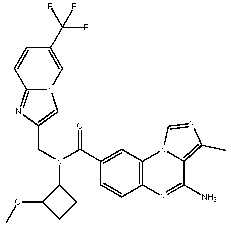 |
| 291 | 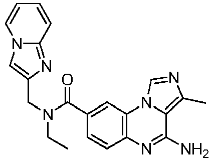 | 292 | 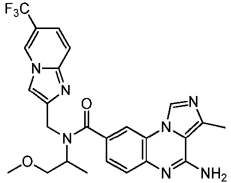 |
| 293 | 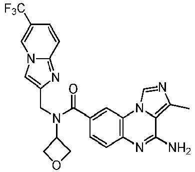 | 294 | 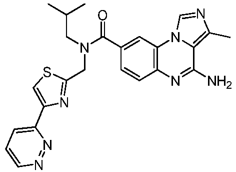 |
| 295 | 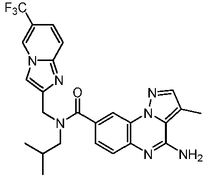 | 296 | 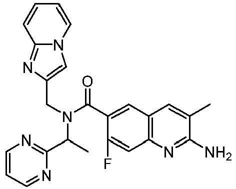 |
| 297 | 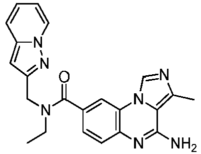 | 298 | 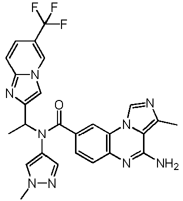 |
| 299 | 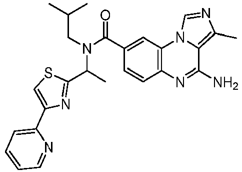 | 300 | 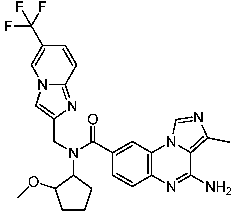 |
| 301 | 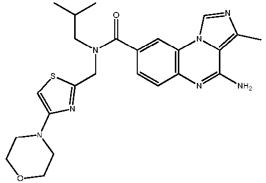 | 302 | 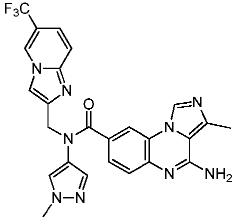 |
| 303 | 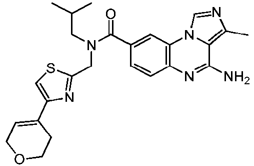 | 304 | 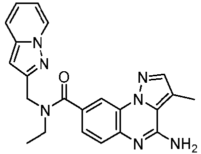 |
| 305 | 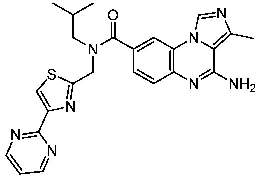 | 306 | 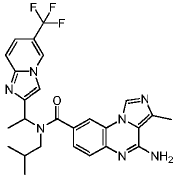 |
| 307 | 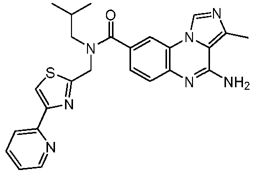 | 308 | 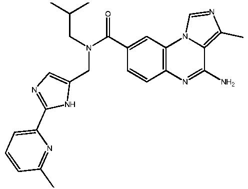 |
| 309 | 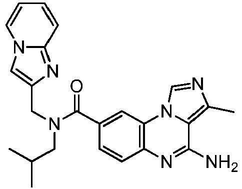 | 310 | 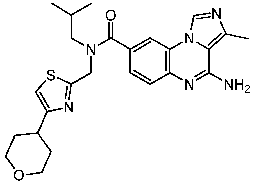 |
| 311 | 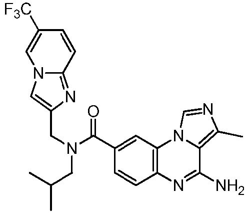 | 312 | 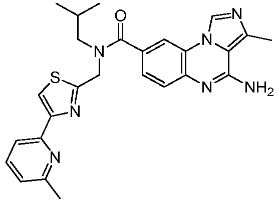 |
| 313 | 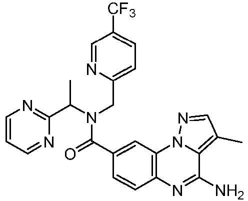 | 314 | 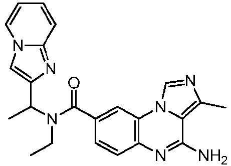 |
| 315 | 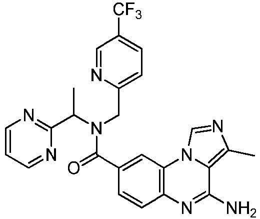 | 316 | 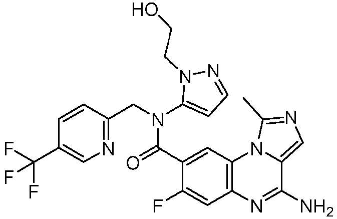 |
| 317 | 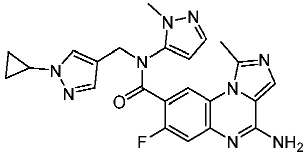 | 318 | 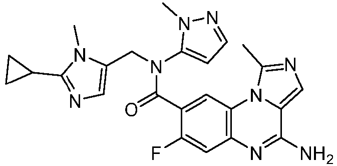 |
| 319 | 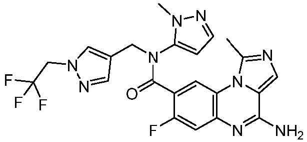 | 320 | 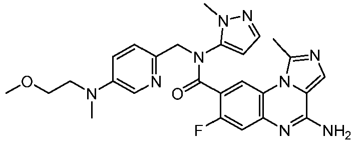 |
| 321 | 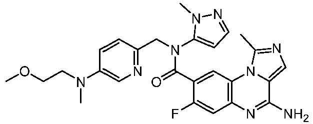 | 322 | 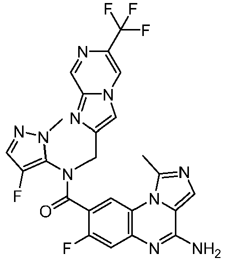 |
| 323 | 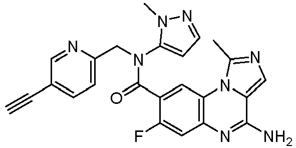 | 324 | 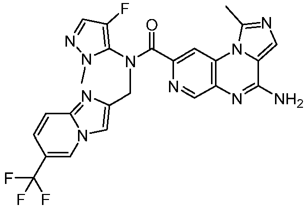 |
| 325 | 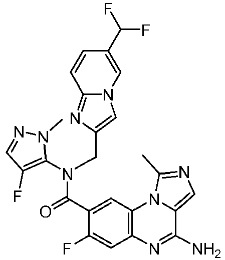 | 326 | 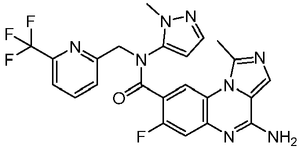 |
| 327 | 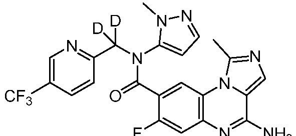 | 328 | 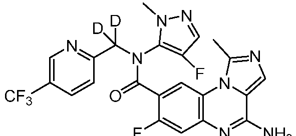 |
| 329 | 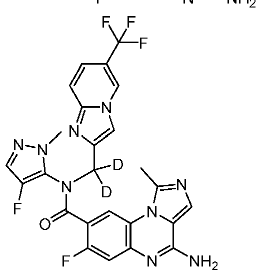 | 330 | 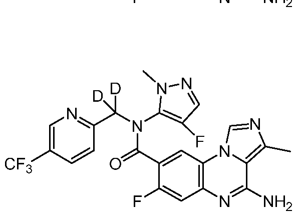 |

12. A compound selected from the following group, or a pharmaceutically acceptable stereoisomer, a salt or a deuterated product thereof:
**Table 2**
| | | | |
|---|---|---|---|
| 401 | 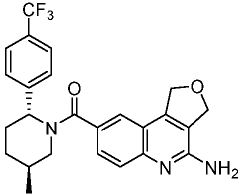 | 402 | 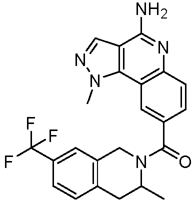 |
| 403 | 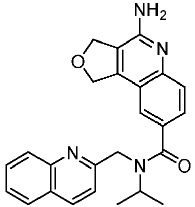 | 404 | 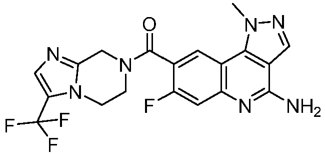 |
| 237 | 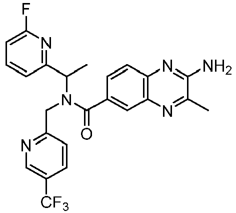 | 406 | 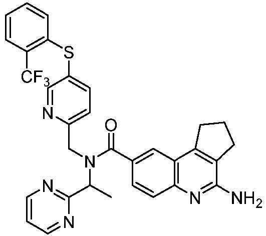 |
| 407 | 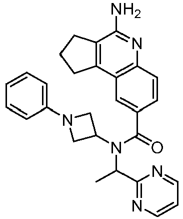 | 408 | 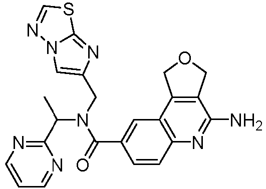 |
| 409 | 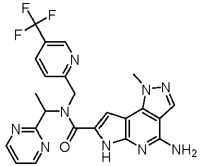 | 410 | 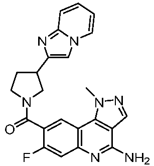 |
| 411 | 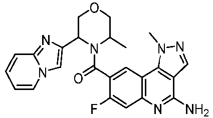 | 412 | 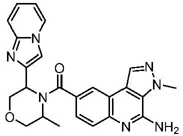 |
| 413 | 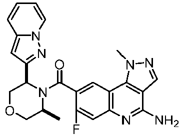 | 414 | 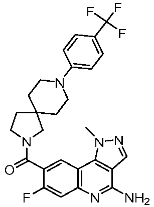 |
| 415 | 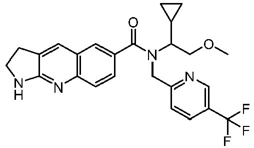 | 416 | 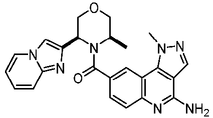 |
| 417 | 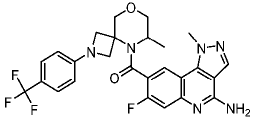 | 418 | 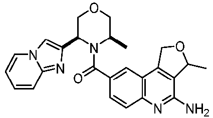 |
| 419 | 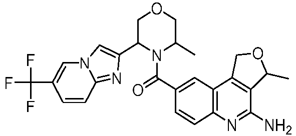 | 420 | 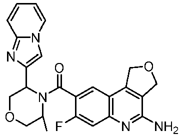 |
| 421 | 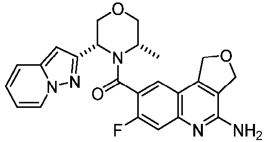 | 422 | 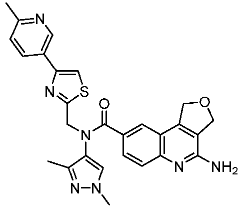 |
| 423 | 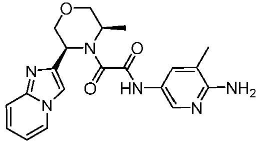 | 425 | 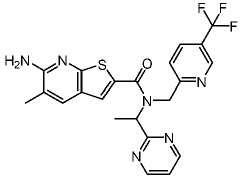 |
| 401 | 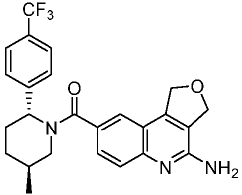 | 402 | 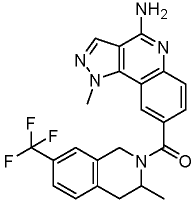 |
| 403 | 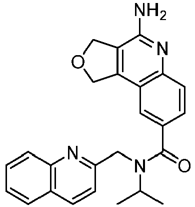 | 404 | 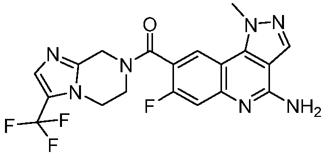 |
| 237 | 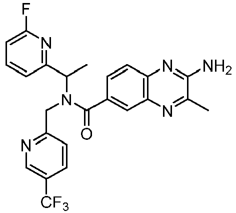 | 406 | 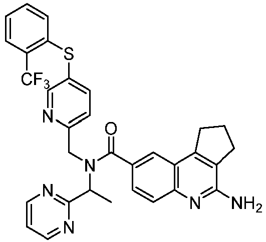 |
| 407 | 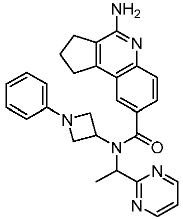 | 408 | 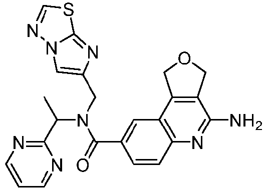 |
| 409 | 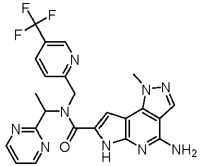 | 410 | 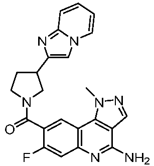 |
| 411 | 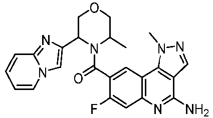 | 412 | 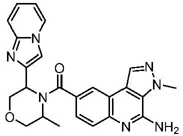 |
| 413 | 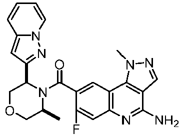 | 414 | 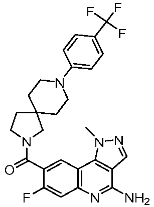 |
| 415 | 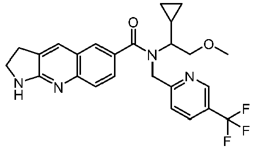 | 416 | 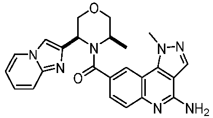 |
| 417 | 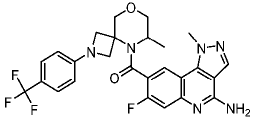 | 418 | 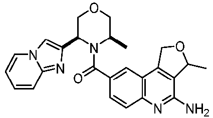 |
| 419 | 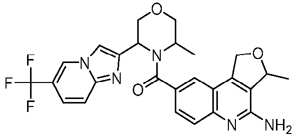 | 420 | 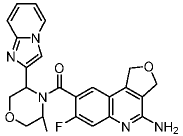 |
| 421 | 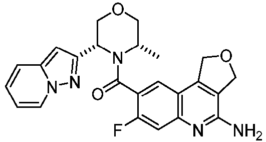 | 422 | 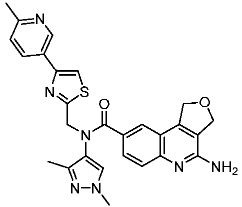 |
| 423 | 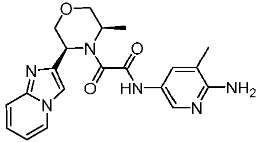 | 425 | 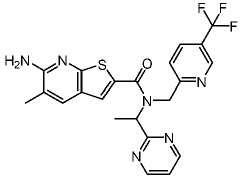 |

13. A pharmaceutical composition comprising a therapeutically effective amount of one or more of the compound according to any one of claims 1-12, a pharmaceutically acceptable salt, a racemate, an optical isomer, a stereoisomer, or a tautomer thereof, and one or more pharmaceutically acceptable carriers, excipients, adjuvants, accessories, and/or diluents.

14. A use of the compound according to any one of claims 1-12, a racemate, a stereoisomer, or a pharmaceutically acceptable salt thereof in the preparation of drugs for the treatment or prevention of diseases associated with abnormal gene levels or abnormal expression of PRMT5(such as the corresponding nucleic acid mutations, deletions, or abnormal MTAP gene level, or the methyltransferase is ectopic or fused or overexpressed).

15. The use according to claim 14, wherein, the disease is selected from the group consisting of: the disease or disorder ovarian cancer, esophageal cancer, lung cancer, lymphatic cancer, glioblastoma, colon cancer, melanoma, gastric cancer, pancreatic cancer and bladder cancer.
